# EUROPEAN PATENT SPECIFICATION

(11) **EP 2 822 948 B1**
(45) Date of publication and mention of the grant of the patent: **06.04.2016**
(21) Application number: 13704368.3
(22) Date of filing: 14.02.2013
(51) Int. Cl.: C07D 487/04, A61K 31/519, A61P 29/00

(54) **TRIAZOLOPYRAZINE DERIVATIVES**
TRIAZOLOPYRAZINDERIVATE
DÉRIVÉS DE TRIAZOLOPYRAZINE

(30) Priority: 07.03.2012 EP 12001568
(43) Date of publication of application: 14.01.2015
(73) Proprietor: Merck Patent GmbH, 64293 Darmstadt (DE)
(72) Inventor: SCHIEMANN, Kai, 64342 Seeheim-Jugenheim (DE); DEUTSCH, Carl, 64287 Darmstadt (DE); HOELZEMANN, Guenter, 64342 Seeheim-Jugenheim (DE); KUHN, Daniel, 64380 Rossdorf (DE); WEGENER, Ansgar, 63150 Heusenstamm (DE); SWINNEN, Dominique, B-1420 Braine L'Alleud (BE); COMAS, Horacio, CH-1006 Lausanne (CH)
(86) International application number: PCT/EP2013/000440
(87) International publication number: WO 2013/131609

(56) References cited:
- WO-A1-2012/025186

## Description

### BACKGROUND OF THE INVENTION

The invention had the object of finding novel compounds having valuable properties, in particular those which can be used for the preparation of medicaments.

The present invention relates to compounds and to the use of compounds in which the inhibition, regulation and/or modulation of signal transduction by protein kinases, in particular immune-modulatory or stress response kinases, furthermore to pharmaceutical compositions which comprise these compounds, and to the use of the compounds for the treatment of kinase-induced diseases.

Because protein kinases regulate nearly every cellular process, including metabolism, cell proliferation, cell differentiation, and cell survival, they are attractive targets for therapeutic intervention for various disease states. For example, cell-cycle control, immune modulation, stress response and angiogenesis, in which protein kinases play a pivotal role are cellular processes associated with numerous disease conditions such as but not limited to cancer, inflammatory diseases, neurodegenerative diseases, chronic infections, abnormal angiogenesis and diseases related thereto, atherosclerosis, macular degeneration, diabetes, obesity, and pain.

Compounds of formula I inhibit the stress response eIF2 kinase EIF2AK4 called general control nonderepressible 2 (GCN2).

Many strategies of cancer treatment of solid tumors focus on the surgically removal of the tumor mass as far as possible and the subsequent eradication of any residual tumor cells by radiotherapy and chemotherapy with cytotoxic agents or inhibitors that target cancer cell pathways more specifically. However, the success of such approach is limited and often does not persist. This is mainly due to the narrow therapeutic window for such cytotoxic agents (specificity and side effects) and to the capability of cancer calls to adapt to the selective pressure applied by cytotoxic or other inhibitory agents. The survival of a small number of tumor (stem) cells that acquired resistance to the initial treatment can be sufficient to seed the regrowth of a tumor. These relapses are in most cases more difficult to treat compared to that of the initial tumors. As a consequence the more successful targeting of tumor cells may require targeting multiple survival and escape mechanism of tumor cells in parallel (Muller & Prendegast 2007).

Development of malignancies is accompanied by a major roll up of the cellular physiology. During this process several qualities are acquired by the cancer cells that are basis for immortalization or insensitivity to growth inhibitory signals. In addition the tumor cells also modify the interaction with the microenvironment and beyond. The latter area includes the strategies of tumor cells to escape from the immunological surveillance (Muller & Prendegast 2007). The immune surveillance limits malignant growth but also provides a selective pressure triggering the evolution of mechanisms for evading the immune response as reviewed by [Dunn et al. 2004]. Essentially it has been frequently observed that ablation of T cell immunity is sufficient to increase tumor incidence [Shankaran et al. 2001] and it is believed that immune escape is affecting tumor dormancy versus progression, promoting invasion and metastasis and negatively impacts on therapeutic response.

Several mechanistic studies discovered that immune escape has an important interface with metabolic alterations within the tumor microenvironment. Here important roles in mediating immune tolerance to antigens have been associated to the catabolism of the essential amino acids tryptophan and arginine, carried out by the enzymes indoleamine 2,3-dioxygenase (IDO) and arginase I (ARG), respectively (Bronte and Zanovello, 2005; Muller et al., 2005b; Muller and Prendergast, 2007; Munn and Mellor, 2007; Popovic et al., 2007).

IDO is a single-chain oxidoreductase that catalyzes the degradation of tryptophan to kynurenine. IDO is not responsible for catabolizing excess dietary tryptophan but to modulate tryptophan level in a local environment. Elevations in tryptophan catabolism in cancer patients manifest in significantly altered serum concentration of tryptophan or catabolites and this was correlated to IDO which is commonly elevated in tumors and draining lymph nodes. According to several publications IDO over-expression is associated with poor prognosis in cancer [Okamoto et al 2005; Brandacher et al, 2006]. T cells appear to be preferentially sensitive to IDO activation, such that when starved for tryptophan they cannot divide and as a result cannot become activated by an antigen presented to them. Munn and Mellor and their colleagues, revealed that IDO modulates immunity by suppressing T-cell activation and by creating peripheral tolerance to tumor antigens (Mellor and Munn, 2004). These mechanism encompass the subversion of immune cells recruited by the tumor cell to its immediate microenvironment or to the tumor-draining lymph nodes Here the tumor antigens that were scavenged by antigen-presenting cells are cross-presented to the adaptive immune system. In addition to being directly toleragenic, mature DCs have the capacity to expand regulatory Tcells (Tregs) [Moser 2003].

Beside tryptophan catabolism the conversion of arginine is increased in a tumor-conditioned microenvironment, and numerous reports indicate a role for the activation of arginases during tumor growth and development. In tumorinfiltrating myeloid cells, arginine is converted by arginase I (ARG1), arginase II (ARG2) to urea and ornithine and oxidized by the inducible form of nitric oxide synthase (NOS2) to citrulline and nitric oxide (NO).

Increased ARG activity is frequently observed in patients with colon, breast, lung, and prostate cancer [Cederbaum 2004] correlating with the overexpression of ARG and NOS found in prostate cancers [Keskinege et al. 2001, Aaltoma et al. 2001, Wang et al. 2003]. It was shown that ARG activity in infiltrating macrophages impairs antigen-specific T cell responses and the expression of the CD3 receptor. Moreover the cumulative activity of ARG and NOS in tumor associated myeloid cells can generate inhibitory signals to antigen-specific T lymphocytes that eventually lead to apoptosis [Bronte 2003 a; 2003b].

Both, the IDO and the ARG related mechanism merge at the point of sensing the depleted concentration of the respective amino acid concentration. During amino acid deprivation, the eIF2 kinase EIF2AK4 called general control nonderepressible 2 (GCN2) is interacting with the intracellular accumulating deacylated tRNA. As a consequence the GCN2 is assumed to change from an auto-inhibited to an active conformation and further activate by autophosphorylation. Then the only known substrate protein eIF2a becomes phosphorylated and as a consequence the complex for translation initiation is inhibited [Harding et al. 2000,]. This diminishes the general Cap-dependent translation initiation and by this the corresponding protein production. On the other hand this induces the specific expression of stress related target genes mainly by cap-independent initiation via the activating transcription factor 4 (ATF4). By expressing the respective stress response proteins, e.g. enzymes in the in amino acid metabolism, the cell tries to compensate the particular cell stress [Wek et al. 2006]. If the stress persists, the same pathway will switch to promoting cell death via transcription of the pro-apoptotic transcription factor, CCAAT/enhancer-binding protein homologous protein (CHOP) [Oyadomari 2004]. It was shown that, tryptophan starvation triggers a GCN2- dependent stress signaling pathway In T cells altering eIF2aphosphorylation and translational initiation leading to a cell growth arrest (Munn et al. 2005). Sharma, et al. [2007] published on the direct IDO-induced and GCN2-dependent activation of mature Tregs. Similarly Fallarino et al [2006] found a GCN2-dependent conversion of CD4+CD25- cells to CD25+FoxP3+ Tregs producing IL-10 and TGFβ. Rodriguez et al. [2007] identified that activation of the GCN2 pathway via tryptophan or arginine depletion in combination with TCR signaling leads to CD3ζ chain down regulation, cell cycle arrest and anergy.

Importantly the GCN2 pathway is not only important for the tumoral immune escape but also plays an active role in modulating tumor survival directly. Ye et al [2010] found that the aforementioned transcription factor ATF4 is over-expressed inhuman solid tumors, suggesting an important function in tumour progression. Amino acid and glucose deprivation are typical stresses found in solid tumours and activated the GCN2 pathway to up-regulate ATF4 target genes involved in amino acid synthesis and transport. GCN2 activation / overexpression and increased phospho-eIF2a were observed in human and mouse tumors compared with normal tissues and abrogation of ATF4 or GCN2 expression significantly inhibited tumor growth in vivo. It was concluded that the GCN2-eIF2a-ATF4 pathway is critical for maintaining metabolic homeostasis in tumor cells.

Over all the present biology makes an interference with the ARG/IDO pathway attractive for braking up the tumoral immune escape by adaptive mechanism. The interference of GCN2 function is here of particular interest as it is a merging point of the two pathways, the IDO and ARG, as well as it provides additional opportunities to impede with the tumor metabolism directly.

Several pathway inhibitors are already considered as immune modulators. These inhibitors address mainly the enzymatic function of the IDO or ARG proteins (Muller and Scherle, 2006). The application of the arginase inhibitor, N-hydroxy-nor-L-Arg blocks growth of s.c. 3LL lung carcinoma in mice [Rodriguez 2004]. The NO-donating aspirins like NCX 4016 (2-(acetyloxy)-benzoic acid 3-(nitrooxymethyl) phenyl ester) have been reported to interfere with the inhibitory enzymatic activities of myeloid cells. Orally administered NO aspirin normalized the immune status of tumor-bearing hosts, increased the number and function of tumor-antigen-specific T lymphocytes, and enhanced the preventive and therapeutic effectiveness of the antitumor immunity elicited by cancer vaccination (DeSanto 2005).

The substrate analogue 1 methyl-tryptophan (1 MT) and related molecules have been used widely to target IDO in the cancer context and other settings. Studies by Friberg et al. (2002) and Uyttenhove et al. (2003) demonstrated that 1MT can limit the growth of tumors over-expressing IDO. However 1MT was unable to elicit tumor regression in several tumor models, suggesting only modest antitumor efficacy when IDO inhibition was applied as a monotherapy. In contrast, the combinatory treatment with 1 MT and a variety of cytotoxic chemotherapeutic agents elicited regression of established MMTV-neu/HER2 tumors, which responded poorly to any single-agent therapy [Muller et al 2005a]. Immunodepletion of CD4+ or CD8+ T cells from the mice, before treatment abolished the combinatorial efficacy observed in this model, confirming the expectation that 1 MT acted indirectly through activation of T cell-mediated antitumor immunity. Important evidence that IDO targeting is essential to 1 MT action was provided by the demonstration that 1 MT lacks antitumor activity in mice that are genetically deficient for IDO [Hou et al., 2007]

The inhibition of GCN2 would enable to combine the two pathway branches of amino acid starvation induced immunoediting and would reduce the options for the tumor to circumvent the inhibition of either branch. Moreover, as detailed above, the GCN2 inhibition provides the opportunity for interfering with the tumor metabolism at the same time what may enhance the efficacy of a monotherapy or a combination therapy with other anticancer approaches.

As mentioned above, the eIF2 kinase GCN2 is activated by interacting with deacylated tRNA that is accumulating as direct consequence of nutritional deprivation stress. Other cellular stress factors like UV irridation, redox stress or proteasome inhibition can induce GCN2 activation indirectly [Wek et al 2006]. In all known cases eIF2a becomes phosphorylated and this induces the specific expression of stress related target genes mainly by cap-independent initiation via the activating transcription factor 4 (ATF4).

Mitsuda et al (2007) showed that presenilin-1 is induced by activating transcription factor 4 (ATF4), regulated by GCN2. Accumulation of amyloid-β (Aβ), which is generated from amyloid precursor protein by γ-secretase, in cerebral cortex is common and critical incident in Alzheimer disease.

Specifically, presenilin is an essential for γ-secretase activity. Ohata et al. (2010) describe a role of GCN2-eIF2α-ATF4 signaling in the regulation of y-secretase activity in autophagy impaired cells: The impairment of the autophagy-lysosomal system may cause amino acid imbalance in the cell because autophagy is required for maintenance of amino acid level. The autophagy-lysosomal system is discussed as a vital modulator of γ-secretase activity through GCN2, leading to Aβ accumulation in autophagy deterioration, which may be a possible therapeutic target for reducing Aβ production. γ-Secretase plays an important role in the development of Alzheimer disease (AD). γ-Secretase activity is enriched in autophagic vacuoles and it augments amyloid-β (Aβ) synthesis.

Senile plaques are primarily composed of β-amyloid peptides (Aβ) derived from amyloid precursor protein (APP) that has undergone proteolytic processing by β-secretase (BACE-1) and γ-secretase. O'Connor et al.(2008) found that BACE-1 levels are translationally increased by phosphorylation of eIF2α.

Inhibition of GCN2 under such disease conditions that promote activation of γ-secretase or induction of BACE-1 with consequence of accumulation of Aβ and plaque formation in the brain would provide a valuable avenue to temper or even stop the progression of neurodegenerative diseases.

It was described that persistent, not acute, parasite or viral infections are associated to the establishment of immune privileged conditions of even immune competent host towards the infectious organism or particles. This has been associated to the local induction of IDO expression. Makala et al (J Infect Dis. 2011 Mar 1;203(5):715-25) show that cutaneous Leishmania major infection stimulated expression of the immune regulatory enzyme indoleamine 2,3 dioxygenase (IDO) in local lymph nodes. Induced IDO attenuated the T cell stimulatory functions of dendritic cells and suppressed local T cell responses to exogenous and nominal parasite antigens. IDO ablation reduced local inflammation and parasite burdens, as did pharmacologic inhibition of IDO in mice with established infections. de Souza Sales (Clin Exp Immunol. 2011 Aug;165(2):251-63) corroborated the role of indoleamine 2, 3-dioxygenase in lepromatous leprosy immunosuppression. Boasso et al (Blood. 2007 Apr 15;109(8):3351-9) found that HIV inhibits CD4+ T-cell proliferation by inducing indoleamine 2,3-dioxygenase in plasmacytoid dendritic cells and that in vitro inhibition of IDO results in increased CD4(+) T-cell proliferative response in PBMCs from HIV-infected patients

Inhibitor drugs of the IDO/GCN2 pathway could be used to enhance host immunity to chronic and persistent infections.

Literature:
1. Aaltoma, S.H., P.K. Lipponen, and V.M. Kosma. 2001. Inducible nitric oxide synthase (iNOS) expression and its prognostic value in prostate cancer. Anticancer Res. 21:3101-3106.
2. Brandacher, G.; Perathoner, A.; Ladurner, R.; Schneeberger, S.; Obrist, P.; Winkler, C.; Werner, E. R.; Werner-Felmayer, G.; Weiss, H. G.; Gobel, G.; Margreiter, R.; Konigsrainer, A.; Fuchs, D.; Amberger, A. Prognostic value of indoleamine 2,3- dioxygenase expression in colorectal cancer: effect on tumorinfiltrating T cells. Clin. Cancer Res. 2006, 12, 1144-1151.
3. Bronte V, Zanovello P. (2005). Regulation of immune responses by L-arginine metabolism. Nat Rev Immunol 5: 641-654.
4. Bronte, V., P. Serafini, C. De Santo, I. Marigo, V. Tosello, A. Mazzoni, D.M. Segal, C. Staib, M. Lowel, G. Sutter, et al. 2003a. IL-4- induced arginase 1 suppresses alloreactive T cells in tumor-bearing mice. J. Immunol. 170:270-278.
5. Bronte, V., P. Serafini, A. Mazzoni, D.M. Segal, and P. Zanovello. 2003b. L-arginine metabolism in myeloid cells controls T-lymphocyte functions. Trends Immunol. 24:302-306
6. Carmela De Santo, Paolo Serafini, Ilaria Marigo, Luigi Dolcetti, Manlio Bolla,§ Piero Del Soldato, Cecilia Melani, Cristiana Guiducci, Mario P. Colombo, Manuela lezzi, Piero Musiani, Paola Zanovello, and Vincenzo Bronte. Nitroaspirin corrects immune dysfunction in tumor-bearing hosts and promotes tumor eradication by cancer vaccination. Proc Natl Acad Sci USA. 2005 March 15; 102(11): 4185-4190
7. Cederbaum, S.D., H. Yu, W.W. Grody, R.M. Kern, P. Yoo, and R.K. lyer. 2004. Arginases I and II: do their functions overlap? Mol. Genet. Metab. 81:S38-44.
8. T. O'Connor, K.R. Sadleir, E. Maus, R.A. Velliquette, J. Zhao, S.L. Cole, W.A. Eimer, B. Hitt, L.A. Bembinster, S. Lammich, S.F. Lichtenthaler, S.S. Hebert, S.B. De, C. Haass, D.A. Bennett, R. Vassar, Phosphorylation of the translation initiation factor eIF2alpha increases BACE1 levels and promotes amyloidogenesis. Neuron, 60 (2008), pp. 988-1009
9. Dey, M., Cao, C., Sicheri, F. and T.E. Dever. Conserved Intermolecular Salt Bridge Required for Activation of Protein Kinases PKR, GCN2, and PERK. JBC 282(9): 6653, 2007.
10. Dunn, G. P.; Old, L. J.; Schreiber, R. D. The immunobiology of cancer immunosurveillance and immunoediting. Immunity 2004, 21, 137-148.
11. Fallarino, F. U. Grohmann, S. You, B.C. et al. The combined effects fo tryptophan starvation and tryptophan catabolites down-regulate T cell receptor zeta-chain and induce a regulatory phenotype in naïve T cells. J. Immunol. 176:6752, 2006.
12. Friberg M, Jennings R, Alsarraj M, Dessureault S, Cantor A, Extermann M et al. (2002). Indoleamine 2,3-dioxygenase contributes to tumor cell evasion of T cell-mediated rejection. Int. J Cancer 101: 151-155
13. Harding HP, Novoa I, Zhang Y, Zeng H, Wek R, Schapira M, Ron D. Regulated translation initiation controls stress-induced gene expression in mammalian cells. Mol Cell. 2000 Nov;6(5):1099-108.
14. Hou DY, Muller AJ, Sharma MD, DuHadaway J, Banerjee T, Johnson M et al. (2007). Inhibition of indoleamine 2,3-dioxygenase in dendritic cells by stereoisomers of 1-methyl-tryptophan correlates with antitumor responses. Cancer Res 67: 792-801.
15. Keskinege, A., S. Elgun, and E. Yilmaz. 2001. Possible implications of arginase and diamine oxidase in prostatic carcinoma. Cancer Detect. Prev. 25:76-79.
16. Mellor AL, Munn DH. (2004). IDO expression by dendritic cells: tolerance and tryptophan catabolism. Nat Rev Immunol 4: 762-774.
17. Mitsuda T, Hayakawa Y, Itoh M, Ohta K, Nakagawa T. ATF4 regulates gamma-secretase activity during amino acid imbalance, Biochem Biophys Res Commun. 2007 Jan 19;352(3):722-7.
18. Moser, M. Dendritic cells in immunity and tolerance-do they display opposite functions? Immunity 2003, 19, 5-8.
19. Muller, A.J. and P.A. Scherle. Targeting the mechanisms of tumoral immune tolerance with small-molecule inhibitors. Nat. Rev. Cancer. 6:613, 2006.
20. Muller AJ, Prendergast GC. (2007). Indoleamine 2,3-dioxygenase in immune suppression and cancer. Curr Cancer Drug Targets 7: 31-40.
21. Muller AJ, DuHadaway JB, Sutanto-Ward E, Donover PS, Prendergast GC. (2005a). Inhibition of indoleamine 2,3-dioxygenase, an immunomodulatory target of the tumor suppressor gene Bin1, potentiates cancer chemotherapy. Nature Med 11: 312-319.
22. Muller AJ, Malachowski WP, Prendergast GC. (2005b). Indoleamine 2,3-dioxygenase in cancer: targeting pathological immune tolerance with small-molecule inhibitors. Expert Opin Ther Targets 9: 831-849.
23. Munn, D.H., M.D. Sharma, B. Baban, H.P. Harding, Y. Zhang, D. Ron, A.L. Mellor. GCN2 kinase in T cells mediates proliferative arrest and anergy induction in response to indoleamine 2,3-dioxygenase. Immunity. 22:633, 2005
24. Ohta K, Mizuno A, Ueda M, Li S, Suzuki Y, Hida Y, Hayakawa-Yano Y, Itoh M, Ohta E, Kobori M, Nakagawa T. Autophagy impairment stimulates PS1 expression and gamma-secretase activity. Autophagy. 2010 ;6(3):345-52
25. Okamoto, A.; Nikaido, T.; Ochiai, K.; Takakura, S.; Saito, M.; Aoki, Y.; Ishii, N.; Yanaihara, N.; Yamada, K.; Takikawa, O.; Kawaguchi, R.; Isonishi, S.; Tanaka, T.; Urashima, M. Indoleamine 2,3-dioxygenase serves as a marker of poor prognosis in gene expression profiles of serous ovarian cancer cells. Clin. Cancer Res. 2005, 11, 6030-6039.
26. Oyadomari S, Mori M. Roles of CHOP/GADD153 in endoplasmic reticulum stress. Cell Death Differ. 2004 Apr;11(4):381-9.
27. GC Prendergast, Immune escape as a fundamental trait of cancer: focus on IDO. Oncogene (2008) 27, 3889-3900
28. Popovic PJ, Zeh III HJ, Ochoa JB. (2007). Arginine and immunity. J Nutr 137: 1681S-1686 S.
29. Rodriguez, P.C., D.G. Quiceno, J. Zabaleta, B. Ortiz, A.H. Zea, M.B. Piazuelo,A.Delgado, P.Correa, J.Brayer, E.M. Sotomayor, S.Antonia, J.B. Ochoa, and A.C. Ochoa. Arginase I Production in the Tumor Microenvironment by Mature Myeloid Cells Inhibits T-Cell Receptor Expression and Antigen-Specific T-Cell Responses. Canc. Res. 64:5839, 2004
30. Rodriguez, P.C., D.G. Quiceno, and A.C. Ochoa. L-arginine availability regulates T-lymphocyte cell-cycle progresión. Blood. 109:1568, 2007.
31. Shankaran, V.; Ikeda, H.; Bruce, A. T.; White, J. M.; Swanson, P. E.; Old, L. J.; Schreiber, R. D. IFNgamma and lymphocytes prevent primary tumour development and shape tumour immunogenicity. Nature 2001, 410, 1107-1111.
32. Sharma, M.D., B. Baban, P. Chandler, D-Y. Hou, N. Singh, H. Yagita, M. Azuma, B.R. Blazar, A.L. Mellor, and D.H. Munn. Plasmacytoid dendritic cells from mouse tumor-draining lymph nodes directly activate mature Tregs via indoleamine 2,3-dioxygenase. J. Clin. Invest. 117:2570,2007.
33. Uyttenhove C, Pilotte L, Theate I, Stroobant V, Colau D, Parmentier N et al. (2003). Evidence for a tumoral immune resistance mechanism based on tryptophan degradation by indoleamine 2,3- dioxygenase. Nat Med 9: 1269-1274
34. Wang, J., M. Torbenson, Q. Wang, J.Y. Ro, and M. Becich. 2003. Expression of inducible nitric oxide synthase in paired neoplastic and non-neoplastic primary prostate cell cultures and prostatectomy specimen. Urol. Oncol. 21:117-122.
35. Wek RC, Jiang HY, Anthony TG. Coping with stress: eIF2 kinases and translational control. Biochem Soc Trans. 2006 Feb;34 (Pt 1):7-11.
36. Ye J, Kumanova M, Hart LS, Sloane K, Zhang H, De Panis DN, Bobrovnikova-Marjon E, Diehl JA, Ron D, Koumenis C. The GCN2-ATF4 pathway is critical for tumour cell survival and proliferation in response to nutrient deprivation. EMBO J. 2010 Jun 16;29(12):2082-96.

In particular, the present invention relates to compounds and to the use of compounds in which the inhibition, regulation and/or modulation of signal transduction by GCN2 plays a role.

The synthesis of small compounds which specifically inhibit, regulate and/or modulate signal transduction by immune-modulatory or stress response kinases in particular GCN2, is therefore desirable and an aim of the present invention.

Moreover, aim of this invention is the synthesis of new compounds for the prevention and treatment of neoplastic malignancies including, but without being limited to, solid tumor cancers, cancers of the lymphatic or blood system, of neurodegenerative diseases and chronic infections.

It has been found that the compounds according to the invention and salts thereof have very valuable pharmacological properties while being well tolerated.

The compounds of the formula I can furthermore be used for the isolation and investigation of the activity or expression of GCN2. In addition, they are particularly suitable for use in diagnostic methods for diseases in connection with unregulated or disturbed GCN2 activity.

Compounds of formula I can also inhibit tyrosine kinases FMS (CSF1 R), FLT3 or FLT4 or combinations of these kinases, preferentially in addition to inhibitory activity towards GCN2.

Fms-like tyrosine kinase 3 (FLT3), which is also known as FLK-2 (fetal liver kinase 2) and STK-I (stem cell kinase 1), plays an important role in the proliferation and differentiation of hematopoietic stem cells. FLT3 receptor kinase is expressed at very high levels on the cells of more than 80% of myelogenous patients and of a fraction of acute lymphoblastic leukemia cells. Furthermore, the enzyme can also be found on cells from patients with chronic myelogenous leukemia in lymphoid blast crisis. It has been reported that FLT3 kinase is mutated in 30% of acute myeloid leukemia (AML) and in a subset of acute lymphoblastic leukemia (ALL) as well (Gilliland et al, Blood 100, 1532-1542 (2002); Stirewalt et al., Nat. Rev. Cancer, 3, 650-665 (2003). Activating mutations in FLT3 mutations have been associated with a poor prognosis (Malempati et al., Blood, 104, 11 (2004). FLT3 inhibitors are being developed and some have shown promising clinical effects against AML (Levis et al Int. J. Hematol, 52, 100- 107 (2005).

It has been reported that some of small-molecule FLT3 inhibitors are effective in inducing apoptosis in cell lines with FLT3-activating mutations and prolonging survival of mice that express mutant FLT3 in their bone marrow cells (Levis et al, Blood, 99, 3885-3891 (2002); Kelly et al, Cancer Cell, 1, 421-432 (2002); Weisberg et al, Cancer Cell, 1, 433-443 (2002); Yee et al, Blood, 100, 2941-2949 (2002).

US patent application 20090054358 describes Flt3 inhibitors for immune suppression and in particular for the treatment of immune related disorders like organ rejection, bone marrow transplant rejection, non-myeloablative bone marrow transplant rejection, ankylosing spondylitis, arthritis, aplastic anemia, Behcet's disease, type 1 diabetes mellitus, graft-versus-host disease, Graves' disease, autoimmune hemolytic anemia, Wegener's granulomatosis, hyper IgE syndrome, idiopathic thrombocytopenia purpura, rheumatoid arthritis, Crohn's disease, multiple sclerosis, Myasthenia gravis, psoriasis, and lupus, among other autoimmune diseases. Flt3 Inhibitors might also be used to treat neurological disorder as neurodegenerative disease, for example a disease caused by axonal degeneration. Neurodegenerative diseases include, for example, multiple sclerosis; demyelinating core disorders, such as multiple sclerosis, acute transverse myelitis without being limited thereto.

Scott et al (Bioorg. Med Chem Let. (2008) 18 (17) p4794) describe CSF-1R inhibitors for the treatment of cancer. CSF-1 R is a member of the class III receptor tyrosine kinases. Colony stimulatory factor 1 (CSF-1), also known as macrophage/monocyte colony stimulatory factor (M-CSF), binds to CSF-1R, resulting in dimerization, autophosphorylation, and activation of signal transduction.1 CSF-1/CSF-1R signaling is essential for normal monocyte development. In cancer, pro-tumorigenic macrophages have been identified and linked to poor prognosis in breast, ovarian, and prostate cancers. Elevated levels of CSF-1 and CSF-1 R have been reported in several tumor types, including breast, ovarian, and endometrial cancers, and have also been linked to invasion and metastasis. Inhibition of CSF-1 R activity could therefore have multiple effects on the tumor through reduction in the levels of tumor-associated macrophages (TAMs) and have direct effects on the tumor itself (C.E. Lewis, J.W. Pollard, Cancer Res., 66 (2006), p. 605; I. Bingle, N. et al., J. Pathol., 196 (2002), p. 254; B.M. Kacinski, Ann. Med., 27 (1995), p. 79; E. Garwood et al. J Clin Oncol 26: 2008).

Su JL et al. (Cancer Cell. 2006 Mar;9(3):209-23) report that the VEGF-C/Flt-4 axis promotes invasion and metastasis of cancer cells. Flt-4, a VEGF receptor, is activated by its specific ligand, VEGF-C. The resultant signaling pathway promotes angiogenesis and/or lymphangiogenesis. VEGF-C/Flt-4 axis enhances cancer cell mobility and invasiveness and contributes to the promotion of cancer cell metastasis. Examination of tumor tissues from various types of cancers revealed high levels of Flt-4 and VEGF-C expression that correlated closely with clinical metastasis and patient survival. Inhibition of Flt-4 kinase could reduce the invasive capacity in different types of cancer

Combining the inhibitory specificity towards GCN2 with that towards FMS (CSF1 R), FLT3 or FLT4 or combinations of these kinases can be of particular advantages for the treatment of neoplastic malignancies at different disease stages. It could combine the effects of stimulating the immune response towards cancer/tumor cells, to reduce the levels of tumor-associated macrophages as well as the invasive capacity of cancers for metastasis formation. In a further aspect the combination of inhibitory activities on GCN2 particularly with inhibition of FLT3 could be advantageous for the treatment of neurodegenerative disorders as it could synergize suppressive effects on inflammatory processes with the modulation of protein deposites generation in the brain. In another aspect the combination of inhibitory activities on GCN2 particularly with inhibition of FLT3 could provide advantages for modulating the immune response to treat immune related disorders and inflammatory or autoimmune diseases.

In a further embodiment the present invention specifically relates to compounds of the formula I which inhibit, regulate and/or modulate signal transduction by GCN2, FMS (CSF1 R), FLT3 or FLT4 or combinations of these kinases, to compositions which comprise these compounds, and to processes for the use thereof for the treatment of diseases and complaints that are - induced or modulated by GCN2, FMS (CSF1 R), FLT3 or FLT4 or combinations of these kinases..

Further aim of this invention is the synthesis of new compounds for the prevention and treatment of neoplastic malignancies including, but without being limited to, solid tumor cancers, cancers of the lymphatic or blood system, of neurodegenerative diseases, immune related disorders like arthritis, psoriasis, lupus, multiple sclerosis or other autoimmune diseases as well as chronic infections.

The compounds of the formula I can furthermore be used for the isolation and investigation of the activity or expression of Syk, GCN2, FMS (CSF1 R), FLT3 or FLT4. In addition, they are particularly suitable for use in diagnostic methods for diseases in connection with unregulated or disturbed Syk, GCN2, FMS (CSF1R), FLT3 or FLT4activity. The host or patient can belong to any mammalian species, for example a primate species, particularly humans; rodents, including mice, rats and hamsters; rabbits; horses, cows, dogs, cats, etc. Animal models are of interest for experimental investigations, providing a model for treatment of human disease.

The susceptibility of a particular cell to treatment with the compounds according to the invention can be determined by in vitro tests. Typically, a culture of the cell is combined with a compound according to the invention at various concentrations for a period of time which is sufficient to allow active agents such as anti IgM to induce a cellular response such as expression of a surface marker, usually between about one hour and one week. In vitro testing can be carried out using cultivated cells from blood or from a biopsy sample. The amount of surface marker expressed are assessed by flow cytometry using specific antibodies recognising the marker.

The dose varies depending on the specific compound used, the specific disease, the patient status, etc. A therapeutic dose is typically sufficient considerably to reduce the undesired cell population in the target tissue while the viability of the patient is maintained. The treatment is generally continued until a considerable reduction has occurred, for example an at least about 50% reduction in the cell burden, and may be continued until essentially no more undesired cells are detected in the body.

For identification of a signal transduction pathway and for detection of interactions between various signal transduction pathways, various scientists have developed suitable models or model systems, for example cell culture models (for example Khwaja et al., EMBO, 1997, 16, 2783-93) and models of transgenic animals (for example White et al., Oncogene, 2001, 20, 7064-7072). For the determination of certain stages in the signal transduction cascade, interacting compounds can be utilised in order to modulate the signal (for example Stephens et al., Biochemical J., 2000, 351, 95-105). The compounds according to the invention can also be used as reagents for testing kinase-dependent signal transduction pathways in animals and/or cell culture models or in the clinical diseases mentioned in this application.

Measurement of the kinase activity is a technique which is well known to the person skilled in the art. Generic test systems for the determination of the kinase activity using substrates, for example histone (for example Alessi et al., FEBS Lett. 1996, 399, 3, pages 333-338) or the basic myelin protein, are described in the literature (for example Campos-González, R. and Glenney, Jr., J.R. 1992, J. Biol. Chem. 267, page 14535).

For the identification of kinase inhibitors, various assay systems are available. In scintillation proximity assay (Sorg et al., J. of. Biomolecular Screening, 2002, 7, 11-19) and flashplate assay, the radioactive phosphorylation of a protein or peptide as substrate with γATP is measured. In the presence of an inhibitory compound, a decreased radioactive signal, or none at all, is detectable. Furthermore, homogeneous time-resolved fluorescence resonance energy transfer (HTR-FRET) and fluorescence polarisation (FP) technologies are suitable as assay methods (Sills et al., J. of Biomolecular Screening, 2002, 191-214).

Other non-radioactive ELISA assay methods use specific phospho-antibodies (phospho-ABs). The phospho-AB binds only the phosphorylated substrate. This binding can be detected by chemiluminescence using a second peroxidase-conjugated anti-sheep antibody (Ross et al., 2002, Biochem. J.).

Moreover,, the present invention relates to compounds and to the use of compounds in which the inhibition, regulation and/or modulation of signal transduction by Syk plays a role.

One of the key events in the signaling pathway following the activation of mast cells is activation of the tyrosine kinase Syk. Mast cells play a critical role in asthma and allergic disorders by releasing pro-inflammatory mediators and cytokines. Antigen-mediated aggregation of FcεRJ, the high-affinity receptor for IgE, results in activation of mast cells. This triggers a series of signaling events resulting in the release of mediators, including histamine, proteases, leukotrienes and cytokines. These mediators cause increased vascular permeability, mucus production, bronchoconstriction, tissue degradation and inflammation, thus playing key roles in the etiology and symptoms of asthma and allergic disorders. Syk kinase acts as a central initiator of all subsequent signaling leading to mediator release. The critical role of Syk kinase in the signaling path was demonstrated by the complete inhibition of mediator release by a protein containing the SH2 domains of Syk kinase that functioned as an inhibitor of Syk kinase (J. A.Taylor et al, Molec. and Cell Biol, 15: 4149-4157 (1995)).

Syk (Spleen-Tyrosine-Kinase) is a 72 kDa non-receptor tyrosine kinase belonging to the subfamily of intracellular tyrosine kinases that comprises ZAP70, Pyk2, Abl, Tie2, KDR and HER, among others. Syk is a major regulator of FcR (FcγRI, II, III, FcεRI, FcαR) and BCR signaling and is expressed throughout hematopoietic lineage, as well as in fibroblasts, osteoclasts, hepatocytes, epithelial and neuronal cells. In addition to the C terminal kinase domain, SYK exhibits two SH2 domains and over 10 autophosphorylation sites¹.

By means of both its SH2 domains SYK is specifically recruited to phosphorylated ITAMs (Immunoreceptor Tyrosine-based Activation Motifs present in immunoreceptors such as FcγRI, IIA, IIIA, FcαR, FcεRI and BCR, expressed by monocytes, macrophages, mast cells, neutrophils and B cells) and specifically mediates immunoreceptor signaling triggered by activation of those receptors in mast cells, B cells, macrophages, monocytes, neutrophils, eosinophils, NK cells, DC cells platelets and osteoclasts^{1,2}.

Upon BCR cross linking, tyrosine residues at the ITAM motifs of the cytosolic tail of the Igα/Igβ are phosphorylated by the Src-family kinase Lyn, generating docking sites for SYK that is thus recruited to the BCR immunocomplex. SYK is then phosphorylated and activated by the Src-family kinase Lyn. Upon activation, SYK will phosphorylate the adaptor protein BLNK allowing its interaction with both BTK and PLCγ₂ via their respective SH2 domains. SYK phosphorylated -and thus activated- BTK will in turn phosphorylate and activate PLCγ₂ leading to IP₃ formation, Ca²⁺ mobilization, PKC and MAPK activation and consequent NFAT, AP-1 and NFκB transcription factor activation, resulting in activation and surface marker expression, cytokine release, survival and proliferation of B cells³. In mast cells, allergen activated FcεRI is phosphorylated by LYN and FYN and recruits SYK which is in turn phosphorylated by LYN and further autophosphorylated, becoming fully activated. Activated SYK phosphorylates the two adaptor molecules NTAL and LAT creating more docking sites for SH2 containing proteins such as PLCγ₁, vav, and the p85 regulatory subunit of PI3K, resulting in mast cell degranulation and cytokine production⁴. Syk's critical role in signal transduction of mast cells is confirmed by reproducible observation that the 10-15% of basophils (circulating mast cells) from human donors that cannot degranulate have reduced amounts of Syk protein^{5,6}. In addition, SYK is required for the bone resorption activity of osteoclasts. Upon stimulation of osteoclasts by αvβ3 integrin, SYK becomes phosphorylated, most likely by c-Src, in a DAP-12 / FcγRII dependent mechanism, leading to SPL-76 and Vav3 phosphorylation and subsequent cytoskeletal reorganisation. SYK deficient osteoclasts are inactive and show defective cytoskeletal reorganisation. In correlation with this, SYK deficient embryos show defective skeletal mass^{7,8}.

BCR-mediated activation of B-cells in the lymph nodes, as well as FcR-mediated activation of dendritic cells, monocytes, macrophages, neutrophils and mast cells in the joints, are essential components of the cellular patho-physiological mechanisms taking place during rheumaoid arthritis (RA). Moreover, activation of osteoclasts leads to the bone and cartilage destruction which are hallmarks of this pathology⁹. SYK signaling should therefore play a pivotal role during the development of arthritis, both at the periphery and on the site of inflammation¹⁰. Indeed, an orally available Syk inhibitor R406 -developed by Rigel- induced a significant improvement of clinical scores and significantly reduced serum cytokine concentrations, as well as bone erosion, in a murine model of RA^{11,12}. Moreover, this inhibitor has shown efficacy (ACR scores improvement) and good tolerability in RA Phase II studies in humans^{13,14,15}.

In SLE B cells contribute essentially towards pathogenesis via production of autoanibodies resulting in immune complex formation, stimulation of Fc receptors and finally in an excessive and chronic activation of inflammation. In a murine model of SLE treatment with a Syk inhibitor resulted in a reduction of numbers of class-switched germinal center, marginal zone, newly formed and follicular B cells and therefore in disease mitigating effects¹⁸.

Although TCR signals are transmited by the intracellular tyrosine kinase ZAP-70 in thymocytes and naïve T cells, several studies indicate that differentiated effector T cells, such as those involved in the pathophysiology of Multiple sclerosis (MS) or systemic lupus erythematosus (SLE), show a down regulation of the TCRzeta chain and a concomitant upregulation of the TCR/CD3 chain and its interaction with FcRγ. Those studies show that the TCR/CD3/FcRgamma complex in effector cells recruits and activates Syk, instead of ZAP-70, tyrosine kinase. This physiologic switch in TCR signaling occurs exclusively in effector, and not naive or memory T cells^{16,17,18}. Not surprisingly then, SYK inhibitors have been shown to delay disease progression and to improve survival in murine models of SLE^{17,18,19,20,21}.

SYK inhibitors may also find a use in asthma, allergy, multiple sclerosis and other diseases such as thrombocytopenia purpura and T or B cell lymphomas^{1,10,14,22-35}. Treatment of prediseased NZB/W mice with a Syk inhibitor prevented the development of renal disease demonstrated by reduced glomerular sclerosis, tubular damage, proteinuria and BUN levels¹⁸.

### References

1. Turner, M., Schweighoffer, E., Colucci, F., Di Santo, J.P. & Tybulewicz, V.L. Tyrosine kinase SYK: essential functions for immunoreceptor signalling. Immunol Today 21, 148-154 (2000).
2. Ghosh, D. & Tsokos, G.C. Spleen tyrosine kinase: an Src family of non-receptor kinase has multiple functions and represents a valuable therapeutic target in the treatment of autoimmune and inflammatory diseases. Autoimmunity 43, 48-55.
3. Lindvall, J.M., et al. Bruton's tyrosine kinase: cell biology, sequence conservation, mutation spectrum, siRNA modifications, and expression profiling. Immunol Rev 203, 200-215 (2005).
4. Gilfillan, A.M. & Tkaczyk, C. Integrated signalling pathways for mast-cell activation. Nat Rev Immunol 6, 218-230 (2006).
5. Gomez, G., Schwartz, L. & Kepley, C. Syk deficiency in human non-releaser lung mast cells. Clin Immunol 125, 112-115 (2007).
6. Kepley, C.L., Youssef, L., Andrews, R.P., Wilson, B.S. & Oliver, J.M. Syk deficiency in nonreleaser basophils. J Allergy Clin Immunol 104, 279-284 (1999).
7. Zou, W., et al. Syk, c-Src, the alphavbeta3 integrin, and ITAM immunoreceptors, in concert, regulate osteoclastic bone resorption. J Cell Biol 176, 877-888 (2007).
8. Reeve, J.L., et al. SLP-76 couples Syk to the osteoclast cytoskeleton. J Immunol 183, 1804-1812 (2009).
9. Klareskog, L., Catrina, A.I. & Paget, S. Rheumatoid arthritis. Lancet 373, 659-672 (2009).
10. Wong, B.R., Grossbard, E.B., Payan, D.G. & Masuda, E.S. Targeting Syk as a treatment for allergic and autoimmune disorders. Expert Opin Investig Drugs 13, 743-762 (2004).
11. Braselmann, S., et al. R406, an orally available spleen tyrosine kinase inhibitor blocks fc receptor signaling and reduces immune complex-mediated inflammation. J Pharmacol Exp Ther 319, 998-1008 (2006).
12. Pine, P.R., et al. Inflammation and bone erosion are suppressed in models of rheumatoid arthritis following treatment with a novel Syk inhibitor. Clin Immunol 124, 244-257 (2007).
13. Tomillero, A. & Moral, M.A. Gateways to clinical trials. Methods Find Exp Clin Pharmacol 31, 47-57 (2009).
14. Bajpai, M. Fostamatinib, a Syk inhibitor prodrug for the treatment of inflammatory diseases. IDrugs 12, 174-185 (2009).
15. Weinblatt, M.E., et al. Treatment of rheumatoid arthritis with a Syk kinase inhibitor: a twelve-week, randomized, placebo-controlled trial. Arthritis Rheum 58, 3309-3318 (2008).
16. Krishnan, S., Warke, V.G., Nambiar, M.P., Tsokos, G.C. & Farber, D.L. The FcR gamma subunit and Syk kinase replace the CD3 zeta-chain and ZAP-70 kinase in the TCR signaling complex of human effector CD4 T cells. J Immunol 170, 4189-4195 (2003).
17. Krishnan, S., et al. Differential expression and molecular associations of Syk in systemic lupus erythematosus T cells. J Immunol 181, 8145-8152 (2008).
18. Bahjat, F.R., et al. An orally bioavailable spleen tyrosine kinase inhibitor delays disease progression and prolongs survival in murine lupus. Arthritis Rheum 58, 1433-1444 (2008).
19. Smith, J., et al. A Spleen Tyrosine Kinase Inhibitor Reduces the Severity of Established Glomerulonephritis. J Am Soc Nephrol (2009).
20. Enyedy, E.J., et al. Fc epsilon receptor type I gamma chain replaces the deficient T cell receptor zeta chain in T cells of patients with systemic lupus erythematosus. Arthritis Rheum 44, 1114-1121 (2001).
21. Perl, A. Systems biology of lupus: mapping the impact of genomic and environmental factors on gene expression signatures, cellular signaling, metabolic pathways, hormonal and cytokine imbalance, and selecting targets for treatment. Autoimmunity 43, 32-47.
22. Smith, J., et al. A spleen tyrosine kinase inhibitor reduces the severity of established glomerulonephritis. J Am Soc Nephrol 21, 231-236.
23. Sanderson, M.P., Gelling, S.J., Rippmann, J.F. & Schnapp, A. Comparison of the anti-allergic activity of Syk inhibitors with optimized Syk siRNAs in FcepsilonRI-activated RBL-2H3 basophilic cells. Cell Immunol 262, 28-34.
24. Podolanczuk, A., Lazarus, A.H., Crow, A.R., Grossbard, E. & Bussel, J.B. Of mice and men: an open-label pilot study for treatment of immune thrombocytopenic purpura by an inhibitor of Syk. *Blood* **113**, 3154-3160 (2009).
25. Bajpai, M., Chopra, P., Dastidar, S.G. & Ray, A. Spleen tyrosine kinase: a novel target for therapeutic intervention of rheumatoid arthritis. *Expert Opin Investig Drugs* **17**, 641-659 (2008).
26. Friedberg, J.W., *et al.* Inhibition of Syk with fostamatinib disodium has significant clinical activity in non-Hodgkin lymphoma and chronic lymphocytic leukemia. *Blood* **115**, 2578-2585.
27. Gao, C., *et al.* Eptifibatide-induced thrombocytopenia and thrombosis in humans require FcgammaRIIa and the integrin beta3 cytoplasmic domain. *J Clin Invest* **119**, 504-511 (2009).
28. Marjon, K.D., Marnell, L.L., Mold, C. & Du Clos, T.W. Macrophages activated by C-reactive protein through Fc gamma RI transfer suppression of immune thrombocytopenia. J Immunol 182, 1397-1403 (2009).
29. Chen, L., et al. SYK-dependent tonic B-cell receptor signaling is a rational treatment target in diffuse large B-cell lymphoma. Blood 111, 2230-2237 (2008).
30. Ponzoni, M., et al. Syk expression patterns differ among B-cell lymphomas. Leuk Res (2010).
31. Pechloff, K., et al. The fusion kinase ITK-SYK mimics a T cell receptor signal and drives oncogenesis in conditional mouse models of peripheral T cell lymphoma. J Exp Med 207, 1031-1044 (2009).
32. Uckun, F.M., Ek, R.O., Jan, S.T., Chen, C.L. & Qazi, S. Targeting SYK kinase-dependent anti-apoptotic resistance pathway in B-lineage acute lymphoblastic leukaemia (ALL) cells with a potent SYK inhibitory pentapeptide mimic. Br J Haematol 149, 508-517 (2010).
33. Wilcox, R.A., et al. Inhibition of Syk protein tyrosine kinase induces apoptosis and blocks proliferation in T-cell non-Hodgkin's lymphoma cell lines. Leukemia 24, 229-232 (2009).
34. Feldman, A.L., et al. Overexpression of Syk tyrosine kinase in peripheral T-cell lymphomas. Leukemia 22, 1139-1143 (2008).
35. Wang, L., et al. Alternative splicing disrupts a nuclear localization signal in spleen tyrosine kinase that is required for invasion suppression in breast cancer. Cancer Res 63, 4724-4730 (2003).

In addition to mast cells, Syk is expressed in other hematopoietic cells including B cells, where it is thought to play an essential role in transducing signals required for the transition of immature B cells into mature recirculating B cells (M. Turner et al, Immunology Today, 21: 148 (2000)). B cells are reported to play an important role in some inflammatory conditions such as lupus (O. T. Chan etal., Immunological Rev, 169: 107-121 (1999)) and rheumatoid arthritis (A. Gause et al, Biodrugs, 15(2): 73-79 (2001)).

Syk was also reported to be an element of the signaling cascade in beta-amyloid and prion fibrils leading to production of neurotoxic products (C. K. Combs et al., J. Neuroscl, 19: 928-939 (1999)). Furthermore, an inhibitor of Syk blocked the production of these neurotoxic products. Thus furopyridine derivatives would potentially be useful in the treatment of Alzheimer's disease and related neuroinflammatory diseases. Another report (Y. Kuno et al., Blood, 97, 1050-1055 (2001)) demonstrates that Syk plays an important role in malignant progression. A TEL-Syk fusion protein was found to transform hematopoietic cells suggesting a role in the pathogenesis of hematopoietic malignancies. Therefore compounds presently claimed may be useful in the treatment of certain types of cancers.

### PRIOR ART

Triazolopyrimidine derivatives are described as GSK3 inhibitors for the treatment of diseases like Alzheimer or diabtes in WO 2005/012307 A1 and in WO 2006/075023 A2. WO 2012/025186 A1 discloses triazolopyrazine derivatives, which are always substituted at the 8-position. The compounds are SYK inhibitors.

### SUMMARY OF THE INVENTION

The invention relates to compounds of the formula I 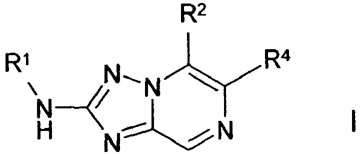 in which
- R¹: denotes Ar, COHet or Het,
- R²: denotes H, Ar¹, NHHet or Het,
- R³: denotes H or A',
- R⁴: denotes H, A, Ar¹, Het, Hal, NHAr¹ or CN,
- Ar: denotes phenyl or naphthyl which is unsubstituted or mono- or disubstituted by Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚN(R³)₂, [C(R³)₂]ₚHet¹, NR³SO₂A, SO₂N(R³)₂, S(O)ₙA, COHet¹, O[C(R³)₂]ₘN(R³)₂ and/or O[C(R³)₂]ₚHet¹,
- Ar¹: denotes phenyl which is unsubstituted or mono- or disubstituted by Hal, A, phenyl, CONH₂, [C(R³)₂]ₚOR³, [C(R³)₂]ₚN(R³)₂, [C(R³)₂]ₚCN, [C(R³)₂]ₚHet¹ and/or O[C(R³)₂]ₚHet¹,
- Het: denotes furyl, thienyl, pyrrolyl, imidazolyl, pyrazolyl, oxazolyl, isoxazolyl, oxadiazolyl, thiazolyl, triazolyl, tetrazolyl, pyridyl, pyrimidyl, pyridazinyl, pyrazinyl, indolyl, isoindolyl, benzimidazolyl, indazolyl, quinolyl, 1,3-benzodioxolyl, benzothiophenyl, benzofuranyl, imidazopyridyl, dihydroindolyl, 2,3-dihydro-benzo[1,4]dioxinyl or furo[3,2-b]pyridyl which is unsubstituted or mono-, di- or trisubstituted by Hal, A, [C(R³)₂]ₚOR³, [C(R³)₂]ₚN(R³)₂, [C(R³)₂]ₚHet¹, NO₂, CN, [C(R⁶)₂]ₚCOOR³, CON(R³)₂, NR³COA, NR³SO₂A, SO₂N(R³)₂, S(O)ₙA, COHet¹, O[C(R³)₂]ₘN(R³)², O[C(R³)₂]ₚHet¹ and/or =O,

- Het¹: denotes dihydropyrrolyl, pyrrolidinyl, azetidinyl, oxetanyl, tetrahydroimidazolyl, dihydropyrazolyl, tetrahydropyrazolyl, tetrahydrofuranyl, dihydropyridyl, tetrahydropyridyl, piperidinyl, morpholinyl, hexahydropyridazinyl, hexahydropyrimidinyl, [1,3]dioxolanyl, tetrahydropyranyl, pyridyl or piperazinyl, which is unsubstituted or mono- or disubstituted by Hal, CN, OH, OA, COOA, CONH₂, S(O)ₙA, S(O)ₙAr, COA, A and/or =O,
- A: denotes unbranched or branched alkyl with 1-10 C-atoms, wherein one or two non-adjacent CH- and/or CH₂-groups may be replaced by N-, O- and/or S-atoms and wherein 1-7 H-atoms may be replaced by F or Cl,
- Cyc: denotes cyclic alkyl with 3-7 C-atoms, which is unsubstituted or monosubstituted by [C(R³)₂]ₚOH or CN,
- A': denotes unbranched or branched alkyl with 1, 2, 3 or 4 C-atoms,
- Hal: denotes F, Cl, Br or I,
- n: denotes 0, 1 or 2,
- m: denotes 1, 2 or 3,
- p: denotes 0, 1, 2, 3 or 4,
and pharmaceutically usable derivatives, solvates, salts, tautomers and stereoisomers thereof, including mixtures thereof in all ratios.

The invention also relates to the optically active forms (stereoisomers), the enantiomers, the racemates, the diastereomers and the hydrates and solvates of these compounds.

The invention also relates to the solvates of the salts of the compounds of formula I, e.g. the mono- or dihydrate of the hydrochloride.

Moreover, the invention relates to pharmaceutically acceptable derivatives of compounds of formula I.

The term solvates of the compounds is taken to mean adductions of inert solvent molecules onto the compounds which form owing to their mutual attractive force. Solvates are, for example, mono- or dihydrates or alcoholates. The term pharmaceutically acceptable derivatives is taken to mean, for example, the salts of the compounds according to the invention and also so-called prodrug compounds.

As used herein and unless otherwise indicated, the term "prodrug" means a derivative of a compound of formula I that can hydrolyze, oxidize, or otherwise react under biological conditions (in vitro or in vivo) to provide an active compound, particularly a compound of formula I. Examples of prodrugs include, but are not limited to, derivatives and metabolites of a compound of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. In certain embodiments, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well- known methods, such as those described by Burger's Medicinal Chemistry and Drug Discovery 6th ed. (Donald J. Abraham ed., 2001, Wiley) and Design and Application of Prodrugs (H.Bundgaard ed., 1985, Harwood Academic Publishers Gmfh).

The expression "effective amount" denotes the amount of a medicament or of a pharmaceutical active ingredient which causes in a tissue, system, animal or human a biological or medical response which is sought or desired, for example, by a researcher or physician.

In addition, the expression "therapeutically effective amount" denotes an amount which, compared with a corresponding subject who has not received this amount, has the following consequence:
improved treatment, healing, prevention or elimination of a disease, syndrome, condition, complaint, disorder or side-effects or also the reduction in the advance of a disease, complaint or disorder.

The expression "therapeutically effective amount" also encompasses the amounts which are effective for increasing normal physiological function.

The invention also relates to the use of mixtures of the compounds of the formula I, for example mixtures of two diastereomers, for example in the ratio 1:1, 1:2, 1:3, 1:4, 1:5, 1:10, 1:100 or 1:1000.

These are particularly preferably mixtures of stereoisomeric compounds.

"Tautomers" refers to isomeric forms of a compound that are in equilibrium with each other. The concentrations of the isomeric forms will depend on the environment the compound is found in and may be different depending upon, for example, whether the compound is a solid or is in an organic or aqueous solution.

The invention relates to the compounds of the formula I and salts thereof and to a process for the preparation of compounds of the formula I and pharmaceutically usable salts, solvates, tautomers and stereoisomers thereof, characterised in that
a compound of the formula II 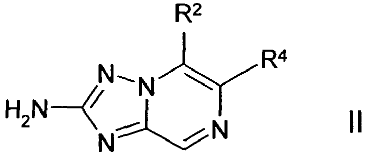
in which R² and R⁴ have the meanings indicated in Claim 1, is reacted with a compound of the formula III

   R¹-L III
in which R¹ has the meaning indicated in Claim 1 and
L denotes Cl or Br,
and/or
a base or acid of the formula I is converted into one of its salts.

Above and below, the radicals R¹, R² and R⁴ have the meanings indicated for the formula I, unless expressly stated otherwise.

A denotes alkyl, this is unbranched (linear) or branched, and has 1, 2, 3, 4, 5, 6, 7, 8, 9 or 10 C atoms. A preferably denotes methyl, furthermore ethyl, propyl, isopropyl, butyl, isobutyl, sec-butyl or tert-butyl, furthermore also pentyl, 1-, 2- or 3-methylbutyl, 1,1- , 1,2- or 2,2-dimethylpropyl, 1-ethylpropyl, hexyl, 1-, 2-, 3- or 4-methylpentyl, 1,1- , 1,2-, 1,3-, 2,2-, 2,3- or 3,3-dimethylbutyl, 1-or 2-ethylbutyl, 1-ethyl-1-methylpropyl, 1-ethyl-2-methylpropyl, 1,1,2- or 1,2,2-trimethylpropyl, furthermore preferably, for example, trifluoromethyl.

A very particularly preferably denotes alkyl having 1, 2, 3, 4, 5 or 6 C atoms, preferably methyl, ethyl, propyl, isopropyl, butyl, isobutyl, sec-butyl, tert-butyl, pentyl, hexyl, trifluoromethyl, pentafluoroethyl or 1,1,1-trifluoroethyl. Moreover, A denotes e.g. CH₂OCH₃, CH₂CH₂OH, OCH₂CH₂NH₂, CH₂NHCH₂ or NHCH₂CH₃.

Cyc preferably denotes cyclopropyl, cyclobutyl, cyclopentyl, cyclohexyl or cycloheptyl.

A' preferably denotes methyl, ethyl, propyl, isopropyl or butyl.

R² preferably denotes furyl, thienyl, pyrrolyl, imidazolyl, pyrazolyl, oxazolyl, isoxazolyl, oxadiazolyl, thiazolyl, triazolyl or tetrazolyl which is unsubstituted or monosubstituted by A, [C(R³)₂]ₚCyc, [C(R³)₂]ₚAr, [C(R³)₂]ₚHet¹, CN or [C(R³)₂]ₚCOOR³.

R³ preferably denotes H or alkyl having 1, 2, 3 or 4 C atoms, particularly preferably H or methyl.

Ar denotes, for example, o-, m- or p-tolyl, o-, m- or p-ethylphenyl, o-, m- or p-propylphenyl, o-, m- or p-isopropylphenyl, o-, m- or p-tert-butylphenyl, o-, m-or p-hydroxyphenyl, o-, m- or p-nitrophenyl, o-, m- or p-aminophenyl, o-, m- or p-(N-methylamino)phenyl, o-, m- or p-(N-methylaminocarbonyl)phenyl, o-, m-or p-methoxyphenyl, o-, m- or p-ethoxyphenyl, o-, m- or p-ethoxycarbonyl-phenyl, o-, m- or p-(N,N-dimethylamino)phenyl, o-, m- or p-(N,N-dimethylaminocarbonyl)phenyl, o-, m- or p-(N-ethylamino)phenyl, o-, m- or p-(N,N-diethylamino)phenyl, o-, m- or p-fluorophenyl, o-, m- or p-bromophenyl, o-, m-or p-chlorophenyl, o-, m- or p-(methylsulfonamido)phenyl, o-, m- or p-(methylsulfonyl)phenyl, o-, m- or p-cyanophenyl, o-, m- or p-carboxyphenyl, o-, m- or p-methoxycarbonylphenyl, o-, m- or p-formylphenyl, o-, m- or p-acetylphenyl, o-, m- or p-aminosulfonylphenyl, o-, m- or p-[2-(morpholin-4-yl)ethoxy]phenyl, o-, m- or p-[3-(N,N-diethylamino)propoxy]phenyl, furthermore preferably 2,3-, 2,4-, 2,5-, 2,6-, 3,4- or 3,5-difluorophenyl, 2,3-, 2,4-, 2,5-, 2,6-, 3,4- or 3,5-dichlorophenyl, 2,3-, 2,4-, 2,5-, 2,6-, 3,4- or 3,5-dibromophenyl, 2,4- or 2,5-dinitrophenyl, 2,5- or 3,4-dimethoxyphenyl, 3-nitro-4-chlorophenyl, 3-amino-4-chloro-, 2-amino-3-chloro-, 2-amino-4-chloro-, 2-amino-5-chloro- or 2-amino-6-chlorophenyl, 2-nitro-4-N,N-dimethylamino- or 3-nitro-4-N,N-dimethylamino-phenyl, 2,3-diaminophenyl, 2,3,4-, 2,3,5-, 2,3,6-, 2,4,6- or 3,4,5-trichlorophenyl, 2,4,6-trimethoxyphenyl, 2-hydroxy-3,5-dichlorophenyl, p-iodophenyl, 3,6-di-chloro-4-aminophenyl, 4-fluoro-3-chlorophenyl, 2-fluoro-4-bromophenyl, 2,5-difluoro-4-bromophenyl, 3-bromo-6-methoxyphenyl, 3-chloro-6-methoxyphenyl, 3-chloro-4-acetamidophenyl, 3-fluoro-4-methoxyphenyl, 3-amino-6-methylphenyl, 3-chloro-4-acetamidophenyl or 2,5-dimethyl-4-chlorophenyl.

Ar furthermore preferably denotes phenyl which is unsubstituted or mono- or disubstituted by Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚHet¹, SO₂N(R³)₂, NR³SO₂A, O[C(R³)₂]ₚHet¹, COHet¹, and/or S(O)ₙA.

Ar¹ preferably denotes phenyl which is unsubstituted or mono- or disubstituted by Hal, A, phenyl, CONH₂, [C(R³)₂]ₚCN, [C(R³)₂]ₚOR³ and/or [C(R³)₂]ₚHet¹.

Het preferably denotes furyl, thienyl, pyrrolyl, imidazolyl, pyrazolyl, oxazolyl, isoxazolyl, oxadiazolyl, thiazolyl, triazolyl, tetrazolyl, pyridyl, pyrimidyl, pyridazinyl, pyrazinyl, indolyl, isoindolyl, benzimidazolyl, indazolyl, quinolyl, 1,3-benzodioxolyl, benzothiophenyl, benzofuranyl, imidazopyridyl, dihydroindolyl, 2,3-dihydro-benzo[1,4]dioxinyl or furo[3,2-b]pyridyl which is unsubstituted or mono-, di- or trisubstituted by A, [C(R³)₂]ₚN(R³)₂ and/or =O.

Het particularly preferably denotes pyrazolyl, pyridyl, benzimidazolyl, indazolyl, quinolyl or dihydroindolyl which is unsubstituted or mono-, di- or trisubstituted by A, [C(R³)₂]ₚHet¹, [C(R³)₂]ₚN(R³)₂ and/or =O.

Het¹ preferably denotes dihydropyrrolyl, pyrrolidinyl, azetidinyl, oxetanyl, tetrahydroimidazolyl, dihydropyrazolyl, tetrahydropyrazolyl, tetrahydrofuranyl, dihydropyridyl, tetrahydropyridyl, piperidinyl, morpholinyl, hexahydropyridazinyl, hexahydropyrimidinyl, [1,3]dioxolanyl, tetrahydropyranyl, pyridyl or piperazinyl, which is unsubstituted or mono- or disubstituted by A.

Het¹ particularly preferably denotes morpholinyl or piperazinyl, which is unsubstituted or mono- or disubstituted by A.

Hal preferably denotes F, Cl or Br, but also I, particularly preferably F or Cl.

Throughout the invention, all radicals which occur more than once may be identical or different, i.e. are independent of one another.

The compounds of the formula I may have one or more chiral centres and can therefore occur in various stereoisomeric forms. The formula I encompasses all these forms.

Accordingly, the invention relates, in particular, to the compounds of the formula I in which at least one of the said radicals has one of the preferred meanings indicated above. Some preferred groups of compounds may be expressed by the following sub-formulae Ia to Ie, which conform to the formula I and in which the radicals not designated in greater detail have the meaning indicated for the formula I, but in which

| | | |
|---|---|---|
| in Ia | Ar | denotes phenyl which is unsubstituted or mono- or disubstituted by Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚHet¹, SO₂N(R³)₂, NR³SO₂A, O[C(R³)₂]ₚHet¹, COHet¹, and/or S(O)ₙA; |
| | | |
| in Ib | Ar¹ | denotes phenyl which is unsubstituted or mono- or disubstituted by Hal, A, phenyl, CONH₂, [C(R³)₂]ₚCN, [C(R³)₂]ₚOR³ and/or [C(R³)₂]ₚHet¹; |
| | | |
| in Ic | Het | denotes furyl, thienyl, pyrrolyl, imidazolyl, pyrazolyl, oxazolyl, isoxazolyl, oxadiazolyl, thiazolyl, triazolyl, tetrazolyl, pyridyl, pyrimidyl, pyridazinyl, pyrazinyl, indolyl, isoindolyl, benzimid-azolyl, indazolyl, quinolyl, 1,3-benzodioxolyl, benzothiophenyl, benzofuranyl, imidazopyridyl, dihydroindolyl, 2,3-dihydro-benzo[1,4]dioxinyl or furo[3,2-b]pyridyl which is unsubstituted or mono-, di- or trisubstituted by A, [C(R³)₂]ₚHet¹, [C(R³)₂]ₚN(R³)₂ and/or =O; |
| | | |
| in Id | Het¹ | denotes dihydropyrrolyl, pyrrolidinyl, azetidinyl, oxetanyl, tetrahydroimidazolyl, dihydropyrazolyl, tetrahydropyrazolyl, tetrahydrofuranyl, dihydropyridyl, tetrahydropyridyl, piperidinyl, morpholinyl, hexahydropyridazinyl, hexahydropyrimidinyl, [1,3]dioxolanyl, tetrahydropyranyl, pyridyl or piperazinyl, which is unsubstituted or mono- or disubstituted by A; |
| | | |
| in Ie | R¹ | denotes Ar, COHet or Het, |
| | R² | denotes H, Ar¹, NHHet or Het, |
| | R³ | denotes H or A', |
| | R⁴ | denotes H, A, Ar¹, Het, Hal, NHAr¹ or CN, |
| | Ar | denotes phenyl which is unsubstituted or mono- or disubstituted by Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚHet¹, SO₂N(R³)₂, NR³SO₂A, O[C(R³)₂]ₚHet¹, COHet¹, and/or S(O)ₙA, |
| | Ar¹ | denotes phenyl which is unsubstituted or mono- or disubstituted by Hal, A, phenyl, CONH₂, [C(R³)₂]ₚCN, [C(R³)₂]ₚOR³ and/or [C(R³)₂]ₚHet¹, |
| | Het | denotes furyl, thienyl, pyrrolyl, imidazolyl, pyrazolyl, oxazolyl, isoxazolyl, oxadiazolyl, thiazolyl, triazolyl, tetrazolyl, pyridyl, pyrimidyl, pyridazinyl, pyrazinyl, indolyl, isoindolyl, benzimidazolyl, indazolyl, quinolyl, 1,3-benzodioxolyl, benzothiophenyl, benzofuranyl, imidazopyridyl, dihydroindolyl, 2,3-dihydro-benzo[1,4]dioxinyl or furo[3,2-b]pyridyl which is unsubstituted or mono-, di- or trisubstituted by A, [C(R³)₂]ₚHet¹, [C(R³)₂]ₚN(R³)₂ and/or =O, |
| | Het¹ | denotes dihydropyrrolyl, pyrrolidinyl, azetidinyl, oxetanyl, tetrahydroimidazolyl, dihydropyrazolyl, tetrahydropyrazolyl, tetrahydrofuranyl, dihydropyridyl, tetrahydropyridyl, piperidinyl, morpholinyl, hexahydropyridazinyl, hexahydropyrimidinyl, [1,3]dioxolanyl, tetrahydropyranyl, pyridyl or piperazinyl, which is unsubstituted or mono- or disubstituted by A, |
| | A | denotes unbranched or branched alkyl with 1-10 C-atoms, and wherein 1-7 H-atoms may be replaced by F or Cl, |
| | Cyc | denotes cyclic alkyl with 3-7 C-atoms, which is unsubstituted or monosubstituted by [C(R³)₂]ₚOH or CN, |
| | A' | denotes unbranched or branched alkyl with 1, 2, 3 or 4 C-atoms, |
| | Hal | denotes F, Cl, Br or I, |
| | n | denotes 0, 1 or 2, |
| | p | denotes 0, 1, 2, 3 or 4; |

and pharmaceutically acceptable salts, solvates, tautomers and stereoisomers thereof, including mixtures thereof in all ratios.

The compounds of the formula I and also the starting materials for their preparation are, in addition, prepared by methods known per se, as described in the literature (for example in the standard works, such as Houben-Weyl, Methoden der organischen Chemie [Methods of Organic Chemistry], Georg-Thieme-Verlag, Stuttgart), to be precise Use can also be made here of variants known per se which are not mentioned here in greater detail.

The starting compounds of the formulae II and III are generally known. If they are novel, however, they can be prepared by methods known per se.

Compounds of the formula I can preferably be obtained by reacting a compound of the formula II with a compound of the formula III.

The reaction is generally carried out under Buchwald-conditions known to the skilled artisan and which are known and suitable for the said reaction. Depending on the conditions used, the reaction time is between a few minutes and 14 days, the reaction temperature is between about 0° and 140°, normally between 20° and 120°, in particular between about 60° and about 110°. Examples of suitable inert solvents are hydrocarbons, such as hexane, petroleum ether, benzene, toluene or xylene; chlorinated hydrocarbons, such as trichloroethylene, 1,2-dichloroethane, carbon tetrachloride, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether, ethylene glycol dimethyl ether (diglyme); ketones, such as acetone or butanone; amides, such as acetamide, dimethylacetamide or dimethylformamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate, or mixtures of the said solvents.

Particular preference is given to aprotic solvents, particularly preferably to THF.

Free amino groups can furthermore be acylated in a conventional manner using an acid chloride or anhydride or alkylated using an unsubstituted or substituted alkyl halide, advantageously in an inert solvent, such as dichloromethane or THF, and/or in the presence of a base, such as triethylamine or pyridine, at temperatures between -60 and +30°.

### Pharmaceutical salts and other forms

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. Pharmaceutically acceptable salt forms of the compounds of the formula I are for the most part prepared by conventional methods. If the compound of the formula I contains a carboxyl group, one of its suitable salts can be formed by reacting the compound with a suitable base to give the corresponding base-addition salt. Such bases are, for example, alkali metal hydroxides, including potassium hydroxide, sodium hydroxide and lithium hydroxide; alkaline earth metal hydroxides, such as barium hydroxide and calcium hydroxide; alkali metal alkoxides, for example potassium ethoxide and sodium propoxide; and various organic bases, such as piperidine, diethanolamine and N-methylglutamine. The aluminium salts of the compounds of the formula I are likewise included. In the case of certain compounds of the formula I, acid-addition salts can be formed by treating these compounds with pharmaceutically acceptable organic and inorganic acids, for example hydrogen halides, such as hydrogen chloride, hydrogen bromide or hydrogen iodide, other mineral acids and corresponding salts thereof, such as sulfate, nitrate or phosphate and the like, and alkyl- and monoarylsulfonates, such as ethanesulfonate, toluenesulfonate and benzenesulfonate, and other organic acids and corresponding salts thereof, such as acetate, trifluoroacetate, tartrate, maleate, succinate, citrate, benzoate, salicylate, ascorbate and the like. Accordingly, pharmaceutically acceptable acid-addition salts of the compounds of the formula I include the following: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemi-sulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, palmoate, pectinate, persulfate, phenylacetate, 3-phenylpropionate, phosphate, phosphonate, phthalate, but this does not represent a restriction.

Furthermore, the base salts of the compounds according to the invention include aluminium, ammonium, calcium, copper, iron(III), iron(II), lithium, magnesium, manganese(III), manganese(II), potassium, sodium and zinc salts, but this is not intended to represent a restriction. Of the above-mentioned salts, preference is given to ammonium; the alkali metal salts sodium and potassium, and the alkaline earth metal salts calcium and magnesium. Salts of the compounds of the formula I which are derived from pharmaceutically acceptable organic non-toxic bases include salts of primary, secondary and tertiary amines, substituted amines, also including naturally occurring substituted amines, cyclic amines, and basic ion exchanger resins, for example arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris(hydroxymethyl)methylamine (tromethamine), but this is not intended to represent a restriction.

Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

The above-mentioned pharmaceutical salts which are preferred include acetate, trifluoroacetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate and tromethamine, but this is not intended to represent a restriction.

Particular preference is given to hydrochloride, dihydrochloride, hydrobromide, maleate, mesylate, phosphate, sulfate and succinate.

The acid-addition salts of basic compounds of the formula I are prepared by bringing the free base form into contact with a sufficient amount of the desired acid, causing the formation of the salt in a conventional manner. The free base can be regenerated by bringing the salt form into contact with a base and isolating the free base in a conventional manner. The free base forms differ in a certain respect from the corresponding salt forms thereof with respect to certain physical properties, such as solubility in polar solvents; for the purposes of the invention, however, the salts otherwise correspond to the respective free base forms thereof.

As mentioned, the pharmaceutically acceptable base-addition salts of the compounds of the formula I are formed with metals or amines, such as alkali metals and alkaline earth metals or organic amines. Preferred metals are sodium, potassium, magnesium and calcium. Preferred organic amines are N,N'-dibenzylethylenediamine, chloroprocaine, choline, diethanolamine, ethylenediamine, N-methyl-D-glucamine and procaine.

The base-addition salts of acidic compounds according to the invention are prepared by bringing the free acid form into contact with a sufficient amount of the desired base, causing the formation of the salt in a conventional manner. The free acid can be regenerated by bringing the salt form into contact with an acid and isolating the free acid in a conventional manner. The free acid forms differ in a certain respect from the corresponding salt forms thereof with respect to certain physical properties, such as solubility in polar solvents; for the purposes of the invention, however, the salts otherwise correspond to the respective free acid forms thereof.

If a compound according to the invention contains more than one group which is capable of forming pharmaceutically acceptable salts of this type, the invention also encompasses multiple salts. Typical multiple salt forms include, for example, bitartrate, diacetate, difumarate, dimeglumine, diphosphate, disodium and trihydrochloride, but this is not intended to represent a restriction.

With regard to that stated above, it can be seen that the expression "pharmaceutically acceptable salt" in the present connection is taken to mean an active ingredient which comprises a compound of the formula I in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

### Isotopes

There is furthermore intended that a compound of the formula I includes isotope-labelled forms thereof. An isotope-labelled form of a compound of the formula I is identical to this compound apart from the fact that one or more atoms of the compound have been replaced by an atom or atoms having an atomic mass or mass number which differs from the atomic mass or mass number of the atom which usually occurs naturally. Exam-ples of isotopes which are readily commercially available and which can be incorporated into a compound of the formula I by well-known methods include isotopes of hydrogen, carbon, nitrogen, oxygen, phos-phorus, fluo-rine and chlorine, for example ²H, ³H, ¹³C, ¹⁴C, ¹⁵N, ¹⁸O, ¹⁷O, ³¹P, ³²P, ³⁵S, ¹⁸F and ³⁶Cl, respectively. A compound of the formula I, a prodrug, thereof or a pharmaceutically acceptable salt of either which contains one or more of the above-mentioned isotopes and/or other iso-topes of other atoms is intended to be part of the present invention. An isotope-labelled compound of the formula I can be used in a number of beneficial ways. For example, an isotope-labelled compound of the formula I into which, for example, a radioisotope, such as ³H or ¹⁴C, has been incorporated is suitable for medicament and/or substrate tissue distribution assays. These radioisotopes, i.e. tritium (³H) and carbon-14 (¹⁴C), are particularly preferred owing to simple preparation and excellent detectability. Incor-po-ra-tion of heavier isotopes, for example deuterium (²H), into a compound of the formula I has therapeutic advantages owing to the higher metabolic stability of this isotope-labelled compound. Higher metabolic stability translates directly into an increased in vivo half-life or lower dosages, which under most circumstances would represent a preferred embodi-ment of the present invention. An isotope-labelled compound of the formula I can usually be prepared by carrying out the procedures dis-closed in the synthesis schemes and the related description, in the example part and in the preparation part in the present text, replacing a non-isotope-labelled reactant by a readily available isotope-labelled reactant.

Deuterium (²H) can also be incorporated into a compound of the formula I for the purpose in order to manipulate the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus cause a reduction in the rate in rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M}/k_{D} = 2-7 are typical. If this rate difference is successfully applied to a com-pound of the formula I that is susceptible to oxidation, the profile of this compound in vivo can be drastically modified and result in improved pharmacokinetic properties.

When discovering and developing therapeutic agents, the person skilled in the art attempts to optimise pharmacokinetic parameters while retaining desirable in vitro properties. It is reasonable to assume that many com-pounds with poor pharmacokinetic profiles are susceptible to oxidative metabolism. In vitro liver microsomal assays currently available provide valuable information on the course of oxidative metabolism of this type, which in turn permits the rational design of deuterated compounds of the formula I with improved stability through resistance to such oxidative meta-bolism. Significant improvements in the pharmacokinetic profiles of compounds of the formula I are thereby obtained, and can be expressed quantitatively in terms of increases in the in vivo half-life (t/2), concen-tra-tion at maximum therapeutic effect (Cₘₐₓ), area under the dose response curve (AUC), and F; and in terms of reduced clearance, dose and materi-als costs.

The following is intended to illustrate the above: a compound of the formula I which has multiple potential sites of attack for oxidative metabolism, for example benzylic hydrogen atoms and hydrogen atoms bonded to a nitrogen atom, is prepared as a series of analogues in which various combinations of hydrogen atoms are replaced by deuterium atoms, so that some, most or all of these hydrogen atoms have been replaced by deuterium atoms. Half-life determinations enable favourable and accurate determination of the extent of the extent to which the improve-ment in resistance to oxidative metabolism has improved. In this way, it is deter-mined that the half-life of the parent compound can be extended by up to 100% as the result of deuterium-hydrogen exchange of this type.

Deuterium-hydrogen exchange in a compound of the formula I can also be used to achieve a favourable modification of the metabolite spectrum of the starting compound in order to diminish or eliminate undesired toxic metabolites. For example, if a toxic metabolite arises through oxidative carbon-hydrogen (C-H) bond cleavage, it can reasonably be assumed that the deuterated analogue will greatly diminish or eliminate production of the unwanted metabolite, even if the particular oxidation is not a rate-determining step. Further information on the state of the art with respect to deuterium-hydrogen exchange may be found, for example in Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al, Biochemistry 33(10) 2927-2937, 1994, and Jarman et al. Carcinogenesis 16(4), 683-688, 1993.

The invention furthermore relates to medicaments comprising at least one compound of the formula I and/or pharmaceutically acceptable salts, solvates, tautomers and stereoisomers thereof, including mixtures thereof in all ratios, and optionally excipients and/or adjuvants.

Pharmaceutical formulations can be administered in the form of dosage units which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water and the like. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethyl-cellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbant, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of non-uniform shape, which are broken up to form granules. The granules can be lubricated by addition of stearic acid, a stearate salt, talc or mineral oil in order to prevent sticking to the tablet casting moulds. The lubricated mixture is then pressed to give tablets. The compounds according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a pre-specified amount of the compound. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The compounds of the formula I and salts, solvates, tautomers and stereoisomers thereof can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds of the formula I and the salts, solvates, tautomers and stereoisomers thereof can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropylmethacrylamidophenol, polyhydroxy-ethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis, as described in general terms in Pharmaceutical Research, 3(6), 318 (1986).

Pharmaceutical compounds adapted for topical administration can be formulated as ointments, creams, suspensions, lotions, powders, solutions, pastes, gels, sprays, aerosols or oils.

For the treatment of the eye or other external tissue, for example mouth and skin, the formulations are preferably applied as topical ointment or cream. In the case of formulation to give an ointment, the active ingredient can be employed either with a paraffinic or a water-miscible cream base. Alternatively, the active ingredient can be formulated to give a cream with an oil-in-water cream base or a water-in-oil base.

Pharmaceutical formulations adapted for topical application to the eye include eye drops, in which the active ingredient is dissolved or suspended in a suitable carrier, in particular an aqueous solvent.

Pharmaceutical formulations adapted for topical application in the mouth encompass lozenges, pastilles and mouthwashes.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil.

Pharmaceutical formulations adapted for administration by inhalation encompass finely particulate dusts or mists, which can be generated by various types of pressurised dispensers with aerosols, nebulisers or insufflators.

Pharmaceutical formulations adapted for vaginal administration can be administered as pessaries, tampons, creams, gels, pastes, foams or spray formulations.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary. Injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound of the formula I depends on a number of factors, including, for example, the age and weight of the animal, the precise condition that requires treatment, and its severity, the nature of the formulation and the method of administration, and is ultimately determined by the treating doctor or vet. However, an effective amount of a compound according to the invention is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as a single dose per day or usually in a series of part-doses (such as, for example, two, three, four, five or six) per day, so that the total daily dose is the same. An effective amount of a salt, solvate, tautomer and stereoisomer thereof can be determined as the fraction of the effective amount of the compound according to the invention *per se.* It can be assumed that similar doses are suitable for the treatment of other conditions mentioned above.

The disclosed compounds of the formula I can be administered in combination with other known therapeutic agents including agents for the treatment of RA (rheumatoid arthritis). As used here, the term "agents for the treatment of RA" relates to any agent which is administered to a patient with RA for the purposes of treating the RA.

The medicaments below are preferably, but not exclusively, combined with the compounds of the formula I:
1. NSAIDs (non-steroidal anti-inflammatory drugs) and analgesics
2. Glucocorticoids (low oral doses)
3. Conventional disease-modifying antirheumatic drugs (DMARDs)
- Methotrexate
- Leflunomide
- Sulfasalazine
- Hydroxycloroquine
- Azathioprine
- Ciclosporin
- Minocycline
- Gold

4. Biologic response modifiers (BRMs) -> target molecules/ immune cells involved in the inflammatory process, and include the following agents:
- TNF inhibitors
   - etanercept (Enbrel)
   - infliximab (Remicade)
   - adalimumab (Humira)
- B-cell-directed therapy
   - rituximab (Rituxan)
- T-cell/B-cell coactivation signal inhibitor
   - abatacept (Orencia)
- IL-1 receptor antagonist
   - anakinra (Kineret)

| | **MECHANISM OF ACTION** |
|---|---|
| Golimumab | Fully humanized monoclonal antibody to TNF |
| Certolizumab pegol | Anti -TNF agent with just the Fab portion attached to the polyethylene glycol |
| Tocilizumab | Humanized monoclonal anti-IL-6 antibody that binds to the soluble and membrane-expresses IL-6 receptor |
| Ocrelizumab | Humanized-second generation anti-CD20 antibody that depletes B cells |
| Ofatumumab | Human monoclonal anti-CD20 IgG1 antibody |
| Denosumab | Fully humanized monoclonal antibody that binds to and inhibits the receptor activator for nuclear factor-kB ligand |
| TRU-015 | New class of CD20-directed protein therapeutics |
| Oral small molecules (JAK, Syk, MAP kinase inhibitors) | Cytoplasmic targets |
| Tolerogens (dnaJP1) | Immunotherapy based on T-cell tolerization |

A combined treatment of this type can be achieved with the aid of simultaneous, consecutive or separate dispensing of the individual components of the treatment. Combination products of this type employ the compounds according to the invention.

The invention furthermore relates to medicaments comprising at least one compound of the formula I and/or pharmaceutically acceptable salts, solvates, tautomers and stereoisomers thereof, including mixtures thereof in all ratios, and at least one further medicament active ingredient.

The invention also relates to a set (kit) consisting of separate packs of
(a) an effective amount of a compound of the formula I and/or pharmaceutically acceptable salts, solvates, tautomers and stereoisomers thereof, including mixtures thereof in all ratios,
   and
(b) an effective amount of a further medicament active ingredient.

The set comprises suitable containers, such as boxes, individual bottles, bags or ampoules. The set may, for example, comprise separate ampoules, each containing an effective amount of a compound of the formula I and/or pharmaceutically acceptable salts, solvates, tautomers and stereoisomers thereof, including mixtures thereof in all ratios,
and an effective amount of a further medicament active ingredient in dissolved or lyophilised form.

"Treating" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The term "effective amount" in connection with a compound of formula (I) can mean an amount capable of alleviating, in whole or in part, symptoms associated with a disorder or disease, or slowing or halting further progression or worsening of those symptoms, or preventing or providing prophylaxis for the disease or disorder in a subject having or at risk for developing a disease disclosed herein, such as inflammatory conditions, immunological conditions, cancer, metabolic conditions, neurodegenerative conditions, chronic infections or conditions treatable or preventable by inhibition of a kinase or a kinase pathway, in one embodiment, the GCN2 pathway. In another embodiment this relates to conditions treatable or preventable by inhibition of a kinase or a kinase pathway, from the group of GCN2, FMS (CSF1 R), FLT3 or FLT4 or combinations thereof. In one embodiment an effective amount of a compound of formula (I) is an amount that inhibits a kinase in a cell, such as, for example, in vitro or in vivo. In some embodiments, the effective amount of the compound of formula (I) inhibits the kinase in a cell by 10%, 20%, 30%, 40%, 50%, 60%, 70%, 80%, 90% or 99%, compared to the activity of the kinase in an untreated cell. The effective amount of the compound of formula (I), for example in a pharmaceutical composition, may be at a level that will exercise the desired effect; for example, about 0.005 mg/kg of a subject's body weight to about 10 mg/kg of a subject's body weight in unit dosage for both oral and parenteral administration.

### USE

The present compounds are suitable as pharmaceutical active ingredients for mammals, especially for humans, in the treatment of immune modulatory and stress response kinase-induced diseases. These diseases include neoplastic malignancies including, but without being limited to, solid tumor cancers, cancers of the lymphatic or blood system, the proliferation of tumour cells, pathological neovascularisation (or angiogenesis) which promotes the growth of solid tumours, neurodegenerative diseases (Alzheimer, demyelinating core disorders multiple sclerosis and the like), immune related disorders like arthritis, psoriasis, lupus, or other autoimmune diseases as well as chronic infections.

The present invention encompasses the use of the compounds of the formula I and/or physiologically acceptable salts and solvates thereof for the preparation of a medicament for the treatment or prevention of cancer. Preferred carcinomas for the treatment originate from the group cerebral carcinoma, urogenital tract carcinoma, carcinoma of the lymphatic system, stomach carcinoma, laryngeal carcinoma and lung carcinoma. A further group of preferred forms of cancer are monocytic leukaemia, lung adenocarcinoma, small-cell lung carcinomas, pancreatic cancer, glioblastomas, melanomas and breast carcinoma. A further group of preferred forms of cancer include, but is not limited to, cervical cancer, neuroblastoma, testicular cancer, macroglobulinemia and sarcomas.

Also encompassed is the use of the compounds according to Claim 1 according to the invention and/or physiologically acceptable salts and solvates thereof for the preparation of a medicament for the treatment or prevention of a neurological disorder, particularly a neurodegenerative disease, for example a disease caused by axonal degeneration or by protein plaque deposition. Neurodegenerative diseases include, for example, demyelinating core disorders, such as multiple sclerosis, acute transverse myelitis, amyotrophic lateral sclerosis, Creutzfeldt-Jakob disease or Alzheimer disease.

Further encompassed is the use of the compounds according to Claim 1 according to the invention and/or physiologically acceptable salts and solvates thereof for the preparation of a medicament for the treatment of chronic infections. Such a chronic infection could relate to parasites like leishmania to leprosy or to viral infection by HIV and the like.

Further encompassed is the use of the compounds according to Claim 1 according to the invention and/or physiologically acceptable salts and solvates thereof for the preparation of a medicament for the treatment or prevention of a disease in which angiogenesis is implicated.

Such a disease in which angiogenesis is implicated is an ocular disease, such as retinal vascularisation, diabetic retinopathy, age-induced macular degeneration and the like.

The present invention encompasses the use of the compounds of the formula I and/or physiologically acceptable salts and solvates thereof for the preparation of a medicament for the treatment or prevention of immune related disorder like ankylosing spondylitis, arthritis, aplastic anemia, Behcet's disease, type 1 diabetes mellitus, graft-versus-host disease, Graves' disease, autoimmune hemolytic anemia, Wegener's granulomatosis, hyper IgE syndrome, idiopathic thrombocytopenia purpura, rheumatoid arthritis, Crohn's disease, multiple sclerosis, Myasthenia gravis, psoriasis, and lupus, among other autoimmune diseases. It might also be used treat organ rejection, bone marrow transplant rejection, non-myeloablative bone marrow transplant rejection,enhance bone marrow engraftment after non-myeloablative conditioning regimens, and combinations thereof

Also encompassed is the use of the compounds of the formula I and/or physiologically acceptable salts and solvates thereof for the preparation of a medicament for the treatment or prevention of a immune-modulatory or stress response kinase-induced disease or a immune-modulatory or stress response kinase-induced condition in a mammal, in which to this method a therapeutically effective amount of a compound according to the invention is administered to a sick mammal in need of such treatment. The therapeutic amount varies according to the specific disease and can be determined by the person skilled in the art without undue effort.

The present invention also encompasses the use compounds of the formula I and/or physiologically acceptable salts and solvates thereof for the preparation of a medicament for the treatment or prevention of retinal vascularisation. The expression "immune-modulatory or stress response kinase-induced diseases or conditions" refers to pathological conditions that depend on the activity of one or more immune-modulatory or stress response kinases. immune-modulatory or stress response kinases either directly or indirectly participate in the signal transduction pathways of a variety of cellular activities, including proliferation, adhesion and migration and differentiation. Diseases associated with immune-modulatory or stress response kinase activity include neoplastic malignancies (solid tumor cancers, cancers of the lymphatic or blood system and the like), of neurodegenerative diseases, immune related disorders like arthritis, psoriasis, lupus, multiple sclerosis or other autoimmune diseases as well as chronic infections.

The present invention specifically relates to compounds of the formula I and pharmaceutically acceptable salts, solvates, tautomers and stereoisomers thereof, including mixtures thereof in all ratios,
for the use for the treatment of diseases in which the inhibition, regulation and/or modulation inhibition of GCN2 plays a role.

The present invention specifically relates to compounds of the formula I and pharmaceutically acceptable salts, solvates, tautomers and stereoisomers thereof, including mixtures thereof in all ratios, for the use for the inhibition of GCN2.

The present invention specifically relates to compounds of the formula I and pharmaceutically acceptable salts, solvates, tautomers and stereoisomers thereof, including mixtures thereof in all ratios, for the use for the treatment of neoplastic malignancies (solid tumor cancers, cancers of the lymphatic or blood system and the like), of neurodegenerative diseases, immune related disorders like arthritis, psoriasis, lupus, multiple sclerosis or other autoimmune diseases as well as chronic infections.

Especial preference is given to the use for the treatment of a disease where the disease is a neoplastic malignancies.

The neoplastic malignancies is preferably selected from the group of tumours of the lung, squamous epithelium, the bladder, the stomach, the kidneys, of head and neck, the oesophagus, the cervix, the thyroid, the intestine, the liver, the brain, the prostate, the urogenital tract, the lymphatic system, the stomach and/or the larynx.

The neoplastic malignancies is furthermore preferably selected from the group lung adenocarcinoma, small-cell lung carcinomas, pancreatic cancer, glioblastomas, colon carcinoma and breast carcinoma.

Preference is furthermore given to the use for the treatment of a neoplastic malignancies of the blood and immune system, preferably for the treatment of a tumour selected from the group of acute myeloid leukaemia, chronic myeloid leukaemia, acute lymphatic leukaemia and/or chronic lymphatic leukaemia.

The present invention specifically relates to methods for treating or preventing an inflammatory condition, immunological condition, autoimmune condition, allergic condition, rheumatic condition, thrombotic condition, cancer, infection, neurodegenerative disease, neuroinflammatory disease, cardiovascular disease or metabolic condition, comprising administering to a subject in need thereof an effective amount of a compound of formula I or a pharmaceutically acceptable salt, tautomer, stereoisomer or solvate thereof.

In another aspect provided herein are methods of inhibiting a kinase in a cell expressing said kinase, comprising contacting said cell with an effective amount of a compound of formula I or a pharmaceutically acceptable salt, tautomer, stereoisomer or solvate thereof. In one embodiment the kinase is GCN2 or mutants or isoforms thereof, or combinations of two or more thereof.

Representative immunological conditions that compounds of formula I are useful for treating or preventing include, but are not limited to, Behcet's syndrome, non-allergy mast cell diseases (e.g., mastocytosis and treatment of anaphylaxis), ankylosing spondylitis, osteoarthritis, rheumatoid arthritis (RA), multiple sclerosis, lupus, inflammatory bowel disease, ulcerative colitis, Crohn's disease, myasthenia gravis, Grave's disease, transplant rejection, humoral transplant rejection, non-humoral transplant rejection, cellular transplant rejection, immune thrombocytopenic purpura (ITP), idiopathic thrombocytopenic purpura, diabetes, immunological response to bacterial, parasitic, helminth infestation or viral infection, eczema, dermatitis, graft versus host disease, Goodpasture's disease, hemolytic disease of the newborn, autoimmune hemolytic anemia, anti-phospholipid syndrome, ANCA-associated vasculitis, Churg-Strauss syndrome, Wegeners granulomatosus, pemphigus vulgaris, serum sickness, mixed cryoglobulinemia, peripheral neuropathy associated with IgM antibody, microscopic polyangiitis, Hashimoto's thyroiditis, Sjogrens syndrome, fibrosing conditions (such as those dependent on the innate or adaptive immune systems or local mesenchyma cells) or primary biliary cirrhosis.

Representative autoimmune conditions that compounds of formula I are useful for treating or preventing include, but are not limited to, autoimmune hemolytic anemia (A1HA), Behcet's syndrome, Crohn's disease, type I diabetes, Goodpasture's disease, Grave's disease, Hashimoto's thyroiditis, idiopathic thrombocytopenic purpura, lupus, multiple sclerosis, amyotrophic lateral sclerosis, myasthenia gravis, pemphigus vulgaris, primary biliary cirrhosis, rheumatoid arthritis, scleroderma, Sjogren's syndrome, ulcerative colitis, or Wegeners granulomatosus.

Representative allergic conditions that compounds of formula I are useful for treating or preventing include, but are not limited to, anaphylaxis, hay fever, allergic conjunctivitis, allergic rhinitis, allergic asthma, atopic dermatitis, eczema, urticaria, mucosal disorders, tissue disorders and certain gastrointestinal disorders.

Representative rheumatic conditions that compounds of formula I are useful for treating or preventing include, but are not limited to, rheumatoid arthritis, gout, ankylosing spondylitis, or osteoarthritis.

Representative inflammatory conditions that compounds of formula I are useful for treating or preventing include, but are not limited to, non-ANCA (anti-neutrophil cytoplasmic autoantibody) vasculitis (e.g., wherein GCN2 function is associated with neutrophil adhesion, diapedesis and/or activation), psoriasis, asthma, allergic rhinitis, allergic conjunctivitis, chronic urticaria, hives, anaphylaxis, bronchitis, chronic obstructive pulmonary disease, cystic fibrosis, inflammatory bowel disease, irritable bowel syndrome, gout, Crohn's disease, mucous colitis, ulcerative colitis, allergy to intestinal antigens (such as gluten enteropathy), diabetes (e.g., Type I diabetes and Type II diabetes) and obesity. In some embodiments, the inflammatory condition is a dermatologic condition, such as, for example, psoriasis, urticaria, hives, eczema, scleroderma, or dermatitis. In other embodiments, the inflammatory condition is an inflammatory pulmonary condition, such as, for example, asthma, bronchitis, chronic obstructive pulmonary disease (COPD), or adult/acute respiratory distress syndrome (ARDS). In other embodiments, the inflammatory condition is a gastrointestinal condition, such as, for example, inflammatory bowel disease, ulcerative colitis, Crohn's disease, idiopathic inflammatory bowel disease, irritable bowel syndrome, or spastic colon.

Representative infections that compounds of formula I are useful for treating or preventing include, but are not limited to, bacterial, parasitic, prion, viral infections or helminth infestation.

Representative cancers that compounds of formula I are useful for treating or preventing include, but are not limited to, cancer of the head, neck, eye, mouth, throat, esophagus, bronchus, larynx, pharynx, chest, bone, lung, colon, rectum, stomach, prostate, urinary bladder, uterine, cervix, breast, ovaries, testicles or other reproductive organs, skin, thyroid, blood, lymph nodes, kidney, liver, pancreas, brain, central nervous system, solid tumors and blood-borne tumors.

Representative cardiovascular diseases that compounds of formula I are useful for treating or preventing include, but are not limited to, restenosis, atherosclerosis and its consequences such as stroke, myocardial infarction, ischemic damage to the heart, lung, gut, kidney, liver, pancreas, spleen or brain.

Representative metabolic conditions that compounds of formula I are useful for treating or preventing include, but are not limited to, obesity and diabetes (e.g. , Type I and II diabetes). In a particular embodiment, provided herein are methods for the treatment or prevention of insulin resistance. In certain embodiments, provided herein are methods for the treatment or prevention of insulin resistance that leads to diabetes (e.g., Type II diabetes). In another embodiment, provided herein are methods for the treatment or prevention of syndrome X or metabolic syndrome. In another embodiment, provided herein are methods for the treatment or prevention of Type II diabetes, Type I diabetes, slow-onset Type I diabetes, diabetes insipidus (e.g., neurogenic diabetes insipidus, nephrogenic diabetes insipidus, dipsogenic diabetes insipidus, or gestagenic diabetes insipidus), diabetes mellitus, gestational diabetes mellitus, polycystic ovarian syndrome, maturity-onset diabetes, juvenile diabetes, insulin-dependant diabetes, non-insulin dependant diabetes, malnutrition-related diabetes, ketosis-prone diabetes, pre-diabetes (e.g. , impaired glucose metabolism), cystic fibrosis related diabetes, hemochromatosis and ketosis-resistant diabetes.

Representative neurodegenerative and neuroinflammatory diseases that compounds of formula I are useful for treating or preventing include, but are not limited to, Huntington's disease, Alzheimer's disease, viral (e.g., HIV) or bacterial-associated encephalitis and damage.

In another embodiment, provided herein are methods for the treatment or prevention of fibrotic diseases and disorders. In a particular embodiment, provided herein are methods for the treatment or prevention of idiopathic pulmonary fibrosis, myelofibrosis, hepatic fibrosis, steatofibrosis and steatohepatitis.

In another embodiment, provided herein are methods for the treatment or prevention of diseases associated with thrombotic events such as but not limited to atherosclerosis, myocardial infarction and ischemic stroke.

The present invention specifically relates to compounds of the formula I and pharmaceutically acceptable salts, solvates, tautomers and stereoisomers thereof, including mixtures thereof in all ratios, for the use for the treatment and/or prevention of inflammatory conditions, immunological conditions, autoimmune conditions, allergic conditions, rheumatic conditions, thrombotic conditions, cancer, infections, neurodegenerative diseases, neuroinflammatory diseases, cardiovascular diseases, and metabolic conditions, the methods comprising administering to a subject in need thereof an effective amount of a compound of claim 1.

Moreover, the present invention specifically relates to compounds for the use for the treatment and/or prevention of cancer,
where the cancer to be treated is a solid tumour or a tumour of the blood and immune system.

Moreover, the present invention specifically relates to compounds, for the use for the treatment and/or prevention of cancer, where the where the tumour originates from the group of acute myeloid leukaemia, chronic myeloid leukaemia, acute lymphatic leukaemia and/or chronic lymphatic leukaemia.

Moreover, the present invention specifically relates to compounds, for the use for the treatment and/or prevention of cancer, where the solid tumour originates from the group of tumours of the epithelium, the bladder, the stomach, the kidneys, of head and neck, the esophagus, the cervix, the thyroid, the intestine, the liver, the brain, the prostate, the uro-genital tract, the lymphatic system, the stomach, the larynx, the bones, including chondosarcoma and Ewing sarcoma, germ cells, including embryonal tissue tumours, and/or the lung, from the group of monocytic leukaemia, lung adenocarcinoma, small-cell lung carcinomas, pancreatic cancer, glioblastomas, neurofibroma, angiosarcoma, breast carcinoma and /or maligna melanoma.

Moreover, the present invention specifically relates to for the use for the treatment and/or prevention of diseases selected from the group rheumatoid arthritis, systemic lupus, asthma, multiple sclerosis, osteoarthritis, ischemic injury, giant cell arteritis, inflammatory bowel disease, diabetes, cystic fibrosis, psoriasis, Sjögrens syndrom and transplant organ rejection.

Moreover, the present invention specifically relates to compounds for the use for the treatment and/or prevention of diseases selected from the group Alzheimer's disease, Down's syndrome, hereditary cerebral hemorrhage with amyloidosis-Dutch Type, cerebral amyloid angiopathy, Creutzfeldt-Jakob disease, frontotemporal dementias, Huntington's disease, Parkinson's disease.

Moreover, the present invention specifically relates to compounds for the use for the treatment and/or prevention of diseases selected from the group leishmania, mycobacteria, including M. leprae, M. tuberculosis and/or M. avium, leishmania, plasmodium, human immunodeficiency virus, Epstein Barr virus, Herpes simplex virus, hepatitis C virus.

The disclosed compounds of the formula I can be administered in combination with other known therapeutic agents, including anticancer agents. As used here, the term "anticancer agent" relates to any agent which is administered to a patient with cancer for the purposes of treating the cancer.

The anti-cancer treatment defined herein may be applied as a sole therapy or may involve, in addition to the compound of the invention, conventional surgery or radiotherapy or chemotherapy. Such chemotherapy may include one or more of the following categories of anti- tumour agents:
(i) antiproliferative/antineoplastic/DNA-damaging agents and combinations thereof, as used in medical oncology, such as alkylating agents (for example cis-platin, carboplatin, cyclophosphamide, nitrogen mustard, melphalan, chloroambucil, busulphan and nitrosoureas); antimetabolites (for example antifolates such as fluoropyrimidines like 5-fluorouracil and tegafur, raltitrexed, methotrexate, cytosine arabinoside, hydroxyurea and gemcitabine); antitumour antibiotics (for example anthracyclines, like adriamycin, bleomycin, doxorubicin, daunomycin, epirubicin, idarubicin, mitomycin-C, dactinomycin and mithramycin) ; antimitotic agents (for example vinca alkaloids, like vincristine, vinblastine, vindesine and vinorelbine, and taxoids, like taxol and taxotere) ; topoisomerase inhibitors (for example epipodophyllotoxins, like etoposide and teniposide, amsacrine, topotecan, irinotecan and camptothecin) and cell-differentiating agents (for example all-trans-retinoic acid, 13-cis-retinoic acid and fenretinide);
(ii) cytostatic agents, such as antioestrogens (for example tamoxifen, toremifene, raloxifene, droloxifene and iodoxyfene), oestrogen receptor downregulators (for example fulvestrant), antiandrogens (for example bicalutamide, flutamide, nilutamide and cyproterone acetate), LHRH antagonists or LHRH agonists (for example goserelin, leuprorelin and buserelin), progesterones (for example megestrol acetate), aromatase inhibitors (for example as anastrozole, letrozole, vorazole and exemestane) and inhibitors of 5α-reductase, such as finasteride;
(iii) agents which inhibit cancer cell invasion (for example metalloproteinase inhibitors, like marimastat, and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbbl antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors, such as N-(3-chloro-4-fluorophenyl)-7-methoxy-6- (3-morpholinopropoxy) quinazolin-4-amine (gefitinib, AZD1839), N-(3-ethynylphenyl)-6,7-bis (2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (Cl 1033)), for example inhibitors of the platelet-derived growth factor family and for example inhibitors of the hepatocyte growth factor family;
(v)antiangiogenic agents, such as those which inhibit the effects of vascular endothelial growth factor, (for example the anti-vascular endothelial cell growth factor antibody bevacizumab [Avastin™], compounds such as those disclosed in published international patent applications WO 97/22596, WO 97/30035, WO 97/32856 and WO 98/13354) and compounds that work by other mechanisms (for example linomide, inhibitors of integrin αvβ3 function and angiostatin);
(vi) vessel-damaging agents, such as combretastatin A4 and compounds disclosed in international patent applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-Ras antisense;
(viii) gene therapy approaches, including, for example, approaches for replacement of aberrant genes, such as aberrant p53 or aberrant BRCA1 or BRCA2, GDEPT (gene-directed enzyme pro-drug therapy) approaches, such as those using cytosine deaminase, thymidine kinase or a bacterial nitroreductase enzyme, and approaches for increasing patient tolerance to chemotherapy or radiotherapy, such as multi-drug resistance gene therapy; and
(ix) immunotherapy approaches, including, for example, ex-vivo and in-vivo approaches for increasing the immunogenicity of patient tumour cells, such as transfection with cytokines, such as interleukin 2, interleukin 4 or granulocyte-macrophage colony stimulating factor, approaches for decreasing T-cell anergy, approaches using transfected immune cells, such as cytokine-transfected dendritic cells, approaches using cytokine-transfected tumour cell lines, and approaches using anti-idiotypic antibodies.

The medicaments from Table 1 below are preferably, but not exclusively, combined with the compounds of the formula 1.

| Table 1. | | |
|---|---|---|
| Alkylating agents | Cyclophosphamide | Lomustine |
| | Busulfan | Procarbazine |
| | Ifosfamide | Altretamine |
| | Melphalan | Estramustine phosphate |
| | Hexamethylmelamine | Mechloroethamine |
| | Thiotepa | Streptozocin |
| | chloroambucil | Temozolomide |
| | Dacarbazine | Semustine |
| | Carmustine | |
| | | |
| Platinum agents | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolites | Azacytidine | Tomudex |
| | Gemcitabine | Trimetrexate |
| | Capecitabine | Deoxycoformycin |
| | 5-fluorouracil | Fludarabine |
| | Floxuridine | Pentostatin |
| | 2-chlorodesoxyadenosine | Raltitrexed |
| | 6-Mercaptopurine | Hydroxyurea |
| | 6-Thioguanine | Decitabine (SuperGen) |
| | Cytarabine | Clofarabine (Bioenvision) |
| | 2-fluorodesoxycytidine | Irofulven (MGI Pharrna) |
| | Methotrexate | DMDC (Hoffmann-La Roche) |
| | Idatrexate | |
| | | Ethynylcytidine (Taiho) |
| | | |
| Topoisomerase inhibitors | Amsacrine | Rubitecan (SuperGen) |
| | Epirubicin | Exatecan mesylate (Daiichi) |
| | Etoposide | |
| | Teniposide or mitoxantrone | Quinamed (ChemGenex) |
| | | Gimatecan (Sigma- Tau) |
| | Irinotecan (CPT-11) | Diflomotecan (Beaufour-Ipsen) |
| | 7-ethyl-10-hydroxycamptothecin | |
| | | TAS-103 (Taiho) |
| | Topotecan | Elsamitrucin (Spectrum) |
| | Dexrazoxanet (TopoTarget) | J-107088 (Merck & Co) |
| | | BNP-1350 (BioNumerik) |
| | Pixantrone (Novuspharrna) | CKD-602 (Chong Kun Dang) |
| | Rebeccamycin analogue (Exelixis) | |
| | | KW-2170 (Kyowa Hakko) |
| | BBR-3576 (Novuspharrna) | |
| | | |
| Antitumour antibiotics | Dactinomycin (Actinomycin D) | Amonafide |
| | | Azonafide |
| | Doxorubicin (Adriamycin) | Anthrapyrazole |
| | Deoxyrubicin | Oxantrazole |
| | Valrubicin | Losoxantrone |
| | Daunorubicin (Daunomycin) | Bleomycin sulfate (Blenoxan) |
| | Epirubicin | Bleomycinic acid |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxo- | |
| | rubicin | |
| | Mitoxantron (Novantron) | |
| | | |
| Antimitotic agents | Paclitaxel | SB 408075 (GlaxoSmith Kline) |
| | Docetaxel | |
| | Colchicine | E7010 (Abbott) |
| | Vinblastine | PG-TXL (Cell Therapeutics) |
| | Vincristine | |
| | Vinorelbine | IDN 5109 (Bayer) |
| | Vindesine | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilone B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunine (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-prodrug (OXiGENE) |
| | BMS 188797 (BMS) | Dolastatin-10 (NrH) |
| | Taxoprexin (Protarga) | CA-4 (OXiGENE) |
| | | |
| Aromatase inhibitors | Aminoglutethimide | Exemestan |
| | Letrozole | Atamestan (BioMedicines) |
| | Anastrazole | YM-511 (Yamanouchi) |
| | Formestan | |
| | | |
| Thymidylate synthase inhibitors | Pemetrexed (Eli Lilly) | Nolatrexed (Eximias) |
| | ZD-9331 (BTG) | CoFactor™ (BioKeys) |
| | | |
| DNA antagonists | Trabectedin (PharmaMar) | Mafosfamide (Baxter International) |
| | Glufosfamide (Baxter International) | |
| | | Apaziquone (Spectrum Pharmaceuticals) |
| | Albumin + 32P (Isotope Solutions) | |
| | | O6-benzylguanine (Paligent) |
| | Thymectacin (NewBiotics) | |
| | Edotreotid (Novartis) | |
| Farnesyl transferase inhibitors | Arglabin (NuOncology Labs) | Tipifarnib (Johnson & Johnson) |
| | lonafarnib (Schering-Plough) | Perillyl alcohol (DOR BioPharma) |
| | BAY-43-9006 (Bayer) | |
| | | |
| Pump inhibitors | CBT-1 (CBA Pharma) | Zosuquidar |
| | Tariquidar (Xenova) | trihydrochloride (Eli Lilly) |
| | MS-209 (Schering AG) | Biricodar dicitrate (Vertex) |
| | | |
| Histone acetyl transferase inhibitors | Tacedinaline (Pfizer) | Pivaloyloxymethyl butyrate (Titan) |
| | SAHA (Aton Pharma) | |
| | MS-275 (Schering AG) | Depsipeptide (Fujisawa) |
| | | |
| Metalloproteinase inhibitors Ribonucleoside reductase inhibitors | Neovastat (Aeterna Laboratories) | CMT -3 (CollaGenex) |
| | | BMS-275291 (Celltech) |
| | Marimastat (British Biotech) | Tezacitabine (Aventis) |
| | | Didox (Molecules for Health) |
| | Gallium maltolate (Titan) | |
| | Triapin (Vion) | |
| | | |
| TNF-alpha agonists/ antagonists | Virulizin (Lorus Therapeutics) | Revimid (Celgene) |
| | CDC-394 (Celgene) | |
| | | |
| Endothelin-A receptor antagonists | Atrasentan (Abbot) | YM-598 (Yamanouchi) |
| | ZD-4054 (AstraZeneca) | |
| | | |
| Retinoic acid receptor agonists | Fenretinide (Johnson & Johnson) | Alitretinoin (Ligand) |
| | LGD-1550 (Ligand) | |
| | | |
| Immunomodulators | Interferon | Dexosome therapy (Anosys) |
| | Oncophage (Antigenics) | |
| | GMK (Progenics) | Pentrix (Australian Cancer Technology) |
| | Adenocarcinoma vaccine (Biomira) | |
| | | JSF-154 (Tragen) |
| | CTP-37 (AVI BioPharma) | Cancer vaccine (Intercell) |
| | JRX-2 (Immuno-Rx) | Norelin (Biostar) |
| | PEP-005 (Peplin Biotech) | BLP-25 (Biomira) |
| | Synchrovax vaccines (CTL Immuno) | MGV (Progenics) |
| | | !3-Alethin (Dovetail) |
| | Melanoma vaccine (CTL Immuno) | CLL-Thera (Vasogen) |
| | p21-RAS vaccine (Gem-Vax) | |
| | | |
| Hormonal and antihormonal agents | Oestrogens | Prednisone |
| | Conjugated oestrogens | Methylprednisolone |
| | Ethynyloestradiol | Prednisolone |
| | chlorotrianisene | Aminoglutethimide |
| | Idenestrol | Leuprolide |
| | Hydroxyprogesterone | Goserelin |
| | caproate | Leuporelin |
| | Medroxyprogesterone | Bicalutamide |
| | Testosterone | Flutamide |
| | Testosterone propionate | Octreotide |
| | Fluoxymesterone | Nilutamide |
| | Methyltestosterone | Mitotan |
| | Diethylstilbestrol | P-04 (Novogen) |
| | Megestrol | 2-Methoxyoestradiol (EntreMed) |
| | Tamoxifen | |
| | Toremofin | Arzoxifen (Eli Lilly) |
| | Dexamethasone | |
| | | |
| Photodynamic agents | Talaporfin (Light Sciences) | Pd-Bacteriopheophorbid (Yeda) |
| | Theralux (Theratechnologies) | |
| | | Lutetium-Texaphyrin (Pharmacyclics) |
| | Motexafin-Gadolinium (Pharmacyclics) | |
| | | Hypericin |
| | | |
| Tyrosine kinase inhibitors | Imatinib (Novartis) | Kahalide F (PharmaMar) |
| | Leflunomide(Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science) | PKC412 (Novartis) |
| | | Phenoxodiol O |
| | Canertjnib (Pfizer) | Trastuzumab (Genentech) |
| | Squalamine (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmith-Kline) | |
| | EKB-509 (Wyeth) | |
| | EKB-569 (Wyeth) | |
| Various agents | SR-27897 (CCK-A inhibitor, Sanofi-Synthelabo) | BCX-1777 (PNP inhibitor, BioCryst) |
| | Tocladesine (cyclic AMP agonist, Ribapharm) | Ranpirnase (ribonuclease stimulant, Alfacell) |
| | Alvocidib (CDK inhibitor, Aventis) | Galarubicin (RNA synthesis inhibitor, Dong-A) |
| | CV-247 (COX-2 inhibitor, Ivy Medical) | Tirapazamine (reducing agent, SRI International) |
| | P54 (COX-2 inhibitor, Phytopharm) | N-Acetylcysteine (reducing agent, Zambon) |
| | CapCell™ (CYP450 stimulant, Bavarian Nordic) | R-Flurbiprofen (NF-kappaB inhibitor, Encore) |
| | GCS-IOO (gal3 antagonist, GlycoGenesys) | 3CPA (NF-kappaB inhibitor, Active Biotech) |
| | G17DT immunogen (gastrin inhibitor, Aphton) | Seocalcitol (vitamin D receptor agonist, Leo) |
| | Efaproxiral (oxygenator, Allos Therapeutics) | 131-I-TM-601 (DNA antagonist, TransMolecular) |
| | PI-88 (heparanase inhibitor, Progen) | |
| | | Eflornithin (ODC inhibitor, ILEX Oncology) |
| | Tesmilifen (histamine antagonist, YM BioSciences) | |
| | | Minodronic acid (osteoclast inhibitor, Yamanouchi) |
| | Histamine (histamine H2 receptor agonist, Maxim) | |
| | Tiazofurin (IMPDH inhibitor, Ribapharm) | Indisulam (p53 stimulant, Eisai) |
| | Cilengitide (integrin antagonist, Merck KGaA) | Aplidin (PPT inhibitor, PharmaMar) |
| | SR-31747 (IL-1 antagonist, Sanofi-Synthelabo) | Rituximab (CD20 antibody, Genentech) |
| | CCI-779 (mTOR kinase inhibitor, Wyeth) | Gemtuzumab (CD33 antibody, Wyeth Ayerst) |
| | Exisulind (PDE-V inhibitor, Cell Pathways) | PG2 (haematopoiesis promoter, Pharmagenesis) |
| | CP-461 (PDE-V inhibitor, Cell Pathways) | Immunol™ (triclosan mouthwash, Endo) |
| | AG-2037 (GART inhibitor, Pfizer) | Triacetyluridine (uridine prodrug, Wellstat) |
| | WX-UK1 (plasminogen activator inhibitor, Wilex) | SN-4071 (sarcoma agent, Signature BioScience) |
| | PBI-1402 (PMN stimulant, ProMetic LifeSciences) | TransMID-107™ (immunotoxin, KS Biomedix) |
| | Bortezomib (proteasome inhibitor, Millennium) | |
| | | PCK-3145 (apoptosis promoter, Procyon) |
| | SRL-172 (T-cell stimulant, SR Pharma) | |
| | | Doranidazole (apoptosis promoter, Pola) |
| | TLK-286 (glutathione-S transferase inhibitor, Telik) | |
| | | CHS-828 (cytotoxic agent, Leo) |
| | PT-100 (growth factor agonist, Point Therapeutics) | Trans-retinic acid (differentiator, NIH) |
| | Midostaurin (PKC inhibitor, | MX6 (apoptosis promoter, |
| | Novartis) | MAXIA) |
| | Bryostatin-1 (PKC stimulant, GPC Biotech) | Apomine (apoptosis promoter, ILEX Oncology) |
| | CDA-II (apoptosis promoter, Everlife) | Urocidin (apoptosis promoter, Bioniche) |
| | SDX-101 (apoptosis promoter, Salmedix) | Ro-31-7453 (apoptosis promoter, La Roche) |
| | Ceflatonin (apoptosis promoter, ChemGenex) | Brostallicin (apoptosis promoter, Pharmacia) |

The disclosed compounds of the formula I and pharmaceutically acceptable solvates, salts, tautomers and stereoisomers thereof, including mixtures thereof in all ratios, preferably can be administered in combination with immunmodulators, preferably with anti-PDL-1- or IL-12.

Moreover, the present invention relates to compounds of the formula I and pharmaceutically acceptable salts, solvates, tautomers and stereoisomers thereof, including mixtures thereof in all ratios, for the use for the inhibition of Syk.

The present invention specifically relates to compounds of the formula I and pharmaceutically acceptable salts, solvates, tautomers and stereoisomers thereof, including mixtures thereof in all ratios, for the use for the treatment of rheumatoid arthritis, systemic lupus, asthma, allergic rhinitis, ITP, multiple sclerosis, leukemia, breast cancer, maligna melanoma.

The present invention specifically relates to methods for treating or preventing an inflammatory condition, immunological condition, autoimmune condition, allergic condition, rheumatic condition, thrombotic condition, cancer, infection, neurodegenerative disease, neuroinflammatory disease, cardiovascular disease or metabolic condition, comprising administering to a subject in need thereof an effective amount of a compound of formula I or a pharmaceutically acceptable salt, tautomer, stereoisomer or solvate thereof.

The following abbreviations refer respectively to the definitions below:
aq (aqueous), h (hour), g (gram), L (liter), mg (milligram), MHz (Megahertz), min. (minute), mm (millimeter), mmol (millimole), mM (millimolar), m.p. (melting point), eq (equivalent), mL (milliliter), L (microliter), ACN (acetonitrile), AcOH (acetic acid), CDCl₃ (deuterated chloroform), CD₃OD (deuterated methanol), CH₃CN (acetonitrile), c-hex (cyclohexane), DCC (dicyclohexyl carbodiimide), DCM (dichloromethane), DIC (diisopropyl carbodiimide), DIEA (diisopropylethyl-amine), DMF (dimethylformamide), DMSO (dimethylsulfoxide), DMSO-d₆ (deuterated dimethylsulfoxide), EDC (1-(3-dimethyl-amino-propyl)-3-ethylcarbodiimide), ESI (Electro-spray ionization), EtOAc (ethyl acetate), Et₂O (diethyl ether), EtOH (ethanol), HATU (dimethylamino-([1,2,3]triazolo[4,5-b]pyridin-3-yloxy)-methylene]-dimethyl-ammonium hexafluorophosphate), HPLC (High Performance Liquid Chromatography), i-PrOH (2-propanol), K₂CO₃ (potassium carbonate), LC (Liquid Chromatography), MeOH (methanol), MgSO₄ (magnesium sulfate), MS (mass spectrometry), MTBE (Methyl tert-butyl ether), NaHCO₃ (sodium bicarbonate), NaBH₄ (sodium borohydride), NMM (N-methyl morpholine), NMR (Nuclear Magnetic Resonance), PyBOP (benzotriazole-1-yl-oxy-tris-pyrrolidino-phosphonium hexafluorophosphate), RT (room temperature), Rt (retention time), SPE (solid phase extraction), TBTU (2-(1-H-benzotriazole-1-yl)-1,1,3,3-tetramethyluromium tetrafluoro borate), TEA (triethylamine), TFA (trifluoroacetic acid), THF (tetrahydrofuran), TLC (Thin Layer Chromatography), UV (Ultraviolet).

### Description of the in vitro assays

### GCN2: Assay principle & conditions

This assay can quantificate the activity of the serin kinase GCN2 (general control non-derepressible-2).

This kinase is involved in the stress metabolism of cells. It is activated upon starvation (amino acid depletion). Its natural substrate is eIF2a (eukaryotic initiation factor 2 alpha subunit), a translation factor, which gets activated (phosphorylated) by GCN2 in case of an amino acid bottleneck in the cells. This in turn leads to a halt of the protein synthesis. Inhibition of GCN2 results in stopping this mechanism: The cell can not stop protein production upon "starvation" stress.

The assay is run in two steps: the enzymatic reaction and the detection step. In the first step GCN2 is incubated with 10 µM ATP and 80 nM of the GFP-labelled substrate eIF2alpha at room temperature.

The enzymatic reaction is stopped by addition of EDTA. The amount of phosphorylated eIF2alpha is determined by TR-FRET (Lanthascreen): A complex is formed consisting of antibody and GFP labelled phospho-eIF2a, which allows a FRET upon exitation at 340 nm.

The GCN2-activity is directly proportional to the ratio of fluorescence units at the emission wavelenghth 520 nm (phosphopeptide-sensitive wavelength = emission of GFP) to the units at 495 nm (reference wavelength = emission of Terbium-chelate).

**Final concentrations in the enzymatic reaction**

| | |
|---|---|
| Hepes, pH 7.0 | 50 mM |
| MgCl₂ | 10 mM |
| MnCl₂ | 5 mM |
| BSA | 0.1% |
| DMSO | 1% |
| ATP | 10 uM |
| DTT | 2 mM |
| GFP-eIF2a | 80 nM (substrate) |
| *GCN2* | *30 nM (enzyme)* |

**Assay procedure**

| | |
|---|---|
| 4 uL | **enzyme** solution (in assay buffer) |
| 1.5 uL | **compound** (in cmpd dilution buffer/6.3% DMSO) |
| Incubation | 20 min at RT |
| 4 uL | **substrate**/ATP mix (in assay buffer) |
| Incubation | 90 min at RT |
| 10 uL | stop/**detection** mix (in antibody dilution buffer) |
| Incubation | 60 min at RT |
| Readout | Lanthascreen 340/495/520 |

### Cellular assay for the determination of compound activities

Human U2OS cells (2000 cells/well) are seeded into 384-well plates and incubated for 20 hours.

The next day, the cells are treated with the test compounds and incubated for 2 hours. Then, tryptophanol, at a final concentration of 600 µM, is added to the cells and those are incubated for 30 minutes.

The analysis of cellular GCN2 activities is done by immunocytochemistry. Briefly, cells are fixated on the well surfaces by formaldehyde and permeabilised with Triton X-100. The primary antibody (anti-phospho-eIF2alpha (Ser51, Cell Signalling Technology, #3398) is incubated on the treated cells for 20 hours, followed by a 60 minutes incubation of the secondary antibody (anti-rabbit-IgG-Alexa 488; Molecular Probes # 11008). The analysis and quantification of phosphorylated GCN2 is done by scanning the plates in the Acumen Explorer system (TTPLabtech). The obtained data are normalised against the untreated control wells (DMSO only) and expressed as % effect values. The determination of IC₅₀ values is done by using the Graph Pad Prism software.

### SYK flash plate assay

The kinase assay is performed either as 384-well Flashplate assay (for e.g. Topcount measurement) or as 384-well Image-Flashplate assay (for LEADseeker measurement).

2.5 nM SYK, 400 nM Biotin-Aha-Aha-KEDPDYEWPSAKK and 10 µM ATP (spiked with 0.3 µCi 33P-ATP/well) are incubated in a total volume of 50 µl (60 mM Hepes, 10 mM MgCl₂, 1.2 mM Dithiothreitol, 0.02 % Brij35, 0.1 % BSA , pH 7.5) with or without test compound for 1 hours at 30°C. The reaction is stopped with 25µl 200 mM EDTA. After 30 Min at 30 °C the liquid is removed and each well washed thrice with 100 µl 0.9% sodium chloride solution. Non-specific reaction is determined in presence of 0.1 µM Staurosporine Radioactivity is measured with Topcount (when using Flashplates) or with LEADseeker (when using Image-Flashplates) respectively. Results (e.g. IC50-values) are calculated with program tools provided by the IT-department (e.g. Symyx Assay Explorer, Genedata Screener).

### Enzymatic assays using the Caliper LifeSciences Technology

The assays described here are performed on the Caliper Life Sciences LC3000 system This technology provides data on enzyme activity via measurement of the relative amounts of phosphorylated or unphosphorylated fluorescently labelled substrate peptide at the end of an enzymatic reaction. These different states of peptide are resolved by applying a potential difference across the sample. The presence of the charged phosphate group on the product (as opposed to the substrate) causes a different peptide mobility between the two peptides. This is visualized by excitation of the fluorescent label on the substrate and product peptides and represented as peaks within the analysis software.

In order to measure inhibitor activity of kinase inhibitors on this technology, a TTP Mosquito liquid handling instrument is used to place 0.25 ul of the appropriate concentration of inhibitor in 100% DMSO (for a dose response curve calculation) into each well of a 384-well plate. To this reaction components are added to a final volume of 25 ul. The table below indicates the sequences and concentrations for the assays described in this report. Standard components are 1 mM DTT (Sigma, D0632), 1 mM MgCl2 (Sigma, M1028), 100 mM HEPES pH 7.5 (Calbiochem, 391338), 0.015% Brij-35 (Sigma, B4184).

| Enzyme | Enzyme Concentratio n (ng/ul) | ATP Concentration (uM) | Peptide Sequence (@ 1 uM) |
|---|---|---|---|
| Syk (BPS Bioscience, CA, USA) | 0.06 | 5 | FITC-AHA-KEDPDYEWPSAKKK-NH2 |

The reaction is incubated for 90 min at 25 C, and then stopped by the addition of 70 ul of Stop buffer (100 mM HEPES pH 7.5, 0.015% Brij-35, 10 mM EDTA (Sigma, E7889)).

The plate is read on a Caliper LC 3000 in an Off-Chip mobility shift assay format, on a 12-sipper chip. Unphosphorylated substrate and phosphorylated product peptide resolve as separate peaks allowing direct measurement of percentage of conversion of substrate to product. The percent conversion can be plotted against concentration of inhibitor to produce a sigmoidal dose response curve, from which an IC50 can be calculated using GeneData Condoseo or a similar product.

### Cellular activity assays

### 1. BCR crosslinking-induced BLNK phosphorylation

Ramos cells incubated overnight in IMDM medium containing 5% FCS were resuspended in IMDM medium without serum (3.3 x10⁶ cells/ml). 90 µl of cell suspension (300'000 cells) were incubated with 10 µl of SYK inhibitors (in 3 % DMSO) for 20 minutes at 37°C, in 96 well plates. After preincubation with inhibitors, cells were activated with 10 µg / ml of goat antihuman anti-IgM for 10 minutes at 37°C. After stimulation, cells were fixed by addition of 80 µl of 4% paraformaldehyde followed by a 10 minutes incubation at RT and fixed in 0.1 % Triton X-100 in PBS. BLNK phosphorylation was detected by flow cytometry after staining of the cells with anti-BLNK-pY84-PE antibodies from BD pharmingen, for 45 minutes at RT.

BLNK phosphorylation in CD19⁺ peripheral blood mononuclear cells (PBMC) isolated from buffy coats of healthy volunteers was performed using the same protocol and staining the cells with a mixture of anti-BLNK-pY84-PE, anti CD-19 PerCp and Anti-IgM APC antibodies from BD Pharmingen.

### 2. BCR crosslinking-induced CD69 up-regulation

To quantify anti-IgM-induced CD69 up-regulation in peripheral blood mononuclear cells, 90 µl of PBMC cell suspension (containing 1 x 10⁶ cells) were preincubated with 10 µl of SYK inhibitors (in 3% DMSO) for 1 h at 37°C/5%CO₂. After preincubation with inhibitors cells were stimulated with 10 µg / ml of goat antihuman anti-IgM during 18 hours at 37°C/5%CO₂. After stimulation cells were stained with a cocktail containing goat IgG (1:200 dilution), CD19-PerCpCy5.5 (5 µl) and CD69-APC (3 µl) antibodies in PBS containing 4 % FCS. CD69 expression in CD19⁺ cells was quantified by flow cytometry.

### In vivo Assays

### CIA

For induction of collagen-induced arthritis (CIA) male DBA/1 mice are injected with 500 µl pristane i.p. on day -21. On day 0 mice are immunized with 100 µg chicken collagen type II (CII) in Complete Freund's Adjuvant (CFA) intradermally, distributed over pinnae and one site on the back on day 0. On day 21, mice will receive an i.p. booster immunization (100 µg) with soluble CII in PBS. Dosing of Syk inhibitor will be prophylactic: starting day 0 and continued until day 10 and before boost starting on day 20 and continued until day 30. Compounds will be administered orally twice a day at doses of 3, 10 and 30 mg/kg.

Body weight and clinical score will be recorded on a daily basis. Arthritis severity is graded using a clinical scoring system based on the assessment of inflammation in individual paws. The scale for this clinical score ranges from 0-4 for each individual paw.

### GIA

For induction of Glucose-6-phosphate isomerase-induced arthritis (GIA) female DBA/1 mice are immunized with 100 µg G6PI in Complete Freund's Adjuvant (CFA) intradermally, distributed over pinnae and one site on the back on day 0. Dosing of Syk inhibitor will be prophylactic starting day 0 and continued until day 14. Compounds will be administered orally twice a day at doses of 3, 10 and 30 mg/kg.

Body weight and clinical score will be recorded on a daily basis. Arthritis severity is graded using a clinical scoring system based on the assessment of inflammation in individual paws. The scale for this clinical score ranges from 0-4 for each individual paw.

### LCMS:

### Method A

Method: A-0.1 % TFA in H₂O, B-0.1 % TFA in ACN: Flow- 2.0 mL/min.
Column: XBridge C8 (50 x 4.6mm, 3.5µm), +ve mode

### Method B

Method: A-0.1 % NH₄HCO₃ in H₂O, B-ACN: Flow- 1.0 mL/min.
Column: XBridge C8 (50 x 4.6 mm, 3.5 µm), -ve mode

### ¹HNMR:

Bruker 400 MHz

### HPLC:

### Method A

Method: A-0.1 % TFA in H₂O B-0.1 % TFA in ACN: Flow - 2.0 mL/min.
Column: XBridge C8 (50 x 4.6 mm, 3.5 µm).

### Method B

Method: A-0.1 % NH₄HCO₃ in H₂O, B-ACN: Flow- 1.0 mL/min.
Column: XBridge C8 (50 x 4.6 mm, 3.5 µm).

Above and below, all temperatures are indicated in°C. In the following examples, "conventional work-up" means: water is added if necessary, the pH is adjusted, if necessary, to values between 2 and 10, depending on the constitution of the end product, the mixture is extracted with ethyl acetate or dichloromethane, the phases are separated, the organic phase is dried over sodium sulfate and evaporated, and the residue is purified by chromatography on silica gel and/or by crystallisation. Rf values on silica gel; eluent: ethyl acetate/methanol 9:1.

### Example 1

### Synthesis of (4-methanesulfonyl-phenyl)-[5-(1-methyl-1H-indazol-5-yl)-[1,2,4]triazolo[1,5-a]pyrazin-2-yl]-amine ("A1")

### 1.1 5-(1-methyl-1H-indazol-5-yl)-[1,2,4]triazolo[1,5-a]pyrazin-2-ylamine

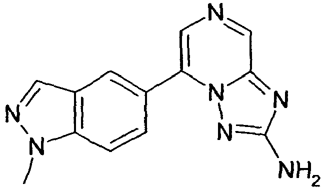

To a solution of 5-chloro-[1,2,4]triazolo[1,5-a]pyrazin-2-ylamine (2.0 g, 11.8 mmol) in 1,4-dioxane/water(9:1, 50 mL), 1-methyl-1H-indazole-5yl-boronic acid (3.1 g, 17.7 mmol), 2-dicyclohexylphosphino-2',4',6'-triisopropylbiphenyl (0.4 g, 0.7 mmol)), palladium acetate (0.08 g, 0.35 mmol) and potassium carbonate (4.9 g, 35.4 mmol) are added and heated in a pressure tube at 110 °C for 10 h. After completion of the reaction (monitored by TLC), the reaction mixture is passed through celite, washed with dichloromethane/methanol (1:1, 75 mL), the filtrate is concentrated to get the crude product. The crude product is purified by column chromatography (silica gel, MEOH/DCM gradient elution); yield: 42 % (1.3 g, yellow solid);
LCMS: (Method A) 266.2 (M+H), RT. 2.2 min, 80.3 % (Max), 92.7 % (254 nm);
¹H NMR (400 MHz, DMSO-d₆): δ [ppm] 8.80 (s, 1H), 8.54 (s, 1H), 8.24-8.21 (m, 2H), 8.03-8.01 (m, 1 H), 7.82 (d, J = 8.84 Hz, 1 H), 6.53 (br s, 2H), 4.11 (s, 3H).

### 1.2 (4-methanesulfonyl-phenyl)-[5-(1-methyl-1 H-indazol-5-yl)-[1,2,4]triazolo[1,5-a]pyrazin-2-yl]-amine ("A1")

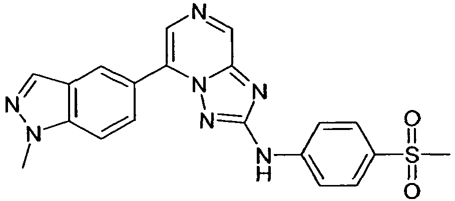

To a solution of 5-(1-methyl-1H-indazol-5-yl)-[1,2,4]triazolo[1,5-a]pyrazin-2-ylamine (0.1 g, 0.37 mmol) in dry *tert*-butanol (5 mL), 4-bromophenylmethyl-sulfone (0.13 g, 0.56 mmol), 2-dicyclohexylphosphino-2'-(N,N-dimethylamino)-biphenyl (0.015 g, 0.04 mmol), tris(dibenzelideneacetone)dipalladium(0) (0.015 g, 0.02 mmol) and lithiumhexamethyldisilylamide (1 M / THF) (1.1 mL) are added, degassed briefly and irradiated in microwave 150 °C for 90 minutes. After completion of the reaction (monitored by TLC), the reaction mixture is filtered through celite washed with dichloromethane/methanol (1:1, 50 mL), the filtrate is concentrated and purified by flash column chromatography (silica gel, MEOH/DCM gradient elution); yield: 5 % (7.7 mg, light brown solid);
LCMS: (Method A) 420.0 (M+H), RT. 3.3 min, 91.6 % (Max), 90.7 % (254 nm); ¹H NMR (400 MHz, DMSO-d₆): δ [ppm]10.65 (s, 1H), 9.14 (s, 1H), 8.60-8.59 (m, 1 H), 8.46 (s, 1 H), 8.27-8.26 (m, 1 H), 8.13 (dd, J = 8.8, 1.6 Hz, 1 H), 7.94-7.89 (m, 3H), 7.85-7.83 (m, 2H), 4.14 (s, 3H), 3.14 (s, 3H);
HPLC: (Method A) RT 3.3 min, 92.5 % (Max), 91.0 % (254 nm).

The following compounds are synthesized analogously to "A1":

### (3-methoxy-phenyl)-[5-(1-methyl-1H-indazol-5-yl)-[1,2,4]triazolo[1,5-a]pyrazin-2-yl]-amine ("A2")

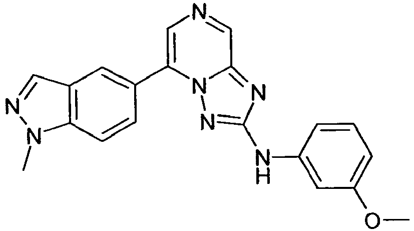

Yield: 18 % (26 mg, light brown solid);
LCMS: (Method A) 372.2 (M+H), RT. 3.9 min, 98.4 % (Max), 99.2 % (254 nm); ¹H NMR (400 MHz, DMSO-d₆): δ [ppm] 10.00 (s, 1H), 9.04 (s, 1H), 8.63 (t, *J* = 0.6 Hz, 1 H), 8.39 (s, 1 H), 8.23 (d, *J* = 0.8 Hz, 1 H), 8.11 (dd, *J* = 8.8, 1.6 Hz, 1H), 7.86 (d, *J* = 8.8 Hz, 1 H), 7.51 (t, *J =* 1.8 Hz, 1 H), 7.20-7.15 (m, 2H), 6.50-6.47 (m, 1H), 4.13 (s, 3H), 3.70 (s, 3H);
HPLC: (Method A) RT 3.8 min, 98.5 % (Max), 98.4 % (254 nm).

### [5-(1-methyl-1 H-indazol-5-yl)-[1,2,4]triazolo[1,5-a]pyrazin-2-yl]-pyridin-3-yl-amine ("A3")

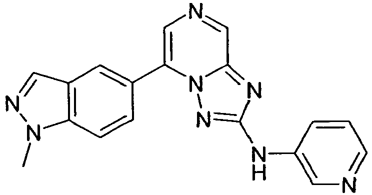

Yield: 36 % (46 mg, light brown solid);
LCMS: (Method A) 343.2 (M+H), RT. 2.3 min, 96.4 % (Max), 94.6 % (254 nm); ¹H NMR (400 MHz, DMSO-d₆): δ [ppm] 10.24 (s, 1H), 9.09 (s, 1H), 8.87 (d, *J* = 2.4 Hz, 1H), 8.59 (d, *J* = 0.7 Hz, 1H), 8.42 (s, 1H), 8.25 (d, *J* = 0.7 Hz, 1H), 8.18-8.10 (m, 3H), 7.88 (d, *J =* 8.8 Hz, 1 H), 7.35-7.32 (m, 1H), 4.14 (s, 3H); HPLC: (Method A) RT 2.3 min, 95.8 % (Max), 94.3 % (254 nm).

### [5-(1-methyl-1 H-indazol-5-yl)-[1,2,4]triazolo[1,5-a]pyrazin-2-yl]-[3-methyl-4-(4-methyl-piperazin-1-yl)-phenyl]-amine ("A4")

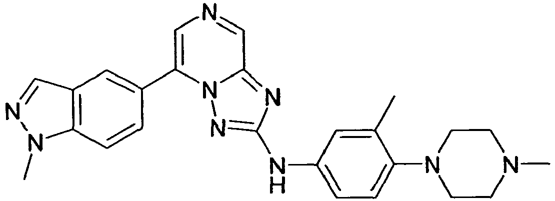

Yield: 65 % (65 mg, pale yellow solid);
LCMS: (Method A) 454.2 (M+H), RT. 3.0 min, 94.5 % (Max), 96.2 % (254 nm); ¹H NMR (400 MHz, CDCl₃): δ [ppm] 8.95 (s, 1H), 8.55 (s, 1H), 8.26 (s, 1H), 8.15 (s, 1H), 8.06 (dd, *J* = 1.5, 8.8 Hz, 1H), 7.61 (d, *J =* 8.8 Hz, 1 H), 7.45 (d, *J* = 2.5 Hz, 1 H), 7.39 (dd, *J =* 8.5, 2.6 Hz, 1 H), 7.06 (d, *J* = 8.5 Hz, 1 H), 6.87 (s, 1 H), 4.19 (s, 3H), 2.99-2.93 (m, 4H), 2.57-2.71 (m, 4H), 2.40 (s, 3H), 2.34 (s, 3H);
HPLC: (Method A) RT 2.9 min, 94.4 % (Max), 95.7 % (254 nm).

### Example 2

### Synthesis of 3,3-dimethyl-6-[5-(1-methyl-1H-indazol-5-yl)-[1,2,4]triazolo[1,5-a]pyrazin-2-ylamino]-1,3-dihydro-indol-2-one ("A5")

### 2.1 2-(4-bromo-2-nitro-phenyl)-malonic acid dimethyl ester

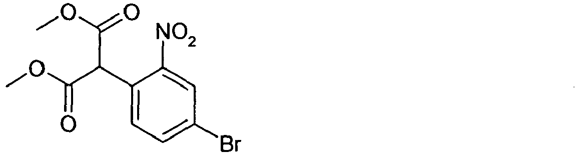

To a suspension of sodium hydride (60 %) (2.13 g, 53.3 mmol) in dry N,N-dimethylformamide (50 mL) at 0 °C, a solution of dimethylmalonate (12 mL, 104.2 mmol) in dry N,N-dimethylformamide (20 mL) is added. The reaction mixture is heated to 100°C for 20 min. 2,5-Dibromonitrobenzene (5 g, 17.8 mmol) in dry N,N-dimethylformamide (20 mL) is added dropwise at RT and is heated to 100 °C for 3 h. After completion of the reaction (monitored by TLC), the reaction mixture is cooled to 0 °C and quenched with cold water. The reaction mixture is concentrated at high vacuum, the residue is taken in ethylacetate (75 mL), washed with water (2 × 75 mL), brine, dried over MgSO₄ and concentrated to get the crude product. The crude product is purified by column chromatography (silica gel, EA/PE gradient elution);
yield: 80 % (4.7 g, light orange solid);
¹H NMR (400 MHz, DMSO-d₆): δ [ppm] 8.30 (d, *J* = 2.16 Hz, 1H), 8.01 (dd, *J* = 8.3, 2.1 Hz, 1 H), 7.50 (d, *J =* 8.3 Hz, 1 H), 5.49 (s, 1 H), 3.69 (s, 6H);
LCMS: (Method B) 330.0 (M-H), RT. 5.8 min, 91.1 % (Max), 90.8 % (254 nm).

### 2.2 4-bromo-2-nitro-benzoic acid methyl ester

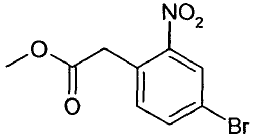

To a solution of 2-(4-bromo-2-nitro-phenyl)-malonic acid dimethyl ester (4.7 g, 14.2 mmol) in DMSO (10 mL), lithium chloride (1.2 g, 28.4 mmol) and water (0.3 mL) are added and heated to 100 °C for 24 h. After completion of the reaction (monitored by TLC), the reaction mixture is concentrated under high vacuum. The residue is diluted with dichloromethane (50 mL), washed with water, brine, dried over MgSO₄ and concentrated to get the crude product. The crude product is purified by column chromatography (silica gel, EA/PE gradient elution);
yield: 20 % (0.8 mg, light brown solid);
¹H NMR (400 MHz, DMSO-d₆): δ [ppm] 8.28 (d, *J =* 2.1 Hz, 1 H), 7.96 (dd, *J* = 8.2, 2.0 Hz, 1 H), 7.54 (d, *J =* 8.2 Hz, 1 H), 4.06 (s, 2H), 3.61 (s, 3H);
LCMS: (Method B) 274.0 (M-H), RT. 5.8 min, 94.0 % (Max).

### 2.3 2-(4-bromo-2-nitro-phenyl)-2-methyl-propionic acid methyl ester

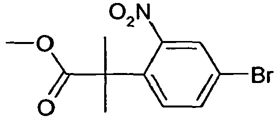

To a suspension of sodium hydride (60%) (0.28 g, 7.22 mmol) in dry N,N-dimethylformamide (15 mL) at 0°C, 4-bromo-2-nitro-benzoic acid methyl ester (0.8 g, 2.9 mmol), iodomethane (0.72 mL, 11.5 mmol) and 18-crown-6 (0.8 g, 0.3 mmol) are added and stirred at RT for 2 h. After completion of the reaction (monitored by TLC), the reaction mixture is cooled to 0 °C and quenched with cold water. The reaction mixture is concentrated at high vacuum, the residue is taken in ethylacetate (30 mL), washed with water (2 × 30 mL), brine, dried over MgSO₄ and concentrated to get the crude product. The crude product is purified by column chromatography (silica gel, EA/PE gradient elution);
yield: 91 % (0.8 g, brown oil);
¹H NMR (400 MHz, DMSO-d₆): δ [ppm] 8.14 (d, *J* = 2.2 Hz, 1H), 7.94 (dd, *J* = 8.6, 2.2 Hz, 1H), 7.71 (d, *J =* 8.6 Hz, 1 H), 3.53 (s, 3H), 1.56 (s, 6H);
LCMS: (Method B) 301.0 (M-H), RT. 6.2 min, 96.3 % (Max), 93.5 % (254 nm);

### 2.4 6-bromo-3,3-dimethyl-1,3-dihydro-indol-2-one

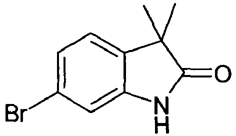

To a solution of 2-(4-bromo-2-nitro-phenyl)-2-methyl-propionic acid methyl ester (0.6 g, 1.96 mmol) in glacial acetic acid (10 mL), iron powder (0.55 g, 9.8 mmol) is added and heated to 100 °C for 2 h. After completion of the reaction (monitored by TLC), the reaction mixture is concentrated at high vacuum, diluted with dichloromethane and passed through celite. The filtrate is concentrated to get the crude product. The crude product is purified by column chromatography (silica gel, EA/PE gradient elution);
yield: 35 % (210 mg, white solid);
¹H NMR (400 MHz, DMSO-d₆): δ [ppm] 10.45 (s, 1H), 7.24 (d, *J* = 7.9 Hz, 1H), 7.13 (dd, *J* = 7.8, 1.7 Hz, 1 H), 6.97 (d, *J =* 1.8 Hz, 1 H), 1.22 (s, 6H);
LCMS: (Method B) 240.0 (M-H), RT. 5.1 min, 99.5 % (Max), 99.0 % (254 nm);

### 2.5 3,3-dimethyl-6-[5-(1-methyl-1H-indazol-5-yl)-[1,2,4]triazolo[1,5-a]pyrazin-2-ylamino]-1,3-dihydro-indol-2-one ("A5")

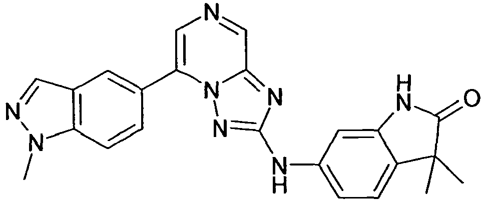

The compound is obtained analogously to "A1";
yield: 58 % (59 mg, yellow solid);
LCMS: (Method A) 425.2 (M+H), RT. 3.6 min, 97.5 % (Max), 81.0, 96.6 % (254 nm);
¹H NMR (400 MHz, DMSO-d₆): δ [ppm] 10.31 (s, 1 H), 9.98 (s, 1H), 9.03 (s, 1 H), 8.59-8.58 (m, 1H), 8.39 (s, 1 H), 8.26 (d, *J* = 0.8 Hz, 1 H), 8.12 (dd, *J* = 8.8, 1.6 Hz, 1 H), 7.88 (d, *J* = 8.8 Hz, 1 H), 7.35 (d, *J* = 1.9 Hz, 1 H), 7.28 (dd, *J* = 8.1, 2.0 Hz, 1 H), 7.16 (d, *J* = 8.0 Hz, 1H), 4.14 (s, 3H), 1.21 (s, 6H).
HPLC: (Method A) RT 3.7 min, 96.8 % (Max), 95.3 % (254 nm).

### Example 3

### Synthesis of (2-dimethylaminomethyl-1H-benzoimidazol-5-yl)-[5-(1-methyl-1H-indazol-5-yl)-[1,2,4]triazolo[1,5-a]pyrazin-2-yl]-amine ("A6")

### 3.1 6-bromo-2-chloromethyl-1 H-benzoimidazole

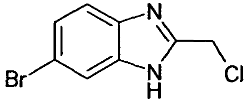

To a solution of 4-bromobenzene-1,2-diamine (3 g, 16 mmol) in absolute alcohol (50 mL), ethyl-2-chloroacetimidate hydrochloride (5 g, 32 mmol) is added and stirred at RT for 12 h. After completion of the reaction (monitored by TLC), the reaction mixture is concentrated under vacuo. The residue is taken in dichloromethane (60 mL), washed with water, brine, dried over MgSO₄ and concentrated to get the crude product. The crude product is purified by column chromatography (silica gel, EA/PE gradient elution);
yield: 30 % (1.2 g, pale brown solid);
¹H NMR (400 MHz, DMSO-d₆): δ [ppm] 7.76 (s, 1H), 7.52 (d, *J* = 8.5 Hz, 1H), 7.34 (dd, *J* = 8.5, 1.8 Hz, 1H), 4.91 (s, 2H);
LCMS: (Method A) 246.0 (M+H), RT. 2.3 min, 97.4 % (Max), 97.5 % (254 nm).

### 3.2 (6-bromo-1H-benzoimidazol-2-ylmethyl)-dimethyl-amine

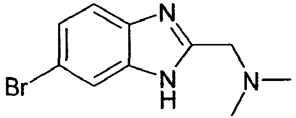

To a solution of 6-bromo-2-chloromethyl-1 H-benzoimidazole (1.2 g, 4.8 mmol) in dry tetrahydrofuran (20 mL), diethylamine (40 %, 5 mL) is added and stirred at RT for 2 h in a sealed tube. After completion of the reaction (monitored by TLC), the reaction mixture is concentrated under vacuo. The residue is taken in dichloromethane (30 mL), washed with water, brine, dried over MgSO₄ and concentrated to get the product;
yield: 69 % (0.85 g, brown solid);
¹H NMR (400 MHz, DMSO-d₆): δ [ppm] 13.41 (br s, 1H), 7.95 (s, 1H), 7.68 (d, *J* = 8.3 Hz, 1 H), 7.44 (dd, *J* = 8.6, 1.5 Hz, 1 H), 5.00 (s, 2H), 3.26 (s, 6H).

### 3.3 [6-bromo-1-(2-trimethylsilanyl-ethoxymethyl)-1H-benzoimidazol-2-ylmethyl]-dimethyl-amine

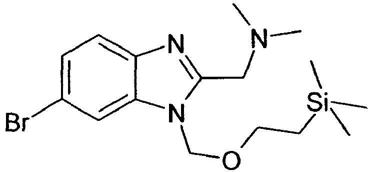

To a suspension of sodium hydride (60 %) (150 mg, 3.8 mmol) in dry N,N-dimethylformamide (15 mL) at 0 °C, a solution of (6-bromo-1H-benzoimidazol-2-ylmethyl)-dimethyl-amine (800 mg, 3.17 mmol) in dry N,N-dimethylformamide (10 mL) is added and stirred for 1 h. (2-(Chloromethoxy)ethyl)trimethylsilane (5.4 mL, 30.6 mmol) is added and stirred at RT for 30 min. After completion of the reaction (monitored by TLC), the reaction mixture is quenched with cold water and concentrated at high vacuum, the residue is taken in ethylacetate, washed with water (2 × 25 mL), brine (1 × 25 mL), dried over MgSO₄ and concentrated to get the crude product. The crude product is purified by column chromatography (silica gel, MeOH/DCM gradient elution) to get the mixture of regioisomers;
yield: 49 % (0.6 g, brown gummy solid);
¹H NMR (400 MHz, DMSO-d₆): δ [ppm] 7.86-7.80 (m, 1H), 7.60-7.55 (m, 1H), 7.35-7.32 (m, 1H), 5.69 (s, 2H), 3.69-3.68 (m, 2H), 3.53-3.48 (m, 2H), 2.20 (s, 6H), 0.84-0.80 (m, 2H), -0.102 (s, 9H);
LCMS: (Method A) 384.0 (M+H), RT. 4.5 min, 85.3 % (Max), 88.7 % (254 nm).

### 3.4 [2-dimethylaminomethyl-1-(3-trimethylsilanyl-propoxymethyl)-1H-benzoimidazol-5-yl]-[5-(1-methyl-1 H-indazol-5-yl)-[1,2,4]triazolo[1,5-a]pyrazin-2-yl]-amine

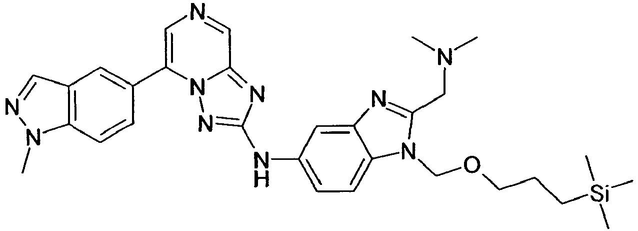

The compound is prepared analogously to "A1";
yield: 17 % (60 mg, brown liquid);
LCMS: (Method A) 569.3 (M+H), RT. 3.8, 4.1 min, 34.3, 30.5 % (Max).

### 3.5 (2-dimethylaminomethyl-1 H-benzoimidazol-5-yl)-[5-(1-methyl-1 H-indazol-5-yl)-[1,2,4]triazolo[1,5-a]pyrazin-2-yl]-amine ("A6")

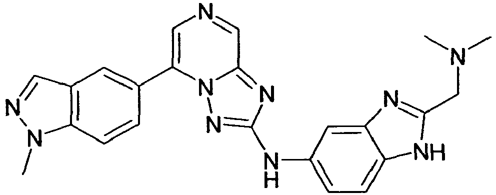

To a solution of [2-dimethylaminomethyl-1-(3-trimethylsilanyl-propoxymethyl)-1H-benzoimidazol-5-yl]-[5-(1-methyl-1H-indazol-5-yl)-[1,2,4]triazolo[1,5-a]pyrazin-2-yl]-amine (60 mg, 0.1 mmol) in dry methanol (3 mL), HCl in methanol (3 mL) is added and stirred at RT for 1 h. After completion of the reaction (monitored by TLC), the reaction mixture is concentrated and the residue is taken in dichloromethane (15 mL), washed with aqueous NaHCO₃ (20 %, 15 mL), water (1 × 15 mL), brine (1 × 15 mL), dried over MgSO₄ and concentrated to get the crude product. The crude product is purified by column chromatography (silica gel, MeOH/DCM gradient elution);
yield: 5 % (5 mg, yellow solid);
LCMS: (Method A) 439.2 (M+H), RT. 2.5 min, 97.7 % (Max), 95.8 % (254 nm); ¹H NMR (400 MHz, DMSO-d₆ δ [ppm] 12.15 (d, *J* = 7.2 Hz, 1 H), 9.91-9.80 (m, 1 H), 9.02 (s, 1 H), 8.65-8.62 (m, 1 H), 8.38-8.36 (m, 1 H), 8.15-8.12 (m, 1 H), 8.04 (s, 1H), 7.97 (d, *J* = 1.6 Hz, 1H), 7.88 (d, *J =* 8.7 Hz, 1H), 7.44-7.41 (m, 1H), 7.36-7.29 (m, 1H), 4.14 (s, 3H), 3.61-3.59 (m, 2H), 2.21 (s, 6H);
HPLC: (Method A) RT 2.4 min, 93.6 % (Max), 92.8 % (254 nm).

### Example 4

### Synthesis of 3-(5-quinolin-3-yl-[1,2,4]triazolo[1,5-a]pyrazin-2-ylamino)-benzenesulfonamide ("A7")

### 4.1 5-quinolin-3-yl-[1,2,4]triazolo[1,5-a]pyrazin-2-ylamine

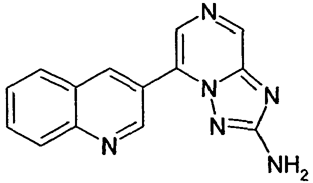

To a solution of 5-chloro-[1,2,4]triazolo[1,5-a]pyrazin-2-ylamine (1.0 g, 5.9 mmol) in 1,2-dimethoxyethane/water (9:1, 20 mL), 3-quinoline-boronic acid pinacol ester (2.24 g, 8.8 mmol), dikis(triphenylphosphino)palladium(II)chloride (0.2 g, 0.3 mmol) and an aqueous solution of sodium carbonate (2M, 5 mL) are added and irradiated in a microwave at 130 °C for 90 minutes. After completion of the reaction (monitored by TLC), the reaction mixture is passed through celite, washed with dichloromethane/methanol (1:1, 75 mL), the filtrate is concentrated to get the crude product. The crude product is purified by column chromatography (silica gel, MEOH/DCM gradient elution);
yield: 55 % (0.85 g, light brown solid);
LCMS: (Method A) 263.0 (M+H), RT. 1.7 min, 96.2 % (Max), 95.7 % (254 nm); ¹H NMR (400 MHz, DMSO-d₆): δ [ppm] 9.48 (d, *J* = 2.2 Hz, 1H), 9.09 (d, *J* = 2.0 Hz, 1 H), 8.91 (s, 1H), 8.42 (s, 1H), 8.13-8.09 (m, 2H), 7.91-7.86 (m, 1H), 7.74-7.70 (m, 1 H), 6.61 (br s, 2H).

### 4.2 3-(5-quinolin-3-yl-[1,2,4]triazolo[1,5-a]pyrazin-2-ylamino)-benzenesulfonamide ("A7")

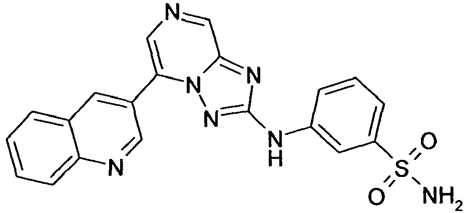

The compound is prepared analogously to "A1";
yield: 36 % (36 mg, off white solid);
LCMS: (Method A) 418.0 (M+H), RT. 2.7 min, 97.8 % (Max), 97.5 % (254 nm); ¹H NMR (400 MHz, DMSO-d₆): δ [ppm] 10.42 (s, 1H), 9.56 (d, *J =* 2.1 Hz, 1H), 9.21 (d, *J* = 1.9 Hz, 1 H), 9.19 (s, 1H), 8.64 (s, 1 H), 8.24 (s, 1 H), 8.19-8.14 (m, 2H), 7.94-7.90 (m, 2H), 7.75 (t, *J =* 7.3 Hz, 1H), 7.50 (t, *J* = 8.0 Hz, 1H), 7.39 (d, *J* = 8.0 Hz, 1 H), 7.34 (s, 2H);
HPLC: (Method A) RT 2.6 min, 96.9 % (Max), 97.1 % (254 nm).

The following compounds are prepared analogously:

### [3-methyl-4-(4-methyl-piperazin-1-yl)-phenyl]-(5-quinolin-3-yl-[1,2,4]triazolo[1,5-a]pyrazin-2-yl)-amine ("A8")

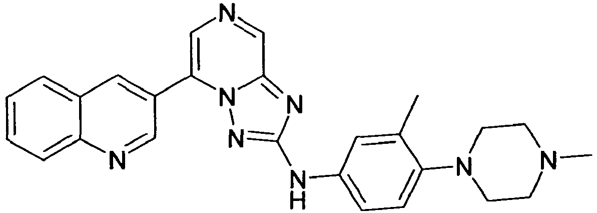

yield: 28 % (28 mg, yellow solid);
LCMS: (Method A) 451.2 (M+H), RT. 2.7 min, 97.3 % (Max), 96.5% (254 nm); ¹H NMR (400 MHz, CDCl₃): δ [ppm] 9.51 (d, *J* = 2.2 Hz, 1 H), 9.08 (d, *J* = 2.1 Hz, 1 H), 9.02 (s, 1 H), 8.39 (s, 1 H), 8.24 (d, *J* = 8.4 Hz, 1 H), 8.01 (d, *J* = 8.2 Hz, 1H), 7.90-7.86 (m, 1H), 7.72-7.68 (m, 1H), 7.45-7.42 (m, 2H), 7.07 (d, *J* = 8.3 Hz, 1 H), 6.90 (s, 1 H), 2.98-2.92 (m, 4H), 2.69-2.61 (m, 4H), 2.41 (s, 3H), 2.33 (s, 3H);
HPLC: (Method A) RT 2.6 min, 96.7 % (Max), 96.2 % (254 nm).

### 3,3-dimethyl-6-(5-quinolin-3-yl-[1,2,4]triazolo[1,5-a]pyrazin-2-ylamino)-1,3-dihydro-indol-2-one ("A9")

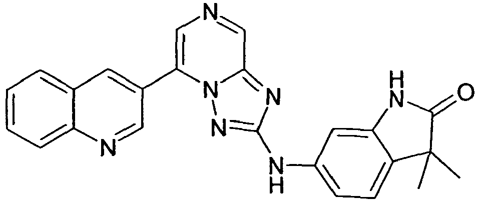

yield: 32 % (32 mg, pale yellow solid);
LCMS: (Method A) 422.2 (M+H), RT. 3.3 min, 96.5 % (Max), 97.3% (254 nm); ¹H NMR (400 MHz, DMSO-d₆): δ [ppm] 10.30 (s, 1H), 10.02 (s, 1H), 9.55 (d, *J* = 2.20 Hz, 1H), 9.16 (d, *J* = 2.12 Hz, 1H), 9.13 (s, 1 H), 8.58 (s, 1H), 8.15 (d, *J* = 8.52 Hz, 2H), 7.92 (dt, *J =* 1.40, 10.77 Hz, 1H), 7.75 (t, *J =* 8.00 Hz, 1H), 7.31-7.29 (m, 2H), 7.16 (d, *J =* 8.64 Hz, 1 H), 1.21 (s, 6H);
HPLC: (Method B) RT 5.0 min, 94.1 % (Max), 92.4 % (254 nm).

### Example 5

### Synthesis of (2-dimethylaminomethyl-1H-benzoimidazol-5-yl)-(5-quinolin-3-yl-[1,2,4]triazolo[1,5-a]pyrazin-2-yl)-amine ("A10")

### 5.1

### [2-dimethylaminomethyl-1-(3-trimethylsilanyl-propoxymethyl)-1H-benzoimidazol-5-yl]-(5-quinolin-3-yl-[1,2,4]triazolo[1,5-a]pyrazin-2-yl)-amine

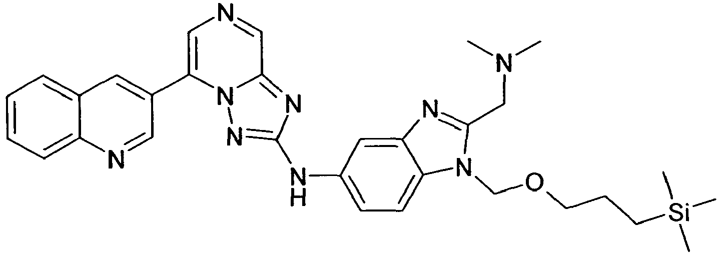

The compound is prepared analogously to "A1";
yield: 20 % (70 mg, brown liquid);
LCMS: (Method A) 566.2 (M+H), RT. 3.7, 3.9 min, 43.5, 37.3 % (Max).

### 5.2 (2-dimethylaminomethyl-1H-benzoimidazol-5-yl)-(5-quinolin-3-yl-[1,2,4]triazolo[1,5-a]pyrazin-2-yl)-amine ("A10")

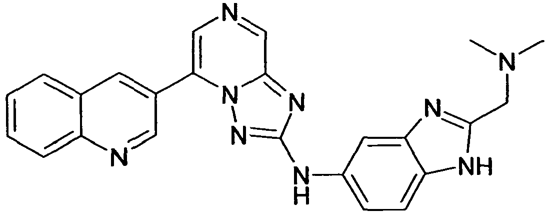

The compound is prepared analogously to "A6";
yield: 10 % (9 mg, yellow solid);
LCMS: (Method A) 436.2 (M+H), RT. 2.4 min, 99.2 % (Max), 99.5% (254 nm). ¹H NMR (400 MHz, DMSO-d₆): δ [ppm] 12.16 (s, 1 H), 9.95-9.86 (m, 1 H), 9.60-9.57 (m, 1 H), 9.25-9.19 (m, 1H), 9.12 (s, 1H), 8.57 (s, 1H), 8.16 (d, *J* = 7.6 Hz, 2H), 7.93-7.90 (m, 2H), 7.75 (t, *J =* 8.0 Hz, 1 H), 7.45-7.30 (m, 2H), 3.61-3.59 (m, 2H), 2.22 (s, 6H);
HPLC: (Method A) RT 2.3 min, 97.1 % (Max), 97.0 % (254 nm).

### Example 6

### Synthesis of (4-methanesulfonyl-phenyl)-[5-(1-methyl-1H-pyrazol-4-yl)-[1,2,4]triazolo[1,5-a]pyrazin-2-yl]-amine ("A11")

### 6.1 5-(1-methyl-1H-pyrazol-4-yl)-[1,2,4]triazolo[1,5-a]pyrazin-2-ylamine

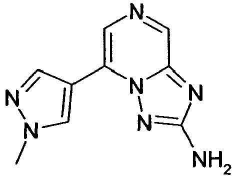

The compound is prepared analogously to "A1", step 1;
yield: 32 % (0.8 g, light brown solid);
LCMS: (Method A) 216.2 (M+H), RT. 1.5 min, 99.8 % (Max), 99.6 % (254 nm);
¹H NMR (400 MHz, DMSO-d₆): δ [ppm] 8.77 (s, 1H), 8.65 (s, 1 H), 8.48 (s, 1H), 8.45 (s, 1 H), 6.52 (br s, 2H), 3.96 (s, 3H).

### 6.2 (4-methanesulfonyl-phenyl)-[5-(1-methyl-1H-pyrazol-4-yl)-[1,2,4]triazolo[1,5-a]pyrazin-2-yl]-amine ("A11")

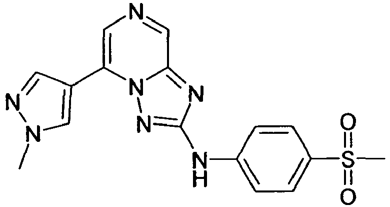

The compound is prepared analogously to "A1";
yield: 29 % (50 mg, light brown solid);
LCMS: (Method A) 370.0 (M+H), RT. 2.8 min, 95.8 % (Max), 92.6 % (220 nm); ¹H NMR (400 MHz, DMSO-d₆): δ [ppm]10.63 (s, 1H), 8.97 (s, 1H), 8.83 (s, 1H), 8.69(s, 1 H), 8.54 (s, 1 H), 7.96-7.89 (m, 4H), 4.03 (s, 3H), 3.16 (s, 3H);
HPLC: (Method A) RT 2.7 min, 94.4 % (Max), 90.5 % (254 nm).

The following compounds are prepared analogously:

### [5-(1-methyl-1H-pyrazol-4-yl)-[1,2,4]triazolo[1,5-a]pyrazin-2-yl]-pyridin-3-yl-amine ("A12")

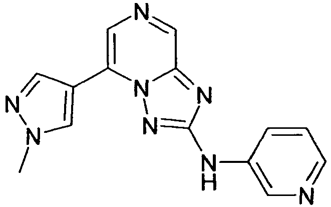

yield: 24 % (33 mg, light brown solid);
LCMS: (Method A) 293.2 (M+H), RT. 1.8 min, 98.8 % (Max), 98.3 % (254 nm);
¹H NMR (400 MHz, DMSO-d₆): δ [ppm] 10.22 (s, 1H), 8.92 (s, 1 H), 8.89 (d, *J* = 2.6 Hz, 1 H), 8.81 (s, 1 H), 8.65 (s, 1 H), 8.53 (s, 1 H), 8.24-8.20 (m, 1 H), 8.17 (dd, *J* = 4.6, 1.3 Hz, 1 H), 7.42-7.38 (m, 1H), 4.00 (s, 3H);
HPLC: (Method A) RT 1.8 min, 99.2 % (Max), 98.6 % (254 nm).

### 3,3-dimethyl-6-[5-(1-methyl-1H-pyrazol-4-yl)-[1,2,4]triazolo[1,5-a]pyrazin-2-ylamino]-1,3-dihydro-indol-2-one ("A13")

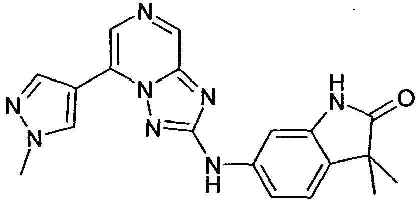

yield: 66 % (70 mg, off white solid);
LCMS: (Method A) 375.2 (M+H), RT. 3.0 min, 97.9 % (Max), 96.8 % (254 nm); ¹H NMR (400 MHz, DMSO-d₆): δ [ppm] 10.37 (s, 1H), 9.96 (s, 1H), 8.86 (s, 1H), 8.82 (s, 1H), 8.63 (s, 1H), 8.53 (s, 1H), 7.42 (d, *J* = 1.8 Hz, 1 H), 7.27-7.20 (m, 2H), 4.01 (s, 3H), 1.23 (s, 6H);
HPLC: (Method A) RT 3.3 min, 97.6 % (Max), 96.9 % (254 nm).

### (2-dimethylaminomethyl-1H-benzoimidazol-5-yl)-[5-(1-methyl-1H-pyrazol-4-yl)-[1,2,4]triazolo[1,5-a]pyrazin-2-yl]-amine ("A14")

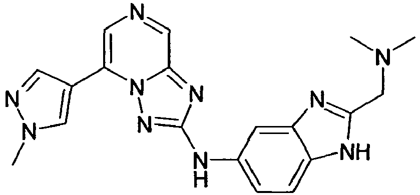

The compound is prepared analogously to "A6";
yield: 11 % (11 mg, yellow solid);
LCMS: (Method A) 389.2 (M+H), RT. 2.1 min, 97.5 % (Max), 97.3 % (254 nm);
¹H NMR (400 MHz, CDCl₃): δ [ppm] 8.84 (s, 1 H), 8.65 (s, 1 H), 8.41 (s, 1 H), 8.30 (s, 1H), 8.11 (d, *J* = 1.8 Hz, 1 H), 7.63-7.61 (m, 1 H), 7.29 (br s, 1 H), 7.12 (s, 1 H), 4.11 (s, 3H), 3.97 (s, 2H), 2.51 (s, 6H);
HPLC: (Method A) RT 2.2 min, 98.0 % (Max), 96.7 % (254 nm).

### Example 7

### Synthesis of 5-[5-(4-morpholin-4-yl-phenyl)-[1,2,4]triazolo[1,5-a]pyrazin-2-ylamino]-1,3-dihydro-indol-2-one ("A15")

### 7.1 5-(4-morpholin-4-yl-phenyl)-[1,2,4]triazolo[1,5-a]pyrazin-2-ylamine

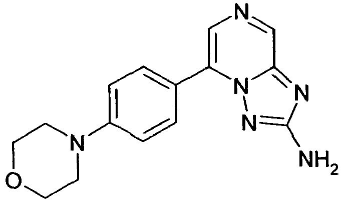

The compound is prepared analogously to "A1", step 1;
yield: 45 % (800 mg, light brown solid);
LCMS: (Method A) 297.2 (M+H), RT. 2.4 min, 89.2 % (Max), 93.2 % (254 nm);

### 7.2 5-[5-(4-morpholin-4-yl-phenyl)-[1,2,4]triazolo[1,5-a]pyrazin-2-ylamino]-1,3-dihydro-indol-2-one ("A15")

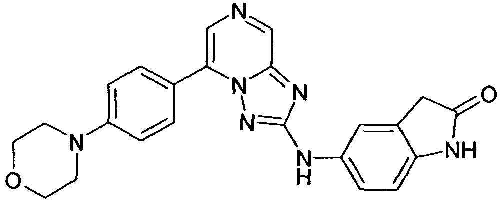

The compound is prepared analogously to "A1";
yield: 30 % (30 mg, yellow solid);
LCMS: (Method A) 428.2 (M+H), RT. 3.1 min, 97.8 % (Max), 96.6 % (254 nm);
¹H NMR (400 MHz, DMSO-d₆): δ [ppm] 10.22 (s, 1H), 9.77 (s, 1H), 8.90 (s, 1H), 8.31 (s, 1 H), 8.10 (d, *J =* 8.0 Hz, 2H), 7.66 (s, 1 H), 7.48 (dd, *J* = 8.4, 2.2 Hz, 1 H), 7.14 (d, *J =* 9.0 Hz, 2H), 6.75 (d, *J* = 8.4 Hz, 1 H), 3.77 (t, *J =* 5.2 Hz, 4H), 3.49 (s, 2H), 3.29-3.26 (m, 4H);
HPLC: (Method A) RT 3.3 min, 96.2 % (Max), 95.5 % (254 nm).

The following compounds are prepared analogously:

### [5-(4-morpholin-4-yl-phenyl)-[1,2,4]triazolo[1,5-a]pyrazin-2-yl]-pyridin-3-yl-amine ("A16")

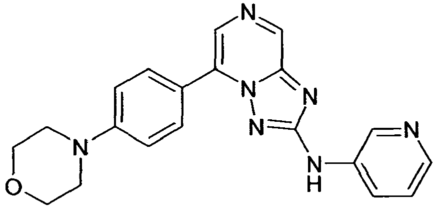

yield: 20 % (26 mg, yellow solid);
LCMS: (Method A) 374.2 (M+H), RT. 2.5 min, 97.1 % (Max), 96.8 % (254 nm);
¹H NMR (400 MHz, DMSO-d₆): δ [ppm]10.62 (s, 1 H), 9.03 (s, 2H), 8.39 (s, 1 H), 8.36 (d, *J* = 7.9 Hz, 1 H), 8.30 (d, *J* = 4.7 Hz, 1 H), 8.09 (d, *J* = 9.0 Hz, 2H), 7.64 (br s, 1 H), 7.15 (d, *J* = 9.0 Hz, 2H), 3.78 (t, *J* = 5.0 Hz, 4H), 3.29 (t, *J* = 4.8 Hz, 4H);
HPLC: (Method A) RT 2.5 min, 98.6 % (Max), 98.1 % (254 nm).

### [3-methyl-4-(4-methyl-piperazin-1-yl)-phenyl]-[5-(4-morpholin-4-yl-phenyl)-[1,2,4]triazolo[1,5-a]pyrazin-2-yl]-amine ("A17")

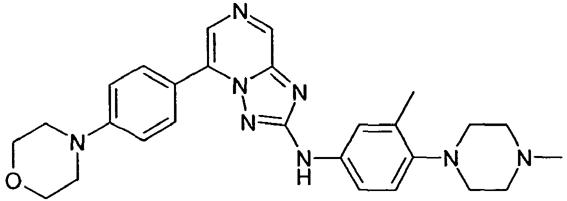

yield: 37 % (37 mg, brown solid);
LCMS: (Method A) 485.3 (M+H), RT. 3.1 min, 97.3 % (Max), 97.8 % (254 nm); ¹H NMR (400 MHz, DMSO-d₆): δ [ppm] 9.76 (s, 1 H), 8.91 (s, 1 H), 8.31 (s, 1 H), 8.12 (d, *J* = 8.9 Hz, 2H), 7.56 (d, *J* = 2.4 Hz, 1H), 7.50 (dd, *J* = 8.6, 2.5 Hz, 1H), 7.14 (d, *J* = 9.0 Hz, 2H), 7.01 (d, *J* = 8.6 Hz, 1 H), 3.78 (t, *J* = 5.0 Hz, 4H), 3.28 (t, *J* = 4.9 Hz, 4H), 3.19-2.69 (m, 8H), 2.53 (s, 3H), 2.25 (s, 3H);
HPLC: (Method A) RT 3.1 min, 97.2 % (Max), 97.8 % (254 nm).

### Example 8

### Synthesis of (6-chloro-[1,2,4]triazolo[1,5-a]pyrazin-2-yl)-(4-methoxy-phenyl)-amine ("A18")

### 8.1 N-(tert-butoxy carbonyl)-O-(mesitylsulfonyl)-hydroxylamine

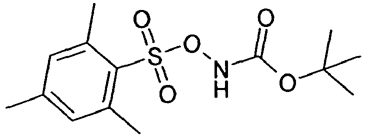

To a solution of 2-mesitylene sulphonyl chloride (2.0 g, 0.00914 mol) in dry THF (50 mL) is added N-Boc-hydroxylamine (1.21 g, 0.00914 mol) and cooled to 0°C under N₂ atmosphere. The reaction mixture is stirred for 5 minutes. To this mixture triethylamine (1.1 g, 0.011 mol) is added slowly over 10 minutes. The reaction mixture is stirred for 1 hour 0°C and then the solvent is removed in vacuo. The residue is dissolved in dichloromethane (50 mL) and washed with water (2 x 50 mL), 10% aqueous NaHCO₃ (50 ml) and dried over MgSO₄. It is then concentrated under reduced pressure at room temperature to get the product as an off white solid. (2.1 g, 73%);
TLC: Pet ether / Ethyl acetate (8/2) R*_{f}*- 0.4;
¹H NMR (DMSO-d₆; 400 MHz): δ [ppm] 11.16 (s, 1H), 7.12 (s, 2H), 2.49 (s, 6H), 2.28 (s, 3H), 1.23 (s, 9H).

### 8.2 2-[(aminooxy)sulfonyl]-1,3,5-trimethylbenzene

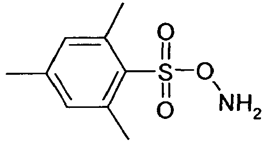

To *N*-(t-butoxy carbonyl)-*O*-(mesitylsulfonyl)-hydroxylamine (2.1 g, 0.0066mol) is added trifluoroacetic acid (20ml) slowly at 0°C under a nitrogen atmosphere. The reaction mixture is stirred for 30 minutes and water (60 mL) is added slowly and the reaction mixture is stirred for 15 minutes. The precipitate is filtered and washed several times with water until the pH of the filtrate is neutral. The white solid (1.4g, 98%) is dried in the Buchner funnel and used immediately for the next reaction.

White solid *CAUTION : 2-[(amin oxy) sulfonyl]-1,3,5-trimethylbenzene, also known as MSH is highly unstable and reactive compound. It is known to explode when handled in large quantities. This compound should always be prepared in batches and used immediately for next reaction.*
¹H NMR (400 MHz, DMSO-d₆): δ [ppm] 6.73 (s, 2H), 2.48 (s, 6H), 2.15 (s, 3H).

### 8.3 1,2-diamino-5-chloro-pyrazinium mesitylenate

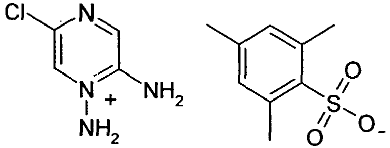

To a solution of 5-amino-2-chloro-pyrazine (1.75 g, 13.5 mmol) in dry dichloromethane (75 ml) at 0°C under N₂ atmosphere, 2-[(aminooxy)sulfonyl]-1, 3,5-trimethylbenzene (3.36 g, 16.88 mmol) in dry dichloromethane (50 ml) is added dropwise and the reaction mixture is stirred for 3 hours at room temperature. The reaction mixture is concentrated to minimum and diethyl ether (100 ml) is added and stirred for 15 minutes. The precipitate is filtered, washed with diethyl ether to get the product as brown solid (3.2g, 69.56%);
¹H NMR: 400 MHz, DMSO-d₆: δ [ppm] 8.99 (br s, 2H), 8.42 (s, 1 H), 8.38 (s, 1H), 7.24-7.22 (m, 2H), 6.74 (s, 2H), 2.48 (s, 6H), 2.16 (s, 3H).

### 8.4 (6-chloro-[1,2,4]triazolo[1,5-a]pyrazin-2-yl)-(4-methoxy-phenyl)-amine

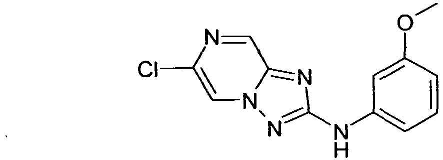

To a solution of 3-methoxyphenylisothiocyanate (0.1 g, 5.87 mmol) in dry dichloromethane (25 ml), 1,2-diamino-5-chloro-pyrazinium mesitylenate (2.52 g, 7.34 mmol) and diisopropylethylamine (3.79 g, 29.35 mmol) are added and stirred for an hour. EDCI (2.24 g, 11.74 mmol) is added and stirred for 6 hours. The reaction mixture is taken in water, separated the layer, the organic layer is washed with water, brine, dried over anhydrous MgSO₄ and concentrated. The crude product is purified by silica column using (60-120) mesh to get the product as light brown solid (0.55 g, 35.71%);
TLC: Chloroform/Methanol (9.5/0.5) R*_{f}*-0.3;
¹H NMR: 400 MHz, DMSO-d₆: δ [ppm] 10.01 (s, 1 H), 9.31 (d, J = 1.16 Hz, 1 H), 8.92 (d, J = 1.12 Hz, 1H), 7.38 (t, J = 2.08 Hz, 1 H), 7.25-7.18 (m, 2H), 6.54-6.51 (m, 1 H), 3.74 (s, 3H);
LCMS: Mass found (M+, 276.0);
Method: A-0.1% TFA in H₂O, B-0.1% TFA in ACN: Flow - 2.0 ml/min Column: XBridge C8 (50 X 4.6 mm, 3.5 µm), +ve mode
Rt (min): 3.79 area % -96.75 (Max), 97.37 (254 nm);

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 3.85 area % -96.68 (Max), 97.02 (254 nm).

The following compounds are prepared analogously:

### (6-chloro-[1,2,4]triazolo[1,5-a]pyrazin-2-yl)-(3-fluoro-phenyl)-amine ("A19")

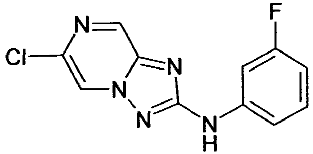

yield: 0.5 g, 29.41 %; color: light brown solid;
TLC: chloroform/methanol (9.5/0.5) R*_{f}*- 0.3;
¹H NMR: 400 MHz, DMSO-d₆: δ [ppm] 10.31 (s, 1H), 9.35 (d, J = 1.24 Hz, 1H), 8.97 (d, J = 1.20 Hz, 1 H), 7.68 (dt, J = 2.32, 7.18 Hz, 1 H), 7.42-7.30 (m, 2H), 6.73-6.77 (m, 1 H);
LCMS: Mass found (M+, 264.0)
Method: A-0.1 % TFA in H₂O B-0.1% TFA in ACN: Flow - 2.0 ml/min. Column: XBridge C8 (50 X 4.6 mm, 3.5 µm), +ve mode
Rt (min): 4.25 area % 99.07(Max), 98.26 (254 nm);

### HPLC

Method: A- A-0.1 % TFA in H₂O, B- % TFA in ACN: Flow - 2.0ml/min.
Column: X Bridge C8 (50 X 4.6 mm, 3.5 µm)
Rt (min): 4.11 area % 99.41 (Max), 98.47(254 nm).

### (6-chloro-[1,2,4]triazolo[1,5-a]pyrazin-2-yl)-pyridin-3-yl-amine ("A20")

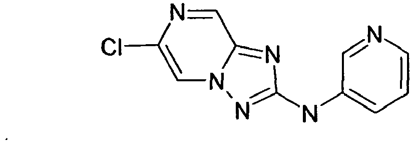

yield: 0.026 g, 37.08 %; color: pale yellow solid;
¹H NMR: 400 MHz, DMSO-d₆: δ [ppm] 10.27 (s, 1H), 9.34 (d, J = 1.24 Hz, 1H), 8.97 (d, J = 1.20 Hz, 1H), 8.85 (d, J = 2.48 Hz, 1H), 8.17-8.13 (m, 2H), 7.37-7.34 (m, 1 H);
LCMS: Mass found (M+, 247.0)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 1.64 area % 97.82 (Max), 96.52 (254 nm);

### HPLC

Method: A- A-0.1% TFA in H₂O B- % TFA in ACN: Flow - 2.0 ml/min.
Column: X Bridge C8 (50 X 4.6 mm, 3.5 µm)
Rt (min): 1.69 area % 98.07 (Max), 96.67 (254 nm).

### (6-chloro-[1,2,4]triazolo[1,5-a]pyrazin-2-yl)-(4-fluoro-phenyl)-amine ("A21")

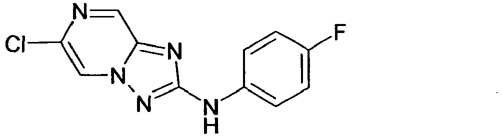

yield: 0.023 g, 49.48 %; color: off white solid;
¹H NMR: 400 MHz, DMSO-d₆: δ [ppm] 10.05 (s, 1 H), 9.30 (d, J = 1.12 Hz, 1 H), 8.91 (d, J = 1.16 Hz, 1 H), 7.71-7.66 (m, 2H), 7.20-7.14 (m, 2H);
LCMS: Mass found (M+, 264.0);
Method: A-0.1% TFA in H₂O B-0.1% TFA in ACN: Flow - 2.0ml/min.
Column: XBridge C8 (50 X 4.6 mm, 3.5 µm), +ve mode
Rt (min): 3.91 area % 99.11 (Max), 98.98 (254 nm).

### HPLC

Method: A- A-0.1% TFA in H₂O B- % TFA in ACN: Flow - 2.0ml/min.
Column: X Bridge C8 (50 X 4.6mm, 3.5 µm)
Rt (min): 3.99 area % 99.3 (Max), 99.42 (254 nm).

### (6-bromo-[1,2,4]triazolo[1,5-a]pyrazin-2-yl)-(3-methoxy-phenyl)-amine ("A22")

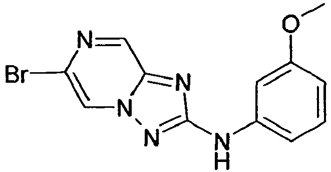

yield: 2.3 g, 60 %; color: light brown solid;
TLC: chloroform/methanol (9.5/0.5) R*_{f}*- 0.4;
¹H NMR: 400 MHz, DMSO-d₆: δ [ppm] 10.01 (s, 1H), 9.36 (d, J = 1.24 Hz, 1H), 8.91 (d, J = 1.20 Hz, 1 H), 7.38 (t, J = 2.16 Hz, 1H), 7.25-7.18 (m, 2H), 6.54-6.51 (m, 1 H), 3.74 (s, 3H);
LCMS: Mass found (M+, 322.0)
Method: A-0.1% TFA in H₂O, B-0.1% TFA in ACN: Flow - 2.0ml/min.
Column: XBridge C8 (50 X 4.6mm, 3.5 µm), +ve mode
Rt (min): 3.88 area % 99.12 (Max), 99.46 (254 nm);

### HPLC

Method: A- A-0.1% TFA in H₂O, B- % TFA in ACN: Flow - 2.0ml/min.
Column: X Bridge C8 (50 X 4.6mm, 3.5 µm)
Rt (min): 3.93 area % 99.46 (Max), 99.80 (254 nm).

### (6-cyano-[1,2,4]triazolo[1,5-a]pyrazin-2-yl)-(3-methoxy-phenyl)-amine ("A23")

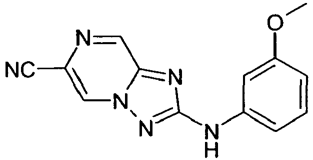

¹H NMR: 400 MHz, DMSO-d₆: δ [ppm] 10.22 (s, 1H), 9.83 (d, J = 1.16 Hz, 1H), 9.14 (d, J = 1.12 Hz, 1H), 7.36 (t, J = 1.64 Hz, 1H), 7.25-7.23 (m, 2H), 6.58-6.55 (m, 1H), 3.75 (s, 3H);
LCMS: Mass found (M+, 267.0)
Method: A-0.1% TFA in H₂O, B-0.1% TFA in ACN: Flow - 2.0ml/min.
Column: XBridge C8 (50 X 4.6 mm, 3.5 µm), +ve mode
Rt (min): 3.67 area % 98.79 (Max), 98.78 (254 nm);

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 3.66 area % 99.57 (Max), 96.80 (254 nm).

### Example 9

### Synthesis of (3-methoxy-phenyl)-(6-methyl-[1,2,4]triazolo[1,5-a]pyrazin-2-yl)-amine ("A24")

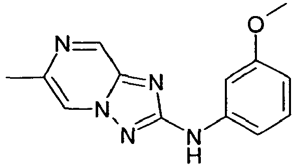

To a solution of (6-bromo-[1,2,4]triazolo[1,5-a]pyrazin-2-yl)-(3-methoxyphenyl)-amine (0.1 g, 0.31 mmol) in acetonitrile/water (1:1, 8ml), methyl boronic acid (0.037 g, 0.62 mmol), bis(triphenylphosphino)dichloropalladium(II) (0.008 g, 0.01 mmol), sodium carbonate (0.09 g, 0.93 mmol) are added and degassed briefly and irradiated in microwave for 30 minutes at 120 °C. The reaction mixture is concentrated and diluted with 50% dichloromethane in methanol and passed through celite, the filtrate is concentrated. The crude product is purified by column using silica gel(60-120) mesh to get the product as off white solid(0.052g, 50.9%);
TLC: Pet ether/ethylacetate(7/3) R*_{f}*- 0.3;
¹H NMR: 400 MHz, CDCl₃: δ [ppm] 8.93 (d, J = 1.32 Hz, 1H), 8.24 (t, J = 1.08 Hz, 1H), 7.36-7.34 (m, 2H), 7.29-7.24 (m, 1H), 7.08-7.11 (m, 1H), 6.61-6.58 (m, 1 H), 3.86 (s, 3H), 2.60 (d, J = 0.60 Hz, 3H);
LCMS: Mass found (M+, 256.30)
Method: A-0.1 % TFA in H₂O, B-0.1% TFA in ACN: Flow - 2.0 ml/min.
Column: XBridge C8 (50 X 4.6 mm, 3.5 µm), +ve mode
Rt (min): 3.18 area % 96.51 (Max), 96.80 (254 nm);

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 3.19 area % 98.85 (Max), 97.92 (254 nm).

### Example 10

### Synthesis of pyridin-3-yl-(6-pyridin-3-yl-[1,2,4]triazolo[1,5-a]pyrazin-2-yl]-amine ("A25")

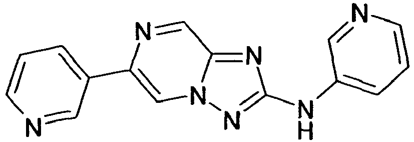

yield : 0.031 g, 21%; color: light brown solid;
¹H NMR: 400 MHz, DMSO-d₆: δ [ppm] 10.25 (s, 1H), 9.70 (s, 1H), 9.31 (s, 1H), 9.23 (s, 1 H), 8.90 (s, 1 H), 8.63 (d, J = 5.04 Hz, 1 H), 8.48 (d, J = 6.96 Hz, 1 H), 8.18 (t, J = 3.80 Hz, 2H), 7.56-7.53 (m, 1H), 7.39-7.36 (m, 1H);
LCMS: Mass found (M+, 290.0)
Method: A-0.1% TFA in H₂O, B-0.1% TFA in ACN: Flow - 2.0 ml/min.
Column: XBridge C8 (50 X 4.6mm, 3.5 µm), +ve mode
Rt (min): 3.67 area % -95.59 (Max), 95.64 (254 nm);

### HPLC

Method: A- A-0.1 % NH₄HCO₃ in H₂O, B- ACN: Flow - 2.0 ml/min.
Column: X Bridge C8 (50 X 4.6 mm, 3.5 µm)
Rt (min): 3.78 area % -98.22 (Max), 97.67 (254 nm).

The following compound is prepared analogously

### (3-methoxy-phenyl)-(6-o-tolyl-[1,2,4]triazolo[1,5-a]pyrazin-2-yl)-amine ("A26")

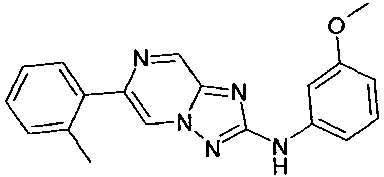

yieid : 0.052 g, 50.9 %; color: off white solid;
TLC: chloroform/methanol (9.5/0.5) R*_{f}*- 0.4,
¹H NMR: 400 MHz, DMSO-d₆: δ [ppm] 9.93 (s, 1H), 9.13 (d, J = 1.36 Hz, 1H), 9.06 (d, J = 1.36 Hz, 1H), 7.49 (d, J = 7.24 Hz, 1H), 7.41 (t, J = 2.20 Hz, 1H), 7.36-7.27 (m, 4H), 7.21 (t, J = 8.12 Hz, 1H), 6.53-6.51 (m, 1H), 3.75 (s, 3H), 2.38 (s, 3H);
LCMS: Mass found (M+, 332.0)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 4.67 area % -98.38 (Max), 99.78 (254 nm);

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 4.78 area % -98.25 (Max), 99.81 (254 nm).

### Example 10 a

### Synthesis of (3-fluoro-phenyl)-[5-(3-Methoxy-phenyl)-[1,2,4]triazolo[1,5-a]pyrazin-2-yl]-amine

### 10a.1 6-(3-methoxy-phenyl)-pyrazin-2ylamine

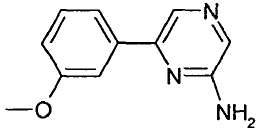

To a solution of 2-amino-6-chloropyrazine (0.5 g, 3.85 mmol) in a mixture of toluene/ethanol (4:1, 10 ml), 3-methoxyphenylboronic acid (0.64 g, 4.24 mmol), tetrakis(triphenylphosphino)palladium(0) (0.13 g, 0.11 mmol) and cesium carbonate (2.51 g, 7.71 mmol) are added, degassed briefly and irradiated in microwave at 120°C for 15 minutes. The reaction mixture is passed through celite, washed with dichloromethane, the filterate is concentrated and purified by column using silica gel (60-120) mesh to get the product as yellow solid (0.34 g, 44.15 %);
TLC: Pet ether/Ethylacetate(7/3) R*_{f}*- 0.2;
¹H NMR: 400 MHz, DMSO-d₆: δ [ppm] 8.27 (s, 1H), 7.83 (s, 1H), 7.57-7.52 (m, 2H), 7.37 (t, J = 7.88 Hz, 1H), 7.00-6.97 (m, 1H), 6.51 (br s, 2H), 3.80 (s, 3H); LCMS: Mass found (M+, 202.3)
Method: A-0.1 % TFA in H₂O, B-0.1% TFA in ACN: Flow - 2.0ml/min.
Column: XBridge C8 (50 X 4.6 mm, 3.5 µm), +ve mode
Rt (min): 2.48 area % -98.79 (Max), 99.35 (254 nm).

### 10a.2 5-(3-methoxy-phenyl)-[1,2,4]triazolo[1,5-a]pyrazin-2-yl-amine

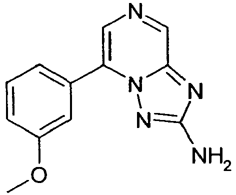

To a solution of 6-(3-methoxy-phenyl)-pyrazin-2ylamine (0.34 g, 1.68 mmol), in dry dichloromethane (25 ml), ethoxycarbonylisothiocyanate (0.24 g, 1.85 mmol) is added and heated to 50°C for 16 hours. The reaction mixture is concentrated and is taken in a mixture of methanol/ethanol(1:1, 35 ml), hydroxylamine hydrochloride (0.58 g, 8.44 mmol) and diisopropylethylamine (0.65 g, 5.06 mmol) are added and refluxed at 80°C for 3 hours. The reaction mixture is concentrated to minimum and cooled, filtered, washed with water, 20% acetonitrile in diethylether to get the product as yellow solid (0.25 g, 61.42 g).

¹H NMR: 400 MHz, DMSO-d₆: δ [ppm] 8.82 (s, 1H), 8.21 (s, 1H), 7.59-7.67 (m, 2H), 7.48 (t, J = 7.80 Hz, 1H), 7.13 (d, J = 7.28 Hz, 1H), 6.53 (br s, 2H), 3.82 (s, 3H);
LCMS: Mass found (M+, 242.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.04 area % -87.06 (Max), 90.54 (254 nm).

### 10a.3 (3-fluoro-phenyl)-[5-(3-Methoxy-phenyl)-[1,2,4]triazolo[1,5-a]pyrazin-2-yl]-amine

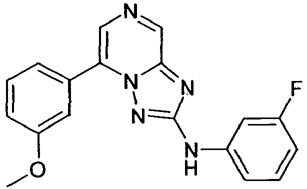

To a solution of 5-(3-methoxy-phenyl)-[1,2,4]triazolo[1,5-a]pyrazin-2-yl-amine (0.1 g, 0.41 mmol) in dry tert-butanol (5ml), 3-chloro-1-fluorobenzene (0.0.094 g, 0.82 mmol), tris(dibenzylideneacetone)dipalladium(0) (0.016 g, 0.017 mmol), 2-dicyclohexylphosphino-2'-(N,N-dimethylamino)biphenyl (0.016 g, 0.041 mmol), and sodiumhexamethyldisilylamide (1M/THF) (0.62 ml, 0.62 mmol) are added, degasified briefly and irradiated in microwave at 120°C for 40 minutes. The reaction mixture is concentrated and directly purified by silica column using (230-400) mesh to get the product as off white solid (0.05 g, 35.90 %); TLC: chloroform/methanol (9.5/0.5) R*_{f}*- 0.4;
¹H NMR: 400 MHz, DMSO-d₆: δ [ppm] 10.32 (s, 1 H), 9.14 (s, 1 H), 8.11 (s, 1 H), 7.74-7.70 (m, 2H), 7.67-7.65 (m, 1H), 7.53 (t, J = 7.88 Hz, 1H), 7.41-7.39 (m, 1 H), 7.31 (q, J = 8.16 Hz, 1 H), 7.19-7.16 (m, 1H), 6.74-6.70 (m, 1H), 3.86 (s, 3H);
LCMS: Mass found (M+, 336.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 4.62 area % -95.42 (Max), 97.75 (254 nm);

### HPLC

Method: A- A-0.1 % TFA in H₂O, B- % TFA in ACN: Flow - 2.0 ml/min.
Column: X Bridge C8 (50 X 4.6 mm, 3.5 µm)
Rt (min): 4.61 area % -97.72 (Max), 98.03 (254 nm).

### Example 11

### [5-(4-fluoro-phenyl)-[1,2,4]triazolo[1,5-a]pyrazin-2-yl]-pyridin-3-yl-amine ("A27") is prepared analogously to example 10a

### 11.1 6-(4-fluoro-phenyl)-pyrazin-2ylamine

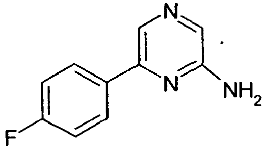

¹H NMR: 400 MHz, DMSO-d₆: δ [ppm] 8.26 (s, 1H), 8.02-8.05 (m, 2H), 7.83 (s, 1H), 7.31-7.27 (m, 2H), 6.52 (br s, 2H);
LCMS: Mass found (M+, 190.0)
Method: A-0.1% TFA in H₂O, B-0.1% TFA in ACN: Flow - 2.0 ml/min.
Column: XBridge C8 (50 X 4.6mm, 3.5 µm), +ve mode
Rt (min): 2.52 area % -96.57 (Max).

### 11.2 5-(4-fluoro-phenyl)-[1,2,4]triazolo[1,5-a]pyrazin-2-ylamine

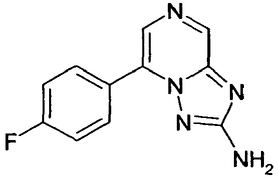

¹H NMR: 400 MHz, DMSO-d₆: δ [ppm] 8.82 (s, 1 H), 8.19 (s, 1 H), 8.13-8.10 (m, 2H), 7.45-7.40 (m, 2H), 6.55 (br s, 2H);
LCMS: Mass found (M+, 230.0)
Method: A-0.1 % TFA in H₂O, B-0.1% TFA in ACN: Flow - 2.0 ml/min.
Column: XBridge C8 (50 X 4.6mm, 3.5 µm), +ve mode
Rt (min): 3.02 area % -97.52 (Max), 97.50 (220nm).

### 11.3 [5-(4-fluoro-phenyl)-[1,2,4]triazolo[1,5-a]pyrazin-2-yl]-pyridin-3-yl-amine ("A27")

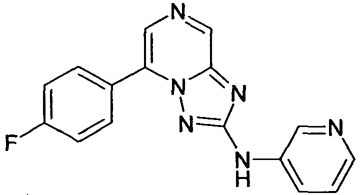

yield : 0.015 g, 11.90 %; color: off white solid
¹H NMR: 400 MHz, DMSO-d₆: δ [ppm] 10.26 (s, 1H), 9.11 (s, 1H), 8.85 (d, J = 2.36 Hz, 1H), 8.37 (s, 1 H), 8.30-8.13 (m, 4H), 7.49 (t, J = 8.84 Hz, 2H), 7.36-7.32 (m, 1 H);
LCMS: Mass found (M+, 307.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.67 area % -92.99 (Max), 93.45 (254 nm).

### HPLC

Method: A- A-0.1 % TFA in H₂O, B- % TFA in ACN: Flow - 2.0 ml/min.
Column: X Bridge C8 (50 X 4.6 mm, 3.5 µm)
Rt (min): 2.58 area % -95.92 (Max), 94.84 (254 nm).

The following compound is pepared analogously

### (3-methoxy-phenyl)-(5-phenyl-[1,2,4]triazolo[1,5-a]pyrazin-2-yl)-amine ("A28")

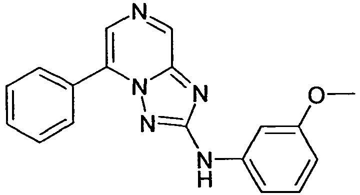

yield : 0.061 g, 40.99 %; color: pale yellow solid;
TLC: chloroform/methanol (9.5/0.5) R*_{f}* - 0.4;
¹H NMR: 400 MHz, DMSO-d₆: δ [ppm] 10.01 (s, 1 H), 9.07 (s, 1 H), 8.34 (s, 1 H), 8.14-8.11 (m, 2H), 7.62-7.59 (m, 3H), 7.51 (t, J = 1.20 Hz, 1H), 7.18-7.14 (m, 2H), 6.50-6.48 (m, 1H), 3.72 (s, 3H);
LCMS: Mass found (M+, 318.3)
Method: A-0.1 % TFA in H₂O, B-0.1% TFA in ACN: Flow - 2.0 ml/min.
Column: XBridge C8 (50 X 4.6 mm, 3.5 µm), +ve mode
Rt (min): 4.46 area % 99.03 (Max), 99.01 (254 nm);

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 4.37 area % 96.18 (Max), 97.66 (254 nm).

### Example 12

### N2-(3-fluoro-phenyl)-N6-(3-methoxy-phenyl)-[1,2,4]triazolo[1,5-a]pyrazine-2,6-diamine

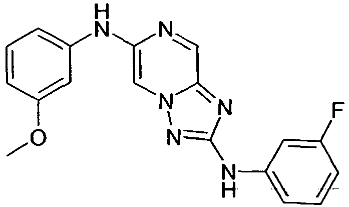

To a solution of (6-bromo-[1,2,4]triazolo[1,5-a]pyrazin-2-yl)-(3-fluoro-phenyl)-amine (0.1 g, 0.31 mmol) in dry 1,4-dioxane (4 ml), m-anisidine (0.034 g, 0.28 mmol), 4,5-bis(diphenylphosphino)-9,9-dimethylxanthene (0.018 g, 0.032 mmol), tris(dibenzylideneacetone)dipalladium(0) (0.14 g, 0.015 mmol), cesium carbonate (0.15 g, 0.46 mmol) were added, briefly degassed, sonicated for 5 minutes and heated in sealed tube at 120°C for 12 hours. The reaction mixture is passed through celite and washed with 30% methanol in dichloromethane (20ml), the filtrate is concentrated and purified by column chromatography using silica gel (230-400) mesh to get the product as off white solid (0.144 g, 43.8%);
TLC: Pet ether/Ethylacetate (5/5) R*_{f}* - 0.2.
¹H NMR: 400 MHz, DMSO-d₆: δ [ppm] 10.05 (s, 1H), 8.89 (s, 1H), 8.86 (d, J = 1.36 Hz, 1H), 8.34 (d, J = 1.36 Hz, 1H), 7.69 (dt, J = 2.32, 7.24 Hz, 1H), 7.40 (dd, J = 1.16, 8.26 Hz, 1H), 7.33-7.28 (m, 1H), 7.16 (t, J = 8.12 Hz, 1H), 7.01 (t, J = 2.24 Hz, 1 H), 6.95 (dd, J = 1.96, 7.80 Hz, 1 H), 6.72-6.68 (m, 1H), 6.47 (dd, J = 2.40, 8.12 Hz, 1H), 3.73 (s, 3H);
LCMS: Mass found (M+, 351.0)
Method: A-0.1% TFA in _{H2O}, B-0.1% TFA in ACN: Flow - 2.0 ml/min.
Column: XBridge C8 (50 X 4.6 mm, 3.5 µm), +ve mode
Rt (min): 4.67 area % 99.40 (Max), 98.45 (254 nm).

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 4.67 area % 99.57 (Max), 99.09 (254 nm).

The following compound is prepared analogously

### N6-(4-fluoro-phenyl)-N2-pyridin-3-yl-[1,2,4]triazolo[1,5-a]pyrazine-2,6-diamine ("A29")

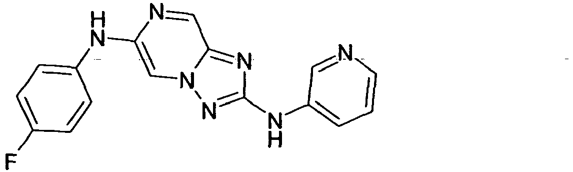

yield : 0.014 g, 3.23 %; color: pale brown solid;
¹H NMR: 400 MHz, DMSO-d₆: δ [ppm] 10.00 (s, 1H), 8.89 (s, 1H), 8.84 (d, J = 2.92 Hz, 2H), 8.28 (d, J = 1.24 Hz, 1H), 8.18 (d, J = 8.48 Hz, 1 H), 8.12 (d, J = 4.52 Hz, 1H), 7.42-7.39 (m, 2H), 7.35-7.31 (m, 1H), 7.12 (t, J = 6.68 Hz, 2H); LCMS: Mass found (M+, 322.0)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 2.81 area % 95.12 (Max), 95.94 (254 nm);

### HPLC

Method: A- A-0.1% TFA in H₂O, B- % TFA in ACN: Flow - 2.0 ml/min.
Column: X Bridge C8 (50 X 4.6 mm, 3.5 µm)
Rt (min): 2.87 area % 96.95 (Max), 95.08 (254 nm).

The following compound is prepared analogously to example 10a

### (3-methoxy-phenyl)-[5-(1-methyl-1H-pyrazol-4-yl)-[1,2,4]triazolo[1,5-a]pyrazin-2-yl]-amine ("A30")

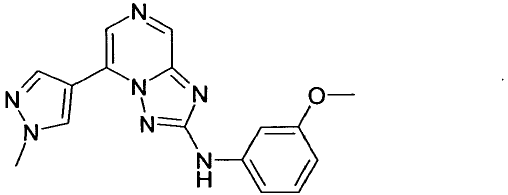

yield : 0.0071 g, 29.72 %; color: light brown solid;
TLC: chloroform/methanol (9.5/0.5) R*_{f}* - 0.4;
¹H NMR: 400 MHz, DMSO-d₆: δ [ppm] 9.99 (s, 1 H), 8.89 (s, 1 H), 8.83 (s, 1 H), 8.62 (s, 1H), 8.55 (s, 1H), 7.49-7.48 (m, 1H), 7.25-7.23 (m, 2H), 6.55-6.52 (m, 1H), 3.99 (s, 3H), 3.79 (s, 3H);
LCMS: Mass found (M+, 323.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.33 area % -94.37 (Max), 94.62 (254 nm).

### HPLC

Method: A- A-0.1 % TFA in H₂O, B- % TFA in ACN: Flow - 2.0 ml/min.
Column: X Bridge C8 (50 X 4.6 mm, 3.5 µm)
Rt (min): 3.34 area % -96.30 (Max), 96.75 (254 nm).

### Example 13

### Synthesis of (4-morpholin-4-yl-phenyl)-[5-(1H-pyrazol-4-yl)-[1,2,4]triazolo[1,5-a]pyrazin-2-yl]-amine ("A31")

### 13.1 5-chloro-[1,2,4]triazolo[1,5-a]pyrazin-2-ylamine

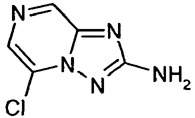

To a solution of 2-amino-6-chloropyrazine (15.0 g, 115.8 mmol) in dry tetrahydrofuran (150 ml), ethoxycarbonylisothiocyante (16.7g, 127.4 mmol) is added and heated to 50°C for 12 hours. The reaction mixture is concentrated and suspended in ethanol/methanol (1:1, 300 ml), hydroxylamine hydrochloride (40.09 g, 576.9 mmol) and diisopropylethylamine (44.73 g, 346.1 mmol) are added and heated to 80°C for 3 hours. The reaction mixture is concentrated and the crude mass is taken in water and filtered, washed with water, cold acetonitrile, diethylether, to get the product as pale yellow solid (14.0 g, 71.42 %); TLC: chloroform/methanol (9.5/0.5) R*_{f}* - 0.2.
¹H NMR: 400 MHz, DMSO-d₆: δ [ppm] 8.80 (s, 1H), 8.18 (s, 1H), 6.75 (br s, 2H);
LCMS: Mass found (M+, 170.0)
Method: A-0.1 % TFA in H₂O, B-0.1% TFA in ACN: Flow - 1.0 ml/min.
Column: XBridge C8 (50 X 4.6 mm, 3.5 µm), +ve mode
Rt (min): 2.01 area % -99.66 (Max), 99.51 (220 nm);

### HPLC

Method: A- A-0.1% TFA in H₂O, B- % TFA in ACN: Flow - 1.0ml/min.
Column: XBridge C8 (50 X 4.6 mm, 3.5 µm)
Rt (min): 2.02 area % - 99.23 (Max), 99.05 (220nm).

### 13.2 4-(4,4,5,5-tetramethyl-[1,3,2]dioxaborolan-2-yl)-1-(2-trimethylsilanyl-ethoxymethyl)-1 H-pyrazole

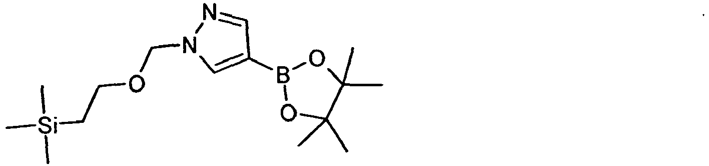

To a solution of 1H-pyrazole-4-boronic acid pinacol ester (0.5 g, 2.57 mmol), in tetrahydrofuran/acetonitrile (3:2, 20ml), 2-(chloromethoxylethyl)trimethylsilane (0.51 g, 3.09 mmol) and cesium carbonate (1.67 g, 5.15 mmol) are added and stirred for 2 hours at room temperature. The reaction mixture is filtered through celite, and concentrated, the crude mass is taken in ethylacetate (30 ml), washed with water, brine solution, dried over anhydrous MgSO₄ and concentrated to get the product as brown oil (0.55 g, 65.94 %); TLC: Pet ether/ethyl acetate(8/2) R*_{f}* - 0.5;
¹H NMR: 400 MHz, DMSO-d₆: δ [ppm] 8.08 (s, 1H), 7.64 (s, 1 H), 5.40 (s, 2H), 3.48-3.54 (m, 2H), 1.24 (s, 12H), 0.81-0.85 (m, 2H), -0.049(s, 9H);

### 13.3 5-[1-(2-trimethylsilanyl-ethoxymethyl)-1H-pyrazol-4-yl]-[1,2,4]triazolo[1,5-a]pyrazin-2-ylamine

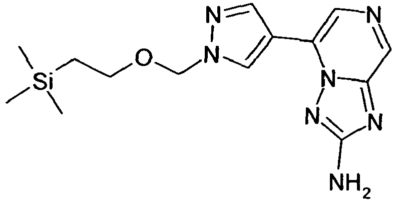

To a solution of 5-chloro-[1,2,4]triazolo[1,5-a]pyrazin-2-ylamine (0.3 g, 1.77 mmol) in 1,2-dimethoxyethane/water (9:1, 10 ml), 4-(4,4,5,5-tetramethyl-[1,3,2]dioxaborolan-2-yl)-1-(2-trimethylsilanyl-ethoxymethyl)-1H-pyrazole (0.86 g, 2.66 mmol), tetrakis(triphenylphosphine) palladium(0 (0.061 g, 0.05 mmol) and sodium bicarbonate(0.44 g, 5.32 mmol) are added, degassed briefly and irradiated in microwave at 120°C for 45 minutes. The reaction mixture is passed through celite, washed with dichloromethane/methanol (1:1, 25ml), the filtrate is concentrated and purified by silica column using (230-400) mesh to get the product as off white solid (0.09 g, 96.77 %);
TLC: chloroform/methanol (9.5/0.5) R*_{f}* - 0.2;
¹H NMR: 400 MHz, DMSO-d₆: δ [ppm] 8.97 (s, 1 H), 8.69 (s, 1 H), 8.56 (s, 1 H), 8.55(s, 1 H), 6.55 (br s, 2H), 5.54 (s, 2H), 3.58 (t, J = 8.12 Hz, 2H), 0.85 (t, J = 8.00 Hz, 2H), -0.051(s, 9H); LCMS: Mass found (M+, 332.3);
Method: A-0.1 % TFA in H₂O, B-0.1% TFA in ACN: Flow - 2.0 ml/min.
Column: XBridge C8 (50 X 4.6 mm, 3.5 µm), +ve mode
Rt (min): 3.92 area % -74.45 (Max).

### 13.4 (4-morpholin-4-yl-phenyl)-{5-[1-(2-trimethylsilanyl-ethoxymethyl)-1H-pyrazol-4-yl]-[1,2,4]triazolo[1,5-a]pyrazin-2-yl}-amine

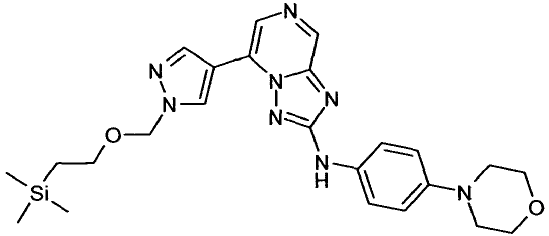

To a solution of 5-[1-(2-trimethylsilanyl-ethoxymethyl)-1H-pyrazol-4-yl]-[1,2,4]triazolo[1,5-a]pyrazin-2-ylamine(0.09 g, 0.27 mmol) in dry *tert*-butanol (5 ml), 4-(4-chlorophenyl)-morpholine (0.08 g, 0.40 mmol), 2-dicyclohexyl-phosphino-2'-(N,N-dimethylamino)biphenyl (0.01 g, 0.03 mmol), tris(dibenzelideneacetone)dipalladium(0) (0.01 g, 0.01 mmol) and sodium hexamethyl disilylamide (1M/THF) (0.4 ml) are added, degassed briefly and irradiated in microwave 150°C for 45 minutes. The reaction mixture is filtered through celite, washed with dichloromethane/methanol (1:1, 10 ml), the filtrate is concentrated and purified by silica column using (230-400) mesh to get the product as yellow solid (0.05 g, 37.59 %);
TLC: chloroform/methanol (9.5/0.5) R*_{f}* - 0.3;
¹H NMR: 400 MHz, DMSO-d₆: δ [ppm] 9.73 (s, 1H), 9.02 (s, 1H), 8.86 (s, 1H), 8.65 (s, 1H), 8.62 (s, 1H), 7.62 (d, J = 9.04 Hz, 2H), 6.77 (d, J = 167.84 Hz, 2H), 5.59-5.58 (m, 2H), 3.74 (t, J = 4.92 Hz, 4H), 3.62 (t, J = 8.12 Hz, 2H), 3.03 (t, J = 4.80 Hz, 4H), 0.86 (t, J = 7.84 Hz, 2H), -0.054 (s, 9H);
LCMS: Mass found (M+, 493.3);
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.98 area % -87.00 (Max), 89.03 (254 nm).

### 13.5 (4-morpholin-4-yl-phenyl)-[5-(1H-pyrazol-4-yl)-[1,2,4]triazolo[1,5-a]pyrazin-2-yl]-amine ("A31")

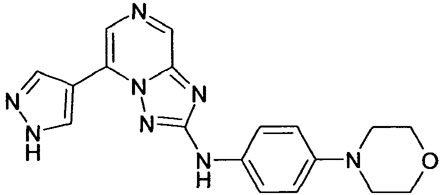

To a solution of (4-morpholin-4-yl-phenyl)-{5-[1-(2-trimethylsilanyl-ethoxy-methyl)-1H-pyrazol-4-yl]-[1,2,4]triazolo[1,5-a]pyrazin-2-yl}-amine (0.05 g, 0.1 mmol) in dry methanol (3 ml), HCl in methanol (5 ml) is added and stirred for 12 hours. The reaction mixture is concentrated and the crude mass is taken in water (15 ml), neutralized with Na₂CO₃ and adjusted to pH∼8, extracted with dichloromethane, dried over anhydrous MgSO₄, concentrated and purified by silica column using (230-400) mesh to get the product as yellow solid (0.004 g, 16.66%);
¹H NMR: 400 MHz, DMSO-d₆: δ [ppm] 13.54 (s, 1H), 9.69 (s, 1H), 8.89 (s, 1 H), 8.83 (s, 1 H), 8.65 (s, 1 H), 8.60 (s, 1 H), 7.61 (d, J = 9.04 Hz, 2H), 6.96 (d, J = 9.08 Hz, 2H), 3.74 (t, J = 4.88 Hz, 4H), 3.04 (t, J = 4.76 Hz, 4H);
LCMS: Mass found (M+, 363.3);
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 1.99 Area % -97.48 (Max), 97.97 (254 nm);

### HPLC

Method: A- A-0.1% TFA in H₂O, B- % TFA in ACN: Flow - 2.0 ml/min.
Column: XBridge C8 (50 X 4.6 mm, 3.5 µm)
Rt (min): 2.04 area % - 98.46 (Max), 98.52 (254 nm).

### Example 14

### Synthesis of (4-morpholin-4-yl-phenyl)-(5-quinolin-3-yl-[1,2,4]triazolo[1,5-a]pyrazin-2-yl)-amine ("A32")

### 14.1 6-quinolin-3-yl-pyrazin-2-ylamine

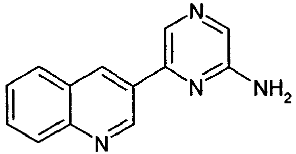

To a solution of 2-amino-6-chloro-pyrazine (0.3 g, 2.31 mmol) in toluene / ethanol (9:1, 10 ml), quinoline-3-boronic acid pinacol ester (0.65 g, 2.54 mmol), tetrakis(triphenylphosphine)palladium(0) (0.08 g, 0.069 mmol) and cesium carbonate (1.5 g, 4.60 mmol) are added, degassed briefly and irradiated in microwave at 130°C for an hour. The reaction mixture is passed through celite, washed with dichloromethane/methanol (1:1, 25 ml), the filtrate is concentrated and purified by silica column using (230-400) mesh to get the product as yellow solid (0.25 g, 49.61 %);
TLC: chloroform/methanol (9.5/0.5) R*_{f}* - 0.4;
LCMS: Mass found (M+, 223.0)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 1.67 area % -99.35 (Max).

### 14.2 5-quinolin-3-yl-[1,2,4]triazolo[1,5-a]pyrazin-2-ylamine

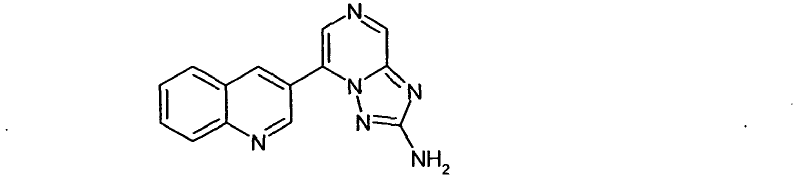

To a solution of 6-quinolin-3-yl-pyrazin-2-ylamine (0.25 g, 1.14 mmol) in dry tetrahydrofuran (30 ml), ethoxycarbonylisothiocyante (0.16 g, 1.26 mmol) is added and heated to 50°C for 12 hours. The reaction mixture is concentrated and suspended in ethanol/methanol (1:1, 50 ml), hydroxylamine hydrochloride (0.49 g, 7.08 mmol) and diisopropylethylamine (0.54 g, 4.24 mmol) are added and heated to 80°C for 6 hours. The reaction mixture is concentrated and the crude mass is taken in water and filtered, washed with water, cold acetonitrile and diethylether to get the product as pale yellow solid (0.05 g, 8%);
TLC: chloroform/methanol (9.5/0.5) R*_{f}* - 0.2;
LCMS: Mass found (M+, 263.0)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 1.76 area % -95.72 (Max), 90.35 (254 nm).

### 14.3 (4-morpholin-4-yl-phenyl)-(5-quinolin-3-yl-[1,2,4]triazolo[1,5-a]pyrazin-2-yl)-amine ("A32")

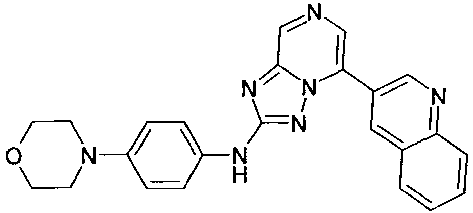

To a solution of 5-quinolin-3-yl-[1,2,4]triazolo[1,5-a]pyrazin-2-ylamine (0.04 g, 0.15 mmol) in dry *tert*-butanol (5 ml), 4-(4-chlorophenyl)-morpholine (0.045 g, 0.22 mmol), 2-dicyclohexylphosphino-2'-(N,N-dimethylamino)biphenyl (0.006 g, 0.015 mmol), tris(dibenzelideneacetone)dipalladium(0) (0.006 g, 0.006 mmol) and sodiumhexamethyldisilylamide (1M/THF) (0.23 ml) are added, degassed briefly and irradiated in microwave 150°C for an hour. The reaction mixture is filtered through celite washed with dichloromethane/methanol (1:1, 10 ml), the filtrate is concentrated and purified by silica column using (230-400) mesh to get the product as yellow solid (0.017 g, 21.6 %);
TLC: chloroform/methanol (9.5/0.5) R*_{f}* - 0.3;
¹H NMR: 400 MHz, DMSO-d₆: δ [ppm] 9.77 (s, 1H), 9.57 (d, J = 2.24 Hz, 1H), 9.17 (d, J = 2.04 Hz, 1H), 9.09 (s, 1H), 8.55 (s, 1 H), 8.13-8.16 (m, 2H), 7.91 (dt, J = 1.48, 6.94 Hz, 1H), 7.75 (dt, J = 1.04, 7.02 Hz, 1H), 7.58 (d, J = 9.04 Hz, 2H), 6.91 (d, J = 9.08 Hz, 2H), 3.72 (t, J = 4.92 Hz, 4H), 3.01 (t, J = 4.08 Hz, 4H);
LCMS: Mass found (M+, 424.3)
Method: A-0.1 % TFA in H₂O, B-0.1% TFA in ACN: Flow - 2.0 ml/min.
Column: XBridge C8 (50 X 4.6 mm, 3.5 µm), +ve mode
Rt (min): 2.49 area % -98.12 (Max), 97.50 (254 nm);

### HPLC

Method: A- A-0.1% TFA in H₂O, B- % TFA in ACN: Flow - 2.0 ml/min.
Column: XBridge C8 (50 X 4.6 mm, 3.5 µm)
Rt (min): 2.50 area % - 97.91 (Max), 97.47 (254 nm).

### Example 15

### Synthesis of (4-morpholin-4-yl-phenyl)-(5-quinolin-6-yl-[1,2,4]triazolo[1,5-a]pyrazin-2-yl)-amine ("A33")

### 15.1 6-quinolin-6-yl-pyrazin-2-ylamine

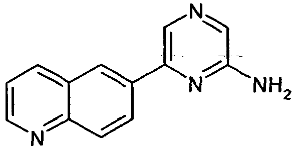

To a solution of 2-amino-6-chloro pyrazine (0.25 g, 3.86 mmol) in toluene / ethanol (9:1, 10 ml), 6-quinoline boronic acid pinacol ester (1.08 g, 4.24 mmol), tetrakis(triphenylphosphine)palladium(0) (0.13 g, 0.11 mmol) and cesium carbonate (2.5 g, 7.7 mmol) are added, degassed briefly and irradiated in microwave at 130°C for an hour. The reaction mixture is passed through celite, washed with dichloromethane/methanol (1:1, 25 ml), the filtrate is concentrated and purified by silica column using (230-400) mesh to get the product as yellow solid (0.51 g, 59.44 %);
TLC: chloroform/methanol (9.5/0.5) R*_{f}* - 0.2;
¹H NMR: 400 MHz, DMSO-d₆: δ [ppm] 8.92 (dd, J = 1.68, 4.20 Hz, 1H), 8.62 (d, J = 1.88 Hz, 1 H), 8.43-8.46 (m, 2H), 8.38 (dd, J = 2.00, 8.86 Hz, 1 H), 8.09 (d, J = 8.84 Hz, 1 H), 7.90 (s, 1 H), 7.55-7.59 (m, 1 H), 6.62 (br s, 2H);
LCMS: Mass found (M+, 223.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 1.41 area % -95.35 (Max), 95.76 (254 nm).

### 15.2 5-quinolin-6-yl-[1,2,4]triazolo[1,5-a]pyrazin-2-ylamine

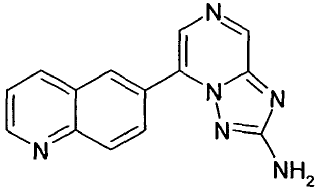

To a solution of 6-quinolin-6-yl-1,2-dihydro-pyrazin-2-ylamine (0.51 g, 2.29 mmol) in dry tetrahydrofuran (20 ml), ethoxycarbonylisothiocyante (0.33 g, 2.52 mmol) is added and heated to 50 °C for 12 hours. The reaction mixture is concentrated and suspended in ethanol/methanol (1:1, 50 ml), hydroxylamine hydrochloride (0.79 g, 11.46 mmol) and diisopropylethylamine (0.88 g, 6.87 mmol) are added and heated to 80 °C for 12 hours. The reaction mixture is concentrated and the crude mass is taken in water and filtered, washed with water, cold acetonitrile and diethylether to get the product as pale yellow solid (0.45 g, 74.98 %);
TLC: chloroform/methanol (9.5/0.5) R*_{f}* - 0.3;
¹H NMR: 400 MHz, DMSO-d₆: δ [ppm] 9.00 (dd, J = 1.60, 4.18 Hz, 1H), 8.88 (s, 1H), 8.70 (d, J = 1.84 Hz, 1H), 8.49 (d, J = 7.68 Hz, 1H), 8.39 (dd, J = 2.00, 8.86 Hz, 1H), 8.33 (s, 1H), 8.18 (d, J = 8.84 Hz, 1H), 7.62-7.65 (m, 1H), 6.59 (br s, 2H);
LCMS: Mass found (M+, 263.3)
Method: A-0.1% TFA in H₂O, B-0.1% TFA in ACN: Flow - 2.0 ml/min.
Column: XBridge C8 (50 X 4. 6mm, 3.5 µm), +ve mode
Rt (min): 1.29 area % -89.08 (Max), 90.99 (254 nm).

### 15.3 (4-morpholin-4-yl-phenyl)-(5-quinolin-6-yl-[1,2,4]triazolo[1,5-a]pyrazin-2-yl)-amine ("A33")

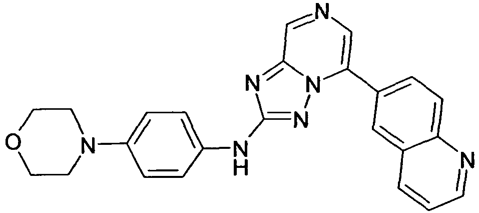

To a solution of 5-quinolin-6-yl-[1,2,4]triazolo[1,5-a]pyrazin-2-ylamine (0.09 g, 0.34 mmol) in dry *tert*-butanol (5 ml), 4-(4-chlorophenyl)-morpholine (0.101 g, 0.51 mmol), 2-dicyclohexylphosphino-2'-(N,N-dimethylamino)biphenyl (0.013 g, 0.03 mmol), tris(dibenzelideneacetone)dipalladium(0) (0.013 g, 0.01 mmol) and sodiumhexamethyldisilylamide (1M/THF) (0.51 ml) are added, degassed briefly and irradiated in microwave 150°C for 30 minutes. The reaction mixture is filtered through celite washed with dichloromethane/methanol (1:1, 10 ml), the filtrate is concentrated and purified by silica column using (230-400) mesh to get the product as orange solid (0.024 g, 16.62 %);
TLC: chloroform/methanol (9.5/0.5) R*_{f}* - 0.3;
¹H NMR: 400 MHz, DMSO-d₆: δ [ppm] 9.76 (br s, 1H), 9.07 (s, 1H), 9.03 (dd, J = 1.76, 4.22 Hz, 1 H), 8.78 (d, J = 2.00 Hz, 1 H), 8.46-8.53 (m, 3H), 8.22-8.24 (m, 1H), 7.65-7.68 (m, 1 H), 7.59 (dd, J = 2.12, 8.02 Hz, 2H), 6.90-6.92 (m, 2H), 3.72 (t, J = 4.92 Hz, 4H), 3.01 (t, J = 4.84 Hz, 4H);
LCMS: Mass found (M+, 424.0)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 2.00 area % -98.18 (Max), 98.12 (254 nm).

### HPLC

Method: A- A-0.1% TFA in H₂O, B- % TFA in ACN: Flow - 2.0 ml/min.
Column: XBridge C8 (50 X 4.6 mm, 3.5 µm)
Rt (min): 1.99 area % - 99.54 (Max), 99.53 (254 nm).

### Example 16

### Synthesis of 2-methyl-2-{4-[2-(4-morpholin-4-yl-phenylamino)-[1,2,4]triazolo[1,5-a]pyrazin-5-yl]-phenyl}-propionitrile ("A34")

### 16.1 2-[4-(6-amino-pyrazin-2-yl)-phenyl]-2-methyl-propionitrile

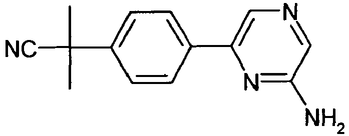

To a solution of 2-amino-6-chloro-pyrazine (0.5 g, 3.86 mmol) in toluene / ethanol (9:1, 10 ml), 4-(1-cyano-1-methylethyl)phenyl boronic acid (0.8 g, 4.24 mmol), tetrakis(triphenylphosphine)palladium(0) (0.13 g, 0.11 mmol) and cesium carbonate (2.5 g, 7.72 mmol) are added, degassed briefly and irradiated in microwave at 130 °C for an hour. The reaction mixture is passed through celite, washed with dichloromethane/methanol (1:1, 25 ml), the filtrate is concentrated and purified by silica column using (230-400) mesh to get the product as yellow solid (0.63 g, 69.28 %);
TLC: chloroform/methanol (9.5/0.5) R*_{f}* - 0.2;
¹H NMR: 400 MHz, DMSO-d₆: δ [ppm] 8.29 (s, 1H), 8.03 (dd, J = 1.92, 6.68 Hz, 2H), 7.85 (s, 1 H), 7.61 (dd, J = 1.88, 6.68 Hz, 2H), 6.54 (br s, 2H), 1.70 (s, 6H);
LCMS: Mass found (M+, 239.3)
Method: A-0.1 % TFA in H₂O, B-0.1% TFA in ACN: Flow - 2.0 ml/min.
Column: XBridge C8 (50 X 4. 6mm, 3.5 µm), +ve mode
Rt (min): 3.06 area % -93.86 (Max), 99.39 (254 nm).

### 16.2 2-[4-(2-amino-[1,2,4]triazolo[1,5-a]pyrazin-5-yl)-phenyl]-2-methylpropionitrile

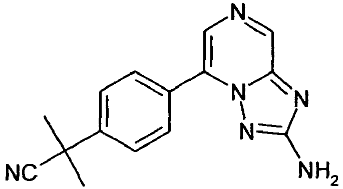

To a solution of 2-[4-(6-amino-pyrazin-2-yl)-phenyl]-2-methyl-propionitrile (0.63 g, 2.67 mmol) in dry tetrahydrofuran (20 ml), ethoxycarbonylisothiocyanate (0.38 g, 2.93 mmol) is added and heated to 50 °C for 12 hours. The reaction mixture is concentrated and suspended in ethanol/methanol (1:1, 50 ml), hydroxylamine hydrochloride (1.5 g, 21.68 mmol) and diisopropylethylamine (1.67 g, 12.97 mmol) are added and heated to 80 °C for 3 hours. The reaction mixture is concentrated and the crude mass is taken in water and filtered, washed with water, cold acetonitrile and diethylether to get the product as pale yellow solid (0.15 g, 20.21%);
TLC: chloroform/methanol (9.5/0.5) R*_{f}* - 0.3;
¹H NMR: 400 MHz, DMSO-d₆: δ [ppm] 8.83 (s, 1 H), 8.20 (s, 1 H), 8.08 (dd, J = 1.96, 6.62 Hz, 2H), 7.71 (dd, J = 1.92, 6.66 Hz, 2H), 6.53 (br s, 2H), 1.75 (s, 6H);
LCMS: Mass found (M+, 279.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.06 area % -96.70 (Max), 95.39 (254 nm).

### 16.3 2-Methyl-2-{4-[2-(4-morpholin-4-yl-phenylamino)-[1,2,4]triazolo[1,5-a]pyrazin-5-yl]-phenyl}-propionitrile ("A34")

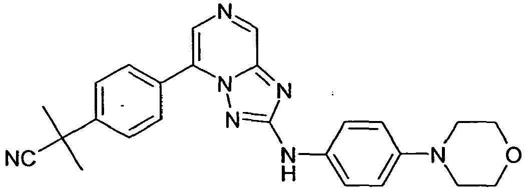

To a solution of 2-[4-(2-amino-[1,2,4]triazolo[1,5-a]pyrazin-5-yl)-phenyl]-2-methyl-propionitrile (0.1 g, 0.36 mmol) in dry *tert*-butanol (5 ml), 4-(4-chlorophenyl)-morpholine (0.106 g, 0.54 mmol), 2-dicyclohexylphosphino-2'-(N,N-dimethylamino)biphenyl (0.014 g, 0.03 mmol), tris(dibenzelideneacetone)-dipalladium(0) (0.014 g, 0.01 mmol) and sodiumhexamethyldisilylamide (1M/THF) (0.54 ml) are added, degassed briefly and irradiated in microwave 150 °C for 30 minutes. The reaction mixture is filtered through celite washed with dichloromethane/methanol (1:1, 10 ml), the filtrate is concentrated and purified by silica column using (230-400) mesh to get the product as yellow solid (0.04 g, 25.39 %); TLC: chloroform/methanol (9.5/0.5) R*_{f}* - 0.3;
¹H NMR: 400 MHz, DMSO-d₆: δ [ppm] 9.73 (s, 1H), 9.02 (s, 1 H), 8.33 (s, 1H), 8.17 (dd, J = 1.96, 6.70 Hz, 2H), 7.76 (dd, J = 1.92, 6.66 Hz, 2H), 7.57 (dd, J = 2.08, 7.02 Hz, 2H), 6.92 (d, J = 9.12 Hz, 2H), 3.73 (t, J = 4.92 Hz, 4H), 3.01 (t, J = 4.84 Hz, 4H), 1.77 (s, 6H);
LCMS: Mass found (M+, 440.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.34 area % -98.72 (Max), 99.21 (254 nm);

### HPLC

Method: A- A-0.1% TFA in H₂O, B- % TFA in ACN: Flow - 2.0 ml/min.
Column: XBridge C8 (50 X 4.6 mm, 3.5 µm)
Rt (min): 3.32 area % - 99.05 (Max), 99.61 (254 nm).

### Example 17

### Synthesis of [5-(1-methyl-1H-pyrazol-4-yl)-[1,2,4]triazolo[1,5-a]pyrazin-2-yl]-(4-morpholin-4-yl-phenyl)-amine ("A35")

### 17.1 6-(1-methyl-1 H-pyrazol-4-yl)-pyrazin-2-ylamine

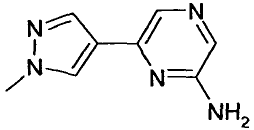

To a solution of 2-amino-6-chloro pyrazine (0.5 g, 3.86 mmol) in toluene / ethanol (9:1, 10 ml), 1-methyl-1H-pyrazole-4-boronic acid pinacol ester (0.8 g, 4.24 mmol), tetrakis(triphenylphosphine)palladium(0) (0.13 g, 0.11 mmol) and cesium carbonate (2.5 g, 7.72 mmol) are added, degassed briefly and irradiated in microwave at 130 °C for an hour. The reaction mixture is passed through celite, washed with dichloromethane/methanol (1:1, 25 ml), the filtrate is concentrated and purified by silica column using (230-400) mesh to get the product as yellow solid (0.51 g, 75.44 %);
TLC: chloroform/methanol (9.5/0.5) R*_{f}* - 0.3;
¹H NMR: 400 MHz, DMSO-d₆: δ [ppm] 8.16 (s, 1H), 8.00 (s, 1H), 7.89 (s, 1H), 7.65 (s, 1 H), 6.32 (br s, 2H), 3.86 (s, 3H);
LCMS: Mass found (M+, 176.0)
Method: A-0.1% TFA in H₂O, B-0.1% TFA in ACN: Flow - 0.6 ml/min.
Column: XBridge C8 (50 X 4.6 mm, 3.5 µm), +ve mode
Rt (min): 1.09 area % -96.86 (Max).

### 17.2 5-(1-Methyl-1H-pyrazol-4-yl)-[1,2,4]triazolo[1,5-a]pyrazin-2-ylamine

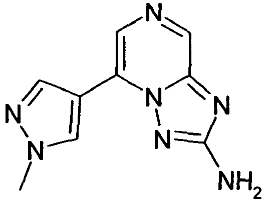

To a solution of 6-(1-methyl-1H-pyrazol-4-yl)-pyrazin-2-ylamine (0.51 g, 2.94 mmol) in dry tetrahydrofuran (30 ml), ethoxycarbonylisothiocyanate (0.42 g, 3.23 mmol) is added and heated to 50 °C for 12 hours. The reaction mixture is concentrated and suspended in ethanol/methanol (1:1, 50 ml), hydroxylamine hydrochloride (1.02 g, 14.72 mmol) and diisopropylethylamine (1.1 g, 8.83 mmol) are added and heated to 80 °C for 3 hours. The reaction mixture is concentrated and the crude mass is taken in water and filtered, washed with water, cold acetonitrile and diethylether to get the product as pale yellow solid (0.05 g, 8 %); TLC: chloroform/methanol (9.5/0.5) R*_{f}* - 0.2;
¹H NMR: 400 MHz, DMSO-d₆: δ [ppm] 8.77 (s, 1 H), 8.65 (s, 1 H), 8.49 (s, 1 H), 8.46 (s, 1 H), 6.53 (s, 2H), 3.96 (s, 3H);

### 17.3 [5-(1-methyl-1H-pyrazol-4-yl)-[1,2,4]triazolo[1,5-a]pyrazin-2-yl]-(4-morpholin-4-yl-phenyl)-amine ("A35")

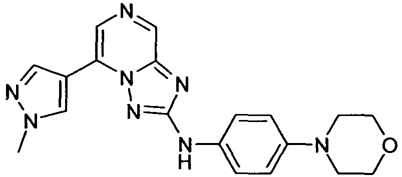

To a solution of 5-(1-methyl-1H-pyrazol-4-yl)-[1,2,4]triazolo[1,5-a]pyrazin-2-ylamine(0.05 g, 0.23 mmol) in dry *tert*-butanol (5 ml), 4-(4-chlorophenyl)-morpholine (0.068 g, 0.34 mmol), 2-dicyclohexylphosphino-2'-(N,N-dimethyl-amino)biphenyl (0.009 g, 0.023 mmol), tris(dibenzelideneacetone)-dipalladium(0) (0.009 g, 0.01 mmol) and sodiumhexamethyldisilylamide (1M/THF) (0.35 ml) are added, degassed briefly and irradiated in microwave 150°C for an hour. The reaction mixture is filtered through celite washed with dichloromethane/methanol (1:1, 10 ml), the filtrate is concentrated and purified by silica column using (230-400) mesh to get the product as yellow solid (0.012 g, 13.8 %); TLC: chloroform/methanol (9.5/0.5) R*_{f}* - 0.3;
¹H NMR: 400 MHz, DMSO-d₆: δ [ppm] 9.70 (s, 1H), 8.83 (s, 1H), 8.81 (s, 1H), 8.59 (s, 1H), 8.52 (s, 1H), 7.61 (dd, J = 2.04, 7.02 Hz, 2H), 6.98 (d, J = 9.08 Hz, 2H), 4.01 (s, 3H), 3.74 (t, J = 4.88 Hz, 4H), 3.04 (t, J = 4.80 Hz, 4H); LCMS: Mass found (M+, 377.3)
Method: A-10 mM NH₄HCO₃, B-ACN: Flow - 1.0 ml/min.
Column: XBridge C8 (50 X 4.6 mm, 3.5 µm), +ve mode
Rt (min): 4.13 area % -96.16 (Max), 97.66 (254 nm);

### HPLC

Method: A- A-0.1% TFA in H₂O, B- % TFA in ACN: Flow - 2.0 ml/min.
Column: XBridge C8 (50 X 4.6 mm, 3.5 µm)
Rt (min): 2.19 area % - 97.26 (Max), 96.49 (254 nm).

### Example 18

### Synthesis of (5-biphenyl-2-yl-[1,2,4]triazolo[1,5-a]pyrazin-2-yl)-(4-morpholin-4-yl-phenyl)-amine ("A36")

### 18.1 6-biphenyl-2-yl-pyrazin-2-ylamine

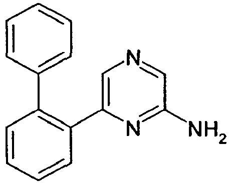

To a solution of 2-amino-6-chloro-pyrazine (0.25 g, 1.93 mmol) in toluene / ethanol (9:1, 10 ml), 2-biphenyl boronic acid (0.42 g, 2.13 mmol), tetrakis(triphenylphosphine)palladium(0) (0.067 g, 0.05 mmol) and cesium carbonate (1.25 g, 3.87 mmol) are added, degassed briefly and irradiated in microwave at 130 °C for an hour. The reaction mixture is passed through celite, washed with dichloromethane/methanol (1:1, 25 ml), the filtrate is concentrated and purified by silica column using (230-400) mesh to get the product as yellow solid (0.3 g, 63.02 %);
TLC: chloroform/methanol (9.5/0.5) R*_{f}* - 0.3.
¹H NMR: 400 MHz, DMSO-d₆: δ [ppm] 7.68 (s, 1 H), 7.58-7.55 (m, 1 H), 7.44-7.51 (m, 2H), 7.39-7.41 (m, 1H), 7.23-7.32 (m, 3H), 7.12-7.15 (m, 3H), 6.42 (br s, 2H);
LCMS: Mass found (M+, 248.3)
Method: A-0.1% TFA in H₂O, B-0.1% TFA in ACN: Flow - 0.6 ml/min.
Column: XBridge C8 (50 X 4.6 mm, 3.5 µm), +ve mode
Rt (min): 3.54 area % -98.72 (Max).

### 18.2 5-biphenyl-2-yl-[1,2,4]triazolo[1,5-a]pyrazin-2-ylamine

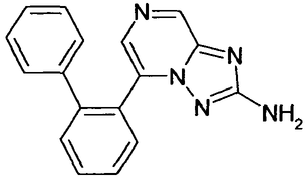

To a solution of 6-biphenyl-2-yl-pyrazin-2-ylamine (0.3 g, 1.21 mmol) in dry tetrahydrofuran (30 ml), ethoxycarbonylisothiocyanate (0.17 g, 1.33 mmol) is added and heated to 50 °C for 12 hours. The reaction mixture is concentrated and suspended in ethanol/methanol (1:1, 50 ml), hydroxylamine hydrochloride (0.54 g, 7.94 mmol) and diisopropylethylamine (0.61 g, 4.74 mmol) are added and heated to 80 °C for 3 hours. The reaction mixture is concentrated and the crude mass is taken in water and filtered, washed with water, cold acetonitrile and diethylether to get the product as pale yellow solid (0.3 g, 63.02 %); TLC: chloroform/methanol (9.5/0.5) R*_{f}* - 0.3;
¹H NMR: 400 MHz, DMSO-d₆: δ [ppm] 8.70 (s, 1 H), 7.62-7.67 (m, 3H), 7.54-7.57 (m, 2H), 7.19 (dd, J = 2.88, 6.30 Hz, 3H), 7.05-7.08 (m, 2H), 6.42 (br s, 2H);
LCMS: Mass found (M+, 288.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.55 area % -78.24 (Max).

### 18.3 (5-biphenyl-2-yl-[1,2,4]triazolo[1,5-a]pyrazin-2-yl)-(4-morpholin-4-yl-phenyl)-amine ("A36")

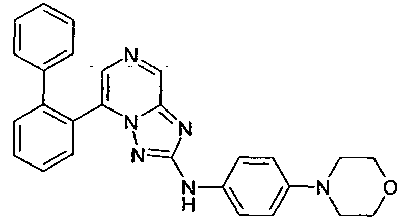

To a solution 5-biphenyl-2-yl-[1,2,4]triazolo[1,5-a]pyrazin-2-ylamine (0.046 g, 0.16 mmol) in dry *tert*-butanol (5 ml), 4-(4-chlorophenyl)-morpholine (0.047 g, 0.24 mmol), 2-dicyclohexylphosphino-2'-(N,N-dimethylamino)biphenyl (0.006 g, 0.016 mmol), tris(dibenzelideneacetone)dipalladium(0) (0.006 g, 0.006 mmol) and sodiumhexamethyldisilylamide (1M/THF) (0.25 ml) are added, degassed briefly and irradiated in microwave 150 °C for an hour. The reaction mixture is filtered through celite washed with dichloromethane/methanol (1:1, 10 ml), the filtrate is concentrated and purified by silica column using (230-400) mesh to get the product as yellow solid (0.009 g, 11.76 %);
TLC: chloroform/methanol (9.5/0.5) R*_{f}* - 0.3;
¹H NMR: 400 MHz, DMSO-d₆: δ [ppm] 9.58 (s, 1H), 8.90 (s, 1H), 7.86 (s, 1H), 7.67-7.73 (m, 2H), 7.57-7.61 (m, 2H), 7.42 (d, J = 9.04 Hz, 2H), 7.12-7.17 (m, 5H), 6.88 (d, J = 9.08 Hz, 2H), 3.72 (t, J = 4.96 Hz, 4H), 3.00 (t, J = 4.84 Hz, 4H);
LCMS: Mass found (M+, 449.3)
Method: A-0.1% TFA in H₂O, B-0.1% TFA in ACN: Flow - 2.0 ml/min.
Column: XBridge C8 (50 X 4.6 mm, 3.5 µm), +ve mode
Rt (min): 3.64 area % -94.69 (Max), 96.26 (254 nm).

### HPLC

Method: A- A-0.1% TFA in H₂O, B- % TFA in ACN: Flow - 2.0 ml/min.
Column: XBridge C8 (50 X 4.6 mm, 3.5 µm)
Rt (min): 3.74 area % - 96.04 (Max), 97.10 (254 nm).

The following compound is prepared analogously

### (5-biphenyl-2-yl-[1,2,4]triazolo[1,5-a]pyrazin-2-yl)-(3,5-dimethoxy-phenyl)-amine ("A37")

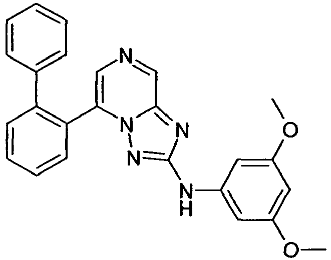

¹H NMR: 400 MHz, DMSO-d₆: δ [ppm] 9.85 (s, 1H), 8.96 (s, 1H), 7.88 (s, 1H), 7.75-7.77 (m, 1H), 7.66-7.68 (m, 1H), 7.57-7.61 (m, 2H), 7.10-7.16 (m, 5H), 6.85 (d, J = 2.16 Hz, 2H), 6.06 (t, J = 2.08 Hz, 1 H), 3.65 (s, 6H).

### Example 19

### Synthesis of 5-[5-(1-methyl-1H-indazol-5-yl)-[1,2,4]triazolo[1,5-a]pyrazin-2-ylamino]-1,3-dihydro-indol-2-one ("A38")

### 19.1 5-(1-methyl-1H-indazol-5-yl)-[1,2,4]triazolo[1,5-a]pyrazin-2-ylamine

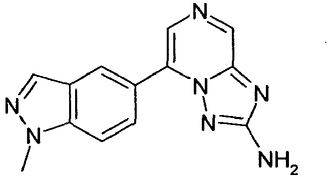

To a solution of 5-chloro-[1,2,4]triazolo[1,5-a]pyrazin-2-ylamine (2.0 g, 11.8 mmol) in 1,4-dioxane/water (9:1, 50 mL), 1-methyl-1H-indazole-5yl-boronic acid (3.1 g, 17.7 mmol), 2-dicyclohexylphosphino-2',4',6'-triisopropylbiphenyl (0.4 g, 0.7 mmol)), palladium acetate (0.08 g, 0.35 mmol) and potassium carbonate (4.9 g, 35.4 mmol) are added and heated in a pressure tube at 110 °C for 10 h. After completion of the reaction (monitored by TLC), the reaction mixture is passed through celite, washed with dichloromethane/methanol (1:1, 75 mL), the filtrate is concentrated to get the crude product. The crude product is purified by column chromatography (silica gel, MEOH/DCM gradient elution); yield: 42 % (1.3 g, yellow solid);
LCMS: (method A) 266.2 (M+H), RT. 2.2 min, 80.3 % (Max), 92.7 % (254 nm); ¹H NMR (400 MHz, DMSO-d₆): δ [ppm] 8.80 (s, 1H), 8.54 (s, 1H), 8.24-8.21 (m, 2H), 8.03-8.01 (m, 1 H), 7.82 (d, J = 8.84 Hz, 1 H), 6.53 (br s, 2H), 4.11 (s, 3H).

### 19.2 5-[5-(1-methyl-1H-indazol-5-yl)-[1,2,4]triazolo[1,5-a]pyrazin-2-ylamino]-1,3-dihydro-indol-2-one ("A38")

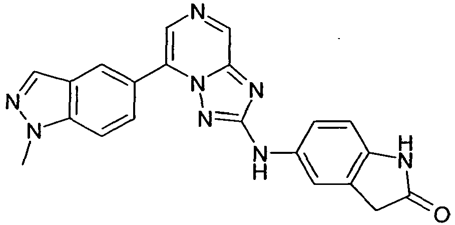

To a solution of 5-(1-methyl-1H-indazol-5-yl)-[1,2,4]triazolo[1,5-a]pyrazin-2-ylamine (250 mg, 0.94 mmol) in dry *tert*-butanol (8 mL), 5-bromo-2-oxindole (298 mg, 1.41 mmol), 2-(dicyclohexylphosphino)-3,6-dimethoxy-2'-4'-6'-tri-i-propyl-1,1'-biphenyl (50 mg, 0.09 mmol), tris(dibenzelideneacetone)-dipalladium(0 (35 mg, 0.037 mmol) and sodiumhexamethyldisilylamide (1 M / THF) (3 mL) are added, degassed briefly and irradiated in microwave 150 °C for 4 h. After completion of the reaction (monitored by TLC), the reaction mixture is filtered through celite, washed with dichloromethane/methanol (1:1, 25 mL), the filtrate is concentrated and purified by flash column chromatography (silica gel, MEOH/DCM gradient elution); yield: 3 % (13 mg, yellow solid); LCMS: (Method A) 397.2 (M+H), RT. 2.9 min, 92.1 % (Max), 92.7 % (254 nm);
¹H NMR (400 MHz, DMSO-d₆): δ [ppm] 10.21 (s, 1 H), 9.81 (s, 1H), 9.00 (s, 1H), 8.64 (d, *J =* 0.7 Hz, 1H), 8.38 (s, 1 H), 8.26 (d, *J* = 0.7 Hz, 1 H), 8.11 (dd, *J* = 8.8, 1.68 Hz, 1H), 7.87 (d, *J =* 8.8 Hz, 1 H), 7.68 (s, 1H), 7.47 (dd, J= 2.2, 8.3 Hz, 1 H), 6.74 (d, *J* = 8.4 Hz, 1 H), 4.13 (s, 3H), 3.48 (s, 2H);
HPLC: (method A) RT 3.1 min, 96.3 % (Max), 94.9 % (254 nm).

### Example 20

### Synthesis of 3-[5-(1-methyl-1H-indazol-5-yl)-[1,2,4]triazolo[1,5-a]pyrazin-2-ylamino]-benzenesulfonamide ("A39")

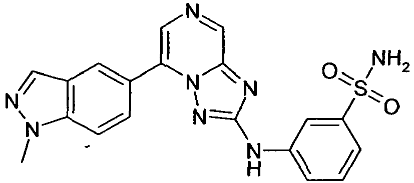

To a solution of 5-(1-methyl-1H-indazol-5-yl)-[1,2,4]triazolo[1,5-a]pyrazin-2-ylamine (250 mg, 0.94 mmol) in dry *tert*-butanol (8 mL), 3-chlorobenzene-sulphonamide (271 mg, 1.41 mmol), 2-dicyclohexylphosphino-2'-(N,N-dimethylamino)biphenyl (37 mg, 0.09 mmol), tris(dibenzelideneacetone)-dipalladium(0) (36 mg, 0.039 mmol) and sodiumhexamethyldisilylamide (1M / THF) (3 mL) are added, degassed briefly and irradiated in microwave 150 °C for 2 h. After completion of the reaction (monitored by TLC), the reaction mixture is filtered through celite washed with dichloromethane/methanol (1:1, 25 mL), the filtrate is concentrated and purified by flash column chromatography (silica gel, MEOH/DCM gradient elution); yield: 4 % (15 mg, off white solid); LCMS: (method A) 421.0 (M+H), RT. 3.1 min, 99.0 % (Max), 99.0 % (254 nm);
¹H NMR (400 MHz, DMSO-d₆): δ [ppm] 10.36 (s, 1H), 9.07 (s, 1H), 8.70 (s, 1H), 8.46 (s, 1 H), 8.34 (t, *J* = 1.9 Hz, 1 H), 8.27 (s, 1 H), 8.15 (dd, *J* = 8.9, 1.6 Hz, 1H), 7.88-7.83 (m, 2H), 7.51-7.47 (m, 1H), 7.39-7.38 (m, 1H), 7.32 (br s, 2H), 4.13 (s, 3H);
HPLC: (method A) RT 3.0 min, 98.5 % (Max), 97.9 % (254 nm).

### Example 21

### Synthesis of 2-methyl-2-(4-{2-[3-methyl-4-(4-methyl-piperazin-1-yl)-phenylamino]-[1,2,4]triazolo[1,5-a]pyrazin-5-yl}-phenyl)-propionitrile ("A40")

### 21.1 2-[4-(6-amino-pyrazin-2-yl)-phenyl]-2-methyl-propionitrile

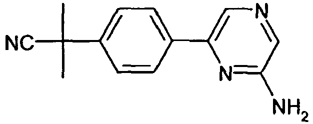

To a solution of 2-amino-6-chloro-pyrazine (0.5 g, 3.86 mmol) in toluene/ethanol (9:1, 10 mL), 4-(1-cyano-1-methylethyl)phenyl boronic acid (0.8 g, 4.24 mmol), tetrakis(triphenylphosphine)palladium(0) (0.13 g, 0.11 mmol) and cesium carbonate (2.5 g, 7.72 mmol) are added, degassed briefly and irradiated in microwave at 130 °C for for 1h. After completion of the reaction (monitored by TLC), the reaction mixture is passed through celite, washed with dichloromethane/methanol (1:1, 25ml), the filtrate is concentrated to get the crude product. The crude product is purified by column chromatography (silica gel, PE/EA gradient elution); yield: 69 % (0.63 g, yellow solid);
¹H NMR (400 MHz, DMSO-d₆): δ [ppm] 8.29 (s, 1H), 8.03 (dd, *J* = 6.6, 1.9 Hz, 2H), 7.85 (s, 1H), 7.61 (dd, *J* = 6.6, 1.8 Hz, 2H), 6.54 (br s, 2H), 1.70 (s, 6H); LCMS: (method A) 239.3 (M+H), RT. 3.0 min, 93.8 % (Max), 99.3 % (254 nm).

### 21.2 2-[4-(2-Amino-[1,2,4]triazolo[1,5-a]pyrazin-5-yl)-phenyl]-2-methyl-propionitrile

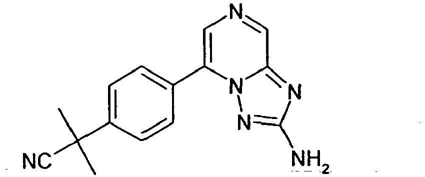

To a solution of 2-[4-(6-amino-pyrazin-2-yl)-phenyl]-2-methyl-propionitrile (0.63 g, 2.67 mmol) in dry tetrahydrofuran (20 mL), ethoxycarbonylisothiocyante (0.38 g, 2.93 mmol) is added and heated to 50 °C for 12 h. After completion of the reaction (monitored by TLC), the reaction mixture is concentrated and suspended in ethanol/methanol (1:1, 50 mL), hydroxylamine hydrochloride (1.5 g, 21.6 mmol) and diisopropylethylamine (2.3 mL, 12.9 mmol) are added and heated to 80° C for 3 hours. After completion of the reaction (monitored by TLC), the reaction mixture is concentrated and the crude mass is taken in water, triturated and filtered, washed with water (2 × 10 mL), acetonitrile (1 × 10 mL), diethylether (2 × 10 mL) to get the product; yield: 20 % (0.15 g, yellow solid);
¹H NMR (400 MHz, DMSO-d₆): δ [ppm] 8.83 (s, 1H), 8.20 (s, 1H), 8.08 (dd, *J* = 6.6, 1.9 Hz, 2H), 7.71 (dd, *J* = 6.6, 1.9 Hz, 2H), 6.53 (br s, 2H), 1.75 (s, 6H); LCMS: (method A) 279.3 (M+H), RT. 3.0 min, 96.7 % (Max), 95.3 % (254 nm).

### 21.3 2-methyl-2-(4-{2-[3-methyl-4-(4-methyl-piperazin-1-yl)-phenylamino]-[1,2,4]triazolo[1,5-a]pyrazin-5-yl}-phenyl)-propionitrile ("A40")

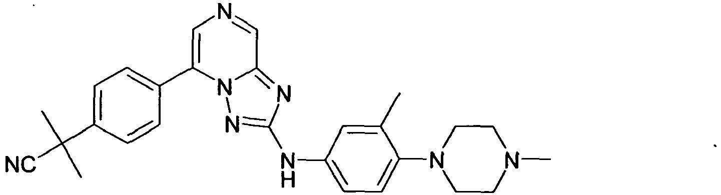

The compound is prepared analogously to "A1"; yield: 16 % (49 mg, pale yellow solid);
LCMS: (method A) 467.2 (M+H), RT. 3.5 min, 97.6 % (Max), 97.2 % (254 nm); ¹H NMR (400 MHz, DMSO-d₆): δ [ppm] 9.77 (s, 1 H), 9.04 (s, 1 H), 8.35 (s, 1 H), 8.18 (dd, *J* = 6.7, 1.8 Hz, 2H), 7.78-7.76 (m, 2H), 7.55 (d, *J* = 2.4 Hz, 1H), 7.44 (dd, *J* = 8.6, 2.5 Hz, 1H), 6.98 (d, *J* = 8.7 Hz, 1H), 2.77-2.79 (m, 4H), 2.50 (s, 3H), 2.26-2.24 (m, 4H), 2.23 (s, 3H), 1.77 (s, 6H);
HPLC: (Method A) RT 3.4 min, 96.0 % (Max), 96.3 % (254 nm).

The following compounds are prepared analogously

### 5-[5-(1-methyl-1H-pyrazol-4-yl)-[1,2,4]triazolo[1,5-a]pyrazin-2-ylamino]-1,3-dihydro-indol-2-one ("A41")

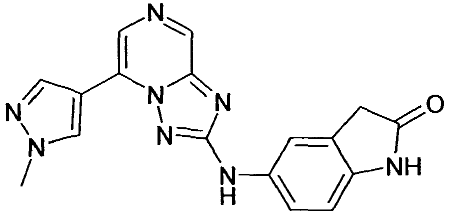

Yield: 21 % (67 mg, yellow solid);
LCMS: (method A) 347.2 (M+H), RT. 2.3 min, 95.0 % (Max), 96.8 % (254 nm); ¹H NMR (400 MHz, DMSO-d₆): δ [ppm] 10.24 (s, 1H), 9.79 (s, 1 H), 8.84 (s, 1 H), 8.82 (s, 1 H), 8.61 (s, 1 H), 8.51 (s, 1H), 7.68 (s, 1 H), 7.51 (dd, *J* = 8.4, 2.2 Hz, 1H), 6.81 (d, *J* = 8.3 Hz, 1H), 4.00 (s, 3H), 3.52 (s, 2H);
HPLC: (method A) RT 2.3 min, 96.1 % (Max), 96.3 % (254 nm).

### 5-(1-methyl-1H-pyrazol-4-yl)-N-(3-methyl-4-(4-methylpiperazin-1-yl)phenyl)-[1,2,4]triazolo[1,5-a]pyrazin-2-amine ("A42")

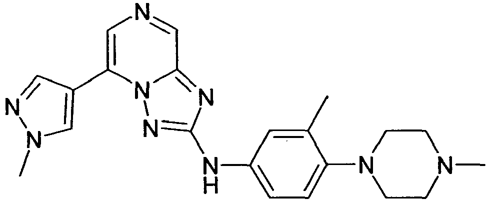

Yield: 22 % (83 mg, brown solid); LCMS: (method A) 404.2 (M+H), RT. 2.4 min, 96.1 % (Max), 97.5 % (254 nm);
¹H NMR (400 MHz, DMSO-d₆): δ [ppm] 9.76 (s, 1 H), 8.84-8.83 (m, 2H), 8.60 (s, 1H), 8.53 (s, 1H), 7.60 (d, *J =* 2.4 Hz, 1 H), 7.48 (dd, *J* = 8.5, 2.5 Hz, 1H), 7.05 (d, *J* = 8.6 Hz, 1 H), 4.01 (s, 3H), 2.83-2.81 (m, 4H), 2.68-2.52 (m, 4H), 2.30-2.28 (m, 6H);
HPLC: (method A) RT 2.7 min, 99.0 % (Max), 99.0 % (254 nm).

### N-(3-(2-((3-methyl-4-(4-methylpiperazin-1-yl)phenyl)amino)-[1,2,4]triazolo[1,5-a]pyrazin-5-yl)phenyl)methanesulfonamide ("A43")

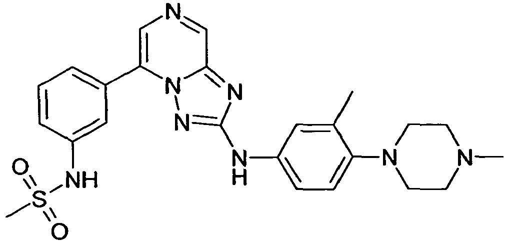

Yield: 22 % (71 mg, yellow solid); LCMS: (Method A) 493.2 (M+H), RT. 2.7 min, 96.6 % (Max), 97.5 % (254 nm);
¹H NMR (400 MHz, DMSO-d₆): δ [ppm] 10.04 (br s, 1 H), 9.75 (s, 1H), 9.03 (s, 1 H), 8.26 (s, 1 H), 7.89 (d, *J* = 1.8 Hz, 1 H), 7.81 (d, *J* = 7.8 Hz, 1 H), 7.58 (t, *J* = 7.8 Hz, 1 H), 7.49-7.46 (m, 2H), 7.42 (dd, *J* = 8.1, 1.3 Hz, 1H), 6.97 (d, *J* = 8.4 Hz, 1 H), 3.07 (s, 3H), 2.86-2.77 (m, 4H), 2.55 (s, 3H), 2.49-2.25 (m, 4H), 2.22 (s, 3H);
HPLC: (Method A) RT 2.8 min, 95.4 % (Max), 97.0 % (254 nm).

### Example 22

### Synthesis of [6-methyl-5-(1-methyl-1H-pyrazol-4-yl)-[1,2,4]triazolo[1,5-a]pyrazin-2-yl]-(4-morpholin-4-yl-phenyl)-amine ("A44")

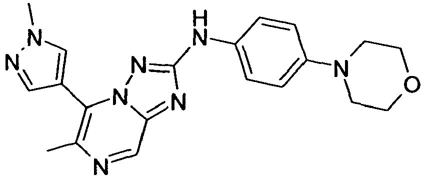

### Step 1

To 2-amino-6-chloropyrazine (1eq) in DMSO and water is added at 0°C NIS (1.1 eq) in 3 portions. The mixture is stirred in the dark for 72 h. The mixture is poured into water, extracted with EtOAc and evaporated. The crude material is purified via flash column chromatography.

### Step 2

6-Chloro-5-iodo-pyrazin-2-ylamine (1eq) is dissolved in dioxane under nitrogen. Ethoxycarbonyl isothiocyanate (1.05 eq) is added and the mixture is stirred for 18h at rt and monitored by HPLC MS. The solvent is removed in vacuum, and the residue washed with cold cyclohexane yielding the desired thiourea as a solid.

### Step 3

Ethyl N-[(6-chloro-5-iodo-pyrazin-2-yl)carbamothioyl]carbamate (1 eq) is given to a mixture of hydroxylammoniumchlorid (5 eq) and N-ethyldiisopropylamine (3 eq) in ethanol. The mixture is stirred for 2 h at rt and then heated to 60 °C for additional 3 h. The reaction is concentrated and the solids are collected by filtration. Washing with cold water and drying in vacuum at 60°C gives 5-chloro-6-iodo-[1,2,4]triazolo[1,5-a]pyrazin-2-ylamine as a solid.

### Step 4

5-Chloro-6-iodo-[1,2,4]triazolo[1,5-a]pyrazin-2-ylamine (1 eq), 2,4,6-trimethyl-cyclotriboroxane (0.8 eq, solution in THF), palladium (II) acetate (0.03 eq), 2-(dicyclohexylphosphino)-2',4',6'-triisopropylbiphenyl (0.1 eq) and potassium carbonate are dissolved in a mixture of acetonitrile and water. The mixture is degassed and heated to 150°C for 1.5 h under microwave irradiation. The solvent is removed in vaccum and the crude material is purified via flash column chromatographie.

### Step 5

5-Chloro-6-methyl-[1,2,4]triazolo[1,5-a]pyrazin-2-ylamine (1eq), 1-methyl-4-(4,4,5,5-tetramethyl-[1,3,2]dioxaborolan-2-yl)-1H-pyrazole (1.5 eq), palladium (II) acetate (0.03 eq), 2-(dicyclohexylphosphino)-2',4',6'-triisopropylbiphenyl (0.1 eq) and potassium carbonate are dissolved in a mixture of acetonitrile and water. The suspension is briefly degassed with nitrogene and heated to 150°C by microwave irradiation for 30 minutes. Upon completion, the mixture is concentrated and purified via flash column chromatography.

### Step 6

6-Methyl-5-(1-methyl-1H-pyrazol-4-yl)-[1,2,4]triazolo[1,5-a]pyrazin-2-ylamine (1 eq.) is dissolved in tert. butanol and 4-(4-chloro-phenyl)-morpholine (1.1 eq) is added. The mixture is briefly degassed with nitrogen before chloro[2-(dicyclo-hexylphosphino)-3,6-dimethoxy-2'-4'-6'-tri-i-propyl-1,1'-biphenyl][2-(2-amino-ethyl)phenyl]Pd(II) (0.014 eq) and LHMDS (2 eq) are added. The mixture is stirred for 2 h at 110°C and monitored by HPLC. Upon completion the reaction is quenched and concentrated. Purification via preparative HPLC gives the title compound as a solid; HPLC-MS purity (method A): 100%, Rt: 1,52 min, observed [M+H] = 391.2;
¹H NMR (400 MHz, DMSO) δ [ppm] 9.57 - 9.52 (s, 1H), 8.83 - 8.78 (s, 1H), 8.54 - 8.49 (s, 1H), 8.24 - 8.19 (d, *J* = 0.8 Hz, 1 H), 7.60 - 7.53 (d, *J* = 9.0 Hz, 2H), 6.96 - 6.89 (d, *J* = 9.0 Hz, 2H), 4.03 - 3.98 (s, 3H), 3.78 - 3.70 (m, 5H), 3.06 - 2.99 (m, 4H), 2.68 - 2.63 (s, 4H).

The following compounds are prepared analogously

### [5-(1-methyl-1H-pyrazol-4-yl)-6-phenyl-[1,2,4]triazolo[1,5-a]pyrazin-2-yl]-(4-morpholin-4-yl-phenyl)-amine ("A45")

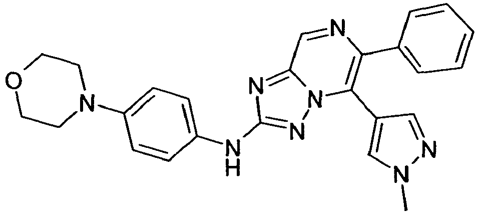

The title compound is synthesized using the methods described for "A44", but using benzene boronic acid in step 4;
HPLC-MS purity (method A): 100%, Rt: 1,95 min, observed [M+H] = 493.2;
¹H NMR (400 MHz, DMSO) δ [ppm] 9.69 - 9.64 (s, 1H), 8.98 - 8.93 (s, 1H), 8.21 - 8.16 (s, 1 H), 7.63 - 7.56 (d, *J* = 8.9 Hz, 2H), 7.53 - 7.39 (m, 6H), 7.30 - 7.25 (s, 1 H), 6.99 - 6.91 (d, *J* = 9.1 Hz, 2H), 6.91 - 6.85 (d, *J* = 5.5 Hz, 1 H), 5.99 - 5.94 (s, 2H), 3.91 - 3.86 (s, 3H), 3.78 - 3.71 (m, 4H), 3.07 - 3.00 (m, 4H).

### [5,6-Bis-(1-methyl-1H-pyrazol-4-yl)-[1,2,4]triazolo[1,5-a]pyrazin-2-yl]-(4-morpholin-4-yl-phenyl)-amine ("A46")

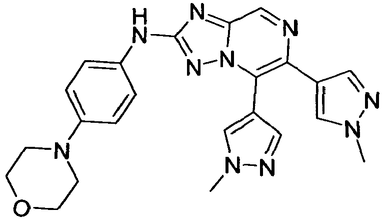

The title compound is synthesized using the methods described for "A44" using 1-methyl-4-(4,4,5,5-tetramethyl-[1,3,2]dioxaborolan-2-yl)-1H-pyrazole in step 4 as coupling partner;
HPLC-MS purity (method A): 100%, Rt: 1,49 min, observed [M+H] = 457.2;
¹H NMR (500 MHz, DMSO) δ [ppm] 9.63 - 9.59 (s, 1H), 8.97 - 8.93 (s, 1H), 8.22 - 8.18 (s, 1H), 7.77 - 7.73 (s, 1 H), 7.71 - 7.67 (s, 1 H), 7.58 - 7.52 (d, *J* = 9.0 Hz, 2H), 7.35 - 7.31 (s, 1 H), 6.95 - 6.89 (d, *J* = 9.1 Hz, 2H), 4.00 - 3.96 (s, 3H), 3.86 - 3.82 (s, 3H), 3.77 - 3.71 (m, 4H), 3.05 - 2.99 (m, 4H).

### 1-{4-[5-(1-methyl-1H-pyrazol-4-yl)-[1,2,4]triazolo[1,5-a]pyrazin-2-ylamino]-phenyl}-cyclopropanecarbonitrile ("A47")

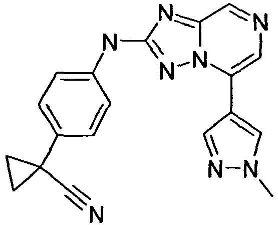

5-(1-Methyl-1H-pyrazol-4-yl)-[1,2,4]triazolo[1,5-a]pyrazin-2-ylamine (1 eq) is coupled under Buchwald Hartwig conditions, using chloro[2-(dicyclohexyl-phosphino)-3,6-dimethoxy-2'-4'-6'-tri-i-propyl-1,1'-biphenyl]2-(2-aminoethyl)-phenyl)Pd(II) (0.05 eq) and LHMDS (2eq) as catalyst and base with 1-(4-chlorophenyl)-1-cyclopropanecarbonitrile (1.5 eq) in tert.- butanol. Usual work up gives the title compound as a solid; HPLC-MS purity (method A): 100%, Rt: 1,90 min, observed [M+H] = 357.2;
¹H NMR (400 MHz, DMSO) δ [ppm] 10.10 - 10.05 (s, 1H), 8.92 - 8.87 (s, 1H), 8.85 - 8.80 (s, 1H), 8.66 - 8.61 (s, 1H), 8.56 - 8.51 (d, *J* = 0.8 Hz, 1H), 7.79 - 7.72 (d, *J* = 8.8 Hz, 2H), 7.38 - 7.31 (d, *J* = 8.7 Hz, 2H), 4.05 - 4.00 (s, 3H), 1.73 - 1.65 (m, 2H), 1.49 - 1.41 (m, 2H).

The following compounds are prepared analogously

| compound nr. | name / structure |
|---|---|
| "A48" | [4-(4-methyl-piperazin-1-yl)-phenyl]-{5-[1-(2-morpholin-4-yl-ethyl)-1 H-pyrazol-4-yl]-[1,2,4]triazolo[1,5-a]pyrazin-2-yl}-amine |
| | 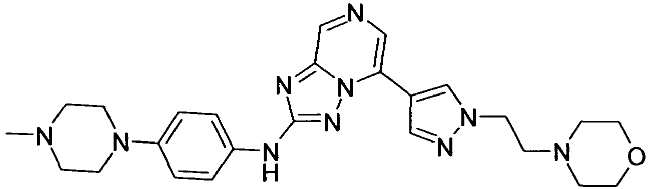 |
| "A49" | 5-{5-[1-(2-morpholin-4-yl-ethyl)-1H-pyrazol-4-yl]-[1,2,4]triazolo[1,5-a]pyrazin-2-ylamino}-1,3-dihydro-indol-2-one |
| | 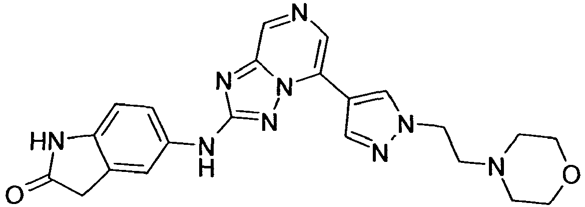 |
| "A50" | [3-methyl-4-(4-methyl-piperazin-1-yl)-phenyl]-{5-[1-(2-morpholin-4-yl-ethyl)-1H-pyrazol-4-yl]-[1,2,4]triazolo[1,5-a]pyrazin-2-yl}-amine |
| | 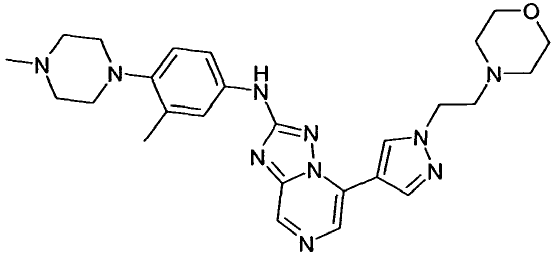 |
| "A51" | {5-[1-(3-methyl-butyl)-1H-pyrazol-4-yl]-[1,2,4]triazolo[1,5-a]pyrazin-2-yl}-[3-methyl-4-(4-methyl-piperazin-1-yl)-phenyl]-amine |
| | 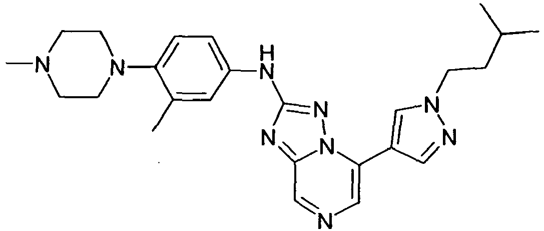 |
| "A52" | 1-methyl-5-{5-[1-(2-morpholin-4-yl-ethyl)-1H-pyrazol-4-yl]-[1,2,4]triazolo[1,5-a]pyrazin-2-ylamino}-1,3-dihydro-indol-2-one |
| | 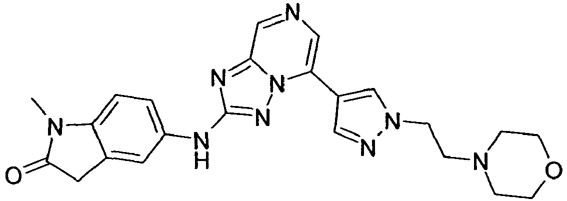 |
| "A53" | N2-[4-(4-methyl-piperazin-1-yl)-phenyl]-N5-(1-methyl-1H-pyrazol-4-yl)-[1,2,4]triazolo[1,5-a]pyrazine-2,5-diamine |
| | 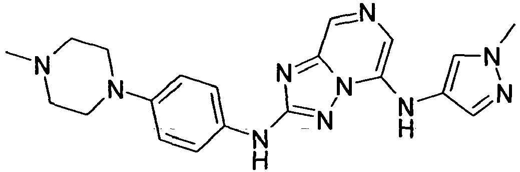 |
| "A54" | 3-{2-[3-methyl-4-(4-methyl-piperazin-1-yl)-phenylamino]-[1,2,4]triazolo[1,5-a]pyrazin-5-yl}-benzamide |
| | 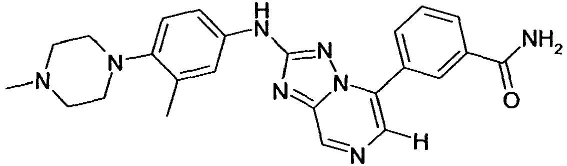 |
| "A55" | [5-(5-methyl-furan-2-yl)-[1,2,4]triazolo[1,5-a]pyrazin-2-yl]-(4-morpholin-4-yl-phenyl)-amine |
| | 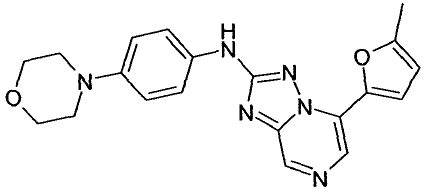 |
| "A56" | {5-[1-(2-morpholin-4-yl-ethyl)-1H-pyrazol-4-yl]-[1,2,4]triazolo[1,5-a]pyrazin-2-yl}-(4-morpholin-4-yl-phenyl)-amine |
| | 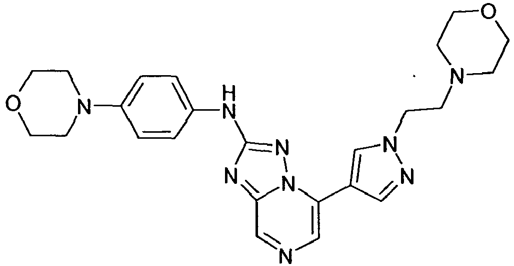 |
| "A57" | 3-[5-(1-methyl-1 H-pyrazol-4-yl)-[1,2,4]triazolo[1,5-a]pyrazin-2-ylamino]-benzonitrile |
| | 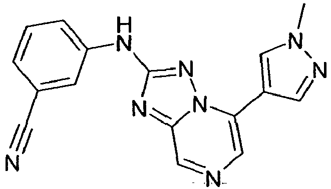 |
| "A58" | 5-[5-(5-morpholin-4-ylmethyl-thiophen-2-yl)-[1,2,4]triazolo[1,5-a]pyrazin-2-ylamino]-1,3-dihydro-indol-2-one |
| | 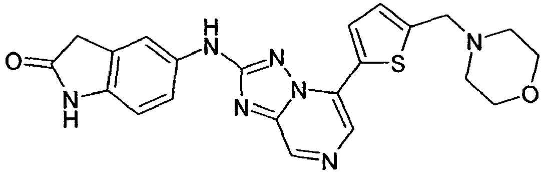 |
| "A59" | (2,3-dihydro-benzo[1,4]dioxin-6-yl)-[5-(1-methyl-1H-pyrazol-4-yl)-[1,2,4]triazolo[1,5-a]pyrazin-2-yl]-amine |
| | 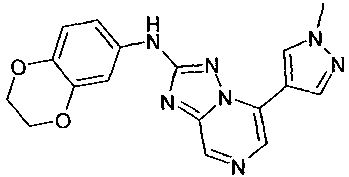 |
| "A60" | [5-(1-methyl-1H-pyrazol-4-yl)-[1,2,4]triazolo[1,5-a]pyrazin-2-yl]-(4-pyridin-4-yl-phenyl)-amine |
| | 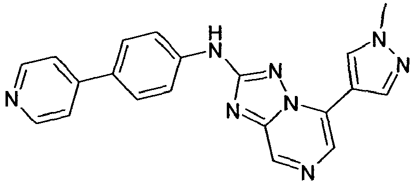 |
| "A61" | [5-(-methyl-1H-pyrazol-4-yl)-[1,2,4]triazolo[1,5-a]pyrazin-2-yl]-(4-morpholin-4-ylmethyl-phenyl)-amine |
| | 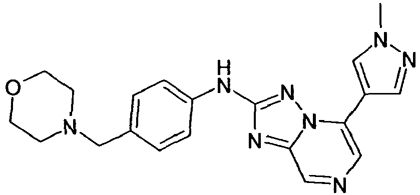 |
| "A62" | [5-(5-morpholin-4-ylmethyl-thiophen-2-yl)-[1,2,4]triazolo[1,5-a]pyrazin-2-yl]-(4-morpholin-4-yl-phenyl)-amine |
| | 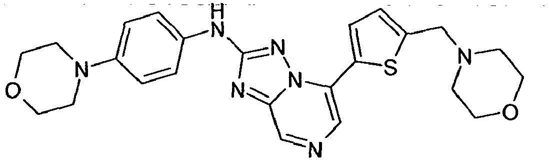 |
| "A63" | 5-[2-(4-morpholin-4-yl-phenylamino)-[1,2,4]triazolo[1,5-a]pyrazin-5-yl]-2-(tetrahydro-pyran-4-yloxy)-benzonitrile |
| | 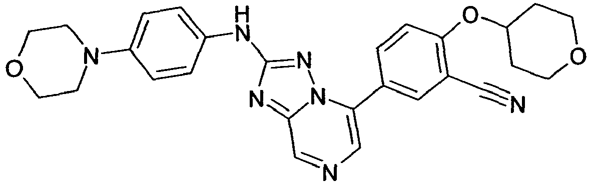 |
| "A64" | [5-(1-methyl-1H-pyrazol-4-yl)-[1,2,4]triazolo[1,5-a]pyrazin-2-yl]-(3-methyl-4-trifluoromethoxy-phenyl)-amine |
| | 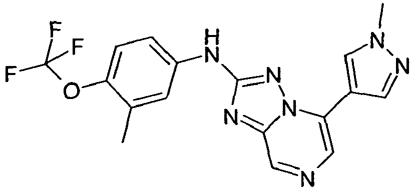 |
| "A65" | cyclobutanecarboxylic acid {2-methyl-4-[5-(1-methyl-1H-pyrazol-4-yl)-[1,2,4]triazolo[1,5-a]pyrazin-2-ylamino]-phenyl}-amide |
| | 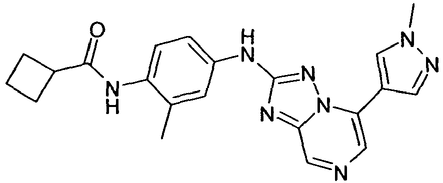 |
| "A66" | {4-[5-(1-methyl-1H-pyrazol-4-yl)-[1,2,4]triazolo[1,5-a]pyrazin-2-ylamino]-phenyl}-morpholin-4-yl-methanone |
| | 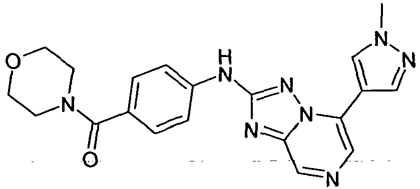 |
| "A67" | [3-methoxy-4-(4-methyl-piperazin-1-yl)-phenyl]-[5-(1-methyl-1 H-pyrazol-4-yl)-[1,2,4]triazolo[1,5-a]pyrazin-2-yl]-amine |
| | 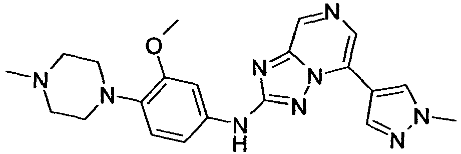 |
| "A68" | (3-methyl-4-morpholin-4-ylmethyl-phenyl)-[5-(1-methyl-1H-pyrazol-4-yl)-[1,2,4]triazolo[1,5-a]pyrazin-2-yl]-amine |
| | 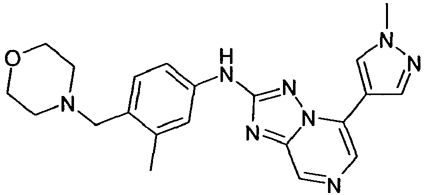 |
| "A69" | N-cyclopropylmethyl-2-methyl-4-[5-(1-methyl-1H-pyrazol-4-yl)-[1,2,4]triazolo[1,5-a]pyrazin-2-ylamino]-benzamide |
| | 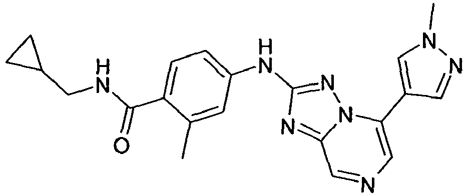 |
| "A70" | N-cyclopropylmethyl-2-methoxy-4-[5-(1-methyl-1H-pyrazol-4-yl)-[1,2,4]triazolo[1,5-a]pyrazin-2-ylamino]-benzamide |
| | 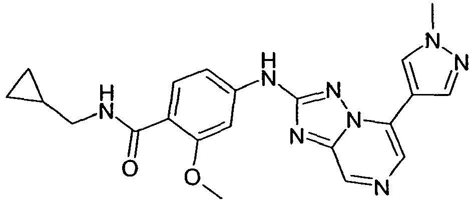 |
| "A71" | N-cyclopropyl-2-methyl-4-[5-(1-methyl-1H-pyrazol-4-yl)-[1,2,4]triazolo[1,5-a]pyrazin-2-ylamino]-benzamide |
| | 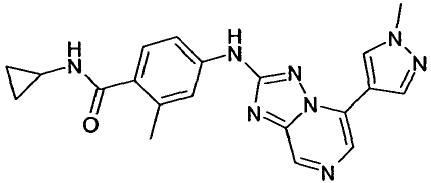 |
| "A72" | N-cyclopropyl-2-methoxy-4-[5-(1-methyl-1H-pyrazol-4-yl)-[1,2,4]triazolo[1,5-a]pyrazin-2-ylamino]-benzamide |
| | 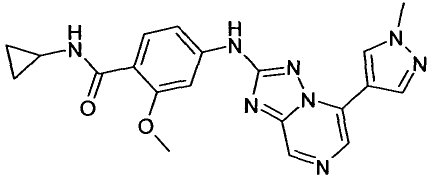 |
| "A73" | {2-methyl-4-[5-(1-methyl-1 H-pyrazol-4-yl)-[1,2,4]triazolo[1,5-a]pyrazin-2-ylamino]-phenyl}-morpholin-4-yl-methanone |
| | 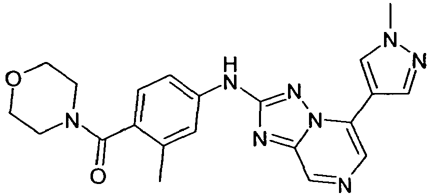 |
| "A74" | piperidine-4-carboxylic acid [5-(1-methyl-1H-pyrazol-4-yl)-[1,2,4]triazolo[1,5-a]pyrazin-2-yl]-amide |
| | 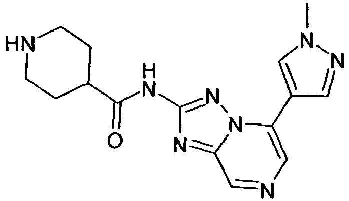 |
| "A75" | [3-methoxy-4-(4-methyl-piperazin-1-yl)-phenyl]-{5-[1-(2-morpholin-4-yl-ethyl)-1H-pyrazol-4-yl]-[1,2,4]triazolo[1,5-a]pyrazin-2-yl}-amine |
| | 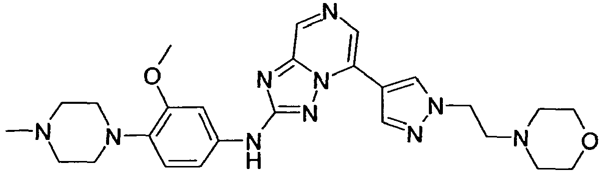 |
| "A76" | [3-methoxy-4-(4-methyl-piperazin-1-yl)-phenyl]-{5-[1-(3-methylbutyl)-1H-pyrazol-4-yl]-[1,2,4]triazolo[1,5-a]pyrazin-2-yl}-amine |
| | 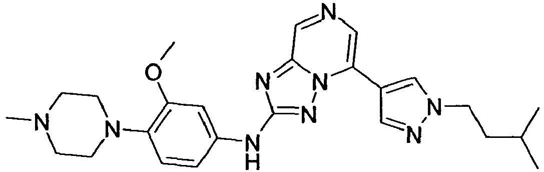 |
| "A77" | [3-methoxy-4-(4-methyl-piperazin-1-yl)-phenyl]-{5-[1-(tetrahydro-pyran-4-yl)-1H-pyrazol-4-yl]-[1,2,4]triazolo[1,5-a]pyrazin-2-yl}-amine |
| | 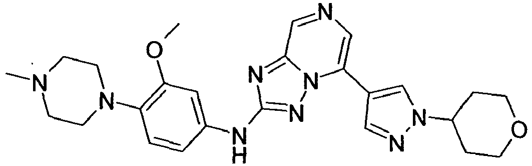 |
| "A78" | (3-methoxy-4-morpholin-4-ylmethyl-phenyl)-[5-(1-methyl-1H-pyrazol-4-yl)-[1,2,4]triazolo[1,5-a]pyrazin-2-yl]-amine |
| | 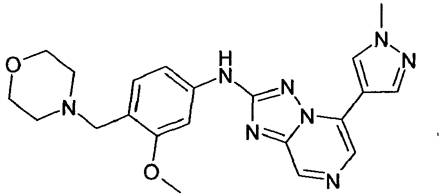 |
| "A79" | [2-methoxy-4-(4-methyl-piperazin-1-yl)-phenyl]-[5-(1-methyl-1H-pyrazol-4-yl)-[1,2,4]triazolo[1,5-a]pyrazin-2-yl]-amine |
| | 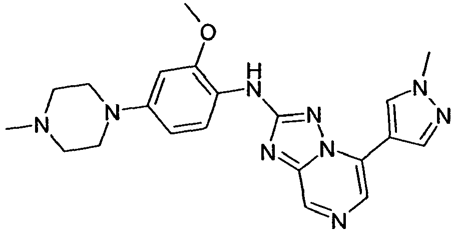 |
| "A80" | [5-(1-methyl-1H-pyrazol-4-yl)-[1,2,4]triazolo[1,5-a]pyrazin-2-yl]-(3-morpholin-4-yl-phenyl)-amine |
| | 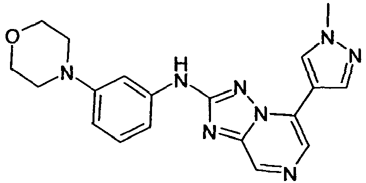 |
| "A81" | N-{2-methyl-4-[5-(1-methyl-1H-pyrazol-4-yl)-[1,2,4]triazolo[1,5-a]pyrazin-2-ylamino]-phenyl}-acetamide |
| | 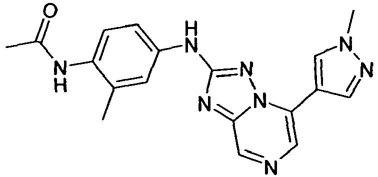 |
| "A82" | piperidine-4-carboxylic acid {2-methyl-4-[5-(1-methyl-1 H-pyrazol-4-yl)-[1,2,4]triazolo[1,5-a]pyrazin-2-ylamino]-phenyl}-amide |
| | 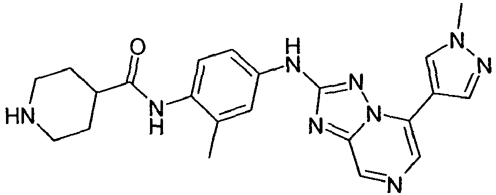 |
| "A83" | tetrahydro-pyran-4-carboxylic acid {2-methyl-4-[5-(1-methyl-1 H-pyrazol-4-yl)-[1,2,4]triazolo[1,5-a]pyrazin-2-ylamino]-phenyl}-amide |
| | 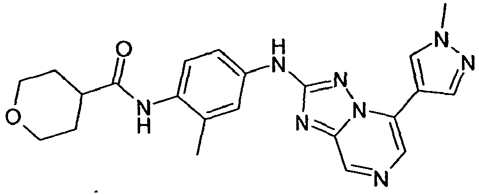 |
| "A84" | piperidine-4-carboxylic acid {2-methoxy-4-[5-(1-methyl-1H-pyrazol-4-yl)-[1,2,4]triazolo[1,5-a]pyrazin-2-ylamino]-phenyl}-amide |
| | 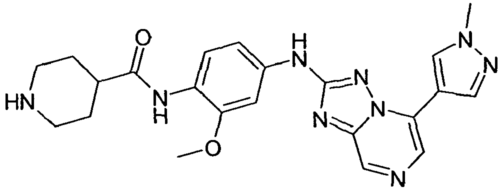 |
| "A85" | N-{2-methoxy-4-[5-(1-methyl-1H-pyrazol-4-yl)-[1,2,4]triazolo[1,5-a]pyrazin-2-ylamino]-phenyl}-acetamide |
| | 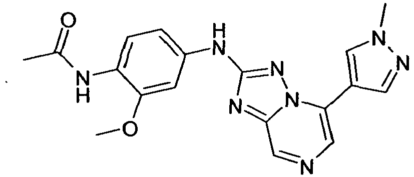 |
| "A86" | 3-{2-[3-methoxy-4-(4-methyl-piperazin-1-yl)-phenylamino]-[1,2,4]triazolo[1,5-a]pyrazin-5-yl}-phenol |
| | 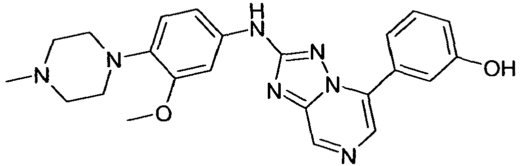 |
| "A87" | 4-{2-[3-methoxy-4-(4-methyl-piperazin-1-yl)-phenylamino]-[1,2,4]triazolo[1,5-a]pyrazin-5-yl}-phenol |
| | 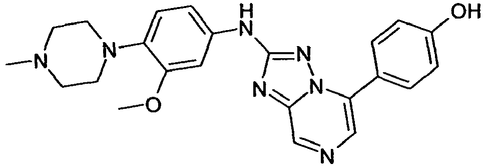 |
| "A88" | [2-(4-methyl-piperazin-1-yl)-pyrimidin-5-yl]-[5-(1-methyl-1H-pyrazol-4-yl)-[1,2,4]triazolo[1,5-a]pyrazin-2-yl]-amine |
| | 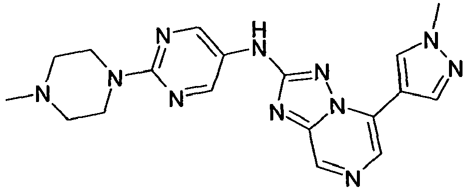 |
| "A89" | tetrahydro-pyran-4-carboxylic acid {2-methoxy-4-[5-(1-methyl-1H-pyrazol-4-yl)-[1,2,4]triazolo[1,5-a]pyrazin-2-ylamino]-phenyl}-amide |
| | 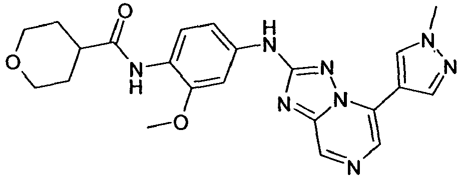 |
| "A90" | [6-(4-methyl-piperazin-1-yl)-pyridin-3-yl]-[5-(1-methyl-1H-pyrazol-4-yl)-[1,2,4]triazolo[1,5-a]pyrazin-2-yl]-amine |
| | 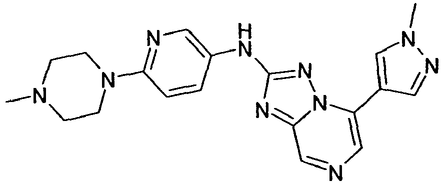 |
| "A91" | [3-methoxy-4-(4-methyl-piperazin-1-yl)-phenyl]-[5-(4-morpholin-4-yl-phenyl)-[1,2,4]triazolo[1,5-a]pyrazin-2-yl]-amine |
| | 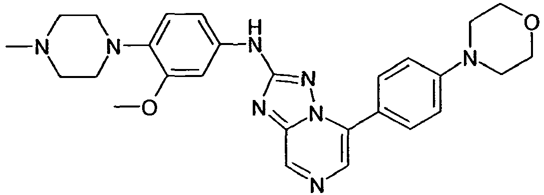 |
| "A92" | [3-methoxy-4-(4-methyl-piperazin-1-yl)-phenyl]-[5-(4-methoxy-phenyl)-[1,2,4]triazolo[1,5-a]pyrazin-2-yl]-amine |
| | 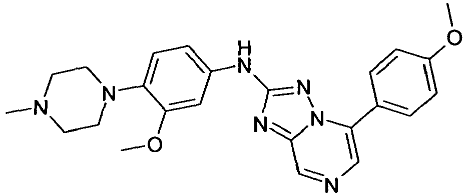 |
| "A93" | N-(4-{2-[3-methoxy-4-(4-methyl-piperazin-1-yl)-phenylamino]-[1,2,4]triazolo[1,5-a]pyrazin-5-yl}-phenyl)-methanesulfonamide |
| | 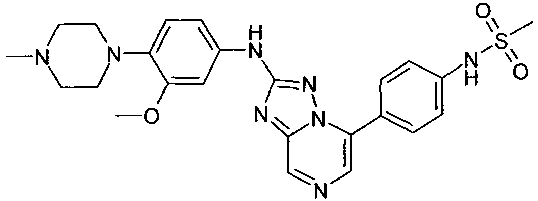 |
| "A94" | 4-{2-[3-methoxy-4-(4-methyl-piperazin-1-yl)-phenylamino]-[1,2,4]triazolo[1,5-a]pyrazin-5-yl}-N-methyl-benzenesulfonamide |
| | 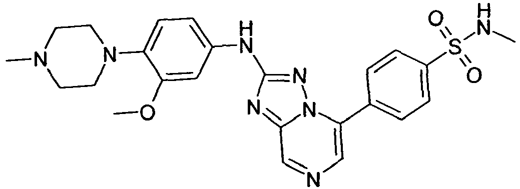 |
| "A95" | N-(3-{2-[3-methoxy-4-(4-methyl-piperazin-1-yl)-phenylamino]-[1,2,4]triazolo[1,5-a]pyrazin-5-yl}-phenyl)-methanesulfonamide |
| | 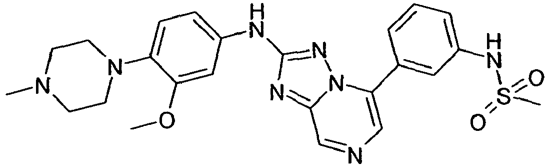 |
| "A96" | 3-{2-[3-methoxy-4-(4-methyl-piperazin-1-yl)-phenylamino]-[1,2,4]triazolo[1,5-a]pyrazin-5-yl}-N-methyl-benzenesulfonamide |
| | 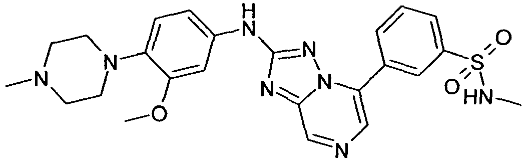 |

| | |
|---|---|
| compound nr. | ¹H NMR; DMSO-d₆; δ [ppm] |
| "A48" | 500 MHz; 9.63 (s, 1H), 8.84 (d, *J* = 25.6 Hz, 2H), 8.56 (d, *J* = 27.7 Hz, 2H), 7.63 - 7.56 (m, 2H), 7.00 - 6.91 (m, 2H), 4.41 (t, *J* = 6.4 Hz, 2H), 3.50 (t, *J* = 4.6 Hz, 4H), 3.13 - 3.04 (m, 4H), 2.80 (t, *J* = 6.4 Hz, 2H), 2.59 - 2.49 (m, 4H), 2.46 - 2.39 (m, 4H), 2.27 (s, 3H). |
| "A49" | 400 MHz; 10.26 (s, 1H), 9.77 (s, 1H), 8.85 (d, *J* = 11.36 Hz, 2H), 8.62 (s, 1H), 8.53 (s, 1H), 7.64 (s, 1H), 7.56-7.53 (m, 1H), 6.80 (d, *J* = 8.36 Hz, 1 H), 4.41 (t, *J* = 6.40 Hz, 2H), 3.51-3.49 (m, 6H), 2.79 (t, *J* = 6.48 Hz, 2H), 2.44-2.32 (m, 4H). |
| "A50" | 400 MHz, CDCl₃; 8.83 (s, 1H), 8.71 (s, 1H), 8.40 (s, 1H), 8.31 (s, 1H), 7.49-7.45 (m, 2H), 7.13 (d, *J* = 8.44 Hz, 1H), 6.89 (s, 1H), 4.40 (t, *J* = 6.48 Hz, 2H), 3.68 (t, *J* = 4.64 Hz, 4H), 3.19-3.05 (m, 4H), 2.98-2.74 (m, 6H), 2.57-2.51 (m, 7H), 2.37 (s, 3H). |
| "A51" | 400 MHz, CDCl₃; 8.81 (s, 1H), 8.69 (s, 1H), 8.39 (s, 1H), 8.28 (s, 1H), 7.47-7.45 (m, 2H), 7.10 (d, *J* = 8.56 Hz, 1H), 6.87 (s, 1H), 4.30 (t, *J* = 7.56 Hz, 2H), 2.95 (t, *J* = 4.68 Hz, 4H), 2.68-2.51 (m, 4H), 2.42-2.34 (m, 6H), 1.93-1.87 (m, 2H), 1.68-1.63 (m, 1H), 1.01 (d, *J* = 6.64 Hz, 6H). |
| "A52" | 500 MHz; 9.82 (s, 1H), 8.88 (d, *J* = 9.0 Hz, 2H), 8.63 (s, 1H), 8.56 (s, 1H), 7.76 - 7.69 (m, 1H), 7.66 (dd, *J* = 8.4, 2.2 Hz, 1H), 6.98 (d, *J* = 8.4 Hz, 1H), 4.48 - 4.29 (m, 2H), 3.59 - 3.49 (m, 4H), 3.13 (s, 3H), 2.87 - 2.74 (m, 4H), 2.49 - 2.39 (m, 4H). |
| "A53" | 500 MHz; 9.42 (s, 1H), 8.73 (s, 1H), 8.26 (s, 1H), 7.96 (s, 1H), 7.71 - 7.65 (m, 2H), 7.59 (s, 1H), 7.44 (s, 1H), 6.93 - 6.86 (m, 2H), 3.88 (s, 3H), 3.06 (t, *J* = 4.9 Hz, 4H), 2.26 (s, 3H). |
| "A55" | 400 MHz; 9.76 (s, 1H), 8.88 (s, 1H), 8.47 (s, 1H), 7.81 (d, *J* = 3.28 Hz, 1H), 7.61 (d, *J* = 9.00 Hz, 2H), 6.97 (d, *J* = 9.08 Hz, 2H), 6.54-6.53 (m, 1H), 3.74 (t, *J* = 4.88 Hz, 4H), 3.04 (t, *J* = 4.76 Hz, 4H), 2.47 (s, 3H). |
| "A56" | 400 MHz, CDCl₃; 8.82 (s, 1H), 8.69 (br s, 1H), 8.38 (s, 1H), 8.34 (s, 1H), 7.56-7.53 (m, 2H), 7.00 (d, *J* = 9.60 Hz, 2H), 6.85 (s, 1H), 4.50-4.35 (m, 2H), 3.90 (t, *J* = 4.84 Hz, 4H), 3.80-3.60 (m, 4H), 3.15 (t, *J* = 4.68 Hz, 4H), 3.00-2.85 (m, 2H), 2.61-2.48 (m, 4H). |
| "A57" | 400 MHz; 10.46 (s, 1H), 8.97 (s, 1H), 8.84 (s, 1 H), 8.68 (s, 1H), 8.52 (s, 1H), 8.30 (t, *J* = 1.72 Hz, 1 H), 7.92 (dd, *J* = 8.36, 1.44 Hz, 1H), 7.56 (t, *J* = 8.08 Hz, 1H), 7.40 (d, *J* = 7.60 Hz, 1H), 4.01 (s, 3H). |
| "A58" | 400 MHz; 10.28 (s, 1H), 9.89 (s, 1H), 8.88 (s, 1H), 8.78 (s, 1H), 8.23 (d, *J* = 3.84 Hz, 1H), 7.79 (s, 1H), 7.57 (dd, *J* = 8.42, 2.16 Hz, 1H), 7.23 (d, *J* = 3.84 Hz, 1H), 6.80 (d, *J* = 8.36 Hz, 1H), 3.83 (s, 2H), 3.62 (t, *J* = 4.44 Hz, 4H), 3.54 (s, 2H), 2.50-2.40 (m, 4H). |
| "A59" | 400 MHz; 9.75 (s, 1H), 8.85 (s, 1H), 8.80 (s, 1H), 8.60 (s, 1H), 8.52 (s, 1H), 7.37 (d, *J* = 2.60 Hz, 1H), 7.13 (dd, *J* = 8.78, 2.60 Hz, 1H), 6.83 (d, *J* = 8.76 Hz, 1H), 4.26-4.24 (m, 2H), 4.21-4.19 (m, 2H), 3.99 (s, 3H). |
| "A60" | 400 MHz; 10.29 (s, 1H), 8.93 (s, 1H), 8.85 (s, 1H), 8.66 (s, 1H), 8.59-8.56 (m, 3H), 7.91-7.85 (m, 4H), 7.71 (d, *J* = 6.12 Hz, 2H), 4.03 (s, 3H). |
| "A61" | 400 MHz; 9.96 (s, 1 H), 8.87 (s, 1H), 8.82 (s, 1H), 8.62 (s, 1 H), 8.53 (s, 1H), 7.69 (d, *J* = 8.48 Hz, 2H), 7.27 (d, *J* = 8.44 Hz, 2H), 4.01 (s, 3H), 3.56 (t, *J* = 4.52 Hz, 4H), 3.40 (s, 2H), 2.38-2.30 (m, 4H). |
| "A62" | 400 MHz, CDCl₃; 8.83 (s, 1H), 8.48 (s, 1H), 8.12 (d, *J* = 3.80 Hz, 1H), 7.61 (d, *J* = 8.88 Hz, 2H), 7.21-7.04 (m, 1H), 7.02-6.96 (m, 3H), 3.92-3.74 (m, 11H), 3.16 (t, *J* = 4.68 Hz, 4H), 2.75-2.05 (m, 3H). |
| "A63" | 400 MHz; 9.74 (s, 1H), 9.00 (s, 1H), 8.63 (d, *J* = 2.32 Hz, 1H), 8.42-8.39 (m, 2H), 7.62-7.56 (m, 3H), 6.92 (d, *J* = 9.12 Hz, 2H), 5.01-4.97 (m, 1H), 3.90-3.85 (m, 2H), 3.73 (t, *J* = 4.96 Hz, 4H), 3.59-3.54 (m, 2H), 3.01 (t, *J* = 4.76 Hz, 4H), 2.06-2.04 (m, 2H), 1.74-1.66 (m, 2H). |
| "A64" | 400 MHz; 10.15 (s, 1H), 8.90 (s, 1H), 8.83 (s, 1H), 8.65 (s, 1H), 8.53 (s, 1H), 7.77 (d, *J* = 2.44 Hz, 1H), 7.62 (dd, *J* = 8.90, 2.52 Hz, 1H), 7.31 (d, *J* = 8.92 Hz, 1H), 4.01 (s, 3H), 2.31 (s, 3H). |
| "A65" | 400 MHz; 9.90 (s, 1H), 9.03 (s, 1H), 8.87 (s, 1H), 8.84 (s, 1H), 8.62 (s, 1H), 8.53 (d, *J* = 0.36 Hz, 1H), 7.63 (d, *J* = 2.32 Hz, 1H), 7.49 (dd, *J* = 8.58, 2.44 Hz, 1H), 7.24 (d, *J* = 8.60 Hz, 1H), 3.34 (s, 3H), 3.27-3.23 (m, 1H), 2.25-2.18 (m, 5H), 2.14-2.10 (m, 2H), 1.94-1.90 (m, 1H), 1.89-1.82 (m, 1H). |
| "A66" | 400 MHz; 10.28 (s, 1H), 8.92 (s, 1H), 8.83 (s, 1H), 8.66 (s, 1H), 8.54 (s, 1H), 7.80 (dd, *J* = 6.90, 1.84 Hz, 2H), 7.45 (dd, *J* = 6.94, 1.72 Hz, 2H), 4.02 (s, 3H), 3.65-3.60 (m, 4H), 3.58-3.52 (m, 4H). |
| "A67" | 500 MHz; 9.76 (s, 1H), 8.84 (s, 1H), 8.83 (s, 1H), 8.59 (s, 1H), 8.54 (s, 1H), 7.48 (d, J=2.4, 1H), 7.20 (dd, J=8.6, 2.4, 1H), 6.91 (d, J=8.6, 1H), 4.00 (s, 3H), 3.85 (s, 3H), 2.99 (s, 4H), 2.68 (s, 4H), 2.39 (s, 3H). |
| "A68" | 400 MHz; 9.88 (s, 1H), 8.85 (d, *J* = 10.36 Hz, 2H), 8.61 (s, 1H), 8.54 (s, 1H), 7.60 (s, 1H), 7.47 (d, *J* = 8.20 Hz, 1H), 7.17 (d, *J* = 8.24 Hz, 1H), 4.01 (s, 3H), 3.58-3.50 (m, 4H), 3.35-3.28 (m, 2H), 2.40-2.30 (m, 7H). |
| "A69" | 400 MHz; 10.11 (s, 1H), 8.91 (s, 1H), 8.84 (s, 1H), 8.64 (s, 1H), 8.55 (s, 1H), 8.18 (t, *J =* 5.76 Hz, 1H), 7.64 (s, 1H), 7.57 (dd, *J* = 8.38, 1.96 Hz, 1H), 7.36 (d, *J* = 8.36 Hz, 1H), 4.01 (s, 3H), 3.15-3.08 (m, 2H), 2.41 (s, 3H), 1.03-0.98 (m, 1H), 0.45-0.40 (m, 2H), 0.24-0.20 (m, 2H). |
| "A70" | 400 MHz; 10.34 (s, 1H), 8.93 (s, 1H), 8.86 (s, 1H), 8.65 (s, 1H), 8.55 (s, 1H), 8.10 (t, *J* = 5.80 Hz, 1H), 7.87 (d, *J* = 8.56 Hz, 1H), 7.68 (d, *J* = 1.68 Hz, 1H), 7.30 (dd, *J* = 8.60, 1.80 Hz, 1H), 4.00 (s, 6H), 3.18 (t, *J* = 5.96 Hz, 2H), 1.06-1.03 (m, 1H), 0.45-0.41 (m, 2H), 0.35-0.20 (m, 2H). |
| "A71" | 400 MHz; 10.10 (s, 1H), 8.90 (d, *J =* 1.52 Hz, 1H), 8.83 (s, 1H), 8.64 (d, *J* = 1.44 Hz, 1H), 8.54 (d, *J* = 0.88 Hz, 1H), 8.14 (d, *J* = 3.28 Hz, 1H), 7.62 (s, 1H), 7.55 (d, *J* = 8.32 Hz, 1H), 7.33 (d, *J =* 8.40 Hz, 1H), 4.01 (s, 3H), 2.81-2.80 (m, 1H), 2.38 (s, 3H), 0.67-0.65 (m, 2H), 0.52-0.50 (m, 2H). |
| "A72" | 400 MHz; 10.32 (s, 1H), 8.92 (s, 1H), 8.84 (s, 1H), 8.64 (s, 1H), 8.54 (s, 1H), 7.92 (d, *J* = 4.08 Hz, 1H), 7.80 (d, *J* = 8.56 Hz, 1H), 7.65 (d, *J =* 1.64 Hz, 1H),7.29 (dd, *J =* 8.56, 1.72 Hz, 1H), 3.99 (s, 3H), 3.95 (s, 3H), 2.84-2.80 (m, 1H), 0.70-0.67 (m, 2H), 0.56-0.53 (m, 2H). |
| "A73" | 400 MHz; 10.12 (s, 1H), 8.90 (s, 1H), 8.84 (s, 1H), 8.64 (s, 1H), 8.54 (s, 1H), 7.69 (d, *J* = 1.88 Hz, 1H), 7.58 (dd, *J* = 8.32, 2.08 Hz, 1H), 7.17 (d, *J* = 8.32 Hz, 1H), 4.01 (s, 3H), 3.70-3.64 (m, 4H), 3.55-3.46 (m, 2H), 3.25-3.17 (m, 2H), 2.26 (s, 3H). |
| "A74" | 400 MHz; 11.20 (br s, 1H), 9.01 (s, 1H), 8.85 (s, 1H), 8.73 (s, 1H), 8.58 (s, 1H), 3.99 (s, 3H), 3.18-3.15 (m, 2H), 2.74-2.68 (m, 2H), 1.88 (d, *J* = 11.00 Hz, 2H), 1.70-1.66 (m, 2H). |
| "A75" | 500 MHz; 9.71 (s, 1H), 8.85 (d, J=17.3, 2H), 8.58 (d, J=15.5, 2H), 7.38 (d, J=2.4, 1H), 7.27 (dd, J=8.6, 2.4, 1H), 6.90 (d, J=8.6, 1H), 4.39 (t, J=6.5, 2H), 3.83 (s, 3H), 3.51 (t, J=4.7, 4H), 2.92 (s, 4H), 2.79 (t, J=6.5, 2H), 2.48 - 2.39 (m, 8H), 2.22 (s, 3H). |
| "A76" | 500 MHz; 9.78 (s, 1H), 8.86 (d, J=13.8, 2H), 8.58 (d, J=16.4, 2H), 7.44 (d, J=2.4, 1H), 7.29 (dd, J=8.5, 2.4, 1H), 6.94 (d, J=8.6, 1H), 4.28 (t, J=7.3, 2H), 3.85 (s, 3H), 3.28 - 2.90 (m, 8H), 2.67 (s, 3H), 1.77 (q, J=7.0, 2H), 1.63 - 1.50 (m, 1H), 0.94 (d, J=6.6, 6H). |
| "A77" | 500 MHz; 9.74 (s, 1H), 8.94 (s, 1H), 8.84 (s, 1H), 8.61 (d, J=6.6, 2H), 7.41 (d, J=2.4, 1 H), 7.28 (dd, J=8.5, 2.4, 1H), 6.91 (d, J=8.6, 1H), 4.62 - 4.53 (m, 1H), 4.05 - 3.98 (m, 2H), 3.84 (s, 3H), 3.53 (td, J=11.7, 2.2, 2H), 2.99 (s, 4H), 2.81-2.53 (m, 4H), 2.38 (s, 3H), 2.14 - 2.07 (m, 2H), 2.07 - 1.96 (m, 2H). |
| "A78" | 400 MHz; 9.97 (s, 1H), 8.85 (d, *J* = 11.32 Hz, 2H), 8.58 (d, *J* = 22.92 Hz, 2H), 7.54 (s, 1H), 7.21 (s, 2H), 3.99 (s, 3H), 3.85 (s, 3H), 3.55-3.49 (m, 6H), 2.41-2.29 (m, 4H). |
| "A79" | 400 MHz; 8.82 (d, *J* = 18.92 Hz, 2H), 8.59 (s, 1H), 8.51 (s, 1H), 8.27 (s, 1H), 7.91 (d, *J* = 8.64 Hz, 1H), 6.67 (s, 1H), 6.57 (d, *J* = 8.76 Hz, 1H), 3.99 (s, 3H), 3.84 (s, 3H), 3.16-3.05 (m, 4H), 2.45-2.39 (m, 4H), 2.22 (s, 3H). |
| "A80" | 400 MHz; 9.84 (s, 1H), 8.86 (s, 1H), 8.81 (s, 1H), 8.58 (d, *J* = 15.44 Hz, 2H), 7.42 (s, 1H), 7.21-7.14 (m, 2H), 6.57 (d, *J =* 7.72 Hz, 1H), 4.00 (s, 3H), 3.76 (t, *J* = 4.84 Hz, 4H), 3.12 (t, *J =* 4.72 Hz, 4H). |
| "A81" | 400 MHz; 9.90 (s, 1H), 9.22 (s, 1H), 8.85 (d, *J* = 12.76 Hz, 2H), 8.62 (s, 1H), 8.54 (s, 1H), 7.63 (d, *J* = 2.16 Hz, 1H), 7.48 (dd, *J* = 8.64, 2.36 Hz, 1H), 7.26 (d, *J* = 8.60 Hz, 1H), 4.00 (s, 3H), 2.21 (s, 3H), 2.02 (s, 3H). |
| "A82" | 400 MHz; 9.90 (s, 1H), 9.10 (s, 1H), 8.85 (d, *J* = 13.40 Hz, 2H), 8.62 (s, 1H), 8.53 (s, 1H), 7.62 (d, *J* = 1.84 Hz, 1H), 7.48 (dd, *J* = 8.50, 2.20 Hz, 1H), 7.22 (d, *J* = 8.64 Hz, 1H), 4.00 (s, 3H), 2.96 (d, *J* = 12.36 Hz, 2H), 2.49-2.40 (m, 2H), 2.18 (s, 3H), 1.99-1.96 (m, 2H), 1.69 (d, *J* = 10.64 Hz, 2H), 1.56-1.46 (m, 2H). |
| "A83" | 400 MHz; 9.91 (s, 1H), 9.18 (s, 1H), 8.87 (s, 1H), 8.84 (s, 1H), 8.62 (s, 1H), 8.53 (s, 1H), 7.63 (d, *J* = 2.20 Hz, 1H), 7.49 (dd, *J* = 8.50, 2.32 Hz, 1H), 7.23 (d, *J* = 8.60 Hz, 1H), 4.01 (s, 3H), 3.92-3.89 (m, 2H), 3.39-3.35 (m, 2H), 2.66-2.59 (m, 1H), 2.19 (s, 3H), 1.71-1.65 (m, 4H). |
| "A84" | 400 MHz; 9.97 (s, 1H), 9.05 (s, 1H), 8.86 (d, *J* = 13.56 Hz, 2H), 8.61 (s, 1H), 8.55 (s, 1H), 7.73 (d, *J* = 8.64 Hz, 1 H), 7.61 (d, *J* = 2.08 Hz, 1H), 7.16 (dd, *J* = 8.66, 2.12 Hz, 1H), 3.99 (s, 3H), 3.89 (s, 3H), 3.30-3.10 (m, 2H), 2.83-2.68 (m, 3H), 1.89-1.86 (m, 2H), 1.76-1.70 (m, 2H). |
| "A85" | 400 MHz; 9.95 (s, 1H), 9.05 (s, 1H), 8.86 (d, *J* = 11.20 Hz, 2H), 8.61 (s, 1H), 8.55 (s, 1H), 7.72 (d, *J* = 8.64 Hz, 1H), 7.60 (d, *J* = 1.80 Hz, 1H), 7.14 (dd, *J* = 7.10, 2.08 Hz, 1H), 3.99 (s, 3H), 3.89 (s, 3H), 2.04 (s, 3H). |
| "A86" | 400 MHz; 9.80 (d, *J* = 11.68 Hz, 2H), 9.00 (s, 1H), 8.24 (s, 1H), 7.54-7.49 (m, 3H), 7.38 (t, *J* = 8.08 Hz, 1H), 7.06- 7.03 (m, 2H), 6.81 (d, *J* = 8.60 Hz, 1H), 3.74 (s, 3H), 2.95-2.80 (m, 8H), 2.20 (s, 3H). |
| "A87" | 400 MHz; 10.12 (s, 1H), 9.75 (s, 1H), 8.92 (s, 1H), 8.26 (s, 1H), 8.05 (d, *J* = 8.72 Hz, 2H), 7.53 (d, *J* = 2.32 Hz, 1H), 7.07 (dd, *J* = 8.58, 2.44 Hz, 1H), 6.95 (d, *J* = 1.96 Hz, 2H), 6.83 (d, *J =* 8.60 Hz, 1H), 3.77 (s, 3H), 2.95-2.82 (m, 4H), 2.45-2.37 (m, 4H), 2.20 (s, 3H). |
| "A88" | 400 MHz; 9.69 (s, 1H), 8.84 (s, 1H), 8.77 (s, 1H), 8.72 (s, 2H), 8.59 (s, 1H), 8.50 (d, *J* = 0.36 Hz, 1H), 3.99 (s, 3H), 3.67 (t, *J =* 5.08 Hz, 4H), 2.36 (t, *J* = 4.96 Hz, 4H), 2.21 (s, 3H). |
| "A89" | 400 MHz; δ 9.95 (s, 1H), 8.96 (s, 1H), 8.86 (d, *J* = 12.80 Hz, 2H), 8.61 (s, 1H), 8.55 (s, 1H), 7.75 (d, *J* = 8.64 Hz, 1H), 7.60 (d, *J* = 2.00 Hz, 1H), 7.16 (dd, *J* = 8.70, 2.12 Hz, 1H), 3.99 (s, 3H), 3.89-3.88 (m, 5H), 3.40-3.36 (m, 1H), 3.33-3.30 (m, 1H), 2.78-2.69 (m, 1H), 1.68-1.62 (m, 4H). |
| "A90" | 400 MHz, + TFA; 9.63 (s, 1H), 9.57 (s, 1H), 9.25-9.23 (m, 2H), 9.07 (s, 1H), 8.35-8.25 (m, 1H), 7.53 (d, *J* = 8.96 Hz, 1H), 4.65-4.52 (m, 2H), 4.47 (s, 3H), 4.20-4.10 (m, 2H), 4.09-4.03 (m, 2H), 3.65-3.50 (m, 2H), 3.20 (s, 3H). |
| "A91" | 400 MHz; 9.76 (s, 1H), 8.90 (s, 1H), 8.30 (s, 1H), 8.11 (d, *J =* 8.96 Hz, 2H), 7.57 (d, *J* = 2.36 Hz, 1H), 7.12 (d, *J* = 9.08 Hz, 2H), 7.06 (dd, *J* = 8.56, 2.36 Hz, 1H), 6.83 (d, *J* = 8.60 Hz, 1H), 3.78-3.76 (m, 7H), 3.28-3.26 (m, 4H), 2.95-2.83 (m, 4H), 2.47-2.40 (m, 4H), 2.22 (s, 3H). |
| "A92" | 400 MHz; 9.79 (s, 1H), 8.96 (s, 1H), 8.29 (s, 1H), 8.14 (d, *J* = 8.84 Hz, 2H), 7.54 (d, *J* = 2.16 Hz, 1H), 7.15 (d, *J* = 8.88 Hz, 2H), 7.07 (dd, *J* = 8.58, 2.20 Hz, 1H), 6.83 (d, *J* = 8.60 Hz, 1H), 3.86 (s, 3H), 3.77 (s, 3H), 3.41-3.38 (m, 4H), 2.98-2.80 (m, 4H), 2.25 (s, 3H). |
| "A93" | 400 MHz; 10.22 (br s, 1H), 9.86 (s, 1H), 8.99 (s, 1H), 8.31 (s, 1H), 8.16 (d, *J* = 8.68 Hz, 2H), 7.62 (d, *J* = 1.84 Hz, 1 H), 7.39 (d, *J* = 8.64 Hz, 2H), 7.04 (dd, *J* = 8.62, 2.00 Hz, 1H), 6.87 (d, *J* = 8.56 Hz, 1H), 3.78 (s, 3H), 3.11 (s, 3H), 3.10-2.90 (m, 6H), 2.68-2.58 (m, 2H). 2.49 (s, 3H). |
| "A94" | 400 MHz; 9.83 (s, 1H), 9.07 (s, 1H), 8.39-8.34 (m, 3H), 7.97 (dd, *J* = 6.78, 1.80 Hz, 2H), 7.69 (d, *J =* 4.92 Hz, 1H), 7.51 (d, *J* = 2.36 Hz, 1H), 7.04 (dd, *J* = 8.56, 2.36 Hz, 1H), 6.82 (d, *J* = 8.60 Hz, 1H), 3.78 (s, 3H), 2.98-2.82 (m, 4H), 2.50-2.42 (m, 7H), 2.19 (s, 3H). |
| "A95" | 400 MHz; 10.05 (br s, 1H), 9.85 (s, 1H), 9.04 (s, 1H), 8.26 (s, 1H), 7.83 (d, *J* = 7.88 Hz, 2H), 7.56 (t, *J* = 7.92 Hz, 1H), 7.50 (d, J= 2.24 Hz, 1H), 7.42-7.40 (m, 1H), 7.13 (dd, J= 8.56, 2.20 Hz, 1H), 6.87 (d, *J* = 8.60 Hz, 1H), 3.75 (s, 3H), 3.12-3.07 (m, 11H), 2.71 (s, 3H). |
| "A96" | 400 MHz; 9.86 (s, 1H), 9.08 (s, 1H), 8.53 (s, 1H), 8.39-8.36 (m, 2H), 7.98 (d, *J* = 8.20 Hz, 1H), 7.85 (t, *J* = 7.88 Hz, 1H), 7.64-7.60 (m, 1H), 7.37 (d, *J* = 2.28 Hz, 1H), 7.24 (dd, *J* = 8.56, 2.28 Hz, 1H), 6.87 (d, *J* = 8.60 Hz, 1H), 3.72 (s, 3H), 3.25-2.95 (m, 8H), 2.70-2.60 (m, 3H), 2.46-2.45 (m, 3H). |

### Pharmacological data

**Table 2 GCN2 inhibition of some representative compounds of the formula I**

| Compound No. | IC₅₀ GCN2 (enzyme assay) | IC₅₀ SYK (enzyme assay) | Compound No. | IC₅₀ GCN2 (enzyme assay) | IC₅₀ SYK (enzyme assay) |
|---|---|---|---|---|---|
| "A1" | C | -18@3* | "A26" | | |
| "A2" | A | -29@3 | "A27" | B | -6@3 |
| "A3" | B | -9@3 | "A28" | A | -2@3 |
| "A4" | A | A | "A29" | B | |
| "A5" | B | A | "A30" | A | -33@3 |
| "A6" | A | B | "A31" | B | A |
| "A7" | C | B | "A32" | B | -35@3 |
| "A8" | B | B | "A33" | B | |
| "A9" | B | B | "A34" | B | B |
| "A10" | A | B | "A35" | A | A |
| "A11" | B | B | "A36" | -4@10 | B |
| "A12" | A | | "A37" | 3@10 | B |
| "A13" | A | A | "A38" | A | |
| "A14" | A | B | "A39" | A | |
| "A15" | A | B | "A40" | A | |
| "A16" | A | 1.5@3 | "A41" | A | |
| "A17" | A | B | "A42" | A | |
| "A18" | A | -5@3 | "A43" | A | |
| "A19" | B | | "A44" | C | B |
| "A20" | B | 1@3 | "A45" | 2@10 | A |
| "A21" | B | | "A46" | -8@10 | B |
| "A22" | A | -15@3 | "A47" | A | A |
| "A23" | B | | "A48" | A | C |
| "A24" | | 1@3 | "A49" | B | -19@3 |
| "A25" | B | -8@3 | "A50" | A | -32@3 |
| "A51" | A | A | "A61" | A | B |
| "A52" | B | -32@3 | "A62" | A | -5@3 |
| "A53" | B | -23@3 | "A63" | B | -30@3 |
| "A54" | B | | "A64" | A | B |
| "A55" | A | A | "A65" | A | C |
| "A56" | B | B | "A66" | A | B |
| "A57" | B | C | "A67" | A | B |
| "A58" | A | -34@3 | "A68" | A | B |
| "A59" | A | B | "A69" | A | B |
| "A60" | B | A | "A70" | A | -12@0.3 |
| "A71" | A | B | "A81" | A | B |
| "A72" | A | C | "A82" | A | B |
| "A73" | A | B | "A83" | A | C |
| "A74" | C | 4@3 | "A84" | A | C |
| "A75" | A | -21@3 | "A85" | A | B |
| "A76" | A | B | "A86" | A | A |
| "A77" | A | B | "A87" | A | A |
| "A78" | A | B | "A88" | A | -13@3 |
| "A79" | C | -9@3 | "A89" | A | C |
| "A80" | A | -26@1 | "A90" | A | |
| "A91" | A | B | | | |
| "A92" | A | A | | | |
| "A93" | A | B | | | |
| "A94" | A | B | | | |
| "A95" | A | B | | | |
| "A96" | B | | | | |

IC₅₀: < 1 µM=A 1 - 5 µM = B 5 - 10 µM = C
* 18@3 means -18% at 3 µM
100% corresponds to zero effect
- 50% corresponds to IC₅₀

The compounds shown in Table 1 are particularly preferred compounds according to the invention.

The following examples relate to medicaments:

### Example A: Injection vials

A solution of 100 g of an active ingredient of the formula I and 5 g of disodium hydrogenphosphate in 3 l of bidistilled water is adjusted to pH 6.5 using 2 N hydrochloric acid, sterile filtered, transferred into injection vials, lyophilised under sterile conditions and sealed under sterile conditions. Each injection vial contains 5 mg of active ingredient.

### Example B: Suppositories

A mixture of 20 g of an active ingredient of the formula I with 100 g of soya lecithin and 1400 g of cocoa butter is melted, poured into moulds and allowed to cool. Each suppository contains 20 mg of active ingredient.

### Example C: Solution

A solution is prepared from 1 g of an active ingredient of the formula I, 9.38 g of NaH₂PO₄ · 2 H₂O, 28.48 g of Na₂HPO₄ · 12 H₂O and 0.1 g of benzalkonium chloride in 940 ml of bidistilled water. The pH is adjusted to 6.8, and the solution is made up to 1 I and sterilised by irradiation. This solution can be used in the form of eye drops.

### Example D: Ointment

500 mg of an active ingredient of the formula I are mixed with 99.5 g of Vaseline under aseptic conditions.

### Example E: Tablets

A mixture of 1 kg of active ingredient of the formula I, 4 kg of lactose, 1.2 kg of potato starch, 0.2 kg of talc and 0.1 kg of magnesium stearate is pressed in a conventional manner to give tablets in such a way that each tablet contains 10 mg of active ingredient.

### Example F: Dragees

Tablets are pressed analogously to Example E and subsequently coated in a conventional manner with a coating of sucrose, potato starch, talc, tragacanth and dye.

### Example G: Capsules

2 kg of active ingredient of the formula I are introduced into hard gelatine capsules in a conventional manner in such a way that each capsule contains 20 mg of the active ingredient.

### Example H: Ampoules

A solution of 1 kg of active ingredient of the formula I in 60 I of bidistilled water is sterile filtered, transferred into ampoules, lyophilised under sterile conditions and sealed under sterile conditions. Each ampoule contains 10 mg of active ingredient.

## Claims

1. Compounds of the formula I 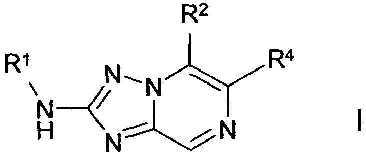 in which
R¹ denotes Ar, COHet or Het,
R² denotes H, Ar¹, NHHet or Het,
R³ denotes H or A',
R⁴ denotes H, A, Ar¹, Het, Hal, NHAr¹ or CN,
Ar denotes phenyl or naphthyl which is unsubstituted or mono- or disubstituted by Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚN(R³)₂, [C(R³)₂]ₚHet¹, NR³SO₂A, SO₂N(R³)₂, S(O)ₙA, COHet¹, O[C(R³)₂]ₘN(R³)₂ and/or O[C(R³)₂]ₚHet¹,
Ar¹ denotes phenyl which is unsubstituted or mono- or disubstituted by Hal, A, phenyl, CONH₂, [C(R³)₂]ₚOR³, [C(R³)₂]ₚN(R³)₂, [C(R³)₂]ₚCN, [C(R³)₂]ₚHet¹ and/or O[C(R³)₂]ₚHet¹,
Het denotes furyl, thienyl, pyrrolyl, imidazolyl, pyrazolyl, oxazolyl, isoxazolyl, oxadiazolyl, thiazolyl, triazolyl, tetrazolyl, pyridyl, pyrimidyl, pyridazinyl, pyrazinyl, indolyl, isoindolyl, benzimidazolyl, indazolyl, quinolyl, 1,3-benzodioxolyl, benzothiophenyl, benzofuranyl, imidazopyridyl, dihydroindolyl, 2,3-dihydro-benzo[1,4]dioxinyl or furo[3,2-b]pyridyl which is unsubstituted or mono-, di- or trisubstituted by Hal, A, [C(R³)₂]ₚOR³, [C(R³)₂]ₚ-N(R³)₂, [C(R³)₂)ₚHet¹, NO₂, CN, [C(R⁶)₂]ₚCOOR³, CON(R³)₂, NR³COA, NR³SO₂A, SO₂N(R³)₂, S(O)ₙA, COHet¹, O[C(R³)₂]ₘN(R³)₂, O[C(R³)₂]ₚHet¹ and/or =O,
Het¹ denotes dihydropyrrolyl, pyrrolidinyl, azetidinyl, oxetanyl, tetra-hydroimidazolyl, dihydropyrazolyl, tetrahydropyrazolyl, tetrahydrofuranyl, dihydropyridyl, tetrahydropyridyl, piperidinyl, morpholinyl, hexahydropyridazinyl, hexahydropyrimidinyl, [1,3]dioxolanyl, tetrahydropyranyl, pyridyl or piperazinyl, which is unsubstituted or mono- or disubstituted by Hal, CN, OH, OA, COOA, CONH₂, S(O)ₙA, S(O)ₙAr, COA, A and/or =O,
A denotes unbranched or branched alkyl with 1-10 C-atoms, wherein one or two non-adjacent CH- and/or CH₂-groups may be replaced by N-, O- and/or S-atoms and wherein 1-7 H-atoms may be replaced by F or Cl,
Cyc denotes cyclic alkyl with 3-7 C-atoms, which is unsubstituted or monosubstituted by [C(R³)₂]ₚOH or CN,
A' denotes unbranched or branched alkyl with 1, 2, 3 or 4 C-atoms,
Hal denotes F, Cl, Br or I,
n denotes 0, 1 or 2,
m denotes 1, 2 or 3,
p denotes 0, 1, 2, 3 or 4,
and pharmaceutically acceptable solvates, salts, tautomers and stereoisomers thereof, including mixtures thereof in all ratios.

2. Compounds according Claim 1 in which
R¹ denotes Ar, COHet or Het,
R² denotes H, Ar¹, NHHet or Het,
R³ denotes H or A',
R⁴ denotes H, A, Ar¹, Het, Hal, NHAr¹ or CN,
Ar denotes phenyl which is unsubstituted or mono- or disubstituted by Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚHet¹, SO₂N(R³)₂, NR³SO₂A, O[C(R³)₂]ₚHet¹, COHet¹, and/or S(O)ₙA,
Ar¹ denotes phenyl which is unsubstituted or mono- or disubstituted by Hal, A, phenyl, CONH₂, [C(R³)₂]ₚCN, [C(R³)₂]ₚOR³ and/or [C(R³)₂]ₚHet¹,
Het denotes furyl, thienyl, pyrrolyl, imidazolyl, pyrazolyl, oxazolyl, isoxazolyl, oxadiazolyl, thiazolyl, triazolyl, tetrazolyl, pyridyl, pyrimidyl, pyridazinyl, pyrazinyl, indolyl, isoindolyl, benzimidazolyl, indazolyl, quinolyl, 1,3-benzodioxolyl, benzothiophenyl, benzofuranyl, imidazopyridyl, dihydroindolyl, 2,3-dihydro-benzo[1,4]dioxinyl or furo[3,2-b]pyridyl which is unsubstituted or mono-, di- or trisubstituted by A, [C(R³)₂]ₚHet¹, [C(R³)₂]ₚN(R³)₂ and/or =O,
Het¹ denotes dihydropyrrolyl, pyrrolidinyl, azetidinyl, oxetanyl, tetra-hydroimidazolyl, dihydropyrazolyl, tetrahydropyrazolyl, tetrahydrofuranyl, dihydropyridyl, tetrahydropyridyl, piperidinyl, morpholinyl, hexahydropyridazinyl, hexahydropyrimidinyl, [1,3]dioxolanyl, tetrahydropyranyl, pyridyl or piperazinyl, which is unsubstituted or mono- or disubstituted by A,
A denotes unbranched or branched alkyl with 1-10 C-atoms, and wherein 1-7 H-atoms may be replaced by F or Cl,
Cyc denotes cyclic alkyl with 3-7 C-atoms, which is unsubstituted or monosubstituted by [C(R³)₂]ₚOH or CN,
A' denotes unbranched or branched alkyl with 1, 2, 3 or 4 C-atoms,
Hal denotes F, Cl, Br or I,
n denotes 0, 1 or 2,
p denotes 0, 1, 2, 3 or 4,
and pharmaceutically acceptable solvates, salts, tautomers and stereoisomers thereof, including mixtures thereof in all ratios.

3. Compounds according to Claim 1 or 2, selected from the group
| No. | Name |
|---|---|
| "A1" | (4-methanesulfonyl-phenyl)-[5-(1-methyl-1H-indazol-5-yl)-[1,2,4]triazolo[1,5-a]pyrazin-2-yl]-amine |
| "A2" | (3-methoxy-phenyl)-[5-(1-methyl-1H-indazol-5-yl)-[1,2,4]triazolo[1,5-a]pyrazin-2-yl]-amine |
| "A3" | [5-(1-methyl-1H-indazol-5-yl)-[1,2,4]triazolo[1,5-a]pyrazin-2-yl]-pyridin-3-yl-amine |
| "A4" | [5-(1-methyl-1H-indazol-5-yl)-[1,2,4]triazolo[1,5-a]pyrazin-2-yl]-[3-methyl-4-(4-methyl-piperazin-1-yl)-phenyl]-amine |
| "A5" | 3,3-dimethyl-6-[5-(1-methyl-1H-indazol-5-yl)-[1,2,4]-triazolo[1,5-a]pyrazin-2-ylamino]-1,3-dihydro-indol-2-one |
| "A6" | (2-dimethylaminomethyl-1H-benzoimidazol-5-yl)-[5-(1-methyl-1H-indazol-5-yl)-[1,2,4]triazolo[1,5-a]pyrazin-2-yl]-amine |
| "A7" | 3-(5-quinolin-3-yl-[1,2,4]triazolo[1,5-a]pyrazin-2-ylamino)-benzenesulfonamide |
| "A8" | [3-methyl-4-(4-methyl-piperazin-1-yl)-phenyl]-(5-quinolin-3-yl-[1,2,4]triazolo[1,5-a]pyrazin-2-yl)-amine |
| "A9" | 3,3-dimethyl-6-(5-quinolin-3-yl-[1,2,4]triazolo[1,5-a]pyrazin-2-ylamino)-1,3-dihydro-indol-2-one |
| "A10" | (2-dimethylaminomethyl-1 H-benzoimidazol-5-yl)-(5-quinolin-3-yl-[1,2,4]triazolo[1,5-a]pyrazin-2-yl)-amine |
| "A11" | (4-methanesulfonyl-phenyl)-[5-(1-methyl-1 H-pyrazol-4-yl)-[1,2,4]triazolo[1,5-a]pyrazin-2-yl]-amine |
| "A12" | [5-(1-methyl-1H-pyrazol-4-yl)-[1,2,4]triazolo[1,5-a]pyrazin-2-yl]-pyridin-3-yl-amine |
| "A13" | 3,3-dimethyl-6-[5-(1-methyl-1H-pyrazol-4-yl)-[1,2,4]-triazolo[1,5-a]pyrazin-2-ylamino]-1,3-dihydro-indol-2-one |
| "A14" | (2-dimethylaminomethyl-1 H-benzoimidazol-5-yl)-[5-(1-methyl-1H-pyrazol-4-yl)-[1,2,4]triazolo[1,5-a]pyrazin-2-yl]-amine |
| "A15" | 5-[5-(4-morpholin-4-yl-phenyl)-[1,2,4]triazolo[1,5-a]pyrazin-2-ylamino]-1,3-dihydro-indol-2-one |
| "A16" | [5-(4-morpholin-4-yl-phenyl)-[1,2,4]triazolo[1,5-a]pyrazin-2-yl]-pyridin-3-yl-amine |
| "A17" | [3-methyl-4-(4-methyl-piperazin-1-yl)-phenyl]-[5-(4-morpholin-4-yl-phenyl)-[1,2,4]triazolo[1,5-a]pyrazin-2-yl]-amine |
| "A18" | (6-chloro-[1,2,4]triazolo[1,5-a]pyrazin-2-yl)-(4-methoxy-phenyl)-amine |
| "A19" | (6-chloro-[1,2,4]triazolo[1,5-a]pyrazin-2-yl)-(3-fluoro-phenyl)-amine |
| "A20" | (6-chloro-[1,2,4]triazolo[1,5-a]pyrazin-2-yl)-pyridin-3-yl-amine |
| "A21" | (6-chloro-[1,2,4]triazolo[1,5-a]pyrazin-2-yl)-(4-fluoro-phenyl)-amine |
| "A22" | (6-bromo-[1,2,4]triazolo[1,5-a]pyrazin-2-yl)-(3-methoxy-phenyl)-amine |
| "A23" | (6-cyano-[1,2,4]triazolo[1,5-a]pyrazin-2-yl)-(3-methoxy-phenyl)-amine |
| "A24" | (3-methoxy-phenyl)-(6-methyl-[1,2,4]triazolo[1,5-a]pyrazin-2-yl)-amine |
| "A25" | pyridin-3-yl-(6-pyridin-3-yl-[1,2,4]triazolo[1,5-a]pyrazin-2-yl]-amine |
| "A26" | (3-methoxy-phenyl)-(6-o-tolyl-[1,2,4]triazolo[1,5-a]pyrazin-2-yl)-amine |
| "A27" | [5-(4-fluoro-phenyl)-[1,2,4]triazolo[1,5-a]pyrazin-2-yl]-pyridin-3-yl-amine |
| "A28" | (3-methoxy-phenyl)-(5-phenyl-[1,2,4]triazolo[1,5-a]pyrazin-2-yl)-amine |
| "A29" | N6-(4-fluoro-phenyl)-N2-pyridin-3-yl-[1,2,4]triazolo[1,5-a]pyrazine-2,6-diamine |
| "A30" | (3-methoxy-phenyl)-[5-(1-methyl-1H-pyrazol-4-yl)-[1,2,4]triazolo[1,5-a]pyrazin-2-yl]-amine |
| "A31" | (4-morpholin-4-yl-phenyl)-[5-(1H-pyrazol-4-yl)-[1,2,4]triazolo[1,5-a]pyrazin-2-yl]-amine |
| "A32" | Synthesis of (4-morpholin-4-yl-phenyl)-(5-quinolin-3-yl-[1,2,4]triazolo[1,5-a]pyrazin-2-yl)-amine |
| "A33" | (4-morpholin-4-yl-phenyl)-(5-quinolin-6-yl-[1,2,4]triazolo[1,5-a]pyrazin-2-yl)-amine |
| "A34" | 2-Methyl-2-{4-[2-(4-morpholin-4-yl-phenylamino)-[1,2,4]triazolo[1,5-a]pyrazin-5-yl]-phenyl}-propionitrile |
| "A35" | [5-(1-methyl-1 H-pyrazol-4-yl)-[1,2,4]triazolo[1,5-a]pyrazin-2-yl]-(4-morpholin-4-yl-phenyl)-amine |
| "A36" | (5-biphenyl-2-yl-[1,2,4]triazolo[1,5-a]pyrazin-2-yl)-(4-morpholin-4-yl-phenyl)-amine |
| "A37" | (5-biphenyl-2-yl-[1,2,4]triazolo[1,5-a]pyrazin-2-yl)-(3,5-dimethoxy-phenyl)-amine |
| "A38" | 5-[5-(1-methyl-1H-indazol-5-yl)-[1,2,4]triazolo[1,5-a]pyrazin-2-ylamino]-1,3-dihydro-indol-2-one |
| "A39" | 3-[5-(1-methyl-1H-indazol-5-yl)-[1,2,4]triazolo[1,5-a]pyrazin-2-ylamino]-benzenesulfonamide |
| "A40" | 2-methyl-2-(4-{2-[3-methyl-4-(4-methyl-piperazin-1-yl)-phenylamino]-[1,2,4]triazolo[1,5-a]pyrazin-5-yl}-phenyl)-propionitrile |
| "A41" | 5-[5-(1-methyl-1H-pyrazol-4-yl)-[1,2,4]triazolo[1,5-a]pyrazin-2-ylamino]-1,3-dihydro-indol-2-one |
| "A42" | 5-(1-methyl-1H-pyrazol-4-yl)-N-(3-methyl-4-(4-methylpiperazin-1-yl)phenyl)-[1,2,4]triazolo[1,5-a]pyrazin-2-amine |
| "A43" | N-(3-(2-((3-methyl-4-(4-methylpiperazin-1-yl)phenyl)amino)-[1,2,4]triazolo[1,5-a]pyrazin-5-yl)phenyl)methanesulfonamide |
| "A44" | [6-methyl-5-(1-methyl-1H-pyrazol-4-yl)-[1,2,4]triazolo[1,5-a]pyrazin-2-yl]-(4-morpholin-4-yl-phenyl)-amine |
| "A45" | [5-(1-methyl-1H-pyrazol-yl)-6-phenyl-[1,2,4]triazolo[1,5-a]pyrazin-2-yl]-(4-morpholin-4-yl-phenyl)-amine |
| "A46" | [5,6-Bis-(1-methyl-1H-pyrazol-4-yl)-[1,2,4]triazolo[1,5-a]pyrazin-2-yl]-(4-morpholin-4-yl-phenyl)-amine |
| "A47" | 1-{4-[5-(1-methyl-1H-pyrazol-4-yl)-[1,2,4]triazolo[1,5-a]pyrazin-2-ylamino]-phenyl}-cyclopropanecarbonitrile |
| "A48" | [4-(4-methyl-piperazin-1-yl)-phenyl]-{5-[1-(2-morpholin-4-yl-ethyl)-1H-pyrazol-4-yl]-[1,2,4]triazolo[1,5-a]pyrazin-2-yl}-amine |
| "A49" | 5-{5-[1-(2-morpholin-4-yl-ethyl)-1H-pyrazol-4-yl]-[1,2,4]triazolo[1,5-a]pyrazin-2-ylamino}-1,3-dihydro-indol-2-one |
| "A50" | [3-methyl-4-(4-methyl-piperazin-1-yl)-phenyl]-{5-[1-(2-morpholin-4-yl-ethyl)-1 H-pyrazol-4-yl]-[1,2,4]triazolo[1,5-a]pyrazin-2-yl}-amine |
| "A51" | {5-[1-(3-methyl-butyl)-1H-pyrazol-4-yl]-[1,2,4]triazolo[1,5-a]pyrazin-2-yl}-[3-methyl-4-(4-methyl-piperazin-1-yl)-phenyl]-amine |
| "A52" | 1-methyl-5-{5-[1-(2-morpholin-4-yl-ethyl)-1H-pyrazol-4-yl]-[1,2,4]triazolo[1,5-a]pyrazin-2-ylamino}-1,3-dihydro-indol-2-one |
| "A53" | N2-[4-(4-methyl-piperazin-1-yl)-phenyl]-N5-(1-methyl-1H-pyrazol-4-yl)-[1,2,4]triazolo[1,5-a]pyrazine-2,5-diamine |
| "A54" | 3-{2-[3-methyl-4-(4-methyl-piperazin-1-yl)-phenylamino]-[1,2,4]triazolo[1,5-a]pyrazin-5-yl}-benzamide |
| "A55" | [5-(5-methyl-furan-2-yl)-[1,2,4]triazolo[1,5-a]pyrazin-2-yl]-(4-morpholin-4-yl-phenyl)-amine |
| "A56" | {5-[1-(2-morpholin-4-yl-ethyl)-1H-pyrazol-4-yl]-[1,2,4]triazolo[1,5-a]pyrazin-2-yl}-(4-morpholin-4-yl-phenyl)-amine |
| "A57" | 3-[5-(1-methyl-1 H-pyrazol-4-yl)-[1,2,4]triazolo[1,5-a]pyrazin-2-ylamino]-benzonitrile |
| "A58" | 5-[5-(5-morpholin-4-ylmethyl-thiophen-2-yl)-[1,2,4]triazolo[1,5-a]pyrazin-2-ylamino]-1,3-dihydro-indol-2-one |
| "A59" | (2,3-dihydro-benzo[1,4]dioxin-6-yl)-[5-(1-methyl-1H-pyrazol-4-yl)-[1,2,4]triazolo[1,5-a]pyrazin-2-yl]-amine |
| "A60" | [5-(1-methyl-1H-pyrazol-4-yl)-[1,2,4]triazolo[1,5-a]pyrazin-2-yl]-(4-pyridin-4-yl-phenyl)-amine |
| "A61" | [5-(1-methyl-1H-pyrazol-4-yl)-[1,2,4]triazolo[1,5-a]pyrazin-2-yl]-(4-morpholin-4-ylmethyl-phenyl)-amine |
| "A62" | [5-(5-morpholin-4-ylmethyl-thiophen-2-yl)-[1,2,4]triazolo[1,5-a]pyrazin-2-yl]-(4-morpholin-4-yl-phenyl)-amine |
| "A63" | 5-[2-(4-morpholin-4-yl-phenylamino)-[1,2,4]triazolo[1,5-a]pyrazin-5-yl]-2-(tetrahydro-pyran-4-yloxy)-benzonitrile |
| "A64" | [5-(1-methyl-1H-pyrazol-4-yl)-[1,2,4]triazolo[1,5-a]pyrazin-2-yl]-(3-methyl-4-trifluoromethoxy-phenyl)-amine |
| "A65" | cyclobutanecarboxylic acid {2-methyl-4-[5-(1-methyl-1H-pyrazol-4-yl)-[1,2,4]triazolo[1,5-a]pyrazin-2-ylamino]-phenyl}-amide |
| "A66" | {4-[5-(1-methyl-1H-pyrazol-4-yl)-[1,2,4]triazolo[1,5-a]pyrazin-2-ylamino]-phenyl}-morpholin-4-yl-methanone |
| "A67" | [3-methoxy-4-(4-methyl-piperazin-1-yl)-phenyl]-[5-(1-methyl-1H-pyrazol-4-yl)-[1,2,4]triazolo[1,5-a]pyrazin-2-yl]-amine |
| "A68" | (3-methyl-4-morpholin-4-ylmethyl-phenyl)-[5-(1-methyl-1H-pyrazol-4-yl)-[1,2,4]triazolo[1,5-a]pyrazin-2-yl]-amine |
| "A69" | N-cyclopropylmethyl-2-methyl-4-[5-(1-methyl-1H-pyrazol-4-yl)-[1,2,4]triazolo[1,5-a]pyrazin-2-ylamino]-benzamide |
| "A70" | N-cyclopropylmethyl-2-methoxy-4-[5-(1-methyl-1H-pyrazol-4-yl)-[1,2,4]triazolo[1,5-a]pyrazin-2-ylamino]-benzamide |
| "A71" | N-cyclopropyl-2-methyl-4-[5-(1-methyl-1 H-pyrazol-4-yl)-[1,2,4]triazolo[1,5-a]pyrazin-2-ylamino]-benzamide |
| "A72" | N-cyclopropyl-2-methoxy-4-[5-(1-methyl-1H-pyrazol-4-yl)-[1,2,4]triazolo[1,5-a]pyrazin-2-ylamino]-benzamide |
| "A73" | {2-methyl-4-[5-(1-methyl-1H-pyrazol-4-yl)-[1,2,4]triazolo[1,5-a]pyrazin-2-ylamino]-phenyl}-morpholin-4-yl-methanone |
| "A74" | piperidine-4-carboxylic acid [5-(1-methyl-1H-pyrazol-4-yl)-[1,2,4]triazolo[1,5-a]pyrazin-2-yl]-amide |
| "A75" | [3-methoxy-4-(4-methyl-piperazin-1-yl)-phenyl]-{5-[1-(2-morpholin-4-yl-ethyl)-1H-pyrazol-4-yl]-[1,2,4]triazolo[1,5-a]pyrazin-2-yl}-amine |
| "A76" | [3-methoxy-4-(4-methyl-piperazin-1-yl)-phenyl]-{5-[1-(3-methyl-butyl)-1H-pyrazol-4-yl]-[1,2,4]triazolo[1,5-a]pyrazin-2-yl}-amine |
| "A77" | [3-methoxy-4-(4-methyl-piperazin-1-yl)-phenyl]-{5-[1-(tetrahydro-pyran-4-yl)-1H-pyrazol-4-yl]-[1,2,4]triazolo[1,5-a]pyrazin-2-yl}-amine |
| "A78" | (3-methoxy-4-morpholin-4-ylmethyl-phenyl)-[5-(1-methyl-1H-pyrazol-4-yl)-[1,2,4]triazolo[1,5-a]pyrazin-2-yl]-amine |
| "A79" | [2-methoxy-4-(4-methyl-piperazin-1-yl)-phenyl]-[5-(1-methyl-1H-pyrazol-4-yl)-[1,2,4]triazolo[1,5-a]pyrazin-2-yl]-amine |
| "A80" | [5-(1-methyl-1 H-pyrazol-4-yl)-[1,2,4]triazolo[1,5-a]pyrazin-2-yl]-(3-morpholin-4-yl-phenyl)-amine |
| "A81" | N-{2-methyl-4-[5-(1-methyl-1H-pyrazol-4-yl)-[1,2,4]triazolo[1,5-a]pyrazin-2-ylamino]-phenyl}-acetamide |
| "A82" | piperidine-4-carboxylic acid {2-methyl-4-[5-(1-methyl-1H-pyrazol-4-yl)-[1,2,4]triazolo[1,5-a]pyrazin-2-ylamino]-phenyl}-amide |
| "A83" | tetrahydro-pyran-4-carboxylic acid {2-methyl-4-[5-(1-methyl-1H-pyrazol-4-yl)-[1,2,4]triazolo[1,5-a]pyrazin-2-ylamino]-phenyl}-amide |
| "A84" | piperidine-4-carboxylic acid {2-methoxy-4-[5-(1-methyl-1H-pyrazol-4-yl)-[1,2,4]triazolo[1,5-a]pyrazin-2-ylamino]-phenyl}-amide |
| "A85" | N-{2-methoxy-4-[5-(1-methyl-1H-pyrazol-4-yl)-[1,2,4]triazolo[1,5-a]pyrazin-2-ylamino]-phenyl}-acetamide |
| "A86" | 3-{2-[3-methoxy-4-(4-methyl-piperazin-1-yl)-phenylamino]-[1,2,4]triazolo[1,5-a]pyrazin-5-yl}-phenol |
| "A87" | 4-{2-[3-methoxy-4-(4-methyl-piperazin-1-yl)-phenylamino]-[1,2,4]triazolo[1,5-a]pyrazin-5-yl}-phenol |
| "A88" | [2-(4-methyl-piperazin-1-yl)-pyrimidin-5-yl]-[5-(1-methyl-1H-pyrazol-4-yl)-[1,2,4]triazolo[1,5-a]pyrazin-2-yl]-amine |
| "A89" | tetrahydro-pyran-4-carboxylic acid {2-methoxy-4-[5-(1-methyl-1H-pyrazol-4-yl)-[1,2,4]triazolo[1,5-a]pyrazin-2-ylamino]-phenyl}-amide |
| "A90" | [6-(4-methyl-piperazin-1-yl)-pyridin-3-yl]-[5-(1-methyl-1H-pyrazol-4-yl)-[1,2,4]triazolo[1,5-a]pyrazin-2-yl]-amine |
| "A91" | [3-methoxy-4-(4-methyl-piperazin-1-yl)-phenyl]-[5-(4-morpholin-4-yl-phenyl)-[1,2,4]triazolo[1,5-a]pyrazin-2-yl]-amine |
| "A92" | [3-methoxy-4-(4-methyl-piperazin-1-yl)-phenyl]-[5-(4-methoxyphenyl)-[1,2,4]triazolo[1,5-a]pyrazin-2-yl]-amine |
| "A93" | N-(4-{2-[3-methoxy-4-(4-methyl-piperazin-1-yl)-phenylamino]-[1,2,4]triazolo[1,5-a]pyrazin-5-yl}-phenyl)-methanesulfonamide |
| "A94" | 4-{2-[3-methoxy-4-(4-methyl-piperazin-1-yl)-phenylamino]-[1,2,4]triazolo[1,5-a]pyrazin-5-yl}-N-methyl-benzenesulfonamide |
| "A95" | N-(3-{2-[3-methoxy-4-(4-methyl-piperazin-1-yl)-phenylamino]-[1,2,4]triazolo[1,5-a]pyrazin-5-yl}-phenyl)-methanesulfonamide |
| "A96" | 3-{2-[3-methoxy-4-(4-methyl-piperazin-1-yl)-phenylamino]-[1,2,4]triazolo[1,5-a]pyrazin-5-yl}-N-methyl-benzenesulfonamide |
and pharmaceutically acceptable solvates, salts, tautomers and stereoisomers thereof, including mixtures thereof in all ratios.

4. Process for the preparation of compounds of the formula I according to Claims 1-3 and pharmaceutically acceptable salts, solvates, tautomers and stereoisomers thereof, **characterised in that**
a compound of the formula II 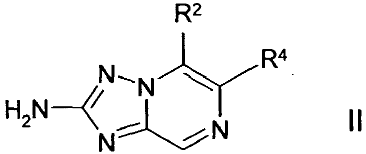
in which R² and R⁴ have the meanings indicated in Claim 1, is reacted with a compound of the formula III
R¹-L III
in which R¹ has the meaning indicated in Claim 1 and
L denotes Cl or Br,
and/or
a base or acid of the formula I is converted into one of its salts.

5. Medicaments comprising at least one compound of the formula I and/or pharmaceutically acceptable salts, solvates, tautomers and stereoisomers thereof, including mixtures thereof in all ratios, and optionally an pharmaceutically acceptable carrier, excipient or vehicle.

6. Compounds of the formula I and pharmaceutically acceptable salts, solvates, tautomers and stereoisomers thereof, including mixtures thereof in all ratios, for use in the treatment and/or prevention of inflammatory conditions, immunological conditions, autoimmune conditions, allergic conditions, rheumatic conditions, thrombotic conditions, cancer, infections, neurodegenerative diseases, neuroinflammatory diseases, cardiovascular diseases, and metabolic conditions, the methods comprising administering to a subject in need thereof an effective amount of a compound of claim 1.

7. Compound for use according to claim 6 in the treatment and/or prevention of cancer,
where the cancer to be treated is a solid tumour or a tumour of the blood and immune system.

8. Compounds for use according to claim 7, where the solid tumour originates from
the group of tumours of the epithelium, the bladder, the stomach, the kidneys, of head and neck, the esophagus, the cervix, the thyroid, the intestine, the liver, the brain, the prostate, the uro-genital tract, the lymphatic system, the stomach, the larynx, the bones, including chondosarcoma and Ewing sarcoma, germ cells, including embryonal tissue tumours, and/or the lung, from the group of monocytic leukaemia, lung adenocarcinoma, small-cell lung carcinomas, pancreatic cancer, glioblastomas, neurofibroma, angiosarcoma, breast carcinoma and /or maligna melanoma.

9. Compound for use according to claim 6 in the treatment and/or prevention of diseases selected from the group rheumatoid arthritis, systemic lupus, asthma, multiple sclerosis, osteoarthritis, ischemic injury, giant cell arteritis, inflammatory bowel disease, diabetes, cystic fibrosis, psoriasis, Sjögrens syndrom and transplant organ rejection.

10. Compound for use according to claim 6 in the treatment and/or prevention of diseases selected from the group Alzheimer's disease, Down's syndrome, hereditary cerebral hemorrhage with amyloidosis-Dutch Type, cerebral amyloid angiopathy, Creutzfeldt-Jakob disease, frontotemporal dementias, Huntington's disease, Parkinson's disease.

11. Compound for use according to claim 6 in the treatment and/or prevention of diseases selected from the group leishmania, mycobacteria, including M. leprae, M. tuberculosis and/or M. avium, leishmania, plasmodium, human immunodeficiency virus, Epstein Barr virus, Herpes simplex virus, hepatitis C virus.

12. Medicaments comprising at least one compound of the formula I and/or pharmaceutically acceptable salts, solvates and stereoisomers thereof, including mixtures thereof in all ratios, and at least one further medicament active ingredient.

13. Set (kit) consisting of separate packs of
(a) an effective amount of a compound of the formula I and/or pharmaceutically acceptable salts, solvates, salts and stereoisomers thereof, including mixtures thereof in all ratios,
and
(b) an effective amount of a further medicament active ingredient.

## Patentansprüche

1. Verbindungen der Formel I 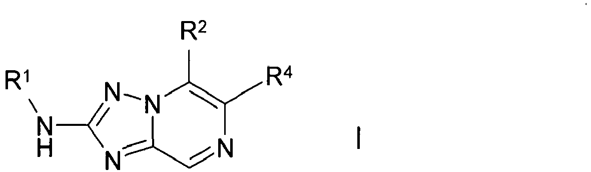 in der
R¹ Ar, COHet oder Het bedeutet,
R² H, Ar¹, NHHet oder Het bedeutet,
R³ H oder A' bedeutet,
R⁴ H, A, Ar¹, Het, Hal, NHAr¹ oder CN bedeutet,
Ar Phenyl oder Naphthyl bedeutet, das unsubstituiert oder ein- oder zweifach durch Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚN(R³)₂, [C(R³)₂]ₚHet¹, NR³SO₂A, SO₂N(R³)₂, S(O)ₙA, COHet¹, O[C(R³)₂]ₘN(R³)₂ und/oder O[C(R³)₂]ₚHet¹ substituiert ist,
Ar¹ Phenyl bedeutet, das unsubstituiert oder ein- oder zweifach durch Hal, A, Phenyl, CONH₂, [C(R³)₂]ₚOR³, [C(R³)₂]ₚN(R³)₂, [C(R³)₂]ₚ-CN, [C(R³)₂]ₚHet¹ und/oder O(C(R³)₂]ₚHet¹ substituiert ist,
Het Furyl, Thienyl, Pyrrolyl, Imidazolyl, Pyrazolyl, Oxazolyl, Isoxazolyl, Oxadiazolyl, Thiazolyl, Triazolyl, Tetrazolyl, Pyridyl, Pyrimidyl, Pyridazinyl, Pyrazinyl, Indolyl, Isoindolyl, Benzimidazolyl, Indazolyl, Chinolyl, 1,3-Benzodioxolyl, Benzothiophenyl, Benzofuranyl, Imidazopyridyl, Dihydroindolyl, 2,3-Dihydro-benzo[1,4]dioxinyl oder Furo[3,2-b]pyridyl bedeutet, das unsubstituiert oder ein-, zwei- oder dreifach durch Hal, A, [C(R³)₂]ₚOR³, [C(R³)₂]ₚN(R³)₂, [C(R³)₂]ₚHet¹, NO₂, CN, [C(R⁶)₂]ₚCOOR³, CON(R³)₂, NR³COA, NR³SO₂A, SO₂N(R³)₂, S(O)ₙA, COHet¹, O[C(R³)₂]ₘN(R³)₂, O[C(R³)₂]ₚHet¹ und/oder =O substituiert ist,
Het¹ Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl, Pyridyl oder Piperazinyl bedeutet, das unsubstituiert oder ein- oder zweifach durch Hal, CN, OH, OA, COOA, CONH₂, S(O)ₙA, S(O)ₙAr, COA, A und/oder =O substituiert ist,
A unverzweigtes oder verzweigtes Alkyl mit 1-10 C-Atomen bedeutet, worin eine oder zwei nicht benachbarte CH- und/oder CH₂-Gruppen durch N-, O- und/oder S-Atome ersetzt sein können und worin 1-7 H-Atome durch F oder Cl ersetzt sein können,
Cyc cyclisches Alkyl mit 3-7 C-Atomen bedeutet, das unsubstituiert oder einfach durch [C(R³)₂]ₚOH oder CN substituiert ist,
A' unverzweigtes oder verzweigtes Alkyl mit 1, 2, 3 oder 4 C-Atomen bedeutet,
Hal F, Cl, Br oder I bedeutet,
n für 0, 1 oder 2 steht,
m für 1, 2 oder 3 steht,
p für 0, 1, 2, 3 oder 4 steht,
und pharmazeutisch unbedenkliche Solvate, Salze, Tautomere und Stereoisomere davon, einschließlich deren Mischungen in allen Verhältnissen.

2. Verbindungen nach Anspruch 1, in denen
R¹ Ar, COHet oder Het bedeutet,
R² H, Ar¹, NHHet oder Het bedeutet,
R³ H oder A' bedeutet,
R⁴ H, A, Ar¹, Het, Hal, NHAr¹ oder CN bedeutet,
Ar Phenyl bedeutet, das unsubstituiert oder ein- oder zweifach durch Hal, A, Cyc, [C(R³)₂]ₚOA, [C(R³)₂]ₚOH, CN, NHCOHet¹, NHCOA, NHCO[C(R³)₂]ₚCyc, CONH[C(R³)₂]ₚCyc, [C(R³)₂]ₚHet¹, SO₂N(R³)₂, NR³SO₂A, O[C(R³)₂]ₚHet¹, COHet¹ und/oder S(O)ₙA substituiert ist,
Ar¹ Phenyl bedeutet, das unsubstituiert oder ein- oder zweifach durch Hal, A, Phenyl, CONH₂, [C(R³)₂]ₚCN, [C(R³)₂]ₚOR³ und/oder [C(R³)₂]ₚHet¹ substituiert ist,
Het Furyl, Thienyl, Pyrrolyl, Imidazolyl, Pyrazolyl, Oxazolyl, Isoxazolyl, Oxadiazolyl, Thiazolyl, Triazolyl, Tetrazolyl, Pyridyl, Pyrimidyl, Pyridazinyl, Pyrazinyl, Indolyl, Isoindolyl, Benzimidazolyl, Indazolyl, Chinolyl, 1,3-Benzodioxolyl, Benzothiophenyl, Benzofuranyl, Imidazopyridyl, Dihydroindolyl, 2,3-Dihydro-benzo[1,4]dioxinyl oder Furo[3,2-b]pyridyl bedeutet, das unsubstituiert oder ein-, zwei- oder dreifach durch A, [C(R³)₂]ₚHet¹, [C(R³)₂]ₚN(R³)₂ und/oder =O substituiert ist,
Het¹ Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl, Pyridyl oder Piperazinyl bedeutet, das unsubstituiert oder ein- oder zweifach durch A substituiert ist,
A unverzweigtes oder verzweigtes Alkyl mit 1-10 C-Atomen, und worin 1-7 H-Atome durch F oder Cl ersetzt sein können, bedeutet,
Cyc cyclisches Alkyl mit 3-7 C-Atomen bedeutet, das unsubstituiert oder einfach durch [C(R³)₂]ₚOH oder CN substituiert ist,
A' unverzweigtes oder verzweigtes Alkyl mit 1, 2, 3 oder 4 C-Atomen bedeutet,
Hal F, Cl, Br oder I bedeutet,
n für 0, 1 oder 2 steht,
p 0, 1, 2, 3 oder 4 steht,
und pharmazeutisch unbedenkliche Solvate, Salze, Tautomere und Stereoisomere davon, einschließlich deren Mischungen in allen Verhältnissen.

3. Verbindungen nach Anspruch 1 oder 2, ausgewählt aus der Gruppe
| Nr. | Name |
|---|---|
| "A1" | (4-Methansulfonyl-phenyl)-[5-(1-methyl-1H-indazol-5-yl)-[1,2,4]triazolo[1,5-a]pyrazin-2-yl]-amin |
| "A2" | (3-Methoxy-phenyl)-[5-(1-methyl-1H-indazol-5-yl)-[1,2,4]triazolo[1,5-a]pyrazin-2-yl]-amin |
| "A3" | [5-(1-Methyl-1H-indazol-5-yl)-[1,2,4]triazolo[1,5-a]pyrazin-2-yl]-pyridin-3-yl-amin |
| "A4" | [5-(1-Methyl-1H-indazol-5-yl)-[1,2,4]triazolo[1,5-a]pyrazin-2-yl]-[3-methyl-4-(4-methyl-piperazin-1-yl)-phenyl]-amin |
| "A5" | 3,3-Dimethyl-6-[5-(1-methyl-1H-indazol-5-yl)-[1,2,4]-triazolo[1,5-a]pyrazin-2-ylamino]-1,3-dihydro-indol-2-on |
| "A6" | (2-Dimethylaminomethyl-1H-benzoimidazol-5-yl)-[5-(1-methyl-1H-indazol-5-yl)-[1,2,4]triazolo[1,5-a]pyrazin-2-yl]-amin |
| "A7" | 3-(5-Chinolin-3-yl-[1,2,4]triazolo[1,5-a]pyrazin-2-ylamino)-benzolsulfonamid |
| "A8" | [3-Methyl-4-(4-methyl-piperazin-1-yl)-phenyl]-(5-chinolin-3-yl-[1,2,4]triazolo[1,5-a]pyrazin-2-yl)-amin |
| "A9" | 3,3-Dimethyl-6-(5-chinolin-3-yl-[1,2,4]triazolo[1,5-a]pyrazin-2-ylamino)-1,3-dihydro-indol-2-on |
| "A10" | (2-Dimethylaminomethyl-1H-benzoimidazol-5-yl)-(5-chinolin-3-yl-[1,2,4]triazolo[1,5-a]pyrazin-2-yl)-amin |
| "A11" | (4-Methansulfonyl-phenyl)-[5-(1-methyl-1H-pyrazol-4-yl)-[1,2,4]triazolo[1,5-a]pyrazin-2-yl]-amin |
| "A12" | [5-(1-Methyl-1H-pyrazol-4-yl)-[1,2,4]triazolo[1,5-a]pyrazin-2-yl]-pyridin-3-yl-amin |
| "A13" | 3,3-Dimethyl-6-[5-(1-methyl-1H-pyrazol-4-yl)-[1,2,4]-triazolo[1,5-a]pyrazin-2-ylamino]-1,3-dihydro-indol-2-on |
| "A14" | (2-Dimethylaminomethyl-1H-benzoimidazol-5-yl)-[5-(1-methyl-1H-pyrazol-4-yl)-[1,2,4]triazolo[1,5-a]pyrazin-2-yl]-amin |
| "A15" | 5-[5-(4-Morpholin-4-yl-phenyl)-[1,2,4]triazolo[1,5-a]pyrazin-2-ylamino]-1,3-dihydro-indol-2-on |
| "A16" | [5-(4-Morpholin-4-yl-phenyl)-[1,2,4]triazolo[1,5-a]pyrazin-2-yl]-pyridin-3-yl-amin |
| "A17" | [3-Methyl-4-(4-methyl-piperazin-1-yl)-phenyl]-[5-(4-morpholin-4-yl-phenyl)-[1,2,4]triazolo[1,5-a]pyrazin-2-yl]-amin |
| "A18" | (6-Chlor-[1,2,4]triazolo[1,5-a]pyrazin-2-yl)-(4-methoxyphenyl)-amin |
| "A19" | (6-Chlor-[1,2,4]triazolo[1,5-a]pyrazin-2-yl)-(3-fluor-phenyl)-amin |
| "A20" | (6-Chlor-[1,2,4]triazolo[1,5-a]pyrazin-2-yl)-pyridin-3-yl-amin |
| "A21" | (6-Chlor-[1,2,4]triazolo[1,5-a]pyrazin-2-yl)-(4-fluor-phenyl)-amin |
| "A22" | (6-Brom-[1,2,4]triazolo[1,5-a]pyrazin-2-yl)-(3-methoxy-phenyl)-amin |
| "A23" | (6-Cyano-[1,2,4]triazolo[1,5-a]pyrazin-2-yl)-(3-methoxyphenyl)-amin |
| "A24" | (3-Methoxy-phenyl)-(6-methyl-[1,2,4]triazolo[1,5-a]pyrazin-2-yl)-amin |
| "A25" | Pyridin-3-yl-(6-pyridin-3-yl-[1,2,4]triazolo[1,5-a]pyrazin-2-yl]-amin |
| "A26" | (3-Methoxy-phenyl)-(6-o-tolyl-[1,2,4]triazolo[1,5-a]pyrazin-2-yl)-amin |
| "A27" | [5-(4-Fluor-phenyl)-[1,2,4]triazolo[1,5-a]pyrazin-2-yl]-pyridin-3-yl-amin |
| "A28" | (3-Methoxy-phenyl)-(5-phenyl-[1,2,4]triazolo[1,5-a]pyrazin-2-yl)-amin |
| "A29" | N6-(4-Fluor-phenyl)-N2-pyridin-3-yl-[1,2,4]triazolo[1,5-a]-pyrazin-2,6-diamin |
| "A30" | (3-Methoxy-phenyl)-[5-(1-methyl-1H-pyrazol-4-yl)-[1,2,4]triazolo[1,5-a]pyrazin-2-yl]-amin |
| "A31" | (4-Morpholin-4-yl-phenyl)-[5-(1H-pyrazol-4-yl)-[1,2,4]triazolo[1,5-a]pyrazin-2-yl]-amin |
| "A32" | (4-Morpholin-4-yl-phenyl)-(5-chinolin-3-yl-[1,2,4]triazolo[1,5-a]pyrazin-2-yl)-amin |
| "A33" | (4-Morpholin-4-yl-phenyl)-(5-chinolin-6-yl-[1,2,4]triazolo-[1,5-a]pyrazin-2-yl)-amin |
| "A34" | 2-Methyl-2-{4-[2-(4-morpholin-4-yl-phenylamino)-[1,2,4]triazolo[1,5-a]pyrazin-5-yl]-phenyl}-propionitril |
| "A35" | [5-(1-Methyl-1H-pyrazol-4-yl)-[1,2,4]triazolo[1,5-a]pyrazin-2-yl]-(4-morpholin-4-yl-phenyl)-amin |
| "A36" | (5-Biphenyl-2-yl-[1,2,4]triazolo[1,5-a]pyrazin-2-yl)-(4-morpholin-4-yl-phenyl)-amin |
| "A37" | (5-Biphenyl-2-yl-[1,2,4]triazolo[1,5-a]pyrazin-2-yl)-(3,5-dimethoxy-phenyl)-amin |
| "A38" | 5-[5-(1-Methyl-1H-indazol-5-yl)-[1,2,4]triazolo[1,5-a]pyrazin-2-ylamino]-1,3-dihydro-indol-2-on |
| "A39" | 3-[5-(1-Methyl-1H-indazol-5-yl)-[1,2,4]triazolo[1,5-a]pyrazin-2-ylamino]-benzolsulfonamid |
| "A40" | 2-Methyl-2-(4-{2-[3-methyl-4-(4-methyl-piperazin-1-yl)-phenylamino]-[1,2,4]triazolo[1,5-a]pyrazin-5-yl}-phenyl)-propionitril |
| "A41" | 5-[5-(1-Methyl-1H-pyrazol-4-yl)-[1,2,4]triazolo[1,5-a]pyrazin-2-ylamino]-1,3-dihydro-indol-2-on |
| "A42" | 5-(1-Methyl-1H-pyrazol-4-yl)-N-(3-methyl-4-(4-methyl-piperazin-1-yl)phenyl)-[1,2,4]triazolo[1,5-a]pyrazin-2-amin |
| "A43" | N-(3-(2-((3-Methyl-4-(4-methylpiperazin-1-yl)phenyl)amino)-[1,2,4]triazolo[1,5-a]pyrazin-5-yl)phenyl)methansulfonamid |
| "A44" | [6-Methyl-5-(1-methyl-1H-pyrazol-4-yl)-[1,2,4]triazolo[1,5-a]-pyrazin-2-yl]-(4-morpholin-4-yl-phenyl)-amin |
| "A45" | [5-(1-Methyl-1H-pyrazol-4-yl)-6-phenyl-[1,2,4]triazolo[1,5-a]-pyrazin-2-yl]-(4-morpholin-4-yl-phenyl)-amin |
| "A46" | [5,6-Bis-(1-methyl-1H-pyrazol-4-yl)-[1,2,4]triazolo[1,5-a]-pyrazin-2-yl]-(4-morpholin-4-yl-phenyl)-amin |
| "A47" | 1-{4-[5-(1-Methyl-1H-pyrazol-4-yl)-[1,2,4]triazolo[1,5-a]-pyrazin-2-ylamino]-phenyl}-cyclopropancarbonitril |
| "A48" | [4-(4-Methyl-piperazin-1-yl)-phenyl]-{5-[1-(2-morpholin-4-yl-ethyl)-1H-pyrazol-4-yl]-[1,2,4]triazolo[1,5-a]pyrazin-2-yl}-amin |
| "A49" | 5-{5-[1-(2-Morpholin-4-yl-ethyl)-1H-pyrazol-4-yl]-[1,2,4]triazolo[1,5-a]pyrazin-2-ylamino}-1,3-dihydro-indol-2-on |
| "A50" | [3-Methyl-4-(4-methyl-piperazin-1-yl)-phenyl]-{5-[1-(2-morpholin-4-yl-ethyl)-1H-pyrazol-4-yl]-[1,2,4]triazolo[1,5-a]-pyrazin-2-yl}-amin |
| "A51" | {5-[1-(3-Methyl-butyl)-1H-pyrazol-4-yl]-[1,2,4]triazolo[1,5-a]-pyrazin-2-yl}-[3-methyl-4-(4-methyl-piperazin-1-yl)-phenyl]-amin |
| "A52" | 1-Methyl-5-{5-[1-(2-morpholin-4-yl-ethyl)-1H-pyrazol-4-yl]-[1,2,4]triazolo[1,5-a]pyrazin-2-ylamino}-1,3-dihydro-indol-2-on |
| "A53" | N2-[4-(4-Methyl-piperazin-1-yl)-phenyl]-N5-(1-methyl-1H-pyrazol-4-yl)-[1,2,4]triazolo[1,5-a]pyrazine-2,5-diamin |
| "A54" | 3-{2-[3-Methyl-4-(4-methyl-piperazin-1-yl)-phenylamino]-[1,2,4]triazolo[1,5-a]pyrazin-5-yl}-benzamid |
| "A55" | [5-(5-Methyl-furan-2-yl)-[1,2,4]triazolo[1,5-a]pyrazin-2-yl]-(4-morpholin-4-yl-phenyl)-amin |
| "A56" | {5-[1-(2-Morpholin-4-yl-ethyl)-1H-pyrazol-4-yl]-[1,2,4]-triazolo[1,5-a]pyrazin-2-yl}-(4-morpholin-4-yl-phenyl)-amin |
| "A57" | 3-[5-(1-Methyl-1 H-pyrazol-4-yl)-[1,2,4]triazolo[1,5-a]pyrazin-2-ylamino]-benzonitril |
| "A58" | 5-[5-(5-Morpholin-4-ylmethyl-thiophen-2-yl)-[1,2,4]triazolo-[1,5-a]pyrazin-2-ylamino]-1,3-dihydro-indol-2-on |
| "A59" | (2,3-Dihydro-benzo[1,4]dioxin-6-yl)-[5-(1-methyl-1H-pyrazol-4-yl)-[1,2,4]triazolo[1,5-a]pyrazin-2-yl]-amin |
| "A60" | [5-(1-Methyl-1H-pyrazol-4-yl)-[1,2,4]triazolo[1,5-a]pyrazin-2-yl]-(4-pyridin-4-yl-phenyl)-amin |
| "A61" | [5-(1-Methyl-1H-pyrazol-4-yl)-[1,2,4]triazolo[1,5-a]pyrazin-2-yl]-(4-morpholin-4-ylmethyl-phenyl)-amin |
| "A62" | [5-(5-Morpholin-4-ylmethyl-thiophen-2-yl)-[1,2,4]triazolo-[1,5-a]pyrazin-2-yl]-(4-morpholin-4-yl-phenyl)-amin |
| "A63" | 5-[2-(4-Morpholin-4-yl-phenylamino)-[1,2,4]triazolo[1,5-a]-pyrazin-5-yl]-2-(tetrahydro-pyran-4-yloxy)-benzonitril |
| "A64" | [5-(1-Methyl-1H-pyrazol-4-yl)-[1,2,4]triazolo[1,5-a]pyrazin-2-yl]-(3-methyl-4-trifluormethoxy-phenyl)-amin |
| "A65" | Cyclobutancarbonsäure-{2-methyl-4-[5-(1-methyl-1H-pyrazol-4-yl)-[1,2,4]triazolo[1,5-a]pyrazin-2-ylamino]-phenyl}-amid |
| "A66" | {4-[5-(1-Methyl-1H-pyrazol-4-yl)-[1,2,4]triazolo[1,5-a]pyrazin-2-ylamino]-phenyl}-morpholin-4-yl-methanon |
| "A67" | [3-Methoxy-4-(4-methyl-piperazin-1-yl)-phenyl]-[5-(1-methyl-1H-pyrazol-4-yl)-[1,2,4]triazolo[1,5-a]pyrazin-2-yl]-amin |
| "A68" | (3-Methyl-4-morpholin-4-ylmethyl-phenyl)-[5-(1-methyl-1H-pyrazol-4-yl)-[1,2,4]triazolo[1,5-a]pyrazin-2-yl]-amin |
| "A69" | N-Cyclopropylmethyl-2-methyl-4-[5-(1-methyl-1H-pyrazol-4-yl)-[1,2,4]triazolo[1,5-a]pyrazin-2-ylamino]-benzamid |
| "A70" | N-Cyclopropylmethyl-2-methoxy-4-[5-(1-methyl-1H-pyrazol-4-yl)-[1,2,4]triazolo[1,5-a]pyrazin-2-ylamino]-benzamid |
| "A71" | N-Cyclopropyl-2-methyl-4-[5-(1-methyl-1H-pyrazol-4-yl)-[1,2,4]triazolo[1,5-a]pyrazin-2-ylamino]-benzamid |
| "A72" | N-Cyclopropyl-2-methoxy-4-[5-(1-methyl-1H-pyrazol-4-yl)-[1,2,4]triazolo[1,5-a]pyrazin-2-ylamino]-benzamid |
| "A73" | {2-Methyl-4-[5-(1-methyl-1H-pyrazol-4-yl)-[1,2,4]triazolo-[1,5-a]pyrazin-2-ylamino]-phenyl}-morpholin-4-yl-methanon |
| "A74" | Piperidin-4-carbonsäure-[5-(1-methyl-1H-pyrazol-4-yl)-[1,2,4]triazolo[1,5-a]pyrazin-2-yl]-amid |
| "A75" | [3-Methoxy-4-(4-methyl-piperazin-1-yl)-phenyl]-{5-[1-(2-morpholin-4-yl-ethyl)-1H-pyrazol-4-yl]-[1,2,4]triazolo[1,5-a]-pyrazin-2-yl}-amin |
| "A76" | [3-Methoxy-4-(4-methyl-piperazin-1-yl)-phenyl]-{5-[1-(3-methyl-butyl)-1H-pyrazol-4-yl]-[1,2,4]triazolo[1,5-a]pyrazin-2-yl}-amin |
| "A77" | [3-Methoxy-4-(4-methyl-piperazin-1-yl)-phenyl]-{5-[1-(tetrahydro-pyran-4-yl)-1H-pyrazol-4-yl]-[1,2,4]triazolo[1,5-a]-pyrazin-2-yl}-amin |
| "A78" | (3-Methoxy-4-morpholin-4-ylmethyl-phenyl)-[5-(1-methyl-1H-pyrazol-4-yl)-[1,2,4]triazolo[1,5-a]pyrazin-2-yl]-amin |
| "A79" | [2-Methoxy-4-(4-methyl-piperazin-1-yl)-phenyl]-[5-(1-methyl-1H-pyrazol-4-yl)-[1,2,4]triazolo[1,5-a]pyrazin-2-yl]-amin |
| "A80" | [5-(1-Methyl-1H-pyrazol-4-yl)-[1,2,4]triazolo[1,5-a]pyrazin-2-yl]-(3-morpholin-4-yl-phenyl)-amin |
| "A81" | N-{2-Methyl-4-[5-(1-methyl-1H-pyrazol-4-yl)-[1,2,4]triazolo[1,5-a]pyrazin-2-ylamino]-phenyl}-acetamid |
| "A82" | Piperidin-4-carbonsäure-{2-methyl-4-[5-(1-methyl-1H-pyrazol-4-yl)-[1,2,4]triazolo[1,5-a]pyrazin-2-ylamino]-phenyl}-amid |
| "A83" | Tetrahydro-pyran-4-carbonsäure-{2-methyl-4-[5-(1-methyl-1 H-pyrazol-4-yl)-[1,2,4]triazolo[1,5-a]pyrazin-2-ylamino]-phenyl}-amid |
| "A84" | Piperidin-4-carbonsäure-{2-methoxy-4-[5-(1-methyl-1H-pyrazol-4-yl)-[1,2,4]triazolo[1,5-a]pyrazin-2-ylamino]-phenyl}-amid |
| "A85" | N-{2-Methoxy-4-[5-(1-methyl-1H-pyrazol-4-yl)-[1,2,4]triazolo[1,5-a]pyrazin-2-ylamino]-phenyl}-acetamid |
| "A86" | 3-{2-[3-Methoxy-4-(4-methyl-piperazin-1-yl)-phenylamino]-[1,2,4]triazolo[1,5-a]pyrazin-5-yl}-phenol |
| "A87" | 4-{2-[3-Methoxy-4-(4-methyl-piperazin-1-yl)-phenylamino]-[1,2,4]triazolo[1,5-a]pyrazin-5-yl}-phenol |
| "A88" | [2-(4-Methyl-piperazin-1-yl)-pyrimidin-5-yl]-[5-(1-methyl-1H-pyrazol-4-yl)-[1,2,4]triazolo[1,5-a]pyrazin-2-yl]-amin |
| "A89" | Tetrahydro-pyran-4-carbonsäure-{2-methoxy-4-[5-(1-methyl-1 H-pyrazol-4-yl)-[1,2,4]triazolo[1,5-a]pyrazin-2-ylamino]-phenyl}-amid |
| "A90" | [6-(4-Methyl-piperazin-1-yl)-pyridin-3-yl]-[5-(1-methyl-1H-pyrazol-4-yl)-[1,2,4]triazolo[1,5-a]pyrazin-2-yl]-amin |
| "A91" | [3-Methoxy-4-(4-methyl-piperazin-1-yl)-phenyl]-[5-(4-morpholin-4-yl-phenyl)-[1,2,4]triazolo[1,5-a]pyrazin-2-yl]-amin |
| "A92" | [3-Methoxy-4-(4-methyl-piperazin-1-yl)-phenyl]-[5-(4-methoxy-phenyl)-[1,2,4]triazolo[1,5-a]pyrazin-2-yl]-amin |
| "A93" | N-(4-{2-[3-Methoxy-4-(4-methyl-piperazin-1-yl)-phenylamino]-[1,2,4]triazolo[1,5-a]pyrazin-5-yl)-phenyl)-methansulfonamid |
| "A94" | 4-{2-[3-Methoxy-4-(4-methyl-piperazin-1-yl)-phenylamino]-[1,2,4]triazolo[1,5-a]pyrazin-5-yl}-N-methyl-benzolsulfonamid |
| "A95" | N-(3-{2-[3-Methoxy-4-(4-methyl-piperazin-1-yl)-phenylamino]-[1,2,4]triazolo[1,5-a]pyrazin-5-yl}-phenyl)-methansulfonamid |
| "A96" | 3-{2-[3-Methoxy-4-(4-methyl-piperazin-1-yl)-phenylamino]-[1,2,4]triazolo[1,5-a]pyrazin-5-yl}-N-methyl-benzolsulfonamid |
und pharmazeutisch unbedenkliche Solvate, Salze, Tautomere und Stereoisomere davon, einschließlich deren Mischungen in allen Verhältnissen.

4. Verfahren zur Herstellung von Verbindungen der Formel I nach den Ansprüchen 1-3 und pharmazeutisch unbedenklicher Salze, Solvate, Tautomere und Stereoisomere davon, **dadurch gekennzeichnet, dass** man
eine Verbindung der Formel II 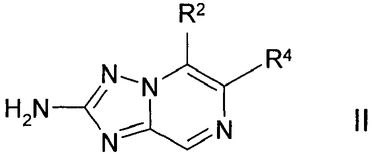
in der R² und R⁴ die in Anspruch 1 angegebenen Bedeutungen besitzen,
mit einer Verbindung der Formel III
R¹-L III
in der R¹ die in Anspruch 1 angegebene Bedeutung besitzt und
L Cl oder Br bedeutet,
umsetzt und/oder
eine Base oder Säure der Formel I in eines ihrer Salze umwandelt.

5. Arzneimittel enthaltend mindestens eine Verbindung der Formel I und/ oder pharmazeutisch unbedenkliche Salze, Solvate, Tautomere und Stereoisomere davon, einschließlich deren Mischungen in allen Verhältnissen, und gegebenenfalls einen pharmazeutisch unbedenklichen Träger, Trägerstoff oder Vehikel.

6. Verbindungen der Formel I und pharmazeutisch unbedenkliche Salze, Solvate, Tautomere und Stereoisomere davon, einschließlich deren Mischungen in allen Verhältnissen, zur Verwendung bei der Behandlung und/oder Prävention von Entzündungsleiden, Immunleiden, Autoimmunleiden, allergischen Leiden, rheumatischen Leiden, thrombotischen Leiden, Krebs, Infektionen, neurodegenerativen Krankheiten, neuroinflammatorischen Krankheiten, Herz-Kreislauf-Krankheiten und Stoffwechselleiden, wobei die Verfahren umfassen, dass man einem Patienten, der dessen bedarf, eine wirksame Menge einer Verbindung des Anspruchs 1 verabreicht.

7. Verbindungen zur Verwendung nach Anspruch 6 bei der Behandlung und/oder Prävention von Krebs,
wobei es sich bei dem zu behandelnden Krebs um einen festen Tumor oder einen Tumor des Blutes und Immunsystems handelt.

8. Verbindungen zur Verwendung nach Anspruch 7, wobei der feste Tumor aus der Gruppe der Tumore des Epitheliums, der Blase, des Magens, der Nieren, von Kopf und Hals, der Speiseröhre, des Muttermundes, der Schilddrüse, des Darms, der Leber, des Gehirns, der Prostata, des Urogenitaltraktes, des Lymphsystems, des Kehlkopfes, der Knochen, einschließlich Chondosarkom und Ewing-Sarkom, Keimzellen, einschließlich Tumoren des embryonalen Gewebes, und/oder der Lunge, aus der Gruppe von monozytischer Leukämie, Lungenadenokarzinom, kleinzelligem Lungenkarzinom, Bauchspeicheldrüsenkrebs, Glioblastomen, Neurofibrom, Angiosarkom, Mammakarzinom und/oder malignen Melanomen stammt.

9. Verbindungen zur Verwendung nach Anspruch 6 bei der Behandlung und/oder Prävention von Krankheiten, die aus der Gruppe rheumatoide Arthritis, systemischer Lupus, Asthma, multiple Sklerose, Osteoarthritis, ischämische Verletzung, Riesenzellarteriitis, chronischentzündliche Darmerkrankung, Diabetes, zystische Fibrose, Psoriasis, Sjögren-Syndrom und Organtransplantatabstoßung ausgewählt sind.

10. Verbindungen zur Verwendung nach Anspruch 6 bei der Behandlung und/oder Prävention von Krankheiten, die aus der Gruppe Alzheimer-Krankheit, Down-Syndrom, erbliche zerebrale Blutungen mit Amyloidose vom holländischen Typ, zerebrale Amyloidangiopathie, Creutzfeldt-Jakob-Krankheit, frontotemporale Demenzen, Chorea Huntington, Parkinson-Krankheit ausgewählt sind.

11. Verbindungen zur Verwendung nach Anspruch 6 bei der Behandlung und/oder Prävention von Krankheiten, die aus der Gruppe Leishmania, Mykobakterien, einschließlich M. leprae, M. tuberculosis und/oder M. avium, Plasmodium, humanes Immundefizienz-Virus, Epstein-Barr-Virus, Herpes-simplex-Virus, Hepatitis-C-Virus ausgewählt sind.

12. Arzneimittel enthaltend mindestens eine Verbindung der Formel I und/ oder pharmazeutisch unbedenkliche Salze, Solvate und Stereoisomere davon, einschließlich deren Mischungen in allen Verhältnissen, und mindestens einen weiteren Arzneimittelwirkstoff.

13. Set (Kit) bestehend aus getrennten Packungen von
(a) einer wirksamen Menge einer Verbindung der Formel I und/oder pharmazeutisch unbedenklicher Salze, Solvate und Stereoisomere davon, einschließlich deren Mischungen in allen Verhältnissen,
und
(b) einer wirksamen Menge eines weiteren Arzneimittelwirkstoffs.

## Revendications

1. Composés de formule I 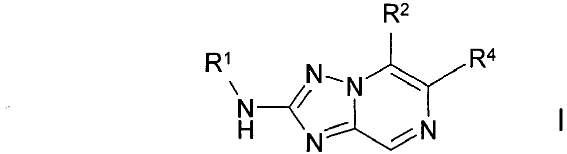 dans lesquels
R¹ désigne Ar, COHét ou Hét,
R² désigne H, Ar¹, NHHét ou Hét,
R³ désigne H ou A',
R⁴ désigne H, A, Ar¹, Hét, Hal, NHAr¹ ou CN,
Ar désigne phényle ou naphtyle qui est non substitué ou mono- ou disubstitué par Hal, A, Cyc, [C(R³)₂]ₚOA, [C(R³)₂]ₚOH, CN, NHCOHét¹, NHCOA, NHCO[C(R³)₂]ₚCyc, CONH[C(R³)₂]ₚCyc, [C(R³)₂]ₚN(R³)₂, [C(R³)₂]ₚHét¹, NR³SO₂A, SO₂N(R³)₂, S(O)ₙA, COHét¹, O[C(R³)₂]ₘN(R³)₂ et/ou O[C(R³)₂]ₚHét¹,
Ar¹ désigne phényle qui est non substitué ou mono- ou disubstitué par Hal, A, phényle, CONH₂, [C(R³)₂]ₚOR³, [C(R³)₂]ₚN(R³)₂, [C(R³)₂]ₚCN, [C(R³)₂]pHét¹ et/ou O[C(R³)₂]ₚHét¹,
Hét désigne furyle, thiényle, pyrrolyle, imidazolyle, pyrazolyle, oxazolyle, isoxazolyle, oxadiazolyle, thiazolyle, triazolyle, tétrazolyle, pyridyle, pyrimidyle, pyridazinyle, pyrazinyle, indolyle, isoindolyle, benzimidazolyle, indazolyle, quinolyle, 1,3-benzodioxolyle, benzothiophényle, benzofuranyle, imidazopyridyle, dihydroindolyle, 2,3-dihydrobenzo[1,4]dioxinyle ou furo[3,2-b]pyridyle qui est non substitué ou mono-, di- ou trisubstitué par Hal, A, [C(R³)₂]ₚOR³, [C(R³)₂]ₚN(R³)₂, [C(R³)₂]ₚHét¹, NO₂, CN, [C(R⁶)₂]ₚCOOR³, CON(R³)₂, NR³COA, NR³SO₂A, SO₂N(R³)₂, S(O)ₙA, COHét¹, O[C(R³)₂]ₘN(R³)₂, O[C(R³)₂]ₚHét¹ et/ou =O,
Hét¹ désigne dihydropyrrolyle, pyrrolidinyle, azétidinyle, oxétanyle, tétrahydroimidazolyle, dihydropyrazolyle, tétrahydropyrazolyle, tétrahydrofuranyle, dihydropyridyle, tétrahydropyridyle, pipéridinyle, morpholinyle, hexahydropyridazinyle, hexahydropyrimidinyle, [1,3]dioxolanyle, tétrahydropyranyle, pyridyle ou pipérazinyle, qui est non substitué ou mono- ou disubstitué par Hal, CN, OH, OA, COOA, CONH₂, S(O)ₙA, S(O)ₙAr, COA, A et/ou =O,
A désigne alkyle non ramifié ou ramifié ayant 1-10 atomes de C, où un ou deux groupements CH et/ou CH₂ non adjacents peuvent être remplacés par atomes de N, O et/ou S et où 1-7 atomes de H peuvent être remplacés par F ou Cl,
Cyc désigne alkyle cyclique ayant 3-7 atomes de C, qui est non substitué ou monosubstitué par [C(R³)₂]ₚOH ou CN,
A' désigne alkyle non ramifié ou ramifié ayant 1, 2, 3 ou 4 atomes deC,
Hal désigne F, Cl, Br ou I,
n désigne 0, 1 ou 2,
m désigne 1, 2 ou 3,
p désigne 0, 1, 2, 3 ou 4,
et les solvats, sels, tautomères et stéréoisomères pharmaceutiquement acceptables de ceux-ci, y compris des mélanges de ceux-ci en toutes proportions.

2. Composés selon la revendication 1, dans lesquels
R¹ désigne Ar, COHét ou Hét,
R² désigne H, Ar¹, NHHét ou Hét,
R³ désigne H ou A',
R⁴ désigne H, A, Ar¹, Hét, Hal, NHAr¹ ou CN,
Ar désigne phényle qui est non substitué ou mono- ou disubstitué par Hal, A, Cyc, [C(R³)₂]ₚOA, [C(R³)₂]ₚOH, CN, NHCOHét¹, NHCOA, NHCO[C(R³)₂]ₚCyc, CONH[C(R³)₂]ₚCyc, [C(R³)₂]ₚHét¹, SO₂N(R³)₂, NR³SO₂A, O[C(R³)₂]ₚHét¹, COHét¹, et/ou S(O)ₙA,
Ar¹ désigne phényle qui est non substitué ou mono- ou disubstitué par Hal, A, phényle, CONH₂, [C(R³)₂]ₚCN, [C(R³)₂]ₚOR³ et/ou [C(R³)²]ₚHét¹,
Hét désigne furyle, thiényle, pyrrolyle, imidazolyle, pyrazolyle, oxazolyle, isoxazolyle, oxadiazolyle, thiazolyle, triazolyle, tétrazolyle, pyridyle, pyrimidyle, pyridazinyle, pyrazinyle, indolyle, isoindolyle, benzimidazolyle, indazolyle, quinolyle, 1,3-benzodioxolyle, benzothiophényle, benzofuranyle, imidazopyridyle, dihydroindolyle, 2,3-dihydrobenzo[1,4]dioxinyle ou furo[3,2-b]pyridyle qui est non substitué ou mono-, di- ou trisubstitué par A, [C(R³)₂]ₚHét¹, [C(R³)₂]ₚN(R³)₂ et/ou =O,
Hét¹ désigne dihydropyrrolyle, pyrrolidinyle, azétidinyle, oxétanyle, tétrahydroimidazolyle, dihydropyrazolyle, tétrahydropyrazolyle, tétrahydrofuranyle, dihydropyridyle, tétrahydropyridyle, pipéridinyle, morpholinyle, hexahydropyridazinyle, hexahydropyrimidinyle, [1,3]dioxolanyle, tétrahydropyranyle, pyridyle ou pipérazinyle, qui est non substitué ou mono- ou disubstitué par A,
A désigne alkyle non ramifié ou ramifié ayant 1-10 atomes de C, et où 1-7 atomes de H peuvent être remplacés par F ou Cl,
Cyc désigne alkyle cyclique ayant 3-7 atomes de C, qui est non substitué ou monosubstitué par [C(R³)₂]ₚOH ou CN,
A' désigne alkyle non ramifié ou ramifié ayant 1, 2, 3 ou 4 atomes de C,
Hal désigne F, Cl, Br ou I,
n désigne 0, 1 ou 2,
p désigne 0, 1, 2, 3 ou 4,
et les solvats, sels, tautomères et stéréoisomères pharmaceutiquement acceptables de ceux-ci, y compris des mélanges de ceux-ci en toutes proportions.

3. Composés selon la revendication 1 ou 2, choisis dans le groupe constitué par
| n° | Nom |
|---|---|
| « A1 » | (4-méthanesulfonylphényl)-[5-(1-méthyl-1H-indazol-5-yl)-[1,2,4]triazolo[1,5-a]pyrazin-2-yl]amine |
| « A2 » | (3-méthoxyphényl)-[5-(1-méthyl-1H-indazol-5-yl)-[1,2,4]triazolo[1,5-a]pyrazin-2-yl]amine |
| « A3 » | [5-(1-méthyl-1H-indazol-5-yl)-[1,2,4]triazolo[1,5-a]pyrazin-2-yl]-pyridin-3-ylamine |
| « A4 » | [5-(1-méthyl-1H-indazol-5-yl)-[1,2,4]triazolo[1,5-a]pyrazin-2-yl]-[3-méthyl-4-(4-méthylpipérazin-1-yl)-phényl]amine |
| « A5 » | 3,3-diméthyl-6-[5-(1-méthyl-1H-indazol-5-yl)-[1,2,4]-triazolo[1,5-a]pyrazin-2-ylamino]-1,3-dihydroindol-2-one |
| « A6 » | (2-diméthylaminométhyl-1H-benzoimidazol-5-yl)-[5-(1-méthyl-1H-indazol-5-yl)-[1,2,4]triazolo[1,5-a]pyrazin-2-yl]-amine |
| « A7 » | 3-(5-quinoléin-3-yl-[1,2,4]triazolo[1,5-a]pyrazin-2-ylamino)-benzènesulfonamide |
| « A8 » | [3-méthyl-4-(4-méthylpipérazin-1-yl)-phényl]-(5-quinoléin-3-yl-[1,2,4]triazolo[1,5-a]pyrazin-2-yl)amine |
| « A9 » | 3,3-diméthyl-6-(5-quinoléin-3-yl-[1,2,4]triazolo[1,5-a]pyrazin-2-ylamino)-1,3-dihydroindol-2-one |
| « A10 » | (2-diméthylaminométhyl-1H-benzoimidazol-5-yl)-(5-quinoléin-3-yl-[1,2,4]triazolo[1,5-a]pyrazin-2-yl)amine |
| « A11 » | (4-méthanesulfonylphényl)-[5-(1-méthyl-1H-pyrazol-4-yl)-[1,2,4]triazolo[1,5-a]pyrazin-2-yl]amine |
| « A12 » | [5-(1-méthyl-1H-pyrazol-4-yl)-[1,2,4]triazolo[1,5-a]pyrazin-2-yl]-pyridin-3-ylamine |
| « A13 » | 3,3-diméthyl-6-[5-(1-méthyl-1H-pyrazol-4-yl)-[1,2,4]-triazolo[1,5-a]pyrazin-2-ylamino]-1,3-dihydroindol-2-one |
| « A14 » | (2-diméthylaminométhyl-1H-benzoimidazol-5-yl)-[5-(1-méthyl-1H-pyrazol-4-yl)-[1,2,4]triazolo[1,5-a]pyrazin-2-yl]-amine |
| « A15 » | 5-[5-(4-morpholin-4-ylphényl)-[1,2,4]triazolo[1,5-a]pyrazin-2-ylamino]-1,3-dihydroindol-2-one |
| « A16 » | [5-(4-morpholin-4-ylphényl)-[1,2,4]triazolo[1,5-a]pyrazin-2-yl]-pyridin-3-ylamine |
| « A17 » | [3-méthyl-4-(4-méthylpipérazin-1-yl)-phényl]-[5-(4-morpholin-4-ylphényl)-[1,2,4]triazolo[1,5-a]pyrazin-2-yl]amine |
| « A18 » | (6-chloro-[1,2,4]triazolo[1,5-a]pyrazin-2-yl)-(4-méthoxyphényl)amine |
| « A19 » | (6-chloro-[1,2,4]triazolo[1,5-a]pyrazin-2-yl)-(3-fluorophényl)amine |
| « A20 » | (6-chloro-[1,2,4]triazolo[1,5-a]pyrazin-2-yl)-pyridin-3-ylamine |
| « A21 » | (6-chloro-[1,2,4]triazolo[1,5-a]pyrazin-2-yl)-(4-fluorophényl)amine |
| « A22 » | (6-bromo-[1,2,4]triazolo[1,5-a]pyrazin-2-yl)-(3-méthoxyphényl)amine |
| « A23 » | (6-cyano-[1,2,4]triazolo[1,5-a]pyrazin-2-yl)-(3-méthoxyphényl)amine |
| « A24 » | (3-méthoxyphényl)-(6-méthyl-[1,2,4]triazolo[1,5-a]pyrazin-2-yl)amine |
| « A25 » | pyridin-3-yl-(6-pyridin-3-yl-[1,2,4]triazolo[1,5-a]pyrazin-2-yl]-amine |
| « A26 » | (3-méthoxyphényl)-(6-o-tolyl-[1,2,4]triazolo[1,5-a]pyrazin-2-yl)amine |
| « A27 » | [5-(4-fluorophényl)-[1,2,4]triazolo[1,5-a]pyrazin-2-yl]-pyridin-3-ylamine |
| « A28 » | (3-méthoxyphényl)-(5-phényl-[1,2,4]triazolo[1,5-a]pyrazin-2-yl)amine |
| « A29 » | N6-(4-fluorophényl)-N2-pyridin-3-yl-[1,2,4]triazolo[1,5-a]-pyrazine-2,6-diamine |
| « A30 » | (3-méthoxyphényl)-[5-(1-méthyl-1H-pyrazol-4-yl)-[1,2,4]triazolo[1,5-a]pyrazin-2-yl]amine |
| « A31 » | (4-morpholin-4-ylphényl)-[5-(1H-pyrazol-4-yl)-[1,2,4]triazolo[1,5-a]pyrazin-2-yl]amine |
| « A32 » | (4-morpholin-4-ylphényl)-(5-quinoléin-3-yl-[1,2,4]triazolo-[1,5-a]pyrazin-2-yl)amine |
| « A33 » | (4-morpholin-4-ylphényl)-(5-quinoléin-6-yl-[1,2,4]triazolo-[1,5-a]pyrazin-2-yl)amine |
| « A34 » | 2-Méthyl-2-{4-[2-(4-morpholin-4-ylphénylamino)-[1,2,4]triazolo[1,5-a]pyrazin-5-yl]-phényl}-propionitrile |
| « A35 » | [5-(1-méthyl-1H-pyrazol-4-yl)-[1,2,4]triazolo[1,5-a]pyrazin-2-yl]-(4-morpholin-4-ylphényl)amine |
| « A36 » | (5-biphényl-2-yl-[1,2,4]triazolo[1,5-a]pyrazin-2-yl)-(4-morpholin-4-ylphényl)amine |
| « A37 » | (5-biphényl-2-yl-[1,2,4]triazolo[1,5-a]pyrazin-2-yl)-(3,5-diméthoxyphényl)amine |
| « A38 » | 5-[5-(1-méthyl-1H-indazol-5-yl)-[1,2,4]triazolo[1,5-a]pyrazin-2-ylamino]-1,3-dihydroindol-2-one |
| « A39 » | 3-[5-(1-méthyl-1H-indazol-5-yl)-[1,2,4]triazolo[1,5-a]pyrazin-2-ylamino]benzènesulfonamide |
| « A40 » | 2-méthyl-2-(4-{2-[3-méthyl-4-(4-méthylpipérazin-1-yl)-phénylamino]-[1,2,4]triazolo[1,5-a]pyrazin-5-yl}-phényl)-propionitrile |
| « A41 » | 5-[5-(1-méthyl-1H-pyrazol-4-yl)-[1,2,4]triazolo[1,5-a]pyrazin-2-ylamino]-1,3-dihydroindol-2-one |
| « A42 » | 5-(1-méthyl-1H-pyrazol-4-yl)-N-(3-méthyl-4-(4-méthyl-pipérazin-1-yl)phényl)-[1,2,4]triazolo[1,5-a]pyrazin-2-amine |
| « A43 » | N-(3-(2-((3-méthyl-4-(4-méthylpipérazin-1-yl)phényl)amino)-[1,2,4]triazolo[1,5-a]pyrazin-5-yl)phényl)-méthanesulfonamide |
| « A44 » | [6-méthyl-5-(1-méthyl-1H-pyrazol-4-yl)-[1,2,4]triazolo[1,5-a]-pyrazin-2-yl]-(4-morpholin-4-ylphényl)amine |
| « A45 » | [5-(1-méthyl-1H-pyrazol-4-yl)-6-phényl-[1,2,4]triazolo[1,5-a]-pyrazin-2-yl]-(4-morpholin-4-ylphényl)amine |
| « A46 » | [5,6-Bis-(1-méthyl-1H-pyrazol-4-yl)-[1,2,4]triazolo[1,5-a]-pyrazin-2-yl]-(4-morpholin-4-ylphényl)amine |
| « A47 » | 1-{4-[5-(1-méthyl-1H-pyrazol-4-yl)-[1,2,4]triazolo[1,5-a]-pyrazin-2-ylamino]-phényl}-cyclopropanecarbonitrile |
| « A48 » | [4-(4-méthylpipérazin-1-yl)-phényl]-{5-[1-(2-morpholin-4-yl-éthyl)-1H-pyrazol-4-yl]-[1,2,4]triazolo[1,5-a]pyrazin-2-yl}-amine |
| « A49 » | 5-{5-[1-(2-morpholin-4-yléthyl)-1H-pyrazol-4-yl]-[1,2,4]-triazolo[1,5-a]pyrazin-2-ylamino}-1,3-dihydroindol-2-one |
| « A50 » | [3-méthyl-4-(4-méthylpipérazin-1-yl)-phényl]-{5-[1-(2-morpholin-4-yléthyl)-1H-pyrazol-4-yl]-[1,2,4]triazolo[1,5-a]-pyrazin-2-yl}-amine |
| « A51 » | {5-[1-(3-méthylbutyl)-1H-pyrazol-4-yl]-[1,2,4]triazolo[1,5-a]-pyrazin-2-yl}-[3-méthyl-4-(4-méthylpipérazin-1-yl)-phényl]amine |
| « A52 » | 1-méthyl-5-{5-[1-(2-morpholin-4-yléthyl)-1H-pyrazol-4-yl]-[1,2,4]triazolo[1,5-a]pyrazin-2-ylamino}-1,3-dihydroindol-2-one |
| « A53 » | N2-[4-(4-méthylpipérazin-1-yl)-phényl]-N5-(1-méthyl-1H-pyrazol-4-yl)-[1,2,4]triazolo[1,5-a]pyrazine-2,5-diamine |
| « A54 » | 3-{2-[3-méthyl-4-(4-méthylpipérazin-1-yl)-phénylamino]-[1,2,4]triazolo[1,5-a]pyrazin-5-yl}-benzamide |
| « A55 » | [5-(5-méthyl-furan-2-yl)-[1,2,4]triazolo[1,5-a]pyrazin-2-yl]-(4-morpholin-4-ylphényl)amine |
| « A56 » | {5-[1-(2-morpholin-4-yléthyl)-1H-pyrazol-4-yl]-[1,2,4]-triazolo[1,5-a]pyrazin-2-yl}-(4-morpholin-4-ylphényl)amine |
| « A57 » | 3-[5-(1-méthyl-1 H-pyrazol-4-yl)-[1,2,4]triazolo[1,5-a]pyrazin-2-ylamino]-benzonitrile |
| « A58 » | 5-[5-(5-morpholin-4-ylméthyl-thiophén-2-yl)-[1,2,4]-triazolo[1,5-a]pyrazin-2-ylamino]-1,3-dihydroindol-2-one |
| « A59 » | (2,3-dihydrobenzo[1,4]dioxin-6-yl)-[5-(1-méthyl-1H-pyrazol-4-yl)-[1,2,4]triazolo[1,5-a]pyrazin-2-yl]amine |
| « A60 » | [5-(1-méthyl-1H-pyrazol-4-yl)-[1,2,4]triazolo[1,5-a]pyrazin-2-yl]-(4-pyridin-4-ylphényl)amine |
| « A61 » | [5-(1-méthyl-1H-pyrazol-4-yl)-[1,2,4]triazolo[1,5-a]pyrazin-2-yl]-(4-morpholin-4-ylméthylphényl)amine |
| « A62 » | [5-(5-morpholin-4-ylméthyl-thiophén-2-yl)-[1,2,4]triazolo[1,5-a]pyrazin-2-yl]-(4-morpholin-4-ylphényl)amine |
| « A63 » | 5-[2-(4-morpholin-4-ylphénylamino)-[1,2,4]triazolo[1,5-a]-pyrazin-5-yl]-2-(tétrahydropyran-4-yloxy)-benzonitrile |
| « A64 » | [5-(1-méthyl-1H-pyrazol-4-yl)-[1,2,4]triazolo[1,5-a]pyrazin-2-yl]-(3-méthyl-4-trifluorométhoxyphényl)amine |
| « A65 » | acide cyclobutanecarboxylique {2-méthyl-4-[5-(1-méthyl-1H-pyrazol-4-yl)-[1,2,4]triazolo[1,5-a]pyrazin-2-ylamino]-phényl}-amide |
| « A66 » | {4-[5-(1-méthyl-1H-pyrazol-4-yl)-[1,2,4]triazolo[1,5-a]pyrazin-2-ylamino]-phényl}-morpholin-4-ylméthanone |
| « A67 » | [3-méthoxy-4-(4-méthylpipérazin-1-yl)-phényl]-[5-(1-méthyl-1H-pyrazol-4-yl)-[1,2,4]triazolo[1,5-a]pyrazin-2-yl]amine |
| « A68 » | (3-méthyl-4-morpholin-4-ylméthylphényl)-[5-(1-méthyl-1H-pyrazol-4-yl)-[1,2,4]triazolo[1,5-a]pyrazin-2-yl]amine |
| « A69 » | N-cyclopropylméthyl-2-méthyl-4-[5-(1-méthyl-1H-pyrazol-4-yl)-[1,2,4]triazolo[1,5-a]pyrazin-2-ylamino]-benzamide |
| « A70 » | N-cyclopropylméthyl-2-méthoxy-4-[5-(1-méthyl-1H-pyrazol-4-yl)-[1,2,4]triazolo[1,5-a]pyrazin-2-ylamino]-benzamide |
| « A71 » | N-cyclopropyl-2-méthyl-4-[5-(1-méthyl-1H-pyrazol-4-yl)-[1,2,4]triazolo[1,5-a]pyrazin-2-ylamino]-benzamide |
| « A72 » | N-cyclopropyl-2-méthoxy-4-[5-(1-méthyl-1H-pyrazol-4-yl)-[1,2,4]triazolo[1,5-a]pyrazin-2-ylamino]-benzamide |
| « A73 » | {2-méthyl-4-[5-(1-méthyl-1H-pyrazol-4-yl)-[1,2,4]triazolo-[1,5-a]pyrazin-2-ylamino]-phényl}-morpholin-4-ylméthanone |
| « A74 » | acide pipéridine-4-carboxylique [5-(1-méthyl-1H-pyrazol-4-yl)-[1,2,4]triazolo[1,5-a]pyrazin-2-yl]-amide |
| « A75 » | [3-méthoxy-4-(4-méthylpipérazin-1-yl)-phényl]-{5-[1-(2-morpholin-4-yléthyl)-1H-pyrazol-4-yl]-[1,2,4]triazolo[1,5-a]-pyrazin-2-yl}-amine |
| « A76 » | [3-méthoxy-4-(4-méthylpipérazin-1-yl)-phényl]-{5-[1-(3-méthylbutyl)-1H-pyrazol-4-yl]-[1,2,4]triazolo[1,5-a]pyrazin-2-yl}-amine |
| « A77 » | [3-méthoxy-4-(4-méthylpipérazin-1-yl)-phényl]-{5-[1-(tétrahydropyran-4-yl)-1H-pyrazol-4-yl]-[1,2,4]triazolo[1,5-a]-pyrazin-2-yl}-amine |
| « A78 » | (3-méthoxy-4-morpholin-4-ylméthylphényl)-[5-(1-méthyl-1H-pyrazol-4-yl)-[1,2,4]triazolo[1,5-a]pyrazin-2-yl]amine |
| « A79 » | [2-méthoxy-4-(4-méthylpipérazin-1-yl)-phényl]-[5-(1-méthyl-1H-pyrazol-4-yl)-[1,2,4]triazolo[1,5-a]pyrazin-2-yl]amine |
| « A80 » | [5-(1-méthyl-1H-pyrazol-4-yl)-[1,2,4]triazolo[1,5-a]pyrazin-2-yl]-(3-morpholin-4-ylphényl)amine |
| « A81 » | N-{2-méthyl-4-[5-(1-méthyl-1H-pyrazol-4-yl)-[1,2,4]triazolo[1,5-a]pyrazin-2-ylamino]-phényl}-acétamide |
| « A82 » | acide pipéridine-4-carboxylique {2-méthyl-4-[5-(1-méthyl-1H-pyrazol-4-yl)-[1,2,4]triazolo[1,5-a]pyrazin-2-ylamino]-phényl}-amide |
| « A83 » | acide tétrahydropyran-4-carboxylique {2-méthyl-4-[5-(1-méthyl-1H-pyrazol-4-yl)-[1,2,4]triazolo[1,5-a]pyrazin-2-yl-amino]-phényl}-amide |
| « A84 » | acide pipéridine-4-carboxylique {2-méthoxy-4-[5-(1-méthyl-1H-pyrazol-4-yl)-[1,2,4]triazolo[1,5-a]pyrazin-2-ylamino]-phényl}-amide |
| « A85 » | N-{2-méthoxy-4-[5-(1-méthyl-1H-pyrazol-4-yl)-[1,2,4]triazolo[1,5-a]pyrazin-2-ylamino]-phényl}-acétamide |
| « A86 » | 3-{2-[3-méthoxy-4-(4-méthylpipérazin-1-yl)-phénylamino]-[1,2,4]triazolo[1,5-a]pyrazin-5-yl}-phénol |
| « A87 » | 4-{2-[3-méthoxy-4-(4-méthylpipérazin-1-yl)-phénylamino]-[1,2,4]triazolo[1,5-a]pyrazin-5-yl}-phénol |
| « A88 » | [2-(4-méthylpipérazin-1-yl)-pyrimidin-5-yl]-[5-(1-méthyl-1H-pyrazol-4-yl)-[1,2,4]triazolo[1,5-a]pyrazin-2-yl]amine |
| « A89 » | acide tétrahydropyran-4-carboxylique {2-méthoxy-4-[5-(1-méthyl-1H-pyrazol-4-yl)-[1,2,4]triazolo[1,5-a]pyrazin-2-ylamino]-phényl}-amide |
| « A90 » | [6-(4-méthylpipérazin-1-yl)-pyridin-3-yl]-[5-(1-méthyl-1H-pyrazol-4-yl)-[1,2,4]triazolo[1,5-a]pyrazin-2-yl]amine |
| « A91 » | [3-méthoxy-4-(4-méthylpipérazin-1-yl)-phényl]-[5-(4-morpholin-4-ylphényl)-[1,2,4]triazolo[1,5-a]pyrazin-2-yl]-amine |
| « A92 » | [3-méthoxy-4-(4-méthylpipérazin-1-yl)-phényl]-[5-(4-méthoxyphényl)-[1,2,4]triazolo[1,5-a]pyrazin-2-yl]amine |
| « A93 » | N-(4-{2-[3-méthoxy-4-(4-méthylpipérazin-1-yl)-phénylamino]-[1,2,4]triazolo[1,5-a]pyrazin-5-yl}-phényl)-méthanesulfonamide |
| « A94 » | 4-{2-[3-méthoxy-4-(4-méthylpipérazin-1-yl)-phénylamino]-[1,2,4]triazolo[1,5-a]pyrazin-5-yl}-N-méthyl-benzènesulfonamide |
| « A95 » | N-(3-{2-[3-méthoxy-4-(4-méthylpipérazin-1-yl)-phénylamino]-[1,2,4]triazolo[1,5-a]pyrazin-5-yl}-phényl)-méthanesulfonamide |
| « A96 » | 3-{2-[3-méthoxy-4-(4-méthylpipérazin-1-yl)-phénylamino]-[1,2,4]triazolo[1,5-a]pyrazin-5-yl}-N-méthyl-benzènesulfonamide |
et les solvats, sels, tautomères et stéréoisomères pharmaceutiquement acceptables de ceux-ci, y compris des mélanges de ceux-ci en toutes proportions.

4. Procédé de préparation de composés de formule I selon les revendications 1-3 et de sels, solvats, tautomères et stéréoisomères pharmaceutiquement acceptables de ceux-ci, **caractérisé en ce que**
un composé de formule II 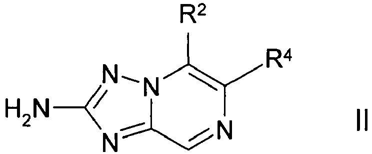
dans lequel R² et R⁴ revêtent les significations indiquées selon la revendication 1,
est réagi avec un composé de formule III
R¹-L III
dans lequel R¹ revêt la signification indiquée selon la revendication 1 et
L désigne Cl ou Br,
et/ou
une base ou un acide de formule I est converti en l'un de ses sels.

5. Médicaments comprenant au moins un composé de formule I et/ou des sels, solvats, tautomères et stéréoisomères pharmaceutiquement acceptables de celui-ci, y compris des mélanges de ceux-ci en toutes proportions, et éventuellement un support, excipient ou véhicule pharmaceutiquement acceptable.

6. Composés de formule I et des sels, solvats, tautomères et stéréoisomères pharmaceutiquement acceptables de ceux-ci, y compris des mélanges de ceux-ci en toutes proportions, pour une utilisation dans le traitement et/ou la prévention de conditions inflammatoires, de conditions immunologiques, de conditions auto-immunes, de conditions allergiques, de conditions rhumatismales, de conditions thrombotiques, du cancer, d'infections, de maladies neurodégénératives, de maladies neuroinflammatoires, de maladies cardiovasculaires, et de conditions métaboliques, les méthodes comprenant l'administration à un sujet en ayant besoin d'une quantité efficace d'un composé selon la revendication 1.

7. Composés pour une utilisation selon la revendication 6, dans le traitement et/ou la prévention du cancer,
où le cancer à traiter est une tumeur solide ou une tumeur du système sanguin et immunitaire.

8. Composés pour une utilisation selon la revendication 7, où la tumeur solide provient du groupe des tumeurs de l'épithélium, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, des os, y compris le chondrosarcome et le sarcome d'Ewing, des cellules souches, y compris les tumeurs du tissu embryonnaire, et/ou du poumon, du groupe de la leucémie monocytique, de l'adénocarcinome du poumon, des carcinomes du poumon à petites cellules, du cancer du pancréas, des glioblastomes, du neurofibrome, de l'angiosarcome, du carcinome du sein et/ou du mélanome malin.

9. Composés pour une utilisation selon la revendication 6, dans le traitement et/ou la prévention de maladies choisies dans le groupe constitué par
la polyarthrite rhumatoïde, le lupus systémique, l'asthme, la sclérose en plaques, l'ostéo-arthrite, les lésions ischémiques, l'artérite à cellules géantes, l'affection abdominale inflammatoire, le diabète, la fibrose kystique, le psoriasis, le syndrome de Sjögren et le rejet de greffe d'organe.

10. Composés pour une utilisation selon la revendication 6, dans le traitement et/ou la prévention de maladies choisies dans le groupe constitué par
la maladie d'Alzheimer, la trisomie 21, l'hémorragie cérébrale héréditaire avec amylose de type néerlandais, l'angiopathie amyloïde cérébrale, la maladie de Creutzfeldt-Jakob, les démences fronto-temporales, la maladie de Huntington, la maladie de Parkinson.

11. Composés pour une utilisation selon la revendication 6, dans le traitement et/ou la prévention de maladies choisies dans le groupe constitué par
leishmania, les mycobactéries, y compris M. leprae, M. tuberculosis et/ou M. avium, leishmania, plasmodium, le virus de l'immunodéficience humaine, le virus d'Epstein-Barr, le virus Herpes simplex, le virus de l'hépatite C.

12. Médicaments comprenant au moins un composé de formule I et/ou des sels, solvats et stéréoisomères pharmaceutiquement acceptables de celui-ci, y compris des mélanges de ceux-ci en toutes proportions, et au moins un autre ingrédient actif médicamenteux.

13. Ensemble (kit) constitué de conditionnements séparés
(a) d'une quantité efficace d'un composé de formule I et/ou de sels, solvats, sels et stéréoisomères pharmaceutiquement acceptables de celui-ci, y compris des mélanges de ceux-ci en toutes proportions, et
(b) d'une quantité efficace d'un autre ingrédient actif médicamenteux.
